# EUROPEAN PATENT SPECIFICATION

(11) **EP 1 774 004 B1**
(45) Date of publication and mention of the grant of the patent: **10.07.2013**
(21) Application number: 05771370.3
(22) Date of filing: 21.07.2005
(51) Int. Cl.: C12N 15/82

(54) **PREPARATION OF ORGANISMS WITH FASTER GROWTH AND/OR HIGHER YIELD**
HERSTELLUNG VON ORGANISMEN MIT SCHNELLEREM WACHSTUM UND/ODER HÖHEREM ERTRAG
PREPARATION D'ORGANISMES A CROISSANCE PLUS RAPIDE ET/OU A MEILLEUR RENDEMENT

(30) Priority: 31.07.2004 EP 04018194
(43) Date of publication of application: 18.04.2007
(73) Proprietor: Metanomics GmbH, 10589 Berlin (DE)
(72) Inventor: PUZIO, Piotr, 13505 Berlin (DE); CHARDONNENS, Agnes, 13158 Berlin (DE)
(74) Representative: Popp, Andreas
(86) International application number: PCT/EP2005/007935
(87) International publication number: WO 2006/013010

(56) References cited:
- WO-A-00/22169
- WO-A-01/26458
- WO-A-01/64928
- WO-A-2004/058980
- WO-A-2004/074440
- WO-A2-03/074653
- US-A1- 2004 034 888
- US-A1- 2004 073 976
- DATABASE UniProt [Online] 1 November 1997 (1997-11-01), "Probable glutamine amidotransferase SNO1 (EC 2.6.-.-)." XP002346368 retrieved from EBI accession no. UNIPROT:SNO1_YEAST Database accession no. Q03144 & RODRIGUEZ-NAVARRO SUSANA ET AL: "Functional analysis of yeast gene families involved in metabolism of vitamins B1 and B6" YEAST, vol. 19, no. 14, October 2002 (2002-10), pages 1261-1276, ISSN: 0749-503X
- ROST B: "Enzyme Function Less Conserved than Anticipated", JOURNAL OF MOLECULAR BIOLOGY, LONDON, GB, vol. 318, no. 2, 26 April 2002 (2002-04-26), pages 595-608, XP004449547, ISSN: 0022-2836, DOI: 10.1016/S0022-2836(02)00016-5
- GUY P M ET AL: "INSECT CELL-EXPRESSED P180ERBB3 POSSESSES AN IMPAIRED TYROSINE KINASE ACTIVITY", PROCEEDINGS OF THE NATIONAL ACADEMY OF SCIENCES OF USA, NATIONAL ACADEMY OF SCIENCE, WASHINGTON, DC; US, vol. 91, no. 17, 16 August 1994 (1994-08-16), pages 8132-8136, XP001184053, ISSN: 0027-8424, DOI: 10.1073/PNAS.91.17.8132
- VAN CAMP ET AL: "Yield enhancement genes: seeds for growth", CURRENT OPINION IN BIOTECHNOLOGY, LONDON, GB, vol. 16, no. 2, 1 April 2005 (2005-04-01), pages 147-153, XP027676708, ISSN: 0958-1669 [retrieved on 2005-04-01]

### SEQUENCE LISTING -

<110> Metanomics GmbH
<120> Preparation of organisms with faster growth and/or higher yield
<130> AE 2003-0644
<140> PF 55769
   <141> 2005-07-07
<160> 139
<170> PatentIN Nr. 3.1 & 3.4
<210> 1
   <211> 675
   <212> DNA
   <213> Saccharomyces cerevisiae
<400> 1
   atg cac aaa acc cac agt aca atg tcc gga aag tcg atg aaa gta att      48
   Met His Lys Thr His Ser Thr Met Ser Gly Lys Ser Met Lys Val Ile
   1               5                   10                  15
   ggg gtt ttg gcg ttg caa ggt gcc ttt ttg gag cat acc aac cat tta      96
   Gly Val Leu Ala Leu Gln Gly Ala Phe Leu Glu His Thr Asn His Leu
               20                  25                  30
   aaa agg tgt ttg gct gaa aac gac tac gga ata aag ata gaa atc aaa      144
   Lys Arg Cys Leu Ala Glu Asn Asp Tyr Gly Ile Lys Ile Glu Ile Lys
           35                  40                  45
   act gta aaa act cct gag gat cta gcc cag tgc gac gcc tta att att      192
   Thr Val Lys Thr Pro Glu Asp Leu Ala Gln Cys Asp Ala Leu Ile Ile
       50                  55                  60
   ccc gga gga gaa tct acg tcg atg tcc ctc atc gct caa aga aca ggc      240
   Pro Gly Gly Glu Ser Thr Ser Met Ser Leu Ile Ala Gln Arg Thr Gly
   65                  70                  75                  80
   tta tat cct tgt tta tac gaa ttt gtt cat aat ccg gaa aag gta gtt      288
   Leu Tyr Pro Cys Leu Tyr Glu Phe Val His Asn Pro Glu Lys Val Val
                   85                  90                  95
   tgg ggt act tgt gct ggt ctc atc ttt tta agc gcg caa tta gaa aac      336
   Trp Gly Thr Cys Ala Gly Leu Ile Phe Leu Ser Ala Gln Leu Glu Asn
               100                 105                 110
   gaa agt gcc cta gta aag act tta ggt gtg ttg aag gtc gac gtg aga      384
   Glu Ser Ala Leu Val Lys Thr Leu Gly Val Leu Lys Val Asp Val Arg
           115                 120                 125
   aga aac gca ttt gga aga caa gct caa tct ttt aca caa aag tgt gat      432
   Arg Asn Ala Phe Gly Arg Gln Ala Gln Ser Phe Thr Gln Lys Cys Asp
       130                 135                 140
   ttt tcc aat ttc ata cct ggc tgt gat aat ttt cct gct aca ttt att      480
   Phe Ser Asn Phe Ile Pro Gly Cys Asp Asn Phe Pro Ala Thr Phe Ile
   145                 150                 155                 160
   cgc gca ccc gtg atc gag aga att ctt gat cct atc gcg gtt aaa agt      528
   Arg Ala Pro Val Ile Glu Arg Ile Leu Asp Pro Ile Ala Val Lys Ser
                   165                 170                 175
   tta tat gaa ttg cca gtg aat gga aag gat gtg gtt gta gct gca acg      576
   Leu Tyr Glu Leu Pro Val Asn Gly Lys Asp Val Val Val Ala Ala Thr
               180                 185                 190
   caa aat cat aat atc ctt gtg act tct ttt cat cca gag ctt gct gac      624
   Gln Asn His Asn Ile Leu Val Thr Ser Phe His Pro Glu Leu Ala Asp
           195                 200                 205
   agt gat aca aga ttt cat gat tgg ttt atc aga cag ttt gtt tct aat      672
   Ser Asp Thr Arg Phe His Asp Trp Phe Ile Arg Gln Phe Val Ser Asn
       210                 215                 220
   taa                                                                  675
<210> 2
   <211> 224
   <212> PRT
   <213> Saccharomyces cerevisiae
<400> 2
   Met His Lys Thr His Ser Thr Met Ser Gly Lys Ser Met Lys Val Ile
   1               5                   10                  15
   Gly Val Leu Ala Leu Gln Gly Ala Phe Leu Glu His Thr Asn His Leu
               20                  25                  30
   Lys Arg Cys Leu Ala Glu Asn Asp Tyr Gly Ile Lys Ile Glu Ile Lys
           35                  40                  45
   Thr Val Lys Thr Pro Glu Asp Leu Ala Gln Cys Asp Ala Leu Ile Ile
       50                  55                  60
   Pro Gly Gly Glu Ser Thr Ser Met Ser Leu Ile Ala Gln Arg Thr Gly
   65                  70                  75                  80
   Leu Tyr Pro Cys Leu Tyr Glu Phe Val His Asn Pro Glu Lys Val Val
                   85                  90                  95
   Trp Gly Thr Cys Ala Gly Leu Ile Phe Leu Ser Ala Gln Leu Glu Asn
               100                 105                 110
   Glu Ser Ala Leu Val Lys Thr Leu Gly Val Leu Lys Val Asp Val Arg
           115                 120                 125
   Arg Asn Ala Phe Gly Arg Gln Ala Gln Ser Phe Thr Gln Lys Cys Asp
       130                 135                 140
   Phe Ser Asn Phe Ile Pro Gly Cys Asp Asn Phe Pro Ala Thr Phe Ile
   145                 150                 155                 160
   Arg Ala Pro Val Ile Glu Arg Ile Leu Asp Pro Ile Ala Val Lys Ser
                   165                 170                 175
   Leu Tyr Glu Leu Pro Val Asn Gly Lys Asp Val Val Val Ala Ala Thr
               180                 185                 190
   Gln Asn His Asn Ile Leu Val Thr Ser Phe His Pro Glu Leu Ala Asp
           195                 200                 205
   Ser Asp Thr Arg Phe His Asp Trp Phe Ile Arg Gln Phe Val Ser Asn
       210                 215                 220
<210> 3
   <211> 591
   <212> DNA
   <213> Pyrococcus abyssi
<400> 3
   atg aag gtt ggc gtt atc ggg tta caa ggt gat gtc agc gag cac atc      48
   Met Lys Val Gly Val Ile Gly Leu Gln Gly Asp Val Ser Glu His Ile
   1               5                   10                  15
   gat gca act aac cta gct ttg aaa aaa tta ggc gtg tct gga gag gcc      96
   Asp Ala Thr Asn Leu Ala Leu Lys Lys Leu Gly Val Ser Gly Glu Ala
               20                  25                  30
   ata tgg ttg aaa aag cca gaa cag ctg aaa gaa gtt tca gct ata ata      144
   Ile Trp Leu Lys Lys Pro Glu Gln Leu Lys Glu Val Ser Ala Ile Ile
           35                  40                  45
   att cct ggg gga gag agc act acc ata tcg agg tta atg cag aaa aca      192
   Ile Pro Gly Gly Glu Ser Thr Thr Ile Ser Arg Leu Met Gln Lys Thr
       50                  55                  60
   ggg ctg ttt gag cca gta aaa aag ttg ata gag gat ggc ctt cca gtt      240
   Gly Leu Phe Glu Pro Val Lys Lys Leu Ile Glu Asp Gly Leu Pro Val
   65                  70                  75                  80
   atg ggg act tgc gcc gga ttg ata atg ctc tct agg gaa gtt cta ggg      288
   Met Gly Thr Cys Ala Gly Leu Ile Met Leu Ser Arg Glu Val Leu Gly
                   85                  90                  95
   gct acc cca gag cag agg ttc ctt gaa gtt cta gac gtt agg gtg aac      336
   Ala Thr Pro Glu Gln Arg Phe Leu Glu Val Leu Asp Val Arg Val Asn
               100                 105                 110
   agg aac gcc tac ggg agg cag gtg gat agt ttc gaa gct cct gtt agg      384
   Arg Asn Ala Tyr Gly Arg Gln Val Asp Ser Phe Glu Ala Pro Val Arg
           115                 120                 125
   tta tct ttc gat gat gaa cct ttc ata ggg gtc ttc ata agg gct ccc      432
   Leu Ser Phe Asp Asp Glu Pro Phe Ile Gly Val Phe Ile Arg Ala Pro
       130                 135                 140
   agg ata gtc gag ttg cta agt gat aga gtt aaa ccc tta gct tgg tta        480
   Arg Ile Val Glu Leu Leu Ser Asp Arg Val Lys Pro Leu Ala Trp Leu
   145                 150                 155                 160
   gag gat agg gtt gtg ggc gtt gag cag gac aac att ata ggc ctc gaa        528
   Glu Asp Arg Val Val Gly Val Glu Gln Asp Asn Ile Ile Gly Leu Glu
                   165                 170                 175
   ttt cac cca gag cta acc gac gat act agg gtt cac gag tac ttc ttg        576
   Phe His Pro Glu Leu Thr Asp Asp Thr Arg Val His Glu Tyr Phe Leu
               180                 185                 190
   aag aag gcg ctc tag                                                    591
   Lys Lys Ala Leu
           195
<210> 4
   <211> 196
   <212> PRT
   <213> Pyrococcus abyssi
<400> 4
   Met Lys Val Gly Val Ile Gly Leu Gln Gly Asp Val Ser Glu His Ile
   1               5                   10                  15
   Asp Ala Thr Asn Leu Ala Leu Lys Lys Leu Gly Val Ser Gly Glu Ala
               20                  25                  30
   Ile Trp Leu Lys Lys Pro Glu Gln Leu Lys Glu Val Ser Ala Ile Ile
           35                  40                  45
   Ile Pro Gly Gly Glu Ser Thr Thr Ile Ser Arg Leu Met Gln Lys Thr
       50                  55                  60
   Gly Leu Phe Glu Pro Val Lys Lys Leu Ile Glu Asp Gly Leu Pro Val
   65                  70                  75                  80
   Met Gly Thr Cys Ala Gly Leu Ile Met Leu Ser Arg Glu Val Leu Gly
                   85                  90                  95
   Ala Thr Pro Glu Gln Arg Phe Leu Glu Val Leu Asp Val Arg Val Asn
               100                 105                 110
   Arg Asn Ala Tyr Gly Arg Gln Val Asp Ser Phe Glu Ala Pro Val Arg
           115                 120                 125
   Leu Ser Phe Asp Asp Glu Pro Phe Ile Gly Val Phe Ile Arg Ala Pro
       130                 135                 140
   Arg Ile Val Glu Leu Leu Ser Asp Arg Val Lys Pro Leu Ala Trp Leu
   145                 150                 155                 160
   Glu Asp Arg Val Val Gly Val Glu Gln Asp Asn Ile Ile Gly Leu Glu
                   165                 170                 175
   Phe His Pro Glu Leu Thr Asp Asp Thr Arg Val His Glu Tyr Phe Leu
               180                 185                 190
   Lys Lys Ala Leu
           195
<210> 5
   <211> 582
   <212> DNA
   <213> Streptococcus pneumoniae
<400> 5
   atg aaa atc gga ata ttg gcc ttg caa ggg gcc ttt gca gaa cat gca      48
   Met Lys Ile Gly Ile Leu Ala Leu Gln Gly Ala Phe Ala Glu His Ala
   1               5                   10                  15
   aaa gtg cta gat caa tta ggt gtc gag agt gta gaa ctc aga aat cta      96
   Lys Val Leu Asp Gln Leu Gly Val Glu Ser Val Glu Leu Arg Asn Leu
               20                  25                  30
   gat gat ttt cag caa gat cag agt gac ttg tcg ggt ttg att ttg cct      144
   Asp Asp Phe Gln Gln Asp Gln Ser Asp Leu Ser Gly Leu Ile Leu Pro
           35                  40                  45
   ggt ggt gag tct aca acc atg ggc aag ctc tta cgt gac cag aac atg      192
   Gly Gly Glu Ser Thr Thr Met Gly Lys Leu Leu Arg Asp Gln Asn Met
       50                  55                  60
   cta ctt ccc ata cga gaa gcc att cta tct ggc tta cca gtg ttt ggg      240
   Leu Leu Pro Ile Arg Glu Ala Ile Leu Ser Gly Leu Pro Val Phe Gly
   65                  70                  75                  80
   acc tgt gcg ggc tta att ttg ctg gct aag gaa atc act tct cag aaa      288
   Thr Cys Ala Gly Leu Ile Leu Leu Ala Lys Glu Ile Thr Ser Gln Lys
                   85                  90                  95
   gag agt cat cta gga act atg gat atg gtg gtc gag cgt aat gct tat      336
   Glu Ser His Leu Gly Thr Met Asp Met Val Val Glu Arg Asn Ala Tyr
               100                 105                 110
   ggg cgc caa tta gga agt ttc tac acg gaa gca gaa tgt aag gga gtt      384
   Gly Arg Gln Leu Gly Ser Phe Tyr Thr Glu Ala Glu Cys Lys Gly Val
           115                 120                 125
   ggc aag att cca atg acc ttt atc cgt ggt ccg att atc agt agt gtt      432
   Gly Lys Ile Pro Met Thr Phe Ile Arg Gly Pro Ile Ile Ser Ser Val
       130                 135                 140
   ggt gag ggt gta gaa att tta gca ata gtg aac aat caa att gtt gca      480
   Gly Glu Gly Val Glu Ile Leu Ala Ile Val Asn Asn Gln Ile Val Ala
   145                 150                 155                 160
   gcc caa gaa aaa aat atg ttg gta agt tct ttt cat cca gaa ttg act      528
   Ala Gln Glu Lys Asn Met Leu Val Ser Ser Phe His Pro Glu Leu Thr
                   165                 170                 175
   gat gat gtg cgc ttg cac cag tac ttt atc aat atg tgt aaa gaa aaa      576
   Asp Asp Val Arg Leu His Gln Tyr Phe Ile Asn Met Cys Lys Glu Lys
               180                 185                 190
   agt tga                                                              582
   Ser
<210> 6
   <211> 193
   <212> PRT
   <213> Streptococcus pneumoniae
<400> 6
   Met Lys Ile Gly Ile Leu Ala Leu Gln Gly Ala Phe Ala Glu His Ala
   1 5 10 15
   Lys Val Leu Asp Gln Leu Gly Val Glu Ser Val Glu Leu Arg Asn Leu
   20 25 30
   Asp Asp Phe Gln Gln Asp Gln Ser Asp Leu Ser Gly Leu Ile Leu Pro
   35 40 45
   Gly Gly Glu Ser Thr Thr Met Gly Lys Leu Leu Arg Asp Gln Asn Met
   50 55 60
   Leu Leu Pro Ile Arg Glu Ala Ile Leu Ser Gly Leu Pro Val Phe Gly
   65 70 75 80
   Thr Cys Ala Gly Leu Ile Leu Leu Ala Lys Glu Ile Thr Ser Gln Lys
   85 90 95
   Glu Ser His Leu Gly Thr Met Asp Met Val Val Glu Arg Asn Ala Tyr
   100 105 110
   Gly Arg Gln Leu Gly Ser Phe Tyr Thr Glu Ala Glu Cys Lys Gly Val
   115 120 125
   Gly Lys Ile Pro Met Thr Phe Ile Arg Gly Pro Ile Ile Ser Ser Val
   130 135 140
   Gly Glu Gly Val Glu Ile Leu Ala Ile Val Asn Asn Gln Ile Val Ala
   145 150 155 160
   Ala Gln Glu Lys Asn Met Leu Val Ser Ser Phe His Pro Glu Leu Thr
   165 170 175
   Asp Asp Val Arg Leu His Gln Tyr Phe Ile Asn Met Cys Lys Glu Lys
   180 185 190
   Ser
<210> 7
   <211> 256
   <212> PRT
   <213> Hordeum vulgare
<400> 7
   Met Ala Ala Val Val Gly Val Leu Ala Leu Gln Gly Ser Tyr Asn Glu
   1 5 10 15
   His Met Ala Ala Leu Arg Arg Ile Gly Ala Lys Gly Val Glu Val Arg
   20 25 30
   Lys Pro Glu Gln Leu Leu Ala Val Asp Ser Leu Ile Ile Pro Gly Gly
   35 40 45
   Glu Ser Thr Thr Met Ala Lys Leu Ala Asn Tyr Asp Asn Leu Phe Pro
   50 55 60
   Ala Leu Arg Glu Phe Val Gly Thr Gly Lys Pro Val Trp Gly Thr Cys
   65 70 75 80
   Ala Gly Leu Ile Phe Leu Ala Asn Lys Ala Val Gly Gln Lys Thr Gly
   85 90 95
   Gly Gln Glu Leu Val Gly Gly Leu Asp Cys Thr Val His Arg Asn Phe
   100 105 110
   Phe Gly Ser Gln Leu Gln Ser Phe Glu Thr Glu Leu Ser Val Pro Met
   115 120 125
   Leu Ala Glu Lys Glu Gly Gly Ser Asn Thr Cys Arg Gly Val Phe Ile
   130 135 140
   Arg Ala Pro Ala Ile Leu Glu Val Gly Gln Asp Val Glu Val Leu Ala
   145 150 155 160
   Asp Cys Pro Val Pro Ala Gly Arg Pro Ser Ile Thr Ile Thr Ser Gly
   165 170 175
   Glu Gly Val Glu Asp Gln Val Tyr Ser Lys Asp Arg Val Ile Val Ala
   180 185 190
   Val Arg Gln Gly Asn Ile Leu Ala Thr Ala Phe His Pro Glu Leu Thr
   195 200 205
   Ser Asp Ser Arg Trp His Gln Leu Phe Leu Asp Met Asp Lys Glu Ser
   210 215 220
   Gln Ala Lys Ala Leu Ala Ser Leu Ser Leu Ser Ala Ser Ser Asn Asn
   225 230 235 240
   Ala Glu Val Gly Ser Lys Asn Lys Ala Pro Asp Leu Pro Ile Phe Glu
   245 250 255
<210> 8
   <211> 567
   <212> DNA
   <213> Listeria monocytogenes
<400> 8
   atg aaa aaa att ggt gtc ctt gca att caa ggt gca gtg gat gaa cat        48
   Met Lys Lys Ile Gly Val Leu Ala Ile Gln Gly Ala Val Asp Glu His
   1               5                   10                  15
   atc caa atg att gaa tca gcc ggt gct ctt gct ttt aaa gta aaa cat        96
   Ile Gln Met Ile Glu Ser Ala Gly Ala Leu Ala Phe Lys Val Lys His
               20                  25                  30
   tca aat gat tta gct ggg ctt gac gga ctt gtt ttg cct ggt ggg gaa        144
   Ser Asn Asp Leu Ala Gly Leu Asp Gly Leu Val Leu Pro Gly Gly Glu
           35                  40                  45
   agc aca acg atg cgc aag att atg aaa cgt tat gat tta atg gaa cca        192
   Ser Thr Thr Met Arg Lys Ile Met Lys Arg Tyr Asp Leu Met Glu Pro
       50                  55                  60
   gtt aaa gca ttt gca agt aaa ggg aaa gct att ttt gga act tgt gct        240
   Val Lys Ala Phe Ala Ser Lys Gly Lys Ala Ile Phe Gly Thr Cys Ala
   65                  70                  75                  80
   ggg ctt gtc ctt ttg tca aaa gaa att gaa ggt ggc gaa gag agc cta        288
   Gly Leu Val Leu Leu Ser Lys Glu Ile Glu Gly Gly Glu Glu Ser Leu
                   85                  90                  95
   ggc ttg att gaa gct acc gcg atc cgt aat ggt ttt ggt agg cag aaa        336
   Gly Leu Ile Glu Ala Thr Ala Ile Arg Asn Gly Phe Gly Arg Gln Lys
               100                 105                 110
   gag agt ttt gaa gcc gaa tta aac gtc gaa gca ttt ggt gaa cct gcg        384
   Glu Ser Phe Glu Ala Glu Leu Asn Val Glu Ala Phe Gly Glu Pro Ala
           115                 120                 125
   ttt gaa gct ata ttt atc cgc gca cca tac tta att gaa ccg agt aat        432
   Phe Glu Ala Ile Phe Ile Arg Ala Pro Tyr Leu Ile Glu Pro Ser Asn
       130                 135                 140
   gag gta gct gtg tta gca aca gtt gaa aat cga atc gta gca gct aaa        480
   Glu Val Ala Val Leu Ala Thr Val Glu Asn Arg Ile Val Ala Ala Lys
   145                 150                 155                 160
   caa gct aat att tta gtt acc gca ttc cat cct gaa ctt act aac gac        528
   Gln Ala Asn Ile Leu Val Thr Ala Phe His Pro Glu Leu Thr Asn Asp
                   165                 170                 175
   aat cgc tgg atg aat tac ttc ctc gaa aaa atg gta taa                    567
   Asn Arg Trp Met Asn Tyr Phe Leu Glu Lys Met Val
                   180                 185
<210> 9
   <211> 188
   <212> PRT
   <213> Listeria monocytogenes
<400> 9
   Met Lys Lys Ile Gly Val Leu Ala Ile Gln Gly Ala Val Asp Glu His
   1               5                   10                  15
   Ile Gln Met Ile Glu Ser Ala Gly Ala Leu Ala Phe Lys Val Lys His
               20                  25                  30
   Ser Asn Asp Leu Ala Gly Leu Asp Gly Leu Val Leu Pro Gly Gly Glu
           35                  40                  45
   Ser Thr Thr Met Arg Lys Ile Met Lys Arg Tyr Asp Leu Met Glu Pro
       50                  55                  60
   Val Lys Ala Phe Ala Ser Lys Gly Lys Ala Ile Phe Gly Thr Cys Ala
   65                  70                  75                  80
   Gly Leu Val Leu Leu Ser Lys Glu Ile Glu Gly Gly Glu Glu Ser Leu
                   85                  90                  95
   Gly Leu Ile Glu Ala Thr Ala Ile Arg Asn Gly Phe Gly Arg Gln Lys
               100                 105                 110
   Glu Ser Phe Glu Ala Glu Leu Asn Val Glu Ala Phe Gly Glu Pro Ala
           115                 120                 125
   Phe Glu Ala Ile Phe Ile Arg Ala Pro Tyr Leu Ile Glu Pro Ser Asn
       130                 135                 140
   Glu Val Ala Val Leu Ala Thr Val Glu Asn Arg Ile Val Ala Ala Lys
   145                 150                 155                 160
   Gln Ala Asn Ile Leu Val Thr Ala Phe His Pro Glu Leu Thr Asn Asp
                   165                 170                 175
   Asn Arg Trp Met Asn Tyr Phe Leu Glu Lys Met Val
               180                 185
<210> 10
   <211> 561
   <212> DNA
   <213> Clostridium acetobutylicum
<400> 10
   atg agg gta ggt gtt tta tcg ttt caa ggt gga gta gtt gaa cac ctg      48
   Met Arg Val Gly Val Leu Ser Phe Gln Gly Gly Val Val Glu His Leu
   1               5                   10                  15
   gag cat ata gaa aaa ctt aat ggt aaa cct gtt aag gtt aga agt tta      96
   Glu His Ile Glu Lys Leu Asn Gly Lys Pro Val Lys Val Arg Ser Leu
               20                  25                  30
   gaa gat tta caa aaa ata gat agg ctt ata ata cca gga gga gaa agt     144
   Glu Asp Leu Gln Lys Ile Asp Arg Leu Ile Ile Pro Gly Gly Glu Ser
           35                  40                  45
   aca act ata gga aag ttt tta aaa caa tct aat atg ctc caa cct ttg     192
   Thr Thr Ile Gly Lys Phe Leu Lys Gln Ser Asn Met Leu Gln Pro Leu
       50                  55                  60
   aga gaa aag ata tat gga ggc atg cca gta tgg gga acc tgc gcg gga     240
   Arg Glu Lys Ile Tyr Gly Gly Met Pro Val Trp Gly Thr Cys Ala Gly
   65                  70                  75                  80
   atg ata ctc tta gca aga aaa ata gaa aac agt gag gtc aac tat ata     288
   Met Ile Leu Leu Ala Arg Lys Ile Glu Asn Ser Glu Val Asn Tyr Ile
                   85                  90                  95
   aat gcc ata gac ata act gta aga aga aat gct tat gga agc caa gtt     336
   Asn Ala Ile Asp Ile Thr Val Arg Arg Asn Ala Tyr Gly Ser Gln Val
               100                 105                 110
   gat agc ttt aat act aag gct tta att gaa gaa ata tct tta aat gaa     384
   Asp Ser Phe Asn Thr Lys Ala Leu Ile Glu Glu Ile Ser Leu Asn Glu
           115                 120                 125
   atg ccg ctt gtt ttt ata aga gct ccg tat ata aca cgc ata gga gaa     432
   Met Pro Leu Val Phe Ile Arg Ala Pro Tyr Ile Thr Arg Ile Gly Glu
       130                 135                 140
   aca gta aaa gca tta tgt act ata gat aaa aat ata gtg gcg gcc aaa     480
   Thr Val Lys Ala Leu Cys Thr Ile Asp Lys Asn Ile Val Ala Ala Lys
   145                 150                 155                 160
   agt aac aat gtt tta gta aca tct ttt cac ccc gaa cta gca gat aat     528
   Ser Asn Asn Val Leu Val Thr Ser Phe His Pro Glu Leu Ala Asp Asn
                   165                 170                 175
   tta gaa ttt cat gaa tat ttt atg aag tta tga                          561
   Leu Glu Phe His Glu Tyr Phe Met Lys Leu
               180                 185
<210> 11
   <211> 186
   <212> PRT
   <213> Clostridium acetobutylicum
<400> 11
   Met Arg Val Gly Val Leu Ser Phe Gln Gly Gly Val Val Glu His Leu
   1               5                   10                  15
   Glu His Ile Glu Lys Leu Asn Gly Lys Pro Val Lys Val Arg Ser Leu
               20                  25                  30
   Glu Asp Leu Gln Lys Ile Asp Arg Leu Ile Ile Pro Gly Gly Glu Ser
           35                  40                  45
   Thr Thr Ile Gly Lys Phe Leu Lys Gln Ser Asn Met Leu Gln Pro Leu
       50                  55                  60
   Arg Glu Lys Ile Tyr Gly Gly Met Pro Val Trp Gly Thr Cys Ala Gly
   65                  70                  75                  80
   Met Ile Leu Leu Ala Arg Lys Ile Glu Asn Ser Glu Val Asn Tyr Ile
                   85                  90                  95
   Asn Ala Ile Asp Ile Thr Val Arg Arg Asn Ala Tyr Gly Ser Gln Val
               100                 105                 110
   Asp Ser Phe Asn Thr Lys Ala Leu Ile Glu Glu Ile Ser Leu Asn Glu
           115                 120                 125
   Met Pro Leu Val Phe Ile Arg Ala Pro Tyr Ile Thr Arg Ile Gly Glu
       130                 135                 140
   Thr Val Lys Ala Leu Cys Thr Ile Asp Lys Asn Ile Val Ala Ala Lys
   145                 150                 155                 160
   Ser Asn Asn Val Leu Val Thr Ser Phe His Pro Glu Leu Ala Asp Asn
                   165                 170                 175
   Leu Glu Phe His Glu Tyr Phe Met Lys Leu
               180                 185
<210> 12
   <211> 597
   <212> DNA
   <213> Mycobacterium tuberculosis
<400> 12
   atg agc gtt cca cgg gtc ggg gtg ctg gcg ctg cag ggc gac acc cgg      48
   Met Ser Val Pro Arg Val Gly Val Leu Ala Leu Gln Gly Asp Thr Arg
   1               5                   10                  15
   gag cac ctg gct gcg ctg cgc gaa tgc ggg gcc gag ccg atg acg gtg      96
   Glu His Leu Ala Ala Leu Arg Glu Cys Gly Ala Glu Pro Met Thr Val
               20                  25                  30
   cgg cgc cgc gac gaa ctt gac gcg gtg gac gcg ctg gtc atc ccg ggc      144
   Arg Arg Arg Asp Glu Leu Asp Ala Val Asp Ala Leu Val Ile Pro Gly
           35                  40                  45
   ggg gaa tcc acc acg atg agc cac ctg ctg ctc gac ctc gac ctg ctg      192
   Gly Glu Ser Thr Thr Met Ser His Leu Leu Leu Asp Leu Asp Leu Leu
       50                  55                  60
   gga ccg ctg cgg gcc cgg ctc gcc gat ggg ctt ccg gcc tat ggt tcg      240
   Gly Pro Leu Arg Ala Arg Leu Ala Asp Gly Leu Pro Ala Tyr Gly Ser
   65                  70                  75                  80
   tgc gcg ggc atg att ctg ttg gcc agc gag atc ctg gac gcc ggt gcg      288
   Cys Ala Gly Met Ile Leu Leu Ala Ser Glu Ile Leu Asp Ala Gly Ala
                   85                  90                  95
   gca ggc cgc cag gcg ctg ccc ctg cgt gcg atg aat atg acg gtg cgg      336
   Ala Gly Arg Gln Ala Leu Pro Leu Arg Ala Met Asn Met Thr Val Arg
               100                 105                 110
   cgc aat gct ttt gga agt cag gtt gac tcg ttt gaa ggc gat atc gag      384
   Arg Asn Ala Phe Gly Ser Gln Val Asp Ser Phe Glu Gly Asp Ile Glu
                   115                     120                     125
   ttc gct ggt cta gac gat ccg gtg cgc gcg gtg ttc atc cgg gcg cca        432
   Phe Ala Gly Leu Asp Asp Pro Val Arg Ala Val Phe Ile Arg Ala Pro
       130                     135                     140
   tgg gtt gag cga gtc ggt gac ggt gtg cag gtg ctg gcc cgc gcg gcg        480
   Trp Val Glu Arg Val Gly Asp Gly Val Gln Val Leu Ala Arg Ala Ala
   145                     150                     155                 160
   ggg cac atc gtc gcg gtg cgc cag ggt gcg gtg ctt gcc acc gcg ttt        528
   Gly His Ile Val Ala Val Arg Gln Gly Ala Val Leu Ala Thr Ala Phe
                   165                     170                     175
   cat ccg gag atg acc ggc gat cgc cgc att cat cag ttg ttc gtc gac        576
   His Pro Glu Met Thr Gly Asp Arg Arg Ile His Gln Leu Phe Val Asp
               180                     185                     190
   atc gtc acc tcc gcg gcg tga                                            597
   Ile Val Thr Ser Ala Ala
           195
<210> 13
   <211> 198
   <212> PRT
   <213> Mycobacterium tuberculosis
<400> 13
   Met Ser Val Pro Arg Val Gly Val Leu Ala Leu Gln Gly Asp Thr Arg
   1               5                   10                  15
   Glu His Leu Ala Ala Leu Arg Glu Cys Gly Ala Glu Pro Met Thr Val
               20                  25                  30
   Arg Arg Arg Asp Glu Leu Asp Ala Val Asp Ala Leu Val Ile Pro Gly
           35                  40                  45
   Gly Glu Ser Thr Thr Met Ser His Leu Leu Leu Asp Leu Asp Leu Leu
       50                  55                  60
   Gly Pro Leu Arg Ala Arg Leu Ala Asp Gly Leu Pro Ala Tyr Gly Ser
   65                  70                  75                  80
   Cys Ala Gly Met Ile Leu Leu Ala Ser Glu Ile Leu Asp Ala Gly Ala
                   85                  90                  95
   Ala Gly Arg Gln Ala Leu Pro Leu Arg Ala Met Asn Met Thr Val Arg
               100                 105                 110
   Arg Asn Ala Phe Gly Ser Gln Val Asp Ser Phe Glu Gly Asp Ile Glu
           115                 120                 125
   Phe Ala Gly Leu Asp Asp Pro Val Arg Ala Val Phe Ile Arg Ala Pro
       130                 135                 140
   Trp Val Glu Arg Val Gly Asp Gly Val Gln Val Leu Ala Arg Ala Ala
   145                 150                 155                 160
   Gly His Ile Val Ala Val Arg Gln Gly Ala Val Leu Ala Thr Ala Phe
                   165                 170                 175
   His Pro Glu Met Thr Gly Asp Arg Arg Ile His Gln Leu Phe Val Asp
               180                 185                 190
   Ile Val Thr Ser Ala Ala
           195
<210> 14
   <211> 561
   <212> DNA
   <213> Aeropyrum pernix
<400> 14
   atg ctt agg agg acc ttc gac cgc ctg ggc gtg cat ggc gag gcg gta        48
   Met Leu Arg Arg Thr Phe Asp Arg Leu Gly Val His Gly Glu Ala Val
   1               5                   10                  15
   gtc gtc aaa aag ccg gag gac ctc aag ggg ctg gac ggc gta att ata        96
   Val Val Lys Lys Pro Glu Asp Leu Lys Gly Leu Asp Gly Val Ile Ile
               20                  25                  30
   ccg ggc ggt gaa agc acg acc atc ggg ata ctg gcg aag agg ctg ggc       144
   Pro Gly Gly Glu Ser Thr Thr Ile Gly Ile Leu Ala Lys Arg Leu Gly
           35                  40                  45
   gtc cta gag cct ctg agg gag cag gtc ctc aac ggc ctc cca gcc atg       192
   Val Leu Glu Pro Leu Arg Glu Gln Val Leu Asn Gly Leu Pro Ala Met
       50                  55                  60
   ggg acg tgc gca ggg gct ata ata ctg gct ggg aag gtt agg gac aag       240
   Gly Thr Cys Ala Gly Ala Ile Ile Leu Ala Gly Lys Val Arg Asp Lys
   65                  70                  75                  80
   gtc gta ggg gag aag agc cag cca cta ctg ggg gtt atg agg gtt gaa       288
   Val Val Gly Glu Lys Ser Gln Pro Leu Leu Gly Val Met Arg Val Glu
                   85                  90                  95
   gtt gtg aga aac ttc ttc ggc agg cag agg gag agc ttc gaa gcc gac       336
   Val Val Arg Asn Phe Phe Gly Arg Gln Arg Glu Ser Phe Glu Ala Asp
               100                 105                 110
   ctg gag ata gag ggt ctc gac ggg agg ttc cgc ggc gtg ttc ata agg       384
   Leu Glu Ile Glu Gly Leu Asp Gly Arg Phe Arg Gly Val Phe Ile Arg
           115                 120                 125
   agc cct gcg ata acg gca gcg gag agt cca gct agg atc ata agc tgg       432
   Ser Pro Ala Ile Thr Ala Ala Glu Ser Pro Ala Arg Ile Ile Ser Trp
       130                 135                 140
   ctc gac tac aac ggt cag agg gtt ggg gtc gcg gca gtt cag ggc ccc       480
   Leu Asp Tyr Asn Gly Gln Arg Val Gly Val Ala Ala Val Gln Gly Pro
   145                 150                 155                 160
   cta ctc gca act agc ttc cac cca gag ctc act ggg gac aca agg ctt       528
   Leu Leu Ala Thr Ser Phe His Pro Glu Leu Thr Gly Asp Thr Arg Leu
                   165                 170                 175
   cac gaa ctc tgg cta agg ctt gtg aaa aga tag                           561
   His Glu Leu Trp Leu Arg Leu Val Lys Arg
               180                 185
<210> 15
   <211> 186
   <212> PRT
   <213> Aeropyrum pernix
<400> 15
   Met Leu Arg Arg Thr Phe Asp Arg Leu Gly Val His Gly Glu Ala Val
   1               5                   10                  15
   Val Val Lys Lys Pro Glu Asp Leu Lys Gly Leu Asp Gly Val Ile Ile
               20                  25                  30
   Pro Gly Gly Glu Ser Thr Thr Ile Gly Ile Leu Ala Lys Arg Leu Gly
           35                  40                  45
   Val Leu Glu Pro Leu Arg Glu Gln Val Leu Asn Gly Leu Pro Ala Met
       50                  55                  60
   Gly Thr Cys Ala Gly Ala Ile Ile Leu Ala Gly Lys Val Arg Asp Lys
   65                  70                  75                  80
   Val Val Gly Glu Lys Ser Gln Pro Leu Leu Gly Val Met Arg Val Glu
                   85                  90                  95
   Val Val Arg Asn Phe Phe Gly Arg Gln Arg Glu Ser Phe Glu Ala Asp
               100                 105                 110
   Leu Glu Ile Glu Gly Leu Asp Gly Arg Phe Arg Gly Val Phe Ile Arg
           115                 120                 125
   Ser Pro Ala Ile Thr Ala Ala Glu Ser Pro Ala Arg Ile Ile Ser Trp
       130                 135                 140
   Leu Asp Tyr Asn Gly Gln Arg Val Gly Val Ala Ala Val Gln Gly Pro
   145                 150                 155                 160
   Leu Leu Ala Thr Ser Phe His Pro Glu Leu Thr Gly Asp Thr Arg Leu
                   165                 170                 175
   His Glu Leu Trp Leu Arg Leu Val Lys Arg
               180                 185
<210> 16
   <211> 612
   <212> DNA
   <213> Halobacterium sp. NRC-1
<400> 16
   atg aca ctg act gcc ggt gtt gtc gcc gtg cag ggc gac gtc tcc gaa      48
   Met Thr Leu Thr Ala Gly Val Val Ala Val Gln Gly Asp Val Ser Glu
   1               5                   10                  15
   cac gcc gcc gcg atc cgc cgc gct gcc gac gct cac ggc cag ccc gcc      96
   His Ala Ala Ala Ile Arg Arg Ala Ala Asp Ala His Gly Gln Pro Ala
               20                  25                  30
   gac gtg cgt gag atc cgg acc gcg ggg gtc gtc ccg gag tgt gac gtg      144
   Asp Val Arg Glu Ile Arg Thr Ala Gly Val Val Pro Glu Cys Asp Val
           35                  40                  45
   ttg ctg ttg ccc ggt ggg gag tcg acg gcc atc tct cgg ctg ctg gac      192
   Leu Leu Leu Pro Gly Gly Glu Ser Thr Ala Ile Ser Arg Leu Leu Asp
       50                  55                  60
   cgc gag ggc atc gac gcc gag atc cgc agc cac gtc gcc gcc ggc aag      240
   Arg Glu Gly Ile Asp Ala Glu Ile Arg Ser His Val Ala Ala Gly Lys
   65                  70                  75                  80
   ccg ctg ctg gcg acg tgc gcg ggc ctc atc gtg tcc tcg acg gac gcc      288
   Pro Leu Leu Ala Thr Cys Ala Gly Leu Ile Val Ser Ser Thr Asp Ala
                   85                  90                  95
   aac gac gac cgc gtc gaa acg ctt gac gtg ctc gac gtg acc gtc gat      336
   Asn Asp Asp Arg Val Glu Thr Leu Asp Val Leu Asp Val Thr Val Asp
               100                 105                 110
   cgg aac gcg ttc ggc cgc cag gtc gac tcc ttc gaa gcc ccc ctg gac      384
   Arg Asn Ala Phe Gly Arg Gln Val Asp Ser Phe Glu Ala Pro Leu Asp
           115                 120                 125
   gtc gac ggg ctc gcc gac ccc ttc ccc gcg gtg ttc atc cgc gcg ccg      432
   Val Asp Gly Leu Ala Asp Pro Phe Pro Ala Val Phe Ile Arg Ala Pro
       130                 135                 140
   gtc atc gac gag gtc ggc gcg gac gcg acg gtg ctt gcg tcc tgg gac      480
   Val Ile Asp Glu Val Gly Ala Asp Ala Thr Val Leu Ala Ser Trp Asp
   145                 150                 155                 160
   ggg cgt ccg gtt gcg atc cgg gac ggc ccc gtg gtt gcg acg tcg ttc      528
   Gly Arg Pro Val Ala Ile Arg Asp Gly Pro Val Val Ala Thr Ser Phe
                   165                 170                 175
   cac ccg gag ctg acc gcc gac gtg cgg ctg cac gaa ctc gcg ttt ttc      576
   His Pro Glu Leu Thr Ala Asp Val Arg Leu His Glu Leu Ala Phe Phe
               180                 185                 190
   gac cga aca ccg tcc gca cag gcc ggt gac gca tga                      612
   Asp Arg Thr Pro Ser Ala Gln Ala Gly Asp Ala
           195                 200
<210> 17
   <211> 203
   <212> PRT
   <213> Halobacterium sp. NRC-1
<400> 17
   Met Thr Leu Thr Ala Gly Val Val Ala Val Gln Gly Asp Val Ser Glu
   1               5                   10                  15
   His Ala Ala Ala Ile Arg Arg Ala Ala Asp Ala His Gly Gln Pro Ala
               20                  25                  30
   Asp Val Arg Glu Ile Arg Thr Ala Gly Val Val Pro Glu Cys Asp Val
           35                  40                  45
   Leu Leu Leu Pro Gly Gly Glu Ser Thr Ala Ile Ser Arg Leu Leu Asp
       50                  55                  60
   Arg Glu Gly Ile Asp Ala Glu Ile Arg Ser His Val Ala Ala Gly Lys
   65                  70                  75                  80
   Pro Leu Leu Ala Thr Cys Ala Gly Leu Ile Val Ser Ser Thr Asp Ala
                   85                  90                  95
   Asn Asp Asp Arg Val Glu Thr Leu Asp Val Leu Asp Val Thr Val Asp
               100                 105                 110
   Arg Asn Ala Phe Gly Arg Gln Val Asp Ser Phe Glu Ala Pro Leu Asp
           115                 120                 125
   Val Asp Gly Leu Ala Asp Pro Phe Pro Ala Val Phe Ile Arg Ala Pro
       130                 135                 140
   Val Ile Asp Glu Val Gly Ala Asp Ala Thr Val Leu Ala Ser Trp Asp
   145                 150                 155                 160
   Gly Arg Pro Val Ala Ile Arg Asp Gly Pro Val Val Ala Thr Ser Phe
                   165                 170                 175
   His Pro Glu Leu Thr Ala Asp Val Arg Leu His Glu Leu Ala Phe Phe
               180                 185                 190
   Asp Arg Thr Pro Ser Ala Gln Ala Gly Asp Ala
           195                 200
<210> 18
   <211> 591
   <212> DNA
   <213> Pyrococcus horikoshii
<400> 18
   atg aag gtt gga gtt gta gga ttg caa gga gat gtt agc gag cac att         48
   Met Lys Val Gly Val Val Gly Leu Gln Gly Asp Val Ser Glu His Ile
   1               5                   10                  15
   gaa gct act aaa atg gcc atc gag aag ctc gag ctt cct ggg gaa gtg         96
   Glu Ala Thr Lys Met Ala Ile Glu Lys Leu Glu Leu Pro Gly Glu Val
               20                  25                  30
   atc tgg ctc aag agg cct gag cag ctt aag ggt gtt gat gcg gta ata        144
   Ile Trp Leu Lys Arg Pro Glu Gln Leu Lys Gly Val Asp Ala Val Ile
           35                  40                  45
   atc cct gga ggg gag agc aca aca ata tca agg ctc atg caa agg acg        192
   Ile Pro Gly Gly Glu Ser Thr Thr Ile Ser Arg Leu Met Gln Arg Thr
       50                  55                  60
   ggg ctt ttt gag ccc att aaa aag atg gtt gag gat ggt tta ccg gtg        240
   Gly Leu Phe Glu Pro Ile Lys Lys Met Val Glu Asp Gly Leu Pro Val
   65                  70                  75                  80
   atg ggg act tgt gca gga tta ata atg ctt gca aag gaa gtc cta ggg        288
   Met Gly Thr Cys Ala Gly Leu Ile Met Leu Ala Lys Glu Val Leu Gly
                   85                  90                  95
   gca act cct gag cag aag ttc tta gag gtt ctg gat gtt aag gta aat        336
   Ala Thr Pro Glu Gln Lys Phe Leu Glu Val Leu Asp Val Lys Val Asn
               100                 105                 110
   agg aac gcc tac gga agg caa gtt gac agc ttt gaa gct cct gtg aag        384
   Arg Asn Ala Tyr Gly Arg Gln Val Asp Ser Phe Glu Ala Pro Val Lys
           115                 120                 125
   tta gca ttt gac gat gaa cct ttc att ggg gta ttc att agg gcc ccc        432
   Leu Ala Phe Asp Asp Glu Pro Phe Ile Gly Val Phe Ile Arg Ala Pro
       130                 135                 140
   agg ata gtt gag tta ttg tcg gag aaa gtt aaa ccc cta gct tgg ctg        480
   Arg Ile Val Glu Leu Leu Ser Glu Lys Val Lys Pro Leu Ala Trp Leu
   145                 150                 155                 160
   gag gat agg gta gtg ggg gtt gag cag gaa aac ata atc ggc ctg gag        528
   Glu Asp Arg Val Val Gly Val Glu Gln Glu Asn Ile Ile Gly Leu Glu
                   165                 170                 175
   ttt cat cca gaa ctt acc aat gac act aga atc cat gag tac ttc tta        576
   Phe His Pro Glu Leu Thr Asn Asp Thr Arg Ile His Glu Tyr Phe Leu
               180                 185                 190
   agg aag gta atc tag                                                    591
   Arg Lys Val Ile
           195
<210> 19
   <211> 196
   <212> PRT
   <213> Pyrococcus horikoshii
<400> 19
   Met Lys Val Gly Val Val Gly Leu Gln Gly Asp Val Ser Glu His Ile
   1               5                   10                  15
   Glu Ala Thr Lys Met Ala Ile Glu Lys Leu Glu Leu Pro Gly Glu Val
               20                  25                  30
   Ile Trp Leu Lys Arg Pro Glu Gln Leu Lys Gly Val Asp Ala Val Ile
           35                  40                  45
   Ile Pro Gly Gly Glu Ser Thr Thr Ile Ser Arg Leu Met Gln Arg Thr
       50                  55                  60
   Gly Leu Phe Glu Pro Ile Lys Lys Met Val Glu Asp Gly Leu Pro Val
   65                  70                  75                  80
   Met Gly Thr Cys Ala Gly Leu Ile Met Leu Ala Lys Glu Val Leu Gly
                   85                  90                  95
   Ala Thr Pro Glu Gln Lys Phe Leu Glu Val Leu Asp Val Lys Val Asn
               100                 105                 110
   Arg Asn Ala Tyr Gly Arg Gln Val Asp Ser Phe Glu Ala Pro Val Lys
           115                 120                 125
   Leu Ala Phe Asp Asp Glu Pro Phe Ile Gly Val Phe Ile Arg Ala Pro
           130                     135                     140
   Arg Ile Val Glu Leu Leu Ser Glu Lys Val Lys Pro Leu Ala Trp Leu
   145                     150                     155                 160
   Glu Asp Arg Val Val Gly Val Glu Gln Glu Asn Ile Ile Gly Leu Glu
                   165                     170                     175
   Phe His Pro Glu Leu Thr Asn Asp Thr Arg Ile His Glu Tyr Phe Leu
               180                     185                     190
   Arg Lys Val Ile
           195
<210> 20
   <211> 597
   <212> DNA
   <213> Archaeoglobus fulgidus
<400> 20
   atg aaa gtt gca gtg gtg ggc gtt cag gga gac gta gag gag cac gtc      48
   Met Lys Val Ala Val Val Gly Val Gln Gly Asp Val Glu Glu His Val
   1               5                   10                  15
   ctg gcg acg aaa agg gcc ctt aaa agg ctt ggg att gat gga gag gtt      96
   Leu Ala Thr Lys Arg Ala Leu Lys Arg Leu Gly Ile Asp Gly Glu Val
               20                  25                  30
   gtt gct aca aga agg aga ggt gtt gtt tca aga agc gat gcc gtt att     144
   Val Ala Thr Arg Arg Arg Gly Val Val Ser Arg Ser Asp Ala Val Ile
           35                  40                  45
   ctt cct ggt ggg gag agc acg aca ata agc aaa ctc att ttt tcc gac     192
   Leu Pro Gly Gly Glu Ser Thr Thr Ile Ser Lys Leu Ile Phe Ser Asp
       50                  55                  60
   ggc att gct gac gaa att ttg cag ctt gca gaa gag gga aag ccg gtt     240
   Gly Ile Ala Asp Glu Ile Leu Gln Leu Ala Glu Glu Gly Lys Pro Val
   65                  70                  75                  80
   atg ggt aca tgt gct ggt ttg ata ctc ctt tcc aaa tat ggc gac gag     288
   Met Gly Thr Cys Ala Gly Leu Ile Leu Leu Ser Lys Tyr Gly Asp Glu
                   85                  90                  95
   cag gtt gaa aaa acg aac acg aag ctt ttg ggt ctg ctg gac gcg aag     336
   Gln Val Glu Lys Thr Asn Thr Lys Leu Leu Gly Leu Leu Asp Ala Lys
               100                 105                 110
   gtt aag aga aac gcc ttc gga agg cag agg gaa agc ttt cag gtg cct     384
   Val Lys Arg Asn Ala Phe Gly Arg Gln Arg Glu Ser Phe Gln Val Pro
           115                 120                 125
   ctg gat gta aag tac gtt gga aag ttc gat gcc gta ttt ata aga gct     432
   Leu Asp Val Lys Tyr Val Gly Lys Phe Asp Ala Val Phe Ile Arg Ala
       130                 135                 140
   ccg gcc ata act gaa gtc ggg aaa gac gtg gag gtg ctt gca acc ttt     480
   Pro Ala Ile Thr Glu Val Gly Lys Asp Val Glu Val Leu Ala Thr Phe
   145                 150                 155                 160
   gag aac ctc atc gtt gca gca agg caa aaa aac gtt tta ggc cta gcc     528
   Glu Asn Leu Ile Val Ala Ala Arg Gln Lys Asn Val Leu Gly Leu Ala
                   165                 170                 175
   ttt cat ccc gaa ctg acg gat gat acg aga att cac gag ttc ttc ctt     576
   Phe His Pro Glu Leu Thr Asp Asp Thr Arg Ile His Glu Phe Phe Leu
               180                 185                 190
   aaa ctt gga gaa acg agc taa                                         597
   Lys Leu Gly Glu Thr Ser
           195
<210> 21
   <211> 198
   <212> PRT
   <213> Archaeoglobus fulgidus
<400> 21
   Met Lys Val Ala Val Val Gly Val Gln Gly Asp Val Glu Glu His Val
   1               5                   10                  15
   Leu Ala Thr Lys Arg Ala Leu Lys Arg Leu Gly Ile Asp Gly Glu Val
               20                  25                  30
   Val Ala Thr Arg Arg Arg Gly Val Val Ser Arg Ser Asp Ala Val Ile
           35                  40                  45
   Leu Pro Gly Gly Glu Ser Thr Thr Ile Ser Lys Leu Ile Phe Ser Asp
       50                  55                  60
   Gly Ile Ala Asp Glu Ile Leu Gln Leu Ala Glu Glu Gly Lys Pro Val
   65                  70                  75                  80
   Met Gly Thr Cys Ala Gly Leu Ile Leu Leu Ser Lys Tyr Gly Asp Glu
                   85                  90                  95
   Gln Val Glu Lys Thr Asn Thr Lys Leu Leu Gly Leu Leu Asp Ala Lys
               100                 105                 110
   Val Lys Arg Asn Ala Phe Gly Arg Gln Arg Glu Ser Phe Gln Val Pro
           115                 120                 125
   Leu Asp Val Lys Tyr Val Gly Lys Phe Asp Ala Val Phe Ile Arg Ala
       130                 135                 140
   Pro Ala Ile Thr Glu Val Gly Lys Asp Val Glu Val Leu Ala Thr Phe
   145                 150                 155                 160
   Glu Asn Leu Ile Val Ala Ala Arg Gln Lys Asn Val Leu Gly Leu Ala
                   165                 170                 175
   Phe His Pro Glu Leu Thr Asp Asp Thr Arg Ile His Glu Phe Phe Leu
               180                 185                 190
   Lys Leu Gly Glu Thr Ser
           195
<210> 22
   <211> 579
   <212> DNA
   <213> Methanobacterium thermoautotrophicum
<400> 22
   atg ata agg ata ggt att ctt gct ctt cag gga gat gta tcc gaa cac       48
   Met Ile Arg Ile Gly Ile Leu Ala Leu Gln Gly Asp Val Ser Glu His
   1               5                   10                  15
   ctc gag atg acc aga agg aca gtc gaa gag atg ggc ata gat gca gag       96
   Leu Glu Met Thr Arg Arg Thr Val Glu Glu Met Gly Ile Asp Ala Glu
               20                  25                  30
   gtt gtg agg gtc agg aca gca gag gaa gcc tcc aca gtc gat gca ata       144
   Val Val Arg Val Arg Thr Ala Glu Glu Ala Ser Thr Val Asp Ala Ile
           35                  40                  45
   ata ata tcc ggc ggc gag agt acg gta ata ggt agg ctg atg gag gag       192
   Ile Ile Ser Gly Gly Glu Ser Thr Val Ile Gly Arg Leu Met Glu Glu
       50                  55                  60
   aca ggg ata aag gac gtc ata atc cgc gaa aag aaa cct gtg atg ggc       240
   Thr Gly Ile Lys Asp Val Ile Ile Arg Glu Lys Lys Pro Val Met Gly
   65                  70                  75                  80
   aca tgt gcc ggc atg gtg ctc ctt gca gat gaa aca gat tat gaa cag       288
   Thr Cys Ala Gly Met Val Leu Leu Ala Asp Glu Thr Asp Tyr Glu Gln
                   85                  90                  95
   ccc ctt ctg gga ctc ata gat atg aag gtt aag aga aac gcc ttt gga       336
   Pro Leu Leu Gly Leu Ile Asp Met Lys Val Lys Arg Asn Ala Phe Gly
               100                 105                 110
   aga cag aga gac tcc ttt gaa gat gag atc gat ata ctt gga agg aaa       384
   Arg Gln Arg Asp Ser Phe Glu Asp Glu Ile Asp Ile Leu Gly Arg Lys
           115                 120                 125
   ttt cat gga ata ttc ata agg gcg ccg gct gtc ctt gaa gtg gga gag       432
   Phe His Gly Ile Phe Ile Arg Ala Pro Ala Val Leu Glu Val Gly Glu
       130                 135                 140
   gga gtt gag gtt ctc tca gaa ctc gat gat atg ata atc gca gta aag       480
   Gly Val Glu Val Leu Ser Glu Leu Asp Asp Met Ile Ile Ala Val Lys
   145                 150                 155                 160
   gac ggc tgc aac ctc gca ctg gcc ttt cac cct gaa ctc gga gag gac       528
   Asp Gly Cys Asn Leu Ala Leu Ala Phe His Pro Glu Leu Gly Glu Asp
                   165                 170                 175
   aca gga ctc cat gaa tac ttt ata aag gag gta ttg aat tgt gtg gaa       576
   Thr Gly Leu His Glu Tyr Phe Ile Lys Glu Val Leu Asn Cys Val Glu
               180                 185                 190
   tag                                                                   579
<210> 23
   <211> 192
   <212> PRT
   <213> Methanobacterium thermoautotrophicum
<400> 23
   Met Ile Arg Ile Gly Ile Leu Ala Leu Gln Gly Asp Val Ser Glu His
   1               5                   10                  15
   Leu Glu Met Thr Arg Arg Thr Val Glu Glu Met Gly Ile Asp Ala Glu
               20                  25                  30
   Val Val Arg Val Arg Thr Ala Glu Glu Ala Ser Thr Val Asp Ala Ile
           35                  40                  45
   Ile Ile Ser Gly Gly Glu Ser Thr Val Ile Gly Arg Leu Met Glu Glu
       50                  55                  60
   Thr Gly Ile Lys Asp Val Ile Ile Arg Glu Lys Lys Pro Val Met Gly
   65                  70                  75                  80
   Thr Cys Ala Gly Met Val Leu Leu Ala Asp Glu Thr Asp Tyr Glu Gln
                   85                  90                  95
   Pro Leu Leu Gly Leu Ile Asp Met Lys Val Lys Arg Asn Ala Phe Gly
               100                 105                 110
   Arg Gln Arg Asp Ser Phe Glu Asp Glu Ile Asp Ile Leu Gly Arg Lys
           115                 120                 125
   Phe His Gly Ile Phe Ile Arg Ala Pro Ala Val Leu Glu Val Gly Glu
       130                 135                 140
   Gly Val Glu Val Leu Ser Glu Leu Asp Asp Met Ile Ile Ala Val Lys
   145                 150                 155                 160
   Asp Gly Cys Asn Leu Ala Leu Ala Phe His Pro Glu Leu Gly Glu Asp
                   165                 170                 175
   Thr Gly Leu His Glu Tyr Phe Ile Lys Glu Val Leu Asn Cys Val Glu
               180                 185                 190
<210> 24
   <211> 528
   <212> DNA
   <213> Haemophilus influenzae
<400> 24
   atg cta gaa aaa tta gga att gaa agt gtc gaa ctg aga aat tta aaa       48
   Met Leu Glu Lys Leu Gly Ile Glu Ser Val Glu Leu Arg Asn Leu Lys
   1               5                   10                  15
   aat ttt caa caa cat tac agt gat tta tca ggt ttg att cta cct ggc       96
   Asn Phe Gln Gln His Tyr Ser Asp Leu Ser Gly Leu Ile Leu Pro Gly
               20                  25                  30
   ggt gag tca acc gcc ata gga aaa ctt tta aga gag ctg tat atg ctg      144
   Gly Glu Ser Thr Ala Ile Gly Lys Leu Leu Arg Glu Leu Tyr Met Leu
           35                  40                  45
   gaa ccg ata aaa caa gct atc tct tct ggc ttt cct gtc ttt gga act      192
   Glu Pro Ile Lys Gln Ala Ile Ser Ser Gly Phe Pro Val Phe Gly Thr
       50                  55                  60
   tgt gct ggt ttg att ctg ttg gct aaa gag att act tct cag aaa gag      240
   Cys Ala Gly Leu Ile Leu Leu Ala Lys Glu Ile Thr Ser Gln Lys Glu
   65                  70                  75                  80
   agt cat ttt gga aca atg gac att gtg gtt gag agg aat gcc tat gga      288
   Ser His Phe Gly Thr Met Asp Ile Val Val Glu Arg Asn Ala Tyr Gly
                   85                  90                  95
   cgc caa ttg gga agt ttc tat aca gaa gca gat tgc aaa ggg gtt ggt      336
   Arg Gln Leu Gly Ser Phe Tyr Thr Glu Ala Asp Cys Lys Gly Val Gly
               100                 105                 110
   aaa att cct atg act ttt atc aga gga cct atc atc agt agt gtt ggt      384
   Lys Ile Pro Met Thr Phe Ile Arg Gly Pro Ile Ile Ser Ser Val Gly
           115                 120                 125
   aaa aaa gtc aat att ctt gca acg gta aat aat aaa atc gtt gca gcc      432
   Lys Lys Val Asn Ile Leu Ala Thr Val Asn Asn Lys Ile Val Ala Ala
       130                 135                 140
   caa gaa aag aat atg ctg gta aca tca ttt cat cct gaa tta aca aat      480
   Gln Glu Lys Asn Met Leu Val Thr Ser Phe His Pro Glu Leu Thr Asn
   145                 150                 155                 160
   aac ttg agt ttg cat aaa tac ttt atc gat ata tgt aaa gta gca          525
   Asn Leu Ser Leu His Lys Tyr Phe Ile Asp Ile Cys Lys Val Ala
                   165                 170                 175
   taa                                                                  528
<210> 25
   <211> 175
   <212> PRT
   <213> Haemophilus influenzae
<400> 25
   Met Leu Glu Lys Leu Gly Ile Glu Ser Val Glu Leu Arg Asn Leu Lys
   1               5                   10                  15
   Asn Phe Gln Gln His Tyr Ser Asp Leu Ser Gly Leu Ile Leu Pro Gly
               20                  25                  30
   Gly Glu Ser Thr Ala Ile Gly Lys Leu Leu Arg Glu Leu Tyr Met Leu
           35                  40                  45
   Glu Pro Ile Lys Gln Ala Ile Ser Ser Gly Phe Pro Val Phe Gly Thr
       50                  55                  60
   Cys Ala Gly Leu Ile Leu Leu Ala Lys Glu Ile Thr Ser Gln Lys Glu
   65                  70                  75                  80
   Ser His Phe Gly Thr Met Asp Ile Val Val Glu Arg Asn Ala Tyr Gly
                   85                  90                  95
   Arg Gln Leu Gly Ser Phe Tyr Thr Glu Ala Asp Cys Lys Gly Val Gly
               100                 105                 110
   Lys Ile Pro Met Thr Phe Ile Arg Gly Pro Ile Ile Ser Ser Val Gly
           115                 120                 125
   Lys Lys Val Asn Ile Leu Ala Thr Val Asn Asn Lys Ile Val Ala Ala
       130                 135                 140
   Gln Glu Lys Asn Met Leu Val Thr Ser Phe His Pro Glu Leu Thr Asn
   145                 150                 155                 160
   Asn Leu Ser Leu His Lys Tyr Phe Ile Asp Ile Cys Lys Val Ala
                   165                 170                 175
<210> 26
   <211> 591
   <212> DNA
   <213> Deinococcus radiodurans
<400> 26
   atg acc gtc ggc gtt ctc gcg ctg caa ggc gcc ttt cgc gag cac cgc      48
   Met Thr Val Gly Val Leu Ala Leu Gln Gly Ala Phe Arg Glu His Arg
   1               5                   10                  15
   cag cgc ctc gag cag ctc ggc gcc ggg gtc cgc gag gtg cgc ctg ccc      96
   Gln Arg Leu Glu Gln Leu Gly Ala Gly Val Arg Glu Val Arg Leu Pro
               20                  25                  30
   gcc gat ctc gcc ggc ctg agc ggg ctg atc ctg ccg ggc ggc gag tcc      144
   Ala Asp Leu Ala Gly Leu Ser Gly Leu Ile Leu Pro Gly Gly Glu Ser
           35                  40                  45
   acg acg atg gtc cgg ctg ctc acg gaa ggc ggc ctc tgg cac ccc ctg      192
   Thr Thr Met Val Arg Leu Leu Thr Glu Gly Gly Leu Trp His Pro Leu
       50                  55                  60
   cgc gac ttt cat gcc gcc ggc ggg gcg ctg tgg ggc acc tgc gcg ggc      240
   Arg Asp Phe His Ala Ala Gly Gly Ala Leu Trp Gly Thr Cys Ala Gly
   65                  70                  75                  80
   gcc atc gtg ctg gcg cgc gag gtg atg ggc ggc agt ccc tcg ctg ccg      288
   Ala Ile Val Leu Ala Arg Glu Val Met Gly Gly Ser Pro Ser Leu Pro
                   85                  90                  95
   ccg cag ccg ggg ctg ggg ctg ctc gac atc acc gtg cag cgc aac gcc      336
   Pro Gln Pro Gly Leu Gly Leu Leu Asp Ile Thr Val Gln Arg Asn Ala
               100                 105                 110
   ttc ggg cgg cag gtg gac tcg ttc acc gcc cca ctc gac att gcc ggg      384
   Phe Gly Arg Gln Val Asp Ser Phe Thr Ala Pro Leu Asp Ile Ala Gly
           115                 120                 125
   ctc gac gcg ccg ttt ccc gcc gtc ttt atc cgc gcc ccg gtc atc acg      432
   Leu Asp Ala Pro Phe Pro Ala Val Phe Ile Arg Ala Pro Val Ile Thr
       130                 135                 140
   cgg gtg ggc ccg gcg gcg cgg gcc ctc gcg acc ctc ggc gac cgg acc      480
   Arg Val Gly Pro Ala Ala Arg Ala Leu Ala Thr Leu Gly Asp Arg Thr
   145                 150                 155                 160
   gcg cac gtg cag cag ggc cgc gtc ctg gcg agt gct ttt cat cct gaa      528
   Ala His Val Gln Gln Gly Arg Val Leu Ala Ser Ala Phe His Pro Glu
                   165                 170                 175
   ctg acg gaa gac aca cgt ctg cac cgg gtg ttt ctc ggc ctc gcg ggc      576
   Leu Thr Glu Asp Thr Arg Leu His Arg Val Phe Leu Gly Leu Ala Gly
               180                 185                 190
   gag cgg gca tac tag                                                  591
   Glu Arg Ala Tyr
           195
<210> 27
   <211> 196
   <212> PRT
   <213> Deinococcus radiodurans
<400> 27
   Met Thr Val Gly Val Leu Ala Leu Gln Gly Ala Phe Arg Glu His Arg
   1               5                   10                  15
   Gln Arg Leu Glu Gln Leu Gly Ala Gly Val Arg Glu Val Arg Leu Pro
               20                  25                  30
   Ala Asp Leu Ala Gly Leu Ser Gly Leu Ile Leu Pro Gly Gly Glu Ser
           35                  40                  45
   Thr Thr Met Val Arg Leu Leu Thr Glu Gly Gly Leu Trp His Pro Leu
       50                  55                  60
   Arg Asp Phe His Ala Ala Gly Gly Ala Leu Trp Gly Thr Cys Ala Gly
   65                  70                  75                  80
   Ala Ile Val Leu Ala Arg Glu Val Met Gly Gly Ser Pro Ser Leu Pro
                   85                  90                  95
   Pro Gln Pro Gly Leu Gly Leu Leu Asp Ile Thr Val Gln Arg Asn Ala
               100                 105                 110
   Phe Gly Arg Gln Val Asp Ser Phe Thr Ala Pro Leu Asp Ile Ala Gly
           115                 120                 125
   Leu Asp Ala Pro Phe Pro Ala Val Phe Ile Arg Ala Pro Val Ile Thr
       130                 135                 140
   Arg Val Gly Pro Ala Ala Arg Ala Leu Ala Thr Leu Gly Asp Arg Thr
   145                 150                 155                 160
   Ala His Val Gln Gln Gly Arg Val Leu Ala Ser Ala Phe His Pro Glu
                   165                 170                 175
   Leu Thr Glu Asp Thr Arg Leu His Arg Val Phe Leu Gly Leu Ala Gly
               180                 185                 190
   Glu Arg Ala Tyr
           195
<210> 28
   <211> 591
   <212> DNA
   <213> Bacillus halodurans
<400> 28
   atg gtg aaa atc ggt gta ttg gca ctt cag gga gcc gtt agg gag cat      48
   Met Val Lys Ile Gly Val Leu Ala Leu Gln Gly Ala Val Arg Glu His
   1               5                   10                  15
   gtc cgc tgc ctc gaa gct cct ggg gtg gaa gtg agc att gtc aag aaa      96
   Val Arg Cys Leu Glu Ala Pro Gly Val Glu Val Ser Ile Val Lys Lys
               20                  25                  30
   gta gag cag ctt gag gat ttg gac ggt ctt gtc ttc cct ggt ggg gaa      144
   Val Glu Gln Leu Glu Asp Leu Asp Gly Leu Val Phe Pro Gly Gly Glu
           35                  40                  45
   agc acg acg atg cgc cgc ctc atc gat aaa tat ggc ttt ttt gaa cct      192
   Ser Thr Thr Met Arg Arg Leu Ile Asp Lys Tyr Gly Phe Phe Glu Pro
       50                  55                  60
   tta aag gca ttc gct gca cag ggc aag ccg gta ttt ggt acg tgt gct      240
   Leu Lys Ala Phe Ala Ala Gln Gly Lys Pro Val Phe Gly Thr Cys Ala
   65                  70                  75                  80
   ggg ttg att tta atg gcg aca cgt att gat gga gag gat cat ggg cat      288
   Gly Leu Ile Leu Met Ala Thr Arg Ile Asp Gly Glu Asp His Gly His
                   85                  90                  95
   ctt gaa tta atg gat atg aca gtg caa cgg aac gct ttt ggt cgt cag      336
   Leu Glu Leu Met Asp Met Thr Val Gln Arg Asn Ala Phe Gly Arg Gln
               100                 105                 110
   cgc gaa agc ttc gaa aca gac ttg att gtg gaa ggc gtt ggc gat gac      384
   Arg Glu Ser Phe Glu Thr Asp Leu Ile Val Glu Gly Val Gly Asp Asp
           115                 120                 125
   gta cgt gcg gtt ttt atc cgt gcc cct tta att cag gaa gtg ggt caa      432
   Val Arg Ala Val Phe Ile Arg Ala Pro Leu Ile Gln Glu Val Gly Gln
       130                 135                 140
   aat gtg gac gtg ctg tcc aag ttt ggc gat gaa att gtt gtc gct aga      480
   Asn Val Asp Val Leu Ser Lys Phe Gly Asp Glu Ile Val Val Ala Arg
   145                 150                 155                 160
   caa ggt cat ttg ctc ggt tgt tca ttc cat cct gaa ctg acg gat gat      528
   Gln Gly His Leu Leu Gly Cys Ser Phe His Pro Glu Leu Thr Asp Asp
                   165                 170                 175
   cgg aga ttt cat caa tac ttc gtc caa atg gta aaa gaa gca aaa acc      576
   Arg Arg Phe His Gln Tyr Phe Val Gln Met Val Lys Glu Ala Lys Thr
               180                 185                 190
   att gct caa tca taa                                                  591
   Ile Ala Gln Ser
           195
<210> 29
   <211> 196
   <212> PRT
   <213> Bacillus halodurans
<400> 29
   Met Val Lys Ile Gly Val Leu Ala Leu Gln Gly Ala Val Arg Glu His
   1               5                   10                  15
   Val Arg Cys Leu Glu Ala Pro Gly Val Glu Val Ser Ile Val Lys Lys
               20                  25                  30
   Val Glu Gln Leu Glu Asp Leu Asp Gly Leu Val Phe Pro Gly Gly Glu
           35                  40                  45
   Ser Thr Thr Met Arg Arg Leu Ile Asp Lys Tyr Gly Phe Phe Glu Pro
       50                  55                  60
   Leu Lys Ala Phe Ala Ala Gln Gly Lys Pro Val Phe Gly Thr Cys Ala
   65                  70                  75                  80
   Gly Leu Ile Leu Met Ala Thr Arg Ile Asp Gly Glu Asp His Gly His
                   85                  90                  95
   Leu Glu Leu Met Asp Met Thr Val Gln Arg Asn Ala Phe Gly Arg Gln
               100                 105                 110
   Arg Glu Ser Phe Glu Thr Asp Leu Ile Val Glu Gly Val Gly Asp Asp
           115                 120                 125
   Val Arg Ala Val Phe Ile Arg Ala Pro Leu Ile Gln Glu Val Gly Gln
       130                 135                 140
   Asn Val Asp Val Leu Ser Lys Phe Gly Asp Glu Ile Val Val Ala Arg
   145                 150                 155                 160
   Gln Gly His Leu Leu Gly Cys Ser Phe His Pro Glu Leu Thr Asp Asp
                   165                 170                 175
   Arg Arg Phe His Gln Tyr Phe Val Gln Met Val Lys Glu Ala Lys Thr
               180                 185                 190
   Ile Ala Gln Ser
           195
<210> 30
   <211> 567
   <212> DNA
   <213> Thermotoga maritima
<400> 30
   atg aag ata ggc gtt ctg ggt gtt cag gga gac gtc aga gaa cac gtg      48
   Met Lys Ile Gly Val Leu Gly Val Gln Gly Asp Val Arg Glu His Val
   1               5                   10                  15
   gaa gct ctc cat aaa ctc gga gtt gag acc ctg ata gtg aaa ctt cca      96
   Glu Ala Leu His Lys Leu Gly Val Glu Thr Leu Ile Val Lys Leu Pro
               20                  25                  30
   gag cag ctg gac atg gtg gat ggc ctc att ctg ccc ggt gga gaa tcg      144
   Glu Gln Leu Asp Met Val Asp Gly Leu Ile Leu Pro Gly Gly Glu Ser
           35                  40                  45
   acc acc atg ata aga att ctc aaa gag atg gat atg gat gaa aag ttg        192
   Thr Thr Met Ile Arg Ile Leu Lys Glu Met Asp Met Asp Glu Lys Leu
       50                  55                  60
   gtg gaa aga ata aac aac ggc ctt ccc gtc ttt gca acg tgt gcc ggt        240
   Val Glu Arg Ile Asn Asn Gly Leu Pro Val Phe Ala Thr Cys Ala Gly
   65                  70                  75                  80
   gtg atc ctt ctc gca aag cgc atc aaa aac tac tct cag gaa aaa cta        288
   Val Ile Leu Leu Ala Lys Arg Ile Lys Asn Tyr Ser Gln Glu Lys Leu
                   85                  90                  95
   gga gtt ttg gac ata acc gtt gaa aga aat gcc tac gga aga cag gtc        336
   Gly Val Leu Asp Ile Thr Val Glu Arg Asn Ala Tyr Gly Arg Gln Val
               100                 105                 110
   gaa agt ttt gag acg ttt gta gag ata ccc gct gta gga aaa gat ccg        384
   Glu Ser Phe Glu Thr Phe Val Glu Ile Pro Ala Val Gly Lys Asp Pro
           115                 120                 125
   ttc aga gcc att ttc ata agg gct ccg agg atc gtt gaa aca gga aag        432
   Phe Arg Ala Ile Phe Ile Arg Ala Pro Arg Ile Val Glu Thr Gly Lys
       130                 135                 140
   aat gtg gaa att ctg gca act tac gac tat gat cct gtt cta gtg aaa        480
   Asn Val Glu Ile Leu Ala Thr Tyr Asp Tyr Asp Pro Val Leu Val Lys
   145                 150                 155                 160
   gaa gga aat ata ctc gcg tgc acg ttt cac cca gaa ctc acc gac gat        528
   Glu Gly Asn Ile Leu Ala Cys Thr Phe His Pro Glu Leu Thr Asp Asp
                   165                 170                 175
   ttg aga ctg cac aga tac ttc ctg gag atg gtg aaa tga                    567
   Leu Arg Leu His Arg Tyr Phe Leu Glu Met Val Lys
               180                 185
<210> 31
   <211> 188
   <212> PRT
   <213> Thermotoga maritima
<400> 31
   Met Lys Ile Gly Val Leu Gly Val Gln Gly Asp Val Arg Glu His Val
   1               5                   10                  15
   Glu Ala Leu His Lys Leu Gly Val Glu Thr Leu Ile Val Lys Leu Pro
               20                  25                  30
   Glu Gln Leu Asp Met Val Asp Gly Leu Ile Leu Pro Gly Gly Glu Ser
           35                  40                  45
   Thr Thr Met Ile Arg Ile Leu Lys Glu Met Asp Met Asp Glu Lys Leu
       50                  55                  60
   Val Glu Arg Ile Asn Asn Gly Leu Pro Val Phe Ala Thr Cys Ala Gly
   65                  70                  75                  80
   Val Ile Leu Leu Ala Lys Arg Ile Lys Asn Tyr Ser Gln Glu Lys Leu
                   85                  90                  95
   Gly Val Leu Asp Ile Thr Val Glu Arg Asn Ala Tyr Gly Arg Gln Val
               100                 105                 110
   Glu Ser Phe Glu Thr Phe Val Glu Ile Pro Ala Val Gly Lys Asp Pro
           115                 120                 125
   Phe Arg Ala Ile Phe Ile Arg Ala Pro Arg Ile Val Glu Thr Gly Lys
       130                 135                 140
   Asn Val Glu Ile Leu Ala Thr Tyr Asp Tyr Asp Pro Val Leu Val Lys
   145                 150                 155                 160
   Glu Gly Asn Ile Leu Ala Cys Thr Phe His Pro Glu Leu Thr Asp Asp
                   165                 170                 175
   Leu Arg Leu His Arg Tyr Phe Leu Glu Met Val Lys
               180                 185
<210> 32
   <211> 603
   <212> DNA
   <213> Sulfolobus solfataricus
<400> 32
   atg aaa ata ggt ata ata gct tat caa ggg agt ttc gaa gaa cat ttt        48
   Met Lys Ile Gly Ile Ile Ala Tyr Gln Gly Ser Phe Glu Glu His Phe
   1               5                   10                  15
   ctt cag tta aag agg gct ttt gat aaa cta tca tta aat ggc gag att        96
   Leu Gln Leu Lys Arg Ala Phe Asp Lys Leu Ser Leu Asn Gly Glu Ile
               20                  25                  30
   att tca ata aag att cct aaa gat cta aag ggt gtg gac gga gta ata       144
   Ile Ser Ile Lys Ile Pro Lys Asp Leu Lys Gly Val Asp Gly Val Ile
           35                  40                  45
   ata ccg gga ggg gaa agc act aca ata gga tta gta gct aaa agg cta       192
   Ile Pro Gly Gly Glu Ser Thr Thr Ile Gly Leu Val Ala Lys Arg Leu
       50                  55                  60
   ggg cta tta gat gaa ctg aaa gag aaa att aca tct ggt tta cca gtc       240
   Gly Leu Leu Asp Glu Leu Lys Glu Lys Ile Thr Ser Gly Leu Pro Val
   65                  70                  75                  80
   tta gga acg tgt gct ggt gct ata atg tta gca aag gaa gta agt gat       288
   Leu Gly Thr Cys Ala Gly Ala Ile Met Leu Ala Lys Glu Val Ser Asp
                   85                  90                  95
   gcc aaa gta ggt aaa acc tca caa cca tta ata gga aca atg aat att       336
   Ala Lys Val Gly Lys Thr Ser Gln Pro Leu Ile Gly Thr Met Asn Ile
               100                 105                 110
   agt gtg att aga aat tat tat gga aga caa aag gaa agt ttt gaa gct       384
   Ser Val Ile Arg Asn Tyr Tyr Gly Arg Gln Lys Glu Ser Phe Glu Ala
           115                 120                 125
   ata gtt gat cta tct aaa ata ggt aag gat aaa gct cat gtg gta ttc       432
   Ile Val Asp Leu Ser Lys Ile Gly Lys Asp Lys Ala His Val Val Phe
       130                 135                 140
   att aga gct cca gca ata gcg aaa gta tgg gga aag gct caa agc tta       480
   Ile Arg Ala Pro Ala Ile Ala Lys Val Trp Gly Lys Ala Gln Ser Leu
   145                 150                 155                 160
   gct gag tta aat ggt gta aca gtt ttc gct gaa gaa aat aat atg ctt       528
   Ala Glu Leu Asn Gly Val Thr Val Phe Ala Glu Glu Asn Asn Met Leu
                   165                 170                 175
   gct act aca ttt cac ccc gaa tta tct gat aca act tcg ata cac gaa       576
   Ala Thr Thr Phe His Pro Glu Leu Ser Asp Thr Thr Ser Ile His Glu
               180                 185                 190
   tat ttc cta cat cta gtt aaa ggg taa                                   603
   Tyr Phe Leu His Leu Val Lys Gly
           195                 200
<210> 33
   <211> 200
   <212> PRT
   <213> Sulfolobus solfataricus
<400> 33
   Met Lys Ile Gly Ile Ile Ala Tyr Gln Gly Ser Phe Glu Glu His Phe
   1               5                   10                  15
   Leu Gln Leu Lys Arg Ala Phe Asp Lys Leu Ser Leu Asn Gly Glu Ile
               20                  25                  30
   Ile Ser Ile Lys Ile Pro Lys Asp Leu Lys Gly Val Asp Gly Val Ile
           35                  40                  45
   Ile Pro Gly Gly Glu Ser Thr Thr Ile Gly Leu Val Ala Lys Arg Leu
       50                  55                  60
   Gly Leu Leu Asp Glu Leu Lys Glu Lys Ile Thr Ser Gly Leu Pro Val
   65                  70                  75                  80
   Leu Gly Thr Cys Ala Gly Ala Ile Met Leu Ala Lys Glu Val Ser Asp
                   85                  90                  95
   Ala Lys Val Gly Lys Thr Ser Gln Pro Leu Ile Gly Thr Met Asn Ile
               100                 105                 110
   Ser Val Ile Arg Asn Tyr Tyr Gly Arg Gln Lys Glu Ser Phe Glu Ala
           115                 120                 125
   Ile Val Asp Leu Ser Lys Ile Gly Lys Asp Lys Ala His Val Val Phe
       130                 135                 140
   Ile Arg Ala Pro Ala Ile Ala Lys Val Trp Gly Lys Ala Gln Ser Leu
   145                 150                 155                 160
   Ala Glu Leu Asn Gly Val Thr Val Phe Ala Glu Glu Asn Asn Met Leu
                   165                 170                 175
   Ala Thr Thr Phe His Pro Glu Leu Ser Asp Thr Thr Ser Ile His Glu
                   180                 185                 190
   Tyr Phe Leu His Leu Val Lys Gly
           195                 200
<210> 34
   <211> 669
   <212> DNA
   <213> Saccharomyces cerevisiae
<400> 34
   atg acc gtc gtt atc gga gtc ttg gca tta cag ggt gcg ttc att gaa      48
   Met Thr Val Val Ile Gly Val Leu Ala Leu Gln Gly Ala Phe Ile Glu
   1               5                   10                  15
   cat gtg cga cac gta gaa aaa tgc atc gtc gaa aac agg gat ttc tat      96
   His Val Arg His Val Glu Lys Cys Ile Val Glu Asn Arg Asp Phe Tyr
               20                  25                  30
   gaa aaa aaa cta tct gtg atg aca gtg aag gat aaa aat caa cta gct      144
   Glu Lys Lys Leu Ser Val Met Thr Val Lys Asp Lys Asn Gln Leu Ala
           35                  40                  45
   caa tgt gat gca ttg atc ata cct ggg gga gag tcg act gca atg tcc      192
   Gln Cys Asp Ala Leu Ile Ile Pro Gly Gly Glu Ser Thr Ala Met Ser
       50                  55                  60
   ctt att gca gaa aga aca gga ttt tac gac gat ctc tac gca ttc gta      240
   Leu Ile Ala Glu Arg Thr Gly Phe Tyr Asp Asp Leu Tyr Ala Phe Val
   65                  70                  75                  80
   cac aac cca agc aag gta acc tgg ggt act tgt gca ggt ttg att tat      288
   His Asn Pro Ser Lys Val Thr Trp Gly Thr Cys Ala Gly Leu Ile Tyr
                   85                  90                  95
   att tca caa caa tta tct aac gaa gca aaa ctg gtc aag acg ctg aat      336
   Ile Ser Gln Gln Leu Ser Asn Glu Ala Lys Leu Val Lys Thr Leu Asn
               100                 105                 110
   tta cta aag gtt aaa gta aaa aga aat gca ttt ggg aga caa gct cag      384
   Leu Leu Lys Val Lys Val Lys Arg Asn Ala Phe Gly Arg Gln Ala Gln
           115                 120                 125
   tct tct acc cgg att tgc gac ttt tca aac ttt att cct cac tgc aat      432
   Ser Ser Thr Arg Ile Cys Asp Phe Ser Asn Phe Ile Pro His Cys Asn
       130                 135                 140
   gat ttt cct gct act ttt ata aga gcc cca gta ata gaa gag gtg ctg      480
   Asp Phe Pro Ala Thr Phe Ile Arg Ala Pro Val Ile Glu Glu Val Leu
   145                 150                 155                 160
   gat cct gaa cat gtg cag gtc ctg tac aaa tta gat ggg aag gat aat      528
   Asp Pro Glu His Val Gln Val Leu Tyr Lys Leu Asp Gly Lys Asp Asn
                   165                 170                 175
   ggt ggt caa gaa cta att gtt gcc gct aag caa aaa aac aat att ctt      576
   Gly Gly Gln Glu Leu Ile Val Ala Ala Lys Gln Lys Asn Asn Ile Leu
               180                 185                 190
   gcg aca tca ttt cat ccg gaa ttg gca gaa aac gat ata cgg ttt cac      624
   Ala Thr Ser Phe His Pro Glu Leu Ala Glu Asn Asp Ile Arg Phe His
           195                 200                 205
   gac tgg ttc atc aga gaa ttt gtt ctt aaa aac tac agt aaa taa          669
   Asp Trp Phe Ile Arg Glu Phe Val Leu Lys Asn Tyr Ser Lys
       210                 215                 220
<210> 35
   <211> 222
   <212> PRT
   <213> Saccharomyces cerevisiae
<400> 35
   Met Thr Val Val Ile Gly Val Leu Ala Leu Gln Gly Ala Phe Ile Glu
   1               5                   10                  15
   His Val Arg His Val Glu Lys Cys Ile Val Glu Asn Arg Asp Phe Tyr
               20                  25                  30
   Glu Lys Lys Leu Ser Val Met Thr Val Lys Asp Lys Asn Gln Leu Ala
           35                  40                  45
   Gln Cys Asp Ala Leu Ile Ile Pro Gly Gly Glu Ser Thr Ala Met Ser
       50                  55                  60
   Leu Ile Ala Glu Arg Thr Gly Phe Tyr Asp Asp Leu Tyr Ala Phe Val
   65                  70                  75                  80
   His Asn Pro Ser Lys Val Thr Trp Gly Thr Cys Ala Gly Leu Ile Tyr
                   85                  90                  95
   Ile Ser Gln Gln Leu Ser Asn Glu Ala Lys Leu Val Lys Thr Leu Asn
               100                 105                 110
   Leu Leu Lys Val Lys Val Lys Arg Asn Ala Phe Gly Arg Gln Ala Gln
           115                 120                 125
   Ser Ser Thr Arg Ile Cys Asp Phe Ser Asn Phe Ile Pro His Cys Asn
       130                 135                 140
   Asp Phe Pro Ala Thr Phe Ile Arg Ala Pro Val Ile Glu Glu Val Leu
   145                 150                 155                 160
   Asp Pro Glu His Val Gln Val Leu Tyr Lys Leu Asp Gly Lys Asp Asn
                   165                 170                 175
   Gly Gly Gln Glu Leu Ile Val Ala Ala Lys Gln Lys Asn Asn Ile Leu
               180                 185                 190
   Ala Thr Ser Phe His Pro Glu Leu Ala Glu Asn Asp Ile Arg Phe His
           195                 200                 205
   Asp Trp Phe Ile Arg Glu Phe Val Leu Lys Asn Tyr Ser Lys
       210                 215                 220
<210> 36
   <211> 591
   <212> DNA
   <213> Bacillus subtilis
<400> 36
   atg tta aca ata ggt gta cta gga ctt caa gga gca gtt aga gag cac        48
   Met Leu Thr Ile Gly Val Leu Gly Leu Gln Gly Ala Val Arg Glu His
   1               5                   10                  15
   atc cat gcg att gaa gca tgc ggc gcg gct ggt ctt gtc gta aaa cgt        96
   Ile His Ala Ile Glu Ala Cys Gly Ala Ala Gly Leu Val Val Lys Arg
               20                  25                  30
   ccg gag cag ctg aac gaa gtt gac ggg ttg att ttg ccg ggc ggt gag       144
   Pro Glu Gln Leu Asn Glu Val Asp Gly Leu Ile Leu Pro Gly Gly Glu
           35                  40                  45
   agc acg acg atg cgc cgt ttg atc gat acg tat caa ttc atg gag ccg       192
   Ser Thr Thr Met Arg Arg Leu Ile Asp Thr Tyr Gln Phe Met Glu Pro
       50                  55                  60
   ctt cgt gaa ttc gct gct cag ggc aaa ccg atg ttt gga aca tgt gcc       240
   Leu Arg Glu Phe Ala Ala Gln Gly Lys Pro Met Phe Gly Thr Cys Ala
   65                  70                  75                  80
   gga tta att ata tta gca aaa gaa att gcc ggt tca gat aat cct cat       288
   Gly Leu Ile Ile Leu Ala Lys Glu Ile Ala Gly Ser Asp Asn Pro His
                   85                  90                  95
   tta ggt ctt ctg aat gtg gtt gta gaa cgt aat tca ttt ggc cgg cag       336
   Leu Gly Leu Leu Asn Val Val Val Glu Arg Asn Ser Phe Gly Arg Gln
               100                 105                 110
   gtt gac agc ttt gaa gct gat tta aca att aaa ggc ttg gac gag cct       384
   Val Asp Ser Phe Glu Ala Asp Leu Thr Ile Lys Gly Leu Asp Glu Pro
           115                 120                 125
   ttt act ggg gta ttc atc cgt gct ccg cat att tta gaa gct ggt gaa       432
   Phe Thr Gly Val Phe Ile Arg Ala Pro His Ile Leu Glu Ala Gly Glu
       130                 135                 140
   aat gtt gaa gtt cta tcg gag cat aat ggt cgt att gta gcc gcg aaa       480
   Asn Val Glu Val Leu Ser Glu His Asn Gly Arg Ile Val Ala Ala Lys
   145                 150                 155                 160
   cag ggg caa ttc ctt ggc tgc tca ttc cat ccg gag ctg aca gaa gat       528
   Gln Gly Gln Phe Leu Gly Cys Ser Phe His Pro Glu Leu Thr Glu Asp
                   165                 170                 175
   cac cga gtg acg cag ctg ttt gtt gaa atg gtt gag gaa tat aag caa       576
   His Arg Val Thr Gln Leu Phe Val Glu Met Val Glu Glu Tyr Lys Gln
               180                 185                 190
   aag gca ctt gta taa                                                   591
   Lys Ala Leu Val
           195
<210> 37
   <211> 196
   <212> PRT
   <213> Bacillus subtilis
<400> 37
   Met Leu Thr Ile Gly Val Leu Gly Leu Gln Gly Ala Val Arg Glu His
   1               5                   10                  15
   Ile His Ala Ile Glu Ala Cys Gly Ala Ala Gly Leu Val Val Lys Arg
               20                  25                  30
   Pro Glu Gln Leu Asn Glu Val Asp Gly Leu Ile Leu Pro Gly Gly Glu
           35                  40                  45
   Ser Thr Thr Met Arg Arg Leu Ile Asp Thr Tyr Gln Phe Met Glu Pro
       50                  55                  60
   Leu Arg Glu Phe Ala Ala Gln Gly Lys Pro Met Phe Gly Thr Cys Ala
   65                  70                  75                  80
   Gly Leu Ile Ile Leu Ala Lys Glu Ile Ala Gly Ser Asp Asn Pro His
                   85                  90                  95
   Leu Gly Leu Leu Asn Val Val Val Glu Arg Asn Ser Phe Gly Arg Gln
               100                 105                 110
   Val Asp Ser Phe Glu Ala Asp Leu Thr Ile Lys Gly Leu Asp Glu Pro
           115                 120                 125
   Phe Thr Gly Val Phe Ile Arg Ala Pro His Ile Leu Glu Ala Gly Glu
       130                 135                 140
   Asn Val Glu Val Leu Ser Glu His Asn Gly Arg Ile Val Ala Ala Lys
   145                 150                 155                 160
   Gln Gly Gln Phe Leu Gly Cys Ser Phe His Pro Glu Leu Thr Glu Asp
                   165                 170                 175
   His Arg Val Thr Gln Leu Phe Val Glu Met Val Glu Glu Tyr Lys Gln
               180                 185                 190
   Lys Ala Leu Val
           195
<210> 38
   <211> 705
   <212> DNA
   <213> Schizosaccharomyces pombe
<400> 38
   atg tct tct gca tcc atg ttc ggg agt ctt aaa acc aat gct gtg gac      48
   Met Ser Ser Ala Ser Met Phe Gly Ser Leu Lys Thr Asn Ala Val Asp
   1               5                   10                  15
   gaa tcc cag ttg aag gct aga att gga gtt tta gct ctc caa gga gca      96
   Glu Ser Gln Leu Lys Ala Arg Ile Gly Val Leu Ala Leu Gln Gly Ala
               20                  25                  30
   ttt att gaa cac att aat ata atg aat tcc att gat gga gta att tct      144
   Phe Ile Glu His Ile Asn Ile Met Asn Ser Ile Asp Gly Val Ile Ser
           35                  40                  45
   ttt cct gtt aaa act gct aag gat tgc gaa aat att gat ggc tta att      192
   Phe Pro Val Lys Thr Ala Lys Asp Cys Glu Asn Ile Asp Gly Leu Ile
       50                  55                  60
   atc cca gga ggt gag tct act acc att ggc aaa tta atc aac att gat      240
   Ile Pro Gly Gly Glu Ser Thr Thr Ile Gly Lys Leu Ile Asn Ile Asp
   65                  70                  75                  80
   gag aag ctt cgt gat cgt ttg gag cac ttg gtt gat caa gga ctt cct      288
   Glu Lys Leu Arg Asp Arg Leu Glu His Leu Val Asp Gln Gly Leu Pro
                   85                  90                  95
   att tgg gga acg tgt gct ggt atg att ctt ctg tcg aaa aag tct cga      336
   Ile Trp Gly Thr Cys Ala Gly Met Ile Leu Leu Ser Lys Lys Ser Arg
               100                 105                 110
   ggt gga aag ttc cca gat cct tat ttg ttg cgc gcc atg gat att gaa      384
   Gly Gly Lys Phe Pro Asp Pro Tyr Leu Leu Arg Ala Met Asp Ile Glu
           115                 120                 125
   gtg act cgt aat tat ttt gga cct caa act atg tct ttt aca act gat      432
   Val Thr Arg Asn Tyr Phe Gly Pro Gln Thr Met Ser Phe Thr Thr Asp
       130                 135                 140
   att aca gtt aca gag tca atg caa ttt gaa gcc act gaa cct tta cat      480
   Ile Thr Val Thr Glu Ser Met Gln Phe Glu Ala Thr Glu Pro Leu His
   145                 150                 155                 160
   tcc ttt tcg gcc act ttt att cgt gct cca gtc gct tcg aca atc ctg      528
   Ser Phe Ser Ala Thr Phe Ile Arg Ala Pro Val Ala Ser Thr Ile Leu
                   165                 170                 175
   tct gat gat att aat gtt tta gct act att gtt cat gaa ggc aac aaa      576
   Ser Asp Asp Ile Asn Val Leu Ala Thr Ile Val His Glu Gly Asn Lys
               180                 185                 190
   gag att gtt gcg gtt gag caa ggt ccc ttt tta ggt aca tcg ttt cac      624
   Glu Ile Val Ala Val Glu Gln Gly Pro Phe Leu Gly Thr Ser Phe His
           195                 200                 205
   ccc gag ctg acc gcc gat aat aga tgg cat gaa tgg tgg gta aaa gag      672
   Pro Glu Leu Thr Ala Asp Asn Arg Trp His Glu Trp Trp Val Lys Glu
       210                 215                 220
   cgt gtt tta cct tta aag gag aaa aag gat tag                          705
   Arg Val Leu Pro Leu Lys Glu Lys Lys Asp
   225                 230
<210> 39
   <211> 234
   <212> PRT
   <213> Schizosaccharomyces pombe
<400> 39
   Met Ser Ser Ala Ser Met Phe Gly Ser Leu Lys Thr Asn Ala Val Asp
   1               5                   10                  15
   Glu Ser Gln Leu Lys Ala Arg Ile Gly Val Leu Ala Leu Gln Gly Ala
               20                  25                  30
   Phe Ile Glu His Ile Asn Ile Met Asn Ser Ile Asp Gly Val Ile Ser
           35                  40                  45
   Phe Pro Val Lys Thr Ala Lys Asp Cys Glu Asn Ile Asp Gly Leu Ile
       50                  55                  60
   Ile Pro Gly Gly Glu Ser Thr Thr Ile Gly Lys Leu Ile Asn Ile Asp
   65                  70                  75                      80
   Glu Lys Leu Arg Asp Arg Leu Glu His Leu Val Asp Gln Gly Leu Pro
                   85                  90                  95
   Ile Trp Gly Thr Cys Ala Gly Met Ile Leu Leu Ser Lys Lys Ser Arg
               100                 105                 110
   Gly Gly Lys Phe Pro Asp Pro Tyr Leu Leu Arg Ala Met Asp Ile Glu
           115                 120                 125
   Val Thr Arg Asn Tyr Phe Gly Pro Gln Thr Met Ser Phe Thr Thr Asp
       130                 135                 140
   Ile Thr Val Thr Glu Ser Met Gln Phe Glu Ala Thr Glu Pro Leu His
   145                 150                 155                     160
   Ser Phe Ser Ala Thr Phe Ile Arg Ala Pro Val Ala Ser Thr Ile Leu
                   165                 170                 175
   Ser Asp Asp Ile Asn Val Leu Ala Thr Ile Val His Glu Gly Asn Lys
               180                 185                 190
   Glu Ile Val Ala Val Glu Gln Gly Pro Phe Leu Gly Thr Ser Phe His
           195                 200                 205
   Pro Glu Leu Thr Ala Asp Asn Arg Trp His Glu Trp Trp Val Lys Glu
       210                 215                 220
   Arg Val Leu Pro Leu Lys Glu Lys Lys Asp
   225                 230
<210> 40
   <211> 570
   <212> DNA
   <213> Haemophilus ducreyi
<400> 40
   atg gct gac tat tct aga tac acg gtt ggt gta tta gcg tta caa ggt      48
   Met Ala Asp Tyr Ser Arg Tyr Thr Val Gly Val Leu Ala Leu Gln Gly
   1               5                   10                  15
   gca gtc aca gaa cat atc tca caa att gag tcg tta ggc gct aaa gca      96
   Ala Val Thr Glu His Ile Ser Gln Ile Glu Ser Leu Gly Ala Lys Ala
               20                  25                  30
   ata gca gta aag caa gtc gaa caa tta aat caa ctt gat gca tta gtt     144
   Ile Ala Val Lys Gln Val Glu Gln Leu Asn Gln Leu Asp Ala Leu Val
           35                  40                  45
   tta ccc gga ggt gaa agt acg gca atg cgc cgt tta atg gaa gca aat     192
   Leu Pro Gly Gly Glu Ser Thr Ala Met Arg Arg Leu Met Glu Ala Asn
    50                  55                  60
   ggt tta ttt gag cgc ttg aaa acc ttt gat aaa cct ata tta ggc act        240
   Gly Leu Phe Glu Arg Leu Lys Thr Phe Asp Lys Pro Ile Leu Gly Thr
   65                  70                  75                  80
   tgt gca gga tta att tta ctt gct gat gaa att att ggc ggt gag caa        288
   Cys Ala Gly Leu Ile Leu Leu Ala Asp Glu Ile Ile Gly Gly Glu Gln
                   85                  90                  95
   gtt cat tta gct aaa atg gca att aaa gta cag cgt aat gca ttt ggt        336
   Val His Leu Ala Lys Met Ala Ile Lys Val Gln Arg Asn Ala Phe Gly
               100                 105                 110
   cgt caa ata gat agt ttt caa acg cca ttg act gtt agt gga tta gat        384
   Arg Gln Ile Asp Ser Phe Gln Thr Pro Leu Thr Val Ser Gly Leu Asp
           115                 120                 125
   aag cct ttt ccg gcg gtg ttt att cgt gca cct tat att act gaa gtg        432
   Lys Pro Phe Pro Ala Val Phe Ile Arg Ala Pro Tyr Ile Thr Glu Val
       130                 135                 140
   ggt gag aat gtt gaa gtg tta gca gaa tgg caa ggt aat gtt gta tta        480
   Gly Glu Asn Val Glu Val Leu Ala Glu Trp Gln Gly Asn Val Val Leu
   145                 150                 155                 160
   gct aaa caa ggc cat ttt ttt gct tgt gca ttt cat cca gaa tta act        528
   Ala Lys Gln Gly His Phe Phe Ala Cys Ala Phe His Pro Glu Leu Thr
                   165                 170                 175
   aat gat aat cgc att atg gca tta tta tta gct cag cta taa                570
   Asn Asp Asn Arg Ile Met Ala Leu Leu Leu Ala Gln Leu
               180                 185
<210> 41
   <211> 189
   <212> PRT
   <213> Haemophilus ducreyi
<400> 41
   Met Ala Asp Tyr Ser Arg Tyr Thr Val Gly Val Leu Ala Leu Gln Gly
   1               5                   10                  15
   Ala Val Thr Glu His Ile Ser Gln Ile Glu Ser Leu Gly Ala Lys Ala
               20                  25                  30
   Ile Ala Val Lys Gln Val Glu Gln Leu Asn Gln Leu Asp Ala Leu Val
           35                  40                  45
   Leu Pro Gly Gly Glu Ser Thr Ala Met Arg Arg Leu Met Glu Ala Asn
       50                  55                  60
   Gly Leu Phe Glu Arg Leu Lys Thr Phe Asp Lys Pro Ile Leu Gly Thr
   65                  70                  75                  80
   Cys Ala Gly Leu Ile Leu Leu Ala Asp Glu Ile Ile Gly Gly Glu Gln
                   85                  90                  95
   Val His Leu Ala Lys Met Ala Ile Lys Val Gln Arg Asn Ala Phe Gly
               100                 105                 110
   Arg Gln Ile Asp Ser Phe Gln Thr Pro Leu Thr Val Ser Gly Leu Asp
           115                 120                 125
   Lys Pro Phe Pro Ala Val Phe Ile Arg Ala Pro Tyr Ile Thr Glu Val
       130                 135                 140
   Gly Glu Asn Val Glu Val Leu Ala Glu Trp Gln Gly Asn Val Val Leu
   145                 150                 155                 160
   Ala Lys Gln Gly His Phe Phe Ala Cys Ala Phe His Pro Glu Leu Thr
                   165                 170                 175
   Asn Asp Asn Arg Ile Met Ala Leu Leu Leu Ala Gln Leu
               180                 185
<210> 42
   <211> 606
   <212> DNA
   <213> Streptomyces avermitilis
<400> 42
   atg aac acc ccc gtg ata ggc gtc ctg gct ctg cag ggc gac gta cgg      48
   Met Asn Thr Pro Val Ile Gly Val Leu Ala Leu Gln Gly Asp Val Arg
   1               5                   10                  15
   gag cac ctg atc gcc ctg gcc gcg gcc gac gcc gtg gcc agg gag gtg      96
   Glu His Leu Ile Ala Leu Ala Ala Ala Asp Ala Val Ala Arg Glu Val
               20                  25                  30
   agg cgc ccc gag gaa ctc gcc gag gtc gac ggc ctc gtc ata ccc ggc      144
   Arg Arg Pro Glu Glu Leu Ala Glu Val Asp Gly Leu Val Ile Pro Gly
           35                  40                  45
   ggc gag tcc acc acc atc tcc aag ctg gcc cat ctc ttc ggc atg atg      192
   Gly Glu Ser Thr Thr Ile Ser Lys Leu Ala His Leu Phe Gly Met Met
       50                  55                  60
   gaa ccc ctc cgc gcg cgc gtg cgc ggc ggc atg ccc gtc tac ggc acc      240
   Glu Pro Leu Arg Ala Arg Val Arg Gly Gly Met Pro Val Tyr Gly Thr
   65                  70                  75                  80
   tgc gcc ggc atg atc atg ctc gcc gac aag atc ctc gac ccg cgc tcg      288
   Cys Ala Gly Met Ile Met Leu Ala Asp Lys Ile Leu Asp Pro Arg Ser
                   85                  90                  95
   ggt cag gag acc atc ggc ggc atc gac atg atc gtg cgc cgc aac gcc      336
   Gly Gln Glu Thr Ile Gly Gly Ile Asp Met Ile Val Arg Arg Asn Ala
               100                 105                 110
   ttc gga cgt cag aac gag tcc ttc gag gcg acg gtc gac gtc aag ggc      384
   Phe Gly Arg Gln Asn Glu Ser Phe Glu Ala Thr Val Asp Val Lys Gly
           115                 120                 125
   gtc ggg ggc gat cct gtc gag ggc gtc ttc atc cgc gcc ccc tgg gtc      432
   Val Gly Gly Asp Pro Val Glu Gly Val Phe Ile Arg Ala Pro Trp Val
       130                 135                 140
   gag tcc gtg ggt gcc gag gcc gag gtg ctc gcc gag cac ggc ggc cac      480
   Glu Ser Val Gly Ala Glu Ala Glu Val Leu Ala Glu His Gly Gly His
   145                 150                 155                 160
   atc gtc gcc gta cgc cag ggc aac gcg ctc gcc acg tcg ttc cac ccg      528
   Ile Val Ala Val Arg Gln Gly Asn Ala Leu Ala Thr Ser Phe His Pro
                   165                 170                 175
   gaa ctg acc ggc gac cac cgc gtg cac ggc ctc ttc gtc gac atg gtg      576
   Glu Leu Thr Gly Asp His Arg Val His Gly Leu Phe Val Asp Met Val
               180                 185                 190
   cgc gcg aac cgg aca ccg gag tcc ttg tag                              606
   Arg Ala Asn Arg Thr Pro Glu Ser Leu
           195                 200
<210> 43
   <211> 201
   <212> PRT
   <213> Streptomyces avermitilis
<400> 43
   Met Asn Thr Pro Val Ile Gly Val Leu Ala Leu Gln Gly Asp Val Arg
   1               5                   10                  15
   Glu His Leu Ile Ala Leu Ala Ala Ala Asp Ala Val Ala Arg Glu Val
               20                  25                  30
   Arg Arg Pro Glu Glu Leu Ala Glu Val Asp Gly Leu Val Ile Pro Gly
           35                  40                  45
   Gly Glu Ser Thr Thr Ile Ser Lys Leu Ala His Leu Phe Gly Met Met
       50                  55                  60
   Glu Pro Leu Arg Ala Arg Val Arg Gly Gly Met Pro Val Tyr Gly Thr
   65                  70                  75                  80
   Cys Ala Gly Met Ile Met Leu Ala Asp Lys Ile Leu Asp Pro Arg Ser
                   85                  90                  95
   Gly Gln Glu Thr Ile Gly Gly Ile Asp Met Ile Val Arg Arg Asn Ala
               100                 105                 110
   Phe Gly Arg Gln Asn Glu Ser Phe Glu Ala Thr Val Asp Val Lys Gly
           115                 120                 125
   Val Gly Gly Asp Pro Val Glu Gly Val Phe Ile Arg Ala Pro Trp Val
       130                 135                 140
   Glu Ser Val Gly Ala Glu Ala Glu Val Leu Ala Glu His Gly Gly His
   145                 150                 155                 160
   Ile Val Ala Val Arg Gln Gly Asn Ala Leu Ala Thr Ser Phe His Pro
                   165                 170                 175
   Glu Leu Thr Gly Asp His Arg Val His Gly Leu Phe Val Asp Met Val
               180                 185                 190
   Arg Ala Asn Arg Thr Pro Glu Ser Leu
           195                 200
<210> 44
   <211> 567
   <212> DNA
   <213> Tropheryma whipplei (strain TW08/27) (Whipple's bacillus)
<400> 44
   atg acc gtt gga gtt ctc tcc ctc cag gga agt ttt tat gag cac cta 48
   Met Thr Val Gly Val Leu Ser Leu Gln Gly Ser Phe Tyr Glu His Leu
   1               5                   10                  15
   tct att ttg agc agg cta aac act gac cac att caa gta aaa act tct 96
   Ser Ile Leu Ser Arg Leu Asn Thr Asp His Ile Gln Val Lys Thr Ser
               20                  25                  30
   gaa gat ctt tcc cgg gtc acg cga ctt ata att ccc ggt ggg gag tct 144
   Glu Asp Leu Ser Arg Val Thr Arg Leu Ile Ile Pro Gly Gly Glu Ser
           35                  40                  45
   act gct atg ctc gct ctg acc cag aag agc ggc ctg ttt gat ttg gtg 192
   Thr Ala Met Leu Ala Leu Thr Gln Lys Ser Gly Leu Phe Asp Leu Val
       50                  55                  60
   aga gac cgc atc atg tct ggc atg cct gtg tac ggc acg tgt gcg ggc 240
   Arg Asp Arg Ile Met Ser Gly Met Pro Val Tyr Gly Thr Cys Ala Gly
   65                  70                  75                  80
   atg att atg cta tcg acg ttt gta gaa gat ttt cct aac caa aag act 288
   Met Ile Met Leu Ser Thr Phe Val Glu Asp Phe Pro Asn Gln Lys Thr
                   85                  90                  95
   ttg tct tgt ctt gat att gcc gtt cgg cgc aat gcc ttt gga agg cag 336
   Leu Ser Cys Leu Asp Ile Ala Val Arg Arg Asn Ala Phe Gly Arg Gln
               100                 105                 110
   ata aac agt ttt gag agc gaa gtt tcc ttt cta aac tca aaa att act 384
   Ile Asn Ser Phe Glu Ser Glu Val Ser Phe Leu Asn Ser Lys Ile Thr
           115                 120                 125
   gtg cct ttt att cgt gcg cca aag att act cag att ggt gag ggc gtt 432
   Val Pro Phe Ile Arg Ala Pro Lys Ile Thr Gln Ile Gly Glu Gly Val
       130                 135                 140
   gat gtt ttg tct cgt ctc gag tcg ggc gat atc gtt gct gta aga cag 480
   Asp Val Leu Ser Arg Leu Glu Ser Gly Asp Ile Val Ala Val Arg Gln
   145                 150                 155                 160
   gga aat gtc atg gca aca gca ttt cat ccc gag ctt acc ggg ggt gca 528
   Gly Asn Val Met Ala Thr Ala Phe His Pro Glu Leu Thr Gly Gly Ala
                   165                 170                 175
   gcc gtg cat gaa tat ttt tta cat ctg ggt cta gaa tag 567
   Ala Val His Glu Tyr Phe Leu His Leu Gly Leu Glu
               180                 185
<210> 45
   <211> 188
   <212> PRT
   <213> Tropheryma whipplei (strain TW08/27) (whipple's bacillus)
<400> 45
   Met Thr Val Gly Val Leu Ser Leu Gln Gly Ser Phe Tyr Glu His Leu
   1               5                   10                  15
   Ser Ile Leu Ser Arg Leu Asn Thr Asp His Ile Gln Val Lys Thr Ser
               20                  25                  30
   Glu Asp Leu Ser Arg Val Thr Arg Leu Ile Ile Pro Gly Gly Glu Ser
           35                  40                  45
   Thr Ala Met Leu Ala Leu Thr Gln Lys Ser Gly Leu Phe Asp Leu Val
       50                  55                  60
   Arg Asp Arg Ile Met Ser Gly Met Pro Val Tyr Gly Thr Cys Ala Gly
   65                  70                  75                  80
   Met Ile Met Leu Ser Thr Phe Val Glu Asp Phe Pro Asn Gln Lys Thr
                   85                  90                  95
   Leu Ser Cys Leu Asp Ile Ala Val Arg Arg Asn Ala Phe Gly Arg Gln
               100                 105                 110
   Ile Asn Ser Phe Glu Ser Glu Val Ser Phe Leu Asn Ser Lys Ile Thr
           115                 120                 125
   Val Pro Phe Ile Arg Ala Pro Lys Ile Thr Gln Ile Gly Glu Gly Val
       130                 135                 140
   Asp Val Leu Ser Arg Leu Glu Ser Gly Asp Ile Val Ala Val Arg Gln
   145                 150                 155                 160
   Gly Asn Val Met Ala Thr Ala Phe His Pro Glu Leu Thr Gly Gly Ala
                   165                 170                 175
   Ala Val His Glu Tyr Phe Leu His Leu Gly Leu Glu
               180                 185
<210> 46
   <211> 558
   <212> DNA
   <213> Staphylococcus epidermidis
<400> 46
   atg aaa att ggt gtt tta gcc tta caa ggt gct gta cgt gaa cat ata      48
   Met Lys Ile Gly Val Leu Ala Leu Gln Gly Ala Val Arg Glu His Ile
   1               5                   10                  15
   cgt cat att gaa tta agt ggt tat gaa ggc att gct ata aaa aga gta      96
   Arg His Ile Glu Leu Ser Gly Tyr Glu Gly Ile Ala Ile Lys Arg Val
               20                  25                  30
   gag caa cta gat gaa att gat ggt cta ata tta cct ggt gga gag tct      144
   Glu Gln Leu Asp Glu Ile Asp Gly Leu Ile Leu Pro Gly Gly Glu Ser
           35                  40                  45
   aca aca tta cgt cgt tta atg gat tta tat gga ttt aaa gaa aag tta      192
   Thr Thr Leu Arg Arg Leu Met Asp Leu Tyr Gly Phe Lys Glu Lys Leu
       50                  55                  60
   caa caa tta gat ttg cca atg ttt gga aca tgt gct gga tta att gtt      240
   Gln Gln Leu Asp Leu Pro Met Phe Gly Thr Cys Ala Gly Leu Ile Val
   65                  70                  75                  80
   ctt gca aaa aat gtt gaa aat gag tct ggt tat tta aat aaa tta gat      288
   Leu Ala Lys Asn Val Glu Asn Glu Ser Gly Tyr Leu Asn Lys Leu Asp
                   85                  90                  95
   ata act gtt gag cgt aat tca ttc ggt aga caa gtc gat agc ttt gaa      336
   Ile Thr Val Glu Arg Asn Ser Phe Gly Arg Gln Val Asp Ser Phe Glu
               100                 105                 110
   tct gaa ctt gat att aaa ggg ata gca aat gat att gag gga gta ttt      384
   Ser Glu Leu Asp Ile Lys Gly Ile Ala Asn Asp Ile Glu Gly Val Phe
           115                 120                 125
   att aga gca cct cat att gct aaa gtg gat aac gga gtg gaa ata ctt      432
   Ile Arg Ala Pro His Ile Ala Lys Val Asp Asn Gly Val Glu Ile Leu
       130                 135                 140
   agt aaa gtt gga ggt aaa ata gta gcc gtc aaa caa gga caa tac ctc      480
   Ser Lys Val Gly Gly Lys Ile Val Ala Val Lys Gln Gly Gln Tyr Leu
   145                 150                 155                 160
   ggt gtt tct ttc cat cca gaa cta act gat gat tat cgt atc act aag      528
   Gly Val Ser Phe His Pro Glu Leu Thr Asp Asp Tyr Arg Ile Thr Lys
               165                 170                 175
   tat ttt att gaa cac atg att aaa cat tga                              558
   Tyr Phe Ile Glu His Met Ile Lys His
               180                 185
<210> 47
   <211> 185
   <212> PRT
   <213> Staphylococcus epidermidis
<400> 47
   Met Lys Ile Gly Val Leu Ala Leu Gln Gly Ala Val Arg Glu His Ile
   1               5                   10                  15
   Arg His Ile Glu Leu Ser Gly Tyr Glu Gly Ile Ala Ile Lys Arg Val
               20                  25                  30
   Glu Gln Leu Asp Glu Ile Asp Gly Leu Ile Leu Pro Gly Gly Glu Ser
           35                  40                  45
   Thr Thr Leu Arg Arg Leu Met Asp Leu Tyr Gly Phe Lys Glu Lys Leu
       50                  55                  60
   Gln Gln Leu Asp Leu Pro Met Phe Gly Thr Cys Ala Gly Leu Ile Val
   65                  70                  75                  80
   Leu Ala Lys Asn Val Glu Asn Glu Ser Gly Tyr Leu Asn Lys Leu Asp
                   85                  90                  95
   Ile Thr Val Glu Arg Asn Ser Phe Gly Arg Gln Val Asp Ser Phe Glu
               100                 105                 110
   Ser Glu Leu Asp Ile Lys Gly Ile Ala Asn Asp Ile Glu Gly Val Phe
           115                 120                 125
   Ile Arg Ala Pro His Ile Ala Lys Val Asp Asn Gly Val Glu Ile Leu
       130                 135                 140
   Ser Lys Val Gly Gly Lys Ile Val Ala Val Lys Gln Gly Gln Tyr Leu
   145                 150                 155                 160
   Gly Val Ser Phe His Pro Glu Leu Thr Asp Asp Tyr Arg Ile Thr Lys
               165                 170                 175
   Tyr Phe Ile Glu His Met Ile Lys His
               180                 185
<210> 48
   <211> 639
   <212> DNA
   <213> Bifidobacterium longum
<400> 48
   atg gtt gta gct gtt gaa tat att tcc aaa gaa gaa tcc gcg gac gcc      48
   Met Val Val Ala Val Glu Tyr Ile Ser Lys Glu Glu Ser Ala Asp Ala
   1               5                   10                  15
   aaa aac gcc aag cac ggc gtg acc ggc atc ctg gcc gta caa ggc gca      96
   Lys Asn Ala Lys His Gly Val Thr Gly Ile Leu Ala Val Gln Gly Ala
               20                  25                  30
   ttc gcc gaa cat gcg gcg gtg ctg gac aag ctc ggt gcg ccg tgg aaa      144
   Phe Ala Glu His Ala Ala Val Leu Asp Lys Leu Gly Ala Pro Trp Lys
           35                  40                  45
   ctg ctg cgc gca gcc gag gat ttc gat gaa tcc atc gac cgc gtg att      192
   Leu Leu Arg Ala Ala Glu Asp Phe Asp Glu Ser Ile Asp Arg Val Ile
       50                  55                  60
   ctg ccc ggc ggc gaa tcc act aca cag ggc aag ctc ctg cat tcg acc      240
   Leu Pro Gly Gly Glu Ser Thr Thr Gln Gly Lys Leu Leu His Ser Thr
   65                  70                  75                  80
   gga ctg ttc gag ccg atc gcc gcc cac atc aag gca ggc aaa ccg gtg      288
   Gly Leu Phe Glu Pro Ile Ala Ala His Ile Lys Ala Gly Lys Pro Val
                   85                  90                  95
   ttt ggc act tgc gcc ggc atg att ctg ctg gct aaa aag ctc gac aat      336
   Phe Gly Thr Cys Ala Gly Met Ile Leu Leu Ala Lys Lys Leu Asp Asn
               100                 105                 110
   gac gac aac gtc tac ttt ggc gcg ctc gac gcc gtc gta cgc cgc aac      384
   Asp Asp Asn Val Tyr Phe Gly Ala Leu Asp Ala Val Val Arg Arg Asn
           115                 120                 125
   gcc tat ggt cgt cag ctc ggt agt ttc cag gct act gcc gat ttt ggt      432
   Ala Tyr Gly Arg Gln Leu Gly Ser Phe Gln Ala Thr Ala Asp Phe Gly
       130                 135                 140
   gca gcg gat gat ccg cag cgt atc acg gac ttc cca ctg gta ttc atc      480
   Ala Ala Asp Asp Pro Gln Arg Ile Thr Asp Phe Pro Leu Val Phe Ile
   145                 150                 155                 160
   cgc gga ccg tac gtg gtg tcg gtc gga ccc gaa gcc acg gtc gaa acc      528
   Arg Gly Pro Tyr Val Val Ser Val Gly Pro Glu Ala Thr Val Glu Thr
                   165                 170                 175
   gaa gtc gat ggc cac gtg gtg ggc ttg cgt caa ggc aat atc ctg gcc      576
   Glu Val Asp Gly His Val Val Gly Leu Arg Gln Gly Asn Ile Leu Ala
               180                 185                 190
   acc gcc ttc cac ccg gaa ctc acg gac gat acc cgc atc cac gag ctc      624
   Thr Ala Phe His Pro Glu Leu Thr Asp Asp Thr Arg Ile His Glu Leu
           195                 200                 205
   ttc ctg tcg ctg tag                                                  639
   Phe Leu Ser Leu
       210
<210> 49
   <211> 212
   <212> PRT
   <213> Bifidobacterium longum
<400> 49
   Met Val Val Ala Val Glu Tyr Ile Ser Lys Glu Glu Ser Ala Asp Ala
   1               5                   10                  15
   Lys Asn Ala Lys His Gly Val Thr Gly Ile Leu Ala Val Gln Gly Ala
               20                  25                  30
   Phe Ala Glu His Ala Ala Val Leu Asp Lys Leu Gly Ala Pro Trp Lys
           35                  40                  45
   Leu Leu Arg Ala Ala Glu Asp Phe Asp Glu Ser Ile Asp Arg Val Ile
       50                  55                  60
   Leu Pro Gly Gly Glu Ser Thr Thr Gln Gly Lys Leu Leu His Ser Thr
   65                  70                  75                  80
   Gly Leu Phe Glu Pro Ile Ala Ala His Ile Lys Ala Gly Lys Pro Val
                   85                  90                  95
   Phe Gly Thr Cys Ala Gly Met Ile Leu Leu Ala Lys Lys Leu Asp Asn
               100                 105                 110
   Asp Asp Asn Val Tyr Phe Gly Ala Leu Asp Ala Val Val Arg Arg Asn
           115                 120                 125
   Ala Tyr Gly Arg Gln Leu Gly Ser Phe Gln Ala Thr Ala Asp Phe Gly
       130                 135                 140
   Ala Ala Asp Asp Pro Gln Arg Ile Thr Asp Phe Pro Leu Val Phe Ile
   145                 150                 155                 160
   Arg Gly Pro Tyr Val Val Ser Val Gly Pro Glu Ala Thr Val Glu Thr
                   165                 170                 175
   Glu Val Asp Gly His Val Val Gly Leu Arg Gln Gly Asn Ile Leu Ala
               180                 185                 190
   Thr Ala Phe His Pro Glu Leu Thr Asp Asp Thr Arg Ile His Glu Leu
           195                 200                 205
   Phe Leu Ser Leu
       210
<210> 50
   <211> 573
   <212> DNA
   <213> Bacillus circulans
<400> 50
   atg aaa gtt ggc gta ttg gct ctg cag gga gcc gta gcg gaa cat atc      48
   Met Lys Val Gly Val Leu Ala Leu Gln Gly Ala Val Ala Glu His Ile
   1               5                   10                  15
   cgc ctg atc gag gcg gtt ggc gga gaa ggc gtc gtt gta aag cgt gcg      96
   Arg Leu Ile Glu Ala Val Gly Gly Glu Gly Val Val Val Lys Arg Ala
               20                  25                  30
   gag cag ctt gcc gaa ctg gac ggt ctg atc att ccc gga ggc gag agt     144
   Glu Gln Leu Ala Glu Leu Asp Gly Leu Ile Ile Pro Gly Gly Glu Ser
           35                  40                  45
   acc acc att ggc aaa ttg atg aga cgc tac ggt ttt atc gaa gcg att     192
   Thr Thr Ile Gly Lys Leu Met Arg Arg Tyr Gly Phe Ile Glu Ala Ile
       50                  55                  60
   cgg gat ttt tcc aat cag gga aaa gcg gtc ttc ggc acg tgt gcc gga     240
   Arg Asp Phe Ser Asn Gln Gly Lys Ala Val Phe Gly Thr Cys Ala Gly
   65                  70                  75                  80
   ctg att gtg atc gcg gat aag att gcg ggt cag gaa gaa gcc cat ctg     288
   Leu Ile Val Ile Ala Asp Lys Ile Ala Gly Gln Glu Glu Ala His Leu
                   85                  90                  95
   gga ctg atg gat atg acc gtg cag cgc aat gcg ttt ggc cgg cag cgg     336
   Gly Leu Met Asp Met Thr Val Gln Arg Asn Ala Phe Gly Arg Gln Arg
               100                 105                 110
   gaa agc ttt gaa acc gat ctg cct gtt aag ggc att gac cgg cct gta     384
   Glu Ser Phe Glu Thr Asp Leu Pro Val Lys Gly Ile Asp Arg Pro Val
           115                 120                 125
   agg gcc gtt ttc atc cgt gcg ccg ctt atc gat cag gtt gga aac ggc     432
   Arg Ala Val Phe Ile Arg Ala Pro Leu Ile Asp Gln Val Gly Asn Gly
       130                 135                 140
   gtg gac gtg tta agc gag tac aac ggg caa atc gtg gcc gcc aga cag     480
   Val Asp Val Leu Ser Glu Tyr Asn Gly Gln Ile Val Ala Ala Arg Gln
   145                 150                 155                 160
   ggc cat ctg ctt gcg gct tcg ttc cat ccc gaa ctg acg gat gat tca     528
   Gly His Leu Leu Ala Ala Ser Phe His Pro Glu Leu Thr Asp Asp Ser
                   165                 170                 175
   agc atg cac gca tat ttt ctg gat atg atc cgg gaa gcc cgt tga         573
   Ser Met His Ala Tyr Phe Leu Asp Met Ile Arg Glu Ala Arg
   180 185 190
<210> 51
   <211> 190
   <212> PRT
   <213> Bacillus circulans
<400> 51
   Met Lys Val Gly Val Leu Ala Leu Gln Gly Ala Val Ala Glu His Ile
   1               5                   10                  15
   Arg Leu Ile Glu Ala Val Gly Gly Glu Gly Val Val Val Lys Arg Ala
               20                  25                  30
   Glu Gln Leu Ala Glu Leu Asp Gly Leu Ile Ile Pro Gly Gly Glu Ser
           35                  40                  45
   Thr Thr Ile Gly Lys Leu Met Arg Arg Tyr Gly Phe Ile Glu Ala Ile
       50                  55                  60
   Arg Asp Phe Ser Asn Gln Gly Lys Ala Val Phe Gly Thr Cys Ala Gly
   65                  70                  75                  80
   Leu Ile Val Ile Ala Asp Lys Ile Ala Gly Gln Glu Glu Ala His Leu
                   85                  90                  95
   Gly Leu Met Asp Met Thr Val Gln Arg Asn Ala Phe Gly Arg Gln Arg
               100                 105                 110
   Glu Ser Phe Glu Thr Asp Leu Pro Val Lys Gly Ile Asp Arg Pro Val
           115                 120                 125
   Arg Ala Val Phe Ile Arg Ala Pro Leu Ile Asp Gln Val Gly Asn Gly
       130                 135                 140
   Val Asp Val Leu Ser Glu Tyr Asn Gly Gln Ile Val Ala Ala Arg Gln
   145                 150                 155                 160
   Gly His Leu Leu Ala Ala Ser Phe His Pro Glu Leu Thr Asp Asp Ser
                   165                 170                 175
   Ser Met His Ala Tyr Phe Leu Asp Met Ile Arg Glu Ala Arg
               180                 185                 190
<210> 52
   <211> 1174
   <212> DNA
   <213> Arabidopsis thaliana (Mouse-ear cress)
<400> 52
   gaatagaaat ccaaatcgtg ggcaaagaaa gaaacacaaa acaaaatcgt cgatggctgt 60
   tacaaaaagg cttttgtgag tgtcccaatt ccattcacaa agttttagtg tttaataata 120
   tctgacactc tctttctttg accgtcgccg ccgca atg acc gtc gga gtt tta 173
   Met Thr Val Gly Val Leu
   1 5
   gct ttg caa ggt tct ttc aat gag cac atc gcg gct ctg cgg cgg ctc 221
   Ala Leu Gln Gly Ser Phe Asn Glu His Ile Ala Ala Leu Arg Arg Leu
   10 15 20
   ggt gtc caa ggc gtc gag att agg aag gct gac cag ctt ctc acc gtt 269
   Gly Val Gln Gly Val Glu Ile Arg Lys Ala Asp Gln Leu Leu Thr Val
   25 30 35
   tct tct ctt atc att cct ggc ggc gag agc acc acc atg gcc aaa ctc 317
   Ser Ser Leu Ile Ile Pro Gly Gly Glu Ser Thr Thr Met Ala Lys Leu
   40 45 50
   gcc gag tat cat aac ttg ttt ccg gct cta cgt gag ttt gtt aag atg 365
   Ala Glu Tyr His Asn Leu Phe Pro Ala Leu Arg Glu Phe Val Lys Met
   55 60 65 70
   ggg aaa cct gtt tgg ggg aca tgc gca ggt ctt ata ttc ttg gca gac 413
   Gly Lys Pro Val Trp Gly Thr Cys Ala Gly Leu Ile Phe Leu Ala Asp
   75 80 85
   aga gca gtt ggt cag aaa gag gga ggt cag gaa tta gtt ggt ggc ctt 461
   Arg Ala Val Gly Gln Lys Glu Gly Gly Gln Glu Leu Val Gly Gly Leu
   90 95 100
   gat tgc acc gta cat agg aac ttc ttc ggt agc cag att caa agt ttt 509
   Asp Cys Thr Val His Arg Asn Phe Phe Gly Ser Gln Ile Gln Ser Phe
   105 110 115
   gaa gct gat atc tta gta cct caa cta aca tct caa gaa ggt ggg cca 557
   Glu Ala Asp Ile Leu Val Pro Gln Leu Thr Ser Gln Glu Gly Gly Pro
   120 125 130
   gag aca tac agg gga gtg ttc ata cgt gct cca gct gtt ctt gat gta 605
   Glu Thr Tyr Arg Gly Val Phe Ile Arg Ala Pro Ala Val Leu Asp Val
   135 140 145 150
   ggt cct gat gtc gaa gtc ctg gcg gat tat ccc gtc cca tca aac aag 653
   Gly Pro Asp Val Glu Val Leu Ala Asp Tyr Pro Val Pro Ser Asn Lys
   155 160 165
   gtc ttg tat tca agc tcc acc gta caa att caa gag gaa gat gct ctt 701
   Val Leu Tyr Ser Ser Ser Thr Val Gln Ile Gln Glu Glu Asp Ala Leu
   170 175 180
   cct gaa aca aaa gtc att gtt gct gtg aag caa gga aac ttg tta gca 749
   Pro Glu Thr Lys Val Ile Val Ala Val Lys Gln Gly Asn Leu Leu Ala
   185 190 195
   act gct ttt cat ccc gag ctt act gca gac act cga tgg cac agt tat 797
   Thr Ala Phe His Pro Glu Leu Thr Ala Asp Thr Arg Trp His Ser Tyr
   200 205 210
   ttc ata aag atg acg aaa gag att gag caa gga gct tct tca agc agt 845
   Phe Ile Lys Met Thr Lys Glu Ile Glu Gln Gly Ala Ser Ser Ser Ser
   215 220 225 230
   agt aag act att gta tct gtt gga gaa aca agt gct ggt ccc gag cca 893
   Ser Lys Thr Ile Val Ser Val Gly Glu Thr Ser Ala Gly Pro Glu Pro
   235 240 245
   gct aag cct gat ctt cct ata ttt caa taactgaaca gagagaagat 940
   Ala Lys Pro Asp Leu Pro Ile Phe Gln
   250 255
   acacacttct taaaataaaa accagagaaa gtgtcagatt ctttatcttt ctaaagatgt 1000
   tttggaaaaa ttgcaagcta gtttgcaatt tgcactcaag aaagtttcac aagactcttt 1060
   aatggattca tgtacttgtt tcttgataca actttatata tacagttgaa tctcaaactt 1120
   ttttgctgat tcaatttggt ctatgtcttg tgaaatgtga aaggtcgttt ggcc 1174
<210> 53
   <211> 255
   <212> PRT
   <213> Arabidopsis thaliana (Mouse-ear cress)
<400> 53
   Met Thr Val Gly Val Leu Ala Leu Gln Gly Ser Phe Asn Glu His Ile
   1               5                   10                  15
   Ala Ala Leu Arg Arg Leu Gly Val Gln Gly Val Glu Ile Arg Lys Ala
               20                  25                  30
   Asp Gln Leu Leu Thr Val Ser Ser Leu Ile Ile Pro Gly Gly Glu Ser
           35                  40                  45
   Thr Thr Met Ala Lys Leu Ala Glu Tyr His Asn Leu Phe Pro Ala Leu
       50                  55                  60
   Arg Glu Phe Val Lys Met Gly Lys Pro Val Trp Gly Thr Cys Ala Gly
   65                  70                  75                  80
   Leu Ile Phe Leu Ala Asp Arg Ala Val Gly Gln Lys Glu Gly Gly Gln
                   85                  90                  95
   Glu Leu Val Gly Gly Leu Asp Cys Thr Val His Arg Asn Phe Phe Gly
               100                 105                 110
   Ser Gln Ile Gln Ser Phe Glu Ala Asp Ile Leu Val Pro Gln Leu Thr
           115                 120                 125
   Ser Gln Glu Gly Gly Pro Glu Thr Tyr Arg Gly Val Phe Ile Arg Ala
       130                 135                 140
   Pro Ala Val Leu Asp Val Gly Pro Asp Val Glu Val Leu Ala Asp Tyr
   145                 150                 155                 160
   Pro Val Pro Ser Asn Lys Val Leu Tyr Ser Ser Ser Thr Val Gln Ile
                   165                 170                 175
   Gln Glu Glu Asp Ala Leu Pro Glu Thr Lys Val Ile Val Ala Val Lys
               180                 185                 190
   Gln Gly Asn Leu Leu Ala Thr Ala Phe His Pro Glu Leu Thr Ala Asp
           195                 200                 205
   Thr Arg Trp His Ser Tyr Phe Ile Lys Met Thr Lys Glu Ile Glu Gln
       210                 215                 220
   Gly Ala Ser Ser Ser Ser Ser Lys Thr Ile Val Ser Val Gly Glu Thr
   225                 230                 235                 240
   Ser Ala Gly Pro Glu Pro Ala Lys Pro Asp Leu Pro Ile Phe Gln
                   245                 250                 255
<210> 54
   <211> 723
   <212> DNA
   <213> Corynebacterium glutamicum (Brevibacterium flavum)
<400> 54
   cctccgtcat tgccgacgta tcccgcggcc tgggtgaagc catggtgggc atcaacgtat      60
   ccgacgttcc agcaccacac cgactcgccg agcgcggctg atg atc gtt gga gtt       115
                                               Met Ile Val Gly Val
                                               1               5
   tta gct ctc cag ggc ggg gtg gaa gaa cac ctc acc gcc ttg gaa gct       163
   Leu Ala Leu Gln Gly Gly Val Glu Glu His Leu Thr Ala Leu Glu Ala
                   10                  15                  20
   ctc gga gcg acg acc cga aaa gta cgt gtg cca aag gac ctt gat ggt       211
   Leu Gly Ala Thr Thr Arg Lys Val Arg Val Pro Lys Asp Leu Asp Gly
               25                  30                  35
   ctc gaa ggc atc gtc atc ccc ggc ggg gaa tcc acc gtg ttg gac aaa       259
   Leu Glu Gly Ile Val Ile Pro Gly Gly Glu Ser Thr Val Leu Asp Lys
           40                  45                  50
   ctg gct cgg aca ttc gac gtg gta gaa cct cta gcg aat ctc att cgc       307
   Leu Ala Arg Thr Phe Asp Val Val Glu Pro Leu Ala Asn Leu Ile Arg
       55                  60                  65
   gac ggc cta ccc gtt ttc gct acc tgc gct ggc ctg atc tat ctg gcg       355
   Asp Gly Leu Pro Val Phe Ala Thr Cys Ala Gly Leu Ile Tyr Leu Ala
   70                  75                  80                  85
   aaa cac ctc gac aac cca gca agg gga caa caa acc ttg gcg gta gtg       403
   Lys His Leu Asp Asn Pro Ala Arg Gly Gln Gln Thr Leu Ala Val Val
                   90                  95                  100
   gac gtg gtg gtg cgt cga aac gca ttt ggc gcc caa cgc gaa tcc ttc       451
   Asp Val Val Val Arg Arg Asn Ala Phe Gly Ala Gln Arg Glu Ser Phe
               105                 110                 115
   gac acc acc gtg gat gtt tcc ttc gac ggt gca aca ttc ccc gga gtg       499
   Asp Thr Thr Val Asp Val Ser Phe Asp Gly Ala Thr Phe Pro Gly Val
           120                 125                 130
   cag gcc tcg ttt atc cga gct ccc atc gtc act gct ttt ggt cct acg       547
   Gln Ala Ser Phe Ile Arg Ala Pro Ile Val Thr Ala Phe Gly Pro Thr
       135                 140                 145
   gta gaa gcg atc gct gct ctc aac ggt ggg gag gtg gtt ggt gta cgc       595
   Val Glu Ala Ile Ala Ala Leu Asn Gly Gly Glu Val Val Gly Val Arg
   150                 155                 160                 165
   caa ggc aac atc atc gcg ctg tct ttc cat ccc gaa gaa acc ggc gat       643
   Gln Gly Asn Ile Ile Ala Leu Ser Phe His Pro Glu Glu Thr Gly Asp
                   170                 175                 180
   tac cgc atc cac caa gcc tgg ctg gac ctg gtg aga aaa cac gct gaa       691
   Tyr Arg Ile His Gln Ala Trp Leu Asp Leu Val Arg Lys His Ala Glu
               185                 190                 195
   ctg gcg att tgatgttttc ggtagcgctc tgt                                723
   Leu Ala Ile
           200
<210> 55
   <211> 200
   <212> PRT
   <213> Corynebacterium glutamicum (Brevibacterium flavum)
<400> 55
   Met Ile Val Gly Val Leu Ala Leu Gln Gly Gly Val Glu Glu His Leu
   1               5                   10                  15
   Thr Ala Leu Glu Ala Leu Gly Ala Thr Thr Arg Lys Val Arg Val Pro
               20                  25                  30
   Lys Asp Leu Asp Gly Leu Glu Gly Ile Val Ile Pro Gly Gly Glu Ser
           35                  40                  45
   Thr Val Leu Asp Lys Leu Ala Arg Thr Phe Asp Val Val Glu Pro Leu
       50                  55                  60
   Ala Asn Leu Ile Arg Asp Gly Leu Pro Val Phe Ala Thr Cys Ala Gly
   65                  70                  75                  80
   Leu Ile Tyr Leu Ala Lys His Leu Asp Asn Pro Ala Arg Gly Gln Gln
                   85                  90                  95
   Thr Leu Ala Val Val Asp Val Val Val Arg Arg Asn Ala Phe Gly Ala
               100                 105                 110
   Gln Arg Glu Ser Phe Asp Thr Thr Val Asp Val Ser Phe Asp Gly Ala
           115                 120                 125
   Thr Phe Pro Gly Val Gln Ala Ser Phe Ile Arg Ala Pro Ile Val Thr
       130                 135                 140
   Ala Phe Gly Pro Thr Val Glu Ala Ile Ala Ala Leu Asn Gly Gly Glu
   145                 150                 155                 160
   Val Val Gly Val Arg Gln Gly Asn Ile Ile Ala Leu Ser Phe His Pro
                   165                 170                 175
   Glu Glu Thr Gly Asp Tyr Arg Ile His Gln Ala Trp Leu Asp Leu Val
               180                 185                 190
   Arg Lys His Ala Glu Leu Ala Ile
           195                 200
<210> 56
   <211> 612
   <212> DNA
   <213> Methanosarcina mazei (Methanosarcina frisia)
<400> 56
   atg gtg ttt tta atg aaa ata ggt gta atc gct att cag gga gcg gtt      48
   Met Val Phe Leu Met Lys Ile Gly Val Ile Ala Ile Gln Gly Ala Val
   1               5                   10                  15
   tct gag cat gtt gat gct tta agg aga gcc ctt aaa gag aga ggg gtt      96
   Ser Glu His Val Asp Ala Leu Arg Arg Ala Leu Lys Glu Arg Gly Val
               20                  25                  30
   gag gct gag gta gtt gag ata aag cac aaa gga att gtg ccg gag tgc      144
   Glu Ala Glu Val Val Glu Ile Lys His Lys Gly Ile Val Pro Glu Cys
           35                  40                  45
   agc gga att gtg att cct ggc ggg gag agt aca acg ctt tgc agg ctg      192
   Ser Gly Ile Val Ile Pro Gly Gly Glu Ser Thr Thr Leu Cys Arg Leu
       50                  55                  60
   ctt gcc cgc gag gga att gca gag gag ata aaa gaa gcg gct gca aag      240
   Leu Ala Arg Glu Gly Ile Ala Glu Glu Ile Lys Glu Ala Ala Ala Lys
   65                  70                  75                  80
   gga gtt cct atc ctc ggg acc tgt gca ggg ctg att gtc att gca aag      288
   Gly Val Pro Ile Leu Gly Thr Cys Ala Gly Leu Ile Val Ile Ala Lys
                   85                  90                  95
   gaa gga gac cgg cag gta gaa aag aca ggt cag gaa ctg ctc ggg att      336
   Glu Gly Asp Arg Gln Val Glu Lys Thr Gly Gln Glu Leu Leu Gly Ile
               100                 105                 110
   atg gat acc agg gtc aac agg aac gcc ttt ggg agg cag agg gat tct      384
   Met Asp Thr Arg Val Asn Arg Asn Ala Phe Gly Arg Gln Arg Asp Ser
           115                 120                 125
   ttt gag gca gaa ctt gag gtg ttt atc ctt gac tct cca ttt acg ggc      432
   Phe Glu Ala Glu Leu Glu Val Phe Ile Leu Asp Ser Pro Phe Thr Gly
       130                 135                 140
   gtg ttt atc cgg gct ccg gga atc gtg agc tgc ggg ccg ggc gtg aag      480
   Val Phe Ile Arg Ala Pro Gly Ile Val Ser Cys Gly Pro Gly Val Lys
   145                 150                 155                 160
   gtg ctt tcc agg ctt gaa ggc atg atc gtt gct gca gag cag gga aat      528
   Val Leu Ser Arg Leu Glu Gly Met Ile Val Ala Ala Glu Gln Gly Asn
                   165                 170                 175
   gtg ctg gca ctt gca ttc cat ccg gaa tta acc gat gac ctt aga att      576
   Val Leu Ala Leu Ala Phe His Pro Glu Leu Thr Asp Asp Leu Arg Ile
               180                 185                 190
   cac cag tat ttc ctg gat aaa gtt ttg aac tgc tag                      612
   His Gln Tyr Phe Leu Asp Lys Val Leu Asn Cys
           195                 200
<210> 57
   <211> 203
   <212> PRT
   <213> Methanosarcina mazei (Methanosarcina frisia)
<400> 57
   Met Val Phe Leu Met Lys Ile Gly Val Ile Ala Ile Gln Gly Ala Val
   1               5                   10                  15
   Ser Glu His Val Asp Ala Leu Arg Arg Ala Leu Lys Glu Arg Gly Val
               20                  25                  30
   Glu Ala Glu Val Val Glu Ile Lys His Lys Gly Ile Val Pro Glu Cys
           35                  40                  45
   Ser Gly Ile Val Ile Pro Gly Gly Glu Ser Thr Thr Leu Cys Arg Leu
       50                  55                  60
   Leu Ala Arg Glu Gly Ile Ala Glu Glu Ile Lys Glu Ala Ala Ala Lys
   65                  70                  75                  80
   Gly Val Pro Ile Leu Gly Thr Cys Ala Gly Leu Ile Val Ile Ala Lys
                   85                  90                  95
   Glu Gly Asp Arg Gln Val Glu Lys Thr Gly Gln Glu Leu Leu Gly Ile
               100                 105                 110
   Met Asp Thr Arg Val Asn Arg Asn Ala Phe Gly Arg Gln Arg Asp Ser
           115                 120                 125
   Phe Glu Ala Glu Leu Glu Val Phe Ile Leu Asp Ser Pro Phe Thr Gly
       130                 135                 140
   Val Phe Ile Arg Ala Pro Gly Ile Val Ser Cys Gly Pro Gly Val Lys
   145                 150                 155                 160
   Val Leu Ser Arg Leu Glu Gly Met Ile Val Ala Ala Glu Gln Gly Asn
                   165                 170                 175
   Val Leu Ala Leu Ala Phe His Pro Glu Leu Thr Asp Asp Leu Arg Ile
               180                 185                 190
   His Gln Tyr Phe Leu Asp Lys Val Leu Asn Cys
           195                 200
<210> 58
   <211> 594
   <212> DNA
   <213> Pyrococcus furiosus
<400> 58
   atg gtc aag ata ggt gtt att ggc ctt cag gga gat gta agc gag cac        48
   Met Val Lys Ile Gly Val Ile Gly Leu Gln Gly Asp Val Ser Glu His
   1               5                   10                  15
   att gaa gct act aaa agg gcc ttg gaa aga tta ggg att gaa ggg agt        96
   Ile Glu Ala Thr Lys Arg Ala Leu Glu Arg Leu Gly Ile Glu Gly Ser
               20                  25                  30
   gtt ata tgg gtc aag aga ccc gaa caa ctc aac caa att gat gga gta       144
   Val Ile Trp Val Lys Arg Pro Glu Gln Leu Asn Gln Ile Asp Gly Val
           35                  40                  45
   ata atc cca gga ggg gaa agc aca aca atc tca aga cta atg cag aga       192
   Ile Ile Pro Gly Gly Glu Ser Thr Thr Ile Ser Arg Leu Met Gln Arg
       50                  55                  60
   aca gga tta ttt gat cca tta aaa aag atg att gag gat ggc ctc ccc       240
   Thr Gly Leu Phe Asp Pro Leu Lys Lys Met Ile Glu Asp Gly Leu Pro
   65                  70                  75                  80
   gca atg ggt act tgt gca ggg ctg ata atg ctt gca aag gaa gtt att       288
   Ala Met Gly Thr Cys Ala Gly Leu Ile Met Leu Ala Lys Glu Val Ile
                   85                  90                  95
   gga gct aca cca gag caa aag ttc ctt gag gtt ctt gat gtg aag gtg       336
   Gly Ala Thr Pro Glu Gln Lys Phe Leu Glu Val Leu Asp Val Lys Val
               100                 105                 110
   aac agg aat gcc tat ggt agg caa gtt gac agc ttt gaa gct cct gta       384
   Asn Arg Asn Ala Tyr Gly Arg Gln Val Asp Ser Phe Glu Ala Pro Val
           115                 120                 125
   aag ttg gca ttt gac gat aaa cca ttc att ggt gtt ttc att agg gct       432
   Lys Leu Ala Phe Asp Asp Lys Pro Phe Ile Gly Val Phe Ile Arg Ala
       130                 135                 140
   ccg agg ata gtt gag ctt ttg tca gac aag gtt aag ccc ctt gct tgg       480
   Pro Arg Ile Val Glu Leu Leu Ser Asp Lys Val Lys Pro Leu Ala Trp
   145                 150                 155                 160
   ctg gaa gat aga gtt gta ggg gtt gaa caa gga aac gtt atc ggt cta       528
   Leu Glu Asp Arg Val Val Gly Val Glu Gln Gly Asn Val Ile Gly Leu
                   165                 170                 175
   gaa ttc cat ccc gag ctt act gac gat act aga att cac gag tat ttc       576
   Glu Phe His Pro Glu Leu Thr Asp Asp Thr Arg Ile His Glu Tyr Phe
               180                 185                 190
   cta aag aag att gtc taa                                               594
   Leu Lys Lys Ile Val
           195
<210> 59
   <211> 197
   <212> PRT
   <213> Pyrococcus furiosus
<400> 59
   Met Val Lys Ile Gly Val Ile Gly Leu Gln Gly Asp Val Ser Glu His
   1               5                   10                  15
   Ile Glu Ala Thr Lys Arg Ala Leu Glu Arg Leu Gly Ile Glu Gly Ser
               20                  25                  30
   Val Ile Trp Val Lys Arg Pro Glu Gln Leu Asn Gln Ile Asp Gly Val
           35                  40                  45
   Ile Ile Pro Gly Gly Glu Ser Thr Thr Ile Ser Arg Leu Met Gln Arg
       50                  55                  60
   Thr Gly Leu Phe Asp Pro Leu Lys Lys Met Ile Glu Asp Gly Leu Pro
   65                  70                  75                  80
   Ala Met Gly Thr Cys Ala Gly Leu Ile Met Leu Ala Lys Glu Val Ile
                   85                  90                  95
   Gly Ala Thr Pro Glu Gln Lys Phe Leu Glu Val Leu Asp Val Lys Val
               100                 105                 110
   Asn Arg Asn Ala Tyr Gly Arg Gln Val Asp Ser Phe Glu Ala Pro Val
           115                 120                 125
   Lys Leu Ala Phe Asp Asp Lys Pro Phe Ile Gly Val Phe Ile Arg Ala
       130                 135                 140
   Pro Arg Ile Val Glu Leu Leu Ser Asp Lys Val Lys Pro Leu Ala Trp
   145                 150                 155                 160
   Leu Glu Asp Arg Val Val Gly Val Glu Gln Gly Asn Val Ile Gly Leu
                   165                 170                 175
   Glu Phe His Pro Glu Leu Thr Asp Asp Thr Arg Ile His Glu Tyr Phe
               180                 185                 190
   Leu Lys Lys Ile Val
           195
<210> 60
   <211> 600
   <212> DNA
   <213> Methanosarcina acetivorans
<400> 60
   atg aag ata ggt gta atc gct att cag gga gcg gtt tcc gag cat gtt      48
   Met Lys Ile Gly Val Ile Ala Ile Gln Gly Ala Val Ser Glu His Val
   1               5                   10                  15
   gat gct ttg agg aga gcc ctt gca gag aga ggg gtt gag gct gag gta      96
   Asp Ala Leu Arg Arg Ala Leu Ala Glu Arg Gly Val Glu Ala Glu Val
               20                  25                  30
   gtt gag ata aag cat aag gga att gtt ccg gag tgc agc gga att gtg      144
   Val Glu Ile Lys His Lys Gly Ile Val Pro Glu Cys Ser Gly Ile Val
           35                  40                  45
   atc ccc ggg ggg gag agc aca acg ctc tgc cgg ctg ctt gcc cgc gaa      192
   Ile Pro Gly Gly Glu Ser Thr Thr Leu Cys Arg Leu Leu Ala Arg Glu
       50                  55                  60
   gga att gga gag gag att aag gag gct gct gca aga gga gtt ccg gtt        240
   Gly Ile Gly Glu Glu Ile Lys Glu Ala Ala Ala Arg Gly Val Pro Val
   65                  70                  75                  80
   ctc ggg acc tgt gcg ggg ctg atc gtg ctt gca aag gaa ggg gac cgg        288
   Leu Gly Thr Cys Ala Gly Leu Ile Val Leu Ala Lys Glu Gly Asp Arg
                   85                  90                  95
   cag gta gaa aaa acc ggg cag gag ctg ctc ggg atc atg gat aca agg        336
   Gln Val Glu Lys Thr Gly Gln Glu Leu Leu Gly Ile Met Asp Thr Arg
               100                 105                 110
   gtt aac agg aac gct ttt ggg agg cag agg gat tcc ttt gag gca gag        384
   Val Asn Arg Asn Ala Phe Gly Arg Gln Arg Asp Ser Phe Glu Ala Glu
           115                 120                 125
   ctt gat gtg gtt att ctt gac tct ccg ttt acc ggg gtg ttc atc cgg        432
   Leu Asp Val Val Ile Leu Asp Ser Pro Phe Thr Gly Val Phe Ile Arg
       130                 135                 140
   gct ccg gga atc att agc tgc ggg cct ggt gtg cgc gtg ctt tcc agg        480
   Ala Pro Gly Ile Ile Ser Cys Gly Pro Gly Val Arg Val Leu Ser Arg
   145                 150                 155                 160
   ctt gaa gac atg att att gct gca gaa cag ggt aat gtg ctg gct ctt        528
   Leu Glu Asp Met Ile Ile Ala Ala Glu Gln Gly Asn Val Leu Ala Leu
                   165                 170                 175
   gct ttc cat ccg gaa tta acc gat gat ctg cgc atc cac cag tat ttc        576
   Ala Phe His Pro Glu Leu Thr Asp Asp Leu Arg Ile His Gln Tyr Phe
               180                 185                 190
   ctg aat aag gtt ttg agt tgt taa                                        600
   Leu Asn Lys Val Leu Ser Cys
           195
<210> 61
   <211> 199
   <212> PRT
   <213> Methanosarcina acetivorans
<400> 61
   Met Lys Ile Gly Val Ile Ala Ile Gln Gly Ala Val Ser Glu His Val
   1               5                   10                  15
   Asp Ala Leu Arg Arg Ala Leu Ala Glu Arg Gly Val Glu Ala Glu Val
               20                  25                  30
   Val Glu Ile Lys His Lys Gly Ile Val Pro Glu Cys Ser Gly Ile Val
           35                  40                  45
   Ile Pro Gly Gly Glu Ser Thr Thr Leu Cys Arg Leu Leu Ala Arg Glu
       50                  55                  60
   Gly Ile Gly Glu Glu Ile Lys Glu Ala Ala Ala Arg Gly Val Pro Val
   65                  70                  75                  80
   Leu Gly Thr Cys Ala Gly Leu Ile Val Leu Ala Lys Glu Gly Asp Arg
                   85                  90                  95
   Gln Val Glu Lys Thr Gly Gln Glu Leu Leu Gly Ile Met Asp Thr Arg
               100                 105                 110
   Val Asn Arg Asn Ala Phe Gly Arg Gln Arg Asp Ser Phe Glu Ala Glu
           115                 120                 125
   Leu Asp Val Val Ile Leu Asp Ser Pro Phe Thr Gly Val Phe Ile Arg
       130                 135                 140
   Ala Pro Gly Ile Ile Ser Cys Gly Pro Gly Val Arg Val Leu Ser Arg
   145                 150                 155                 160
   Leu Glu Asp Met Ile Ile Ala Ala Glu Gln Gly Asn Val Leu Ala Leu
                   165                 170                 175
   Ala Phe His Pro Glu Leu Thr Asp Asp Leu Arg Ile His Gln Tyr Phe
               180                 185                 190
   Leu Asn Lys Val Leu Ser Cys
           195
<210> 62
   <211> 609
   <212> DNA
   <213> Methanopyrus kandleri
<400> 62
   atg aag gtc gct gtc gtc gcc gtg cag gga gcc gtc gag gaa cac gaa        48
   Met Lys Val Ala Val Val Ala Val Gln Gly Ala Val Glu Glu His Glu
   1               5                   10                  15
   tcg atc ctg gaa gcg gcc ggt gag cgg atc ggc gaa gac gtc gag gtg        96
   Ser Ile Leu Glu Ala Ala Gly Glu Arg Ile Gly Glu Asp Val Glu Val
               20                  25                  30
   gta tgg gca agg tac ccg gaa gat ctc gag gac gtg gac gcc gtc gtg        144
   Val Trp Ala Arg Tyr Pro Glu Asp Leu Glu Asp Val Asp Ala Val Val
           35                  40                  45
   att ccg gga gga gag agc acc acg atc gga cgt ctg atg gag cgg cac        192
   Ile Pro Gly Gly Glu Ser Thr Thr Ile Gly Arg Leu Met Glu Arg His
       50                  55                  60
   gac ctg gtt aag ccg ctg ctg gag ctg gcg gag tcg gat act ccc atc        240
   Asp Leu Val Lys Pro Leu Leu Glu Leu Ala Glu Ser Asp Thr Pro Ile
   65                  70                  75                  80
   ctt gga acc tgc gcg ggg atg gtc atc ctc gcg cgt gag gtc gtt ccg        288
   Leu Gly Thr Cys Ala Gly Met Val Ile Leu Ala Arg Glu Val Val Pro
                   85                  90                  95
   cag gct cat cca ggg acg gag gtg gag atc gag cag cct cta cta ggt        336
   Gln Ala His Pro Gly Thr Glu Val Glu Ile Glu Gln Pro Leu Leu Gly
               100                 105                 110
   cta atg gac gtg cgg gta gtc cgg aac gcg ttc ggc cgg cag cgt gaa        384
   Leu Met Asp Val Arg Val Val Arg Asn Ala Phe Gly Arg Gln Arg Glu
           115                 120                 125
   tca ttc gaa gta gat atc gag atc gag ggg ctc gag gac cgg ttc cgg        432
   Ser Phe Glu Val Asp Ile Glu Ile Glu Gly Leu Glu Asp Arg Phe Arg
       130                 135                 140
   gca gtc ttc atc cga gct ccg gcc gtg gac gag gtc ctg tcc gac gat        480
   Ala Val Phe Ile Arg Ala Pro Ala Val Asp Glu Val Leu Ser Asp Asp
   145                 150                 155                 160
   gtg aag gtg ctc gcg gag tac ggc gat tac att gtg gcc gtg gag cag        528
   Val Lys Val Leu Ala Glu Tyr Gly Asp Tyr Ile Val Ala Val Glu Gln
                   165                 170                 175
   gat cac ctg ctc gcc acg gct ttc cac ccg gag ctc acc gac gat ccg        576
   Asp His Leu Leu Ala Thr Ala Phe His Pro Glu Leu Thr Asp Asp Pro
               180                 185                 190
   cgt ctt cac gct tac ttc ctg gag aag gtg tga                            609
   Arg Leu His Ala Tyr Phe Leu Glu Lys Val
           195                 200
<210> 63
   <211> 202
   <212> PRT
   <213> Methanopyrus kandleri
<400> 63
   Met Lys Val Ala Val Val Ala Val Gln Gly Ala Val Glu Glu His Glu
   1               5                   10                  15
   Ser Ile Leu Glu Ala Ala Gly Glu Arg Ile Gly Glu Asp Val Glu Val
               20                  25                  30
   Val Trp Ala Arg Tyr Pro Glu Asp Leu Glu Asp Val Asp Ala Val Val
           35                  40                  45
   Ile Pro Gly Gly Glu Ser Thr Thr Ile Gly Arg Leu Met Glu Arg His
       50                  55                  60
   Asp Leu Val Lys Pro Leu Leu Glu Leu Ala Glu Ser Asp Thr Pro Ile
   65                  70                  75                  80
   Leu Gly Thr Cys Ala Gly Met Val Ile Leu Ala Arg Glu Val Val Pro
                   85                  90                  95
   Gln Ala His Pro Gly Thr Glu Val Glu Ile Glu Gln Pro Leu Leu Gly
               100                 105                 110
   Leu Met Asp Val Arg Val Val Arg Asn Ala Phe Gly Arg Gln Arg Glu
           115                 120                 125
   Ser Phe Glu Val Asp Ile Glu Ile Glu Gly Leu Glu Asp Arg Phe Arg
       130                 135                 140
   Ala Val Phe Ile Arg Ala Pro Ala Val Asp Glu Val Leu Ser Asp Asp
   145                 150                 155                 160
   Val Lys Val Leu Ala Glu Tyr Gly Asp Tyr Ile Val Ala Val Glu Gln
                   165                 170                 175
   Asp His Leu Leu Ala Thr Ala Phe His Pro Glu Leu Thr Asp Asp Pro
                   180                 185                 190
   Arg Leu His Ala Tyr Phe Leu Glu Lys Val
           195                 200
<210> 64
   <211> 1262
   <212> DNA
   <213> Suberites domuncula (Sponge)
<400> 64
<210> 65
   <211> 233
   <212> PRT
   <213> Suberites domuncula (Sponge)
<400> 65
   Met Asp Ser Asn Thr Ile Thr Val Gly Val Leu Cys Ile Gln Gly Ala
   1               5                   10                  15
   Phe Ile Glu His Ile His Lys Leu Thr Thr Leu Ser Ser Thr Asp Lys
               20                  25                  30
   His Arg Asp Leu Thr Ile Thr Ile Val Glu Val Arg Glu Pro Gly Gln
           35                  40                  45
   Leu Ser Asp Leu Asp Gly Leu Ile Ile Pro Gly Gly Glu Ser Thr Thr
       50                  55                  60
   Leu Ser Val Phe Leu Arg Lys Asn Glu Phe Glu Gln Thr Leu Lys Ala
   65                  70                  75                  80
   Trp Ile Ser Asp Lys Gln Arg Pro Gly Val Val Trp Gly Thr Cys Ala
                   85                  90                  95
   Gly Leu Ile Ile Leu Ala Asp Asp Val Val Gly Gln Lys Leu Gly Gly
               100                 105                 110
   Gln Val Thr Ile Gly Gly Leu Asn Ile Gln Cys Thr Arg Asn Met Tyr
           115                 120                 125
   Gly Arg Gln Asn Lys Ser Phe Glu Ser Ala Ile Lys Leu His His Pro
       130                 135                 140
   Pro Leu His Ala Ala Gln Pro Thr Ser Ala Pro Pro Pro Phe Ser Leu
   145                 150                 155                 160
   Ala Asp Asp Glu Cys His Gly Ile Phe Ile Arg Ala Pro Gly Ile Leu
                   165                 170                 175
   Lys Val Asn Ser Pro Asp Val Lys Val Leu Ala Ser Val Asn Asp Asp
               180                 185                 190
   Asn Ile Val Ala Val Gln Gln Asp His Leu Ile Ala Thr Ser Phe His
           195                 200                 205
   Pro Glu Leu Thr Ser Asp Phe Arg Trp His Ser Tyr Phe Val Asp Gln
       210                 215                 220
   Ile Lys Gln His Arg Tyr Pro Gln Tyr
   225                 230
<210> 66
   <211> 615
   <212> DNA
   <213> Pyrobaculum aerophilum
<400> 66
   atg aaa att ggc gtg ttg gcg cta caa gga gat gtg gag gaa cac gca      48
   Met Lys Ile Gly Val Leu Ala Leu Gln Gly Asp Val Glu Glu His Ala
   1               5                   10                  15
   aac gcc ttt aaa gag gcg ggg agg gag gta ggc gtt gat gta gac gta      96
   Asn Ala Phe Lys Glu Ala Gly Arg Glu Val Gly Val Asp Val Asp Val
               20                  25                  30
   gta gag gtg aaa aaa ccc ggg gat tta aaa gac ata aaa gcg cta gcc      144
   Val Glu Val Lys Lys Pro Gly Asp Leu Lys Asp Ile Lys Ala Leu Ala
           35                  40                  45
   att ccg ggg ggc gag tct acc act att ggc cgc ctg gct aaa agg acc      192
   Ile Pro Gly Gly Glu Ser Thr Thr Ile Gly Arg Leu Ala Lys Arg Thr
       50                  55                  60
   ggc ctt tta gat gcc gtg aaa aag gcc att gag ggc ggc gtc ccc gcc      240
   Gly Leu Leu Asp Ala Val Lys Lys Ala Ile Glu Gly Gly Val Pro Ala
   65                  70                  75                  80
   ctc ggg act tgc gca gga gct att ttc atg gct aag gag gtg aaa gac        288
   Leu Gly Thr Cys Ala Gly Ala Ile Phe Met Ala Lys Glu Val Lys Asp
                   85                  90                  95
   gcc gtg gtc ggg gcc aca ggc cag ccc gta ctg ggg gtt atg gac atc        336
   Ala Val Val Gly Ala Thr Gly Gln Pro Val Leu Gly Val Met Asp Ile
               100                 105                 110
   gcc gtg gtc aga aac gcc ttt ggc aga cag agg gag tct ttt gaa gcc        384
   Ala Val Val Arg Asn Ala Phe Gly Arg Gln Arg Glu Ser Phe Glu Ala
           115                 120                 125
   gag gtg gtt tta gaa aat ctc ggc aag cta aag gct gtg ttt atc aga        432
   Glu Val Val Leu Glu Asn Leu Gly Lys Leu Lys Ala Val Phe Ile Arg
       130                 135                 140
   gcg cct gcg ttt gtg agg gcg tgg ggc tct gca aaa ctg ctc gcg cca        480
   Ala Pro Ala Phe Val Arg Ala Trp Gly Ser Ala Lys Leu Leu Ala Pro
   145                 150                 155                 160
   ctt agg cac aac cag ctg ggc ctc gta tat gcc gcg gcc gtg caa aac        528
   Leu Arg His Asn Gln Leu Gly Leu Val Tyr Ala Ala Ala Val Gln Asn
                   165                 170                 175
   aac atg gtg gcc aca gcc ttt cac ccc gag ctg acc acc aca gca gtt        576
   Asn Met Val Ala Thr Ala Phe His Pro Glu Leu Thr Thr Thr Ala Val
               180                 185                 190
   cac aag tgg gtt att aac atg gcg ctg ggc agg ttt taa                    615
   His Lys Trp Val Ile Asn Met Ala Leu Gly Arg Phe
           195                 200
<210> 67
   <211> 204
   <212> PRT
   <213> Pyrobaculum aerophilum
<400> 67
   Met Lys Ile Gly Val Leu Ala Leu Gln Gly Asp Val Glu Glu His Ala
   1               5                   10                  15
   Asn Ala Phe Lys Glu Ala Gly Arg Glu Val Gly Val Asp Val Asp Val
               20                  25                  30
   Val Glu Val Lys Lys Pro Gly Asp Leu Lys Asp Ile Lys Ala Leu Ala
           35                  40                  45
   Ile Pro Gly Gly Glu Ser Thr Thr Ile Gly Arg Leu Ala Lys Arg Thr
       50                  55                  60
   Gly Leu Leu Asp Ala Val Lys Lys Ala Ile Glu Gly Gly Val Pro Ala
   65                  70                  75                  80
   Leu Gly Thr Cys Ala Gly Ala Ile Phe Met Ala Lys Glu Val Lys Asp
                   85                  90                  95
   Ala Val Val Gly Ala Thr Gly Gln Pro Val Leu Gly Val Met Asp Ile
               100                 105                 110
   Ala Val Val Arg Asn Ala Phe Gly Arg Gln Arg Glu Ser Phe Glu Ala
           115                 120                 125
   Glu Val Val Leu Glu Asn Leu Gly Lys Leu Lys Ala Val Phe Ile Arg
       130                 135                 140
   Ala Pro Ala Phe Val Arg Ala Trp Gly Ser Ala Lys Leu Leu Ala Pro
   145                 150                 155                 160
   Leu Arg His Asn Gln Leu Gly Leu Val Tyr Ala Ala Ala Val Gln Asn
                   165                 170                 175
   Asn Met Val Ala Thr Ala Phe His Pro Glu Leu Thr Thr Thr Ala Val
               180                 185                 190
   His Lys Trp Val Ile Asn Met Ala Leu Gly Arg Phe
           195                 200
<210> 68
   <211> 816
   <212> DNA
   <213> Emericella nidulans (Aspergillus nidulans)
<400> 68
   atg att aag att act gtc ggt gtt ctc gcc tta caa ggc gcc ttc ctg      48
   Met Ile Lys Ile Thr Val Gly Val Leu Ala Leu Gln Gly Ala Phe Leu
   1               5                   10                  15
   gag cat tta gag ctg ctg aaa aag gca gcg gcc tcg ctg ggc tcg caa      96
   Glu His Leu Glu Leu Leu Lys Lys Ala Ala Ala Ser Leu Gly Ser Gln
               20                  25                  30
   caa tct tcg ccg cag tgg gaa ttt ctt gag atc cgg acc ccg caa gaa     144
   Gln Ser Ser Pro Gln Trp Glu Phe Leu Glu Ile Arg Thr Pro Gln Glu
           35                  40                  45
   ctc aag aga tgc gat gcg ctc gtc ctg cct ggg ggt gaa agt aca gca     192
   Leu Lys Arg Cys Asp Ala Leu Val Leu Pro Gly Gly Glu Ser Thr Ala
       50                  55                  60
   atc tca ttg gtg gca gct cgg tct aat tta ctt gag cct ttg aga gat     240
   Ile Ser Leu Val Ala Ala Arg Ser Asn Leu Leu Glu Pro Leu Arg Asp
   65                  70                  75                  80
   ttt gtg aag gtc cac cgc aaa cca aca tgg gga acc tgc gcc ggg tta     288
   Phe Val Lys Val His Arg Lys Pro Thr Trp Gly Thr Cys Ala Gly Leu
                   85                  90                  95
   ata ttg ctc gcg gaa tcg gcg aac cgg act aaa aaa ggt ggc cag gag     336
   Ile Leu Leu Ala Glu Ser Ala Asn Arg Thr Lys Lys Gly Gly Gln Glu
               100                 105                 110
   ttg atc gga gga tta gat gtt cga gtt aat cgc aac cac ttt ggc cgg     384
   Leu Ile Gly Gly Leu Asp Val Arg Val Asn Arg Asn His Phe Gly Arg
           115                 120                 125
   caa acg gaa agc ttt cag gcg ccg ctt gat ctg ccg ttc ctc agc aca     432
   Gln Thr Glu Ser Phe Gln Ala Pro Leu Asp Leu Pro Phe Leu Ser Thr
       130                 135                 140
   tcc ggt aca ccc cag cag ccc ttt ccg gca gtc ttc att cgt gcg ccg     480
   Ser Gly Thr Pro Gln Gln Pro Phe Pro Ala Val Phe Ile Arg Ala Pro
   145                 150                 155                 160
   gta gtt gag aaa atc ttg ccg cat cac gac ggt att cag gtg gac gaa     528
   Val Val Glu Lys Ile Leu Pro His His Asp Gly Ile Gln Val Asp Glu
                   165                 170                 175
   gct aag aga gtc gag acc gtt gtt gct cct tcg cga caa gcc gag agc     576
   Ala Lys Arg Val Glu Thr Val Val Ala Pro Ser Arg Gln Ala Glu Ser
               180                 185                 190
   gaa gcg tcc cgg agg gca atg tca cgc gac gtt gaa gta ttg gct agt     624
   Glu Ala Ser Arg Arg Ala Met Ser Arg Asp Val Glu Val Leu Ala Ser
           195                 200                 205
   ctt ccc ggg agg gct gcg cat tta gct gtc agt gga aca cct att cgt     672
   Leu Pro Gly Arg Ala Ala His Leu Ala Val Ser Gly Thr Pro Ile Arg
       210                 215                 220
   gcg gat gag gaa act ggt gat att gtt gcc gtg aga caa ggc aac gtc     720
   Ala Asp Glu Glu Thr Gly Asp Ile Val Ala Val Arg Gln Gly Asn Val
   225                 230                 235                 240
   ttt ggt aca agc ttc cac cct gag ttg act ggt gac gaa aga atc cat     768
   Phe Gly Thr Ser Phe His Pro Glu Leu Thr Gly Asp Glu Arg Ile His
                   245                 250                 255
   gcc tgg tgg ctg cgc caa gtg gaa gat tct gta aaa cga ttg caa         813
   Ala Trp Trp Leu Arg Gln Val Glu Asp Ser Val Lys Arg Leu Gln
               260                 265                 270
   tga                                                                 816
<210> 69
   <211> 271
   <212> PRT
   <213> Emericella nidulans (Aspergillus nidulans)
<400> 69
   Met Ile Lys Ile Thr Val Gly Val Leu Ala Leu Gln Gly Ala Phe Leu
   1 5 10 15
   Glu His Leu Glu Leu Leu Lys Lys Ala Ala Ala Ser Leu Gly Ser Gln
   20 25 30
   Gln Ser Ser Pro Gln Trp Glu Phe Leu Glu Ile Arg Thr Pro Gln Glu
   35 40 45
   Leu Lys Arg Cys Asp Ala Leu Val Leu Pro Gly Gly Glu Ser Thr Ala
   50 55 60
   Ile Ser Leu Val Ala Ala Arg Ser Asn Leu Leu Glu Pro Leu Arg Asp
   65 70 75 80
   Phe Val Lys Val His Arg Lys Pro Thr Trp Gly Thr Cys Ala Gly Leu
   85 90 95
   Ile Leu Leu Ala Glu Ser Ala Asn Arg Thr Lys Lys Gly Gly Gln Glu
   100 105 110
   Leu Ile Gly Gly Leu Asp Val Arg Val Asn Arg Asn His Phe Gly Arg
   115 120 125
   Gln Thr Glu Ser Phe Gln Ala Pro Leu Asp Leu Pro Phe Leu Ser Thr
   130 135 140
   Ser Gly Thr Pro Gln Gln Pro Phe Pro Ala Val Phe Ile Arg Ala Pro
   145 150 155 160
   Val Val Glu Lys Ile Leu Pro His His Asp Gly Ile Gln Val Asp Glu
   165 170 175
   Ala Lys Arg Val Glu Thr Val Val Ala Pro Ser Arg Gln Ala Glu Ser
   180 185 190
   Glu Ala Ser Arg Arg Ala Met Ser Arg Asp Val Glu Val Leu Ala Ser
   195 200 205
   Leu Pro Gly Arg Ala Ala His Leu Ala Val Ser Gly Thr Pro Ile Arg
   210 215 220
   Ala Asp Glu Glu Thr Gly Asp Ile Val Ala Val Arg Gln Gly Asn Val
   225 230 235 240
   Phe Gly Thr Ser Phe His Pro Glu Leu Thr Gly Asp Glu Arg Ile His
   245 250 255
   Ala Trp Trp Leu Arg Gln Val Glu Asp Ser Val Lys Arg Leu Gln
   260 265 270
<210> 70
   <211> 603
   <212> DNA
   <213> Sulfolobus tokodaii
<400> 70
   atg aaa att gga att gtt gca tat caa ggt agc ttt gaa gaa cat gcg        48
   Met Lys Ile Gly Ile Val Ala Tyr Gln Gly Ser Phe Glu Glu His Ala
   1               5                   10                  15
   tta cag act aaa aga gct ttg gac aat ttg aaa att caa gga gat ata        96
   Leu Gln Thr Lys Arg Ala Leu Asp Asn Leu Lys Ile Gln Gly Asp Ile
               20                  25                  30
   gtt gct gtg aaa aaa cct aat gat ttg aaa gat gtt gat gct ata ata       144
   Val Ala Val Lys Lys Pro Asn Asp Leu Lys Asp Val Asp Ala Ile Ile
           35                  40                  45
   ata cct ggc gga gag agt aca acc att ggc gtt gtt gct caa aaa ctt       192
   Ile Pro Gly Gly Glu Ser Thr Thr Ile Gly Val Val Ala Gln Lys Leu
       50                  55                  60
   ggt att tta gat gaa tta aaa gag aaa ata aat tct ggg ata cca act       240
   Gly Ile Leu Asp Glu Leu Lys Glu Lys Ile Asn Ser Gly Ile Pro Thr
   65                  70                  75                  80
   tta ggt act tgt gct gga gca ata att tta gca aaa gat gtt aca gac       288
   Leu Gly Thr Cys Ala Gly Ala Ile Ile Leu Ala Lys Asp Val Thr Asp
                   85                  90                  95
   gcc aaa gtc ggt aaa aaa tct cag ccg tta att ggt tca atg gat att       336
   Ala Lys Val Gly Lys Lys Ser Gln Pro Leu Ile Gly Ser Met Asp Ile
               100                 105                 110
   tct gtg att aga aac tat tat ggt aga caa aga gaa agt ttt gaa gca       384
   Ser Val Ile Arg Asn Tyr Tyr Gly Arg Gln Arg Glu Ser Phe Glu Ala
           115                 120                 125
   act gtt gat tta tca gaa ata ggg gga gga aag act aga gtt gtg ttt       432
   Thr Val Asp Leu Ser Glu Ile Gly Gly Gly Lys Thr Arg Val Val Phe
       130                 135                 140
   ata aga gct cct gct ata gtc aaa aca tgg gga gat gca aag cca tta       480
   Ile Arg Ala Pro Ala Ile Val Lys Thr Trp Gly Asp Ala Lys Pro Leu
   145                 150                 155                 160
   tca aaa ctt aat gat gta ata att atg gct atg gag aga aat atg gtt       528
   Ser Lys Leu Asn Asp Val Ile Ile Met Ala Met Glu Arg Asn Met Val
                   165                 170                 175
   gct aca aca ttt cat cca gag tta tct tca act act gta att cac gag       576
   Ala Thr Thr Phe His Pro Glu Leu Ser Ser Thr Thr Val Ile His Glu
               180                 185                 190
   ttt ctc att aaa atg gca aag aaa tag                                   603
   Phe Leu Ile Lys Met Ala Lys Lys
           195                 200
<210> 71
   <211> 200
   <212> PRT
   <213> Sulfolobus tokodaii
<400> 71
   Met Lys Ile Gly Ile Val Ala Tyr Gln Gly Ser Phe Glu Glu His Ala
   1               5                   10                  15
   Leu Gln Thr Lys Arg Ala Leu Asp Asn Leu Lys Ile Gln Gly Asp Ile
               20                  25                  30
   Val Ala Val Lys Lys Pro Asn Asp Leu Lys Asp Val Asp Ala Ile Ile
           35                  40                  45
   Ile Pro Gly Gly Glu Ser Thr Thr Ile Gly Val Val Ala Gln Lys Leu
       50                  55                  60
   Gly Ile Leu Asp Glu Leu Lys Glu Lys Ile Asn Ser Gly Ile Pro Thr
   65                  70                  75                  80
   Leu Gly Thr Cys Ala Gly Ala Ile Ile Leu Ala Lys Asp Val Thr Asp
                   85                  90                  95
   Ala Lys Val Gly Lys Lys Ser Gln Pro Leu Ile Gly Ser Met Asp Ile
               100                 105                 110
   Ser Val Ile Arg Asn Tyr Tyr Gly Arg Gln Arg Glu Ser Phe Glu Ala
           115                 120                 125
   Thr Val Asp Leu Ser Glu Ile Gly Gly Gly Lys Thr Arg Val Val Phe
       130                 135                 140
   Ile Arg Ala Pro Ala Ile Val Lys Thr Trp Gly Asp Ala Lys Pro Leu
   145                 150                 155                 160
   Ser Lys Leu Asn Asp Val Ile Ile Met Ala Met Glu Arg Asn Met Val
                   165                 170                 175
   Ala Thr Thr Phe His Pro Glu Leu Ser Ser Thr Thr Val Ile His Glu
               180                 185                 190
   Phe Leu Ile Lys Met Ala Lys Lys
           195                 200
<210> 72
   <211> 600
   <212> DNA
   <213> Thermoplasma volcanium
<400> 72
   atg aat gta ggc atc ata ggt ttt caa gga gac gtg gaa gaa cat att      48
   Met Asn Val Gly Ile Ile Gly Phe Gln Gly Asp Val Glu Glu His Ile
   1               5                   10                  15
   gca ata gta aag aag att tcc cgc aga aga aaa gga ata aac gtt tta      96
   Ala Ile Val Lys Lys Ile Ser Arg Arg Arg Lys Gly Ile Asn Val Leu
               20                  25                  30
   cgc att aga aga aag gaa gat ctc gat agg tca gat tcg cta ata att     144
   Arg Ile Arg Arg Lys Glu Asp Leu Asp Arg Ser Asp Ser Leu Ile Ile
           35                  40                  45
   cct ggc ggc gaa agc aca act ata tac aaa cta atc tca gaa tac gga     192
   Pro Gly Gly Glu Ser Thr Thr Ile Tyr Lys Leu Ile Ser Glu Tyr Gly
       50                  55                  60
   ata tac gat gaa ata att aga cgt gca aag gaa ggt atg cct gtc atg     240
   Ile Tyr Asp Glu Ile Ile Arg Arg Ala Lys Glu Gly Met Pro Val Met
   65                  70                  75                  80
   gca act tgc gcc ggc cta ata ctt att tcc aaa gac acc aat gac gat     288
   Ala Thr Cys Ala Gly Leu Ile Leu Ile Ser Lys Asp Thr Asn Asp Asp
                   85                  90                  95
   agg gtt cca gga atg aac ctt ctc gac gta aca ata atg agg aac gct     336
   Arg Val Pro Gly Met Asn Leu Leu Asp Val Thr Ile Met Arg Asn Ala
               100                 105                 110
   tac ggg agg caa gtc aac tca ttc gaa aca gat ata gat ata aag ggc     384
   Tyr Gly Arg Gln Val Asn Ser Phe Glu Thr Asp Ile Asp Ile Lys Gly
           115                 120                 125
   ata ggt act ttt cat gca gta ttc att aga gct cct agg ata aaa gaa     432
   Ile Gly Thr Phe His Ala Val Phe Ile Arg Ala Pro Arg Ile Lys Glu
       130                 135                 140
   tat ggt aac gta gat gtt atg gct agc ctt gat gga tat cct gtc atg     480
   Tyr Gly Asn Val Asp Val Met Ala Ser Leu Asp Gly Tyr Pro Val Met
   145                 150                 155                 160
   gta aga tca gga aat ata tta ggt atg aca ttt cat cca gaa ctc aca      528
   Val Arg Ser Gly Asn Ile Leu Gly Met Thr Phe His Pro Glu Leu Thr
                   165                 170                 175
   gga gat gta agt ata cat gaa tat ttt ctt agc atg ggg gga ggg ggg      576
   Gly Asp Val Ser Ile His Glu Tyr Phe Leu Ser Met Gly Gly Gly Gly
               180                 185                 190
   tac att tcc act gca aca ggt tag                                      600
   Tyr Ile Ser Thr Ala Thr Gly
           195
<210> 73
   <211> 199
   <212> PRT
   <213> Thermoplasma volcanium
<400> 73
   Met Asn Val Gly Ile Ile Gly Phe Gln Gly Asp Val Glu Glu His Ile
   1               5                   10                  15
   Ala Ile Val Lys Lys Ile Ser Arg Arg Arg Lys Gly Ile Asn Val Leu
               20                  25                  30
   Arg Ile Arg Arg Lys Glu Asp Leu Asp Arg Ser Asp Ser Leu Ile Ile
           35                  40                  45
   Pro Gly Gly Glu Ser Thr Thr Ile Tyr Lys Leu Ile Ser Glu Tyr Gly
       50                  55                  60
   Ile Tyr Asp Glu Ile Ile Arg Arg Ala Lys Glu Gly Met Pro Val Met
   65                  70                  75                  80
   Ala Thr Cys Ala Gly Leu Ile Leu Ile Ser Lys Asp Thr Asn Asp Asp
                   85                  90                  95
   Arg Val Pro Gly Met Asn Leu Leu Asp Val Thr Ile Met Arg Asn Ala
               100                 105                 110
   Tyr Gly Arg Gln Val Asn Ser Phe Glu Thr Asp Ile Asp Ile Lys Gly
           115                 120                 125
   Ile Gly Thr Phe His Ala Val Phe Ile Arg Ala Pro Arg Ile Lys Glu
       130                 135                 140
   Tyr Gly Asn Val Asp Val Met Ala Ser Leu Asp Gly Tyr Pro Val Met
   145                 150                 155                 160
   Val Arg Ser Gly Asn Ile Leu Gly Met Thr Phe His Pro Glu Leu Thr
                   165                 170                 175
   Gly Asp Val Ser Ile His Glu Tyr Phe Leu Ser Met Gly Gly Gly Gly
               180                 185                 190
   Tyr Ile Ser Thr Ala Thr Gly
           195
<210> 74
   <211> 759
   <212> DNA
   <213> Neurospora crassa
<400> 74
   atg acc gtc gac gcc gta aac ccc caa caa ata aca gtc ggc gtc cta      48
   Met Thr Val Asp Ala Val Asn Pro Gln Gln Ile Thr Val Gly Val Leu
   1               5                   10                  15
   gcc ctc caa ggc ggc gtg atc gag cac atc tcc ctt ctc caa aag gca      96
   Ala Leu Gln Gly Gly Val Ile Glu His Ile Ser Leu Leu Gln Lys Ala
               20                  25                  30
   gct gcc caa cta tcg tca caa tcc tcg aca cca aca cca caa ttc agc      144
   Ala Ala Gln Leu Ser Ser Gln Ser Ser Thr Pro Thr Pro Gln Phe Ser
           35                  40                  45
   ttc atc caa gtc cgt acc gcc gcc caa ctc tcg caa tgc gac gct ctc      192
   Phe Ile Gln Val Arg Thr Ala Ala Gln Leu Ser Gln Cys Asp Ala Leu
       50                  55                  60
   att atc ccg gga gga gaa agc aca acc atg gct atc gtt gcc aga cgc      240
   Ile Ile Pro Gly Gly Glu Ser Thr Thr Met Ala Ile Val Ala Arg Arg
   65                  70                  75                  80
   ctg gga ttg ctt gat ccg cta cgg gaa ttc gtc aaa gtc caa cac aaa      288
   Leu Gly Leu Leu Asp Pro Leu Arg Glu Phe Val Lys Val Gln His Lys
                   85                  90                  95
   cca aca tgg ggc acc tgc gcc ggc cta gtc atg ctc gcc tcc gcc gcc      336
   Pro Thr Trp Gly Thr Cys Ala Gly Leu Val Met Leu Ala Ser Ala Ala
               100                 105                 110
   tca gca acc aaa caa ggc gga caa gaa ctc atc ggt ggg ctg gac gtc      384
   Ser Ala Thr Lys Gln Gly Gly Gln Glu Leu Ile Gly Gly Leu Asp Val
           115                 120                 125
   aaa gtc ctc aga aac cgc tac ggc aca cag ctc cag agt ttt gtg gga      432
   Lys Val Leu Arg Asn Arg Tyr Gly Thr Gln Leu Gln Ser Phe Val Gly
       130                 135                 140
   gat ttg cgg ttg cct ttt ctg gaa gaa ggg gaa ccc ttc agg gga gta      480
   Asp Leu Arg Leu Pro Phe Leu Glu Glu Gly Glu Pro Phe Arg Gly Val
   145                 150                 155                 160
   ttt atc cgc gca ccg gtt gtg gag gag att atc acc acc acc gct ggg      528
   Phe Ile Arg Ala Pro Val Val Glu Glu Ile Ile Thr Thr Thr Ala Gly
                   165                 170                 175
   gat gat gag gtt acc aag cta aag gga aat ttg gtg gag gta atg ggg      576
   Asp Asp Glu Val Thr Lys Leu Lys Gly Asn Leu Val Glu Val Met Gly
               180                 185                 190
   act tac cca aag cca caa ggg aca gga gaa gga gac gac att gtt gcc      624
   Thr Tyr Pro Lys Pro Gln Gly Thr Gly Glu Gly Asp Asp Ile Val Ala
           195                 200                 205
   gtg cgg cag ggc aac gtt ttc gga acg agt ttc cac ccc gaa cta acg      672
   Val Arg Gln Gly Asn Val Phe Gly Thr Ser Phe His Pro Glu Leu Thr
       210                 215                 220
   gat gat gtc agg ata cat acc tgg tgg ttg aag caa gtt gtt gag ggg      720
   Asp Asp Val Arg Ile His Thr Trp Trp Leu Lys Gln Val Val Glu Gly
   225                 230                 235                 240
   ctg aag tca ggg gga agg gat gtc cag gct cag tcg taa                  759
   Leu Lys Ser Gly Gly Arg Asp Val Gln Ala Gln Ser
                   245                 250
<210> 75
   <211> 252
   <212> PRT
   <213> Neurospora crassa
<400> 75
   Met Thr Val Asp Ala Val Asn Pro Gln Gln Ile Thr Val Gly Val Leu
   1               5                   10                  15
   Ala Leu Gln Gly Gly Val Ile Glu His Ile Ser Leu Leu Gln Lys Ala
               20                  25                  30
   Ala Ala Gln Leu Ser Ser Gln Ser Ser Thr Pro Thr Pro Gln Phe Ser
           35                  40                  45
   Phe Ile Gln Val Arg Thr Ala Ala Gln Leu Ser Gln Cys Asp Ala Leu
       50                  55                  60
   Ile Ile Pro Gly Gly Glu Ser Thr Thr Met Ala Ile Val Ala Arg Arg
   65                  70                  75                  80
   Leu Gly Leu Leu Asp Pro Leu Arg Glu Phe Val Lys Val Gln His Lys
                   85                  90                  95
   Pro Thr Trp Gly Thr Cys Ala Gly Leu Val Met Leu Ala Ser Ala Ala
               100                 105                 110
   Ser Ala Thr Lys Gln Gly Gly Gln Glu Leu Ile Gly Gly Leu Asp Val
           115                 120                 125
   Lys Val Leu Arg Asn Arg Tyr Gly Thr Gln Leu Gln Ser Phe Val Gly
       130                 135                 140
   Asp Leu Arg Leu Pro Phe Leu Glu Glu Gly Glu Pro Phe Arg Gly Val
   145                 150                 155                 160
   Phe Ile Arg Ala Pro Val Val Glu Glu Ile Ile Thr Thr Thr Ala Gly
                   165                 170                 175
   Asp Asp Glu Val Thr Lys Leu Lys Gly Asn Leu Val Glu Val Met Gly
               180                 185                 190
   Thr Tyr Pro Lys Pro Gln Gly Thr Gly Glu Gly Asp Asp Ile Val Ala
           195                 200                 205
   Val Arg Gln Gly Asn Val Phe Gly Thr Ser Phe His Pro Glu Leu Thr
       210                 215                 220
   Asp Asp Val Arg Ile His Thr Trp Trp Leu Lys Gln Val Val Glu Gly
   225                 230                 235                 240
   Leu Lys Ser Gly Gly Arg Asp Val Gln Ala Gln Ser
                   245                 250
<210> 76
   <211> 582
   <212> DNA
   <213> Pasteurella multocida
<400> 76
   atg aaa gac tat tca cat tta cac att ggc gtg tta gct ctg cag gga        48
   Met Lys Asp Tyr Ser His Leu His Ile Gly Val Leu Ala Leu Gln Gly
   1               5                   10                  15
   gca gta agc gaa cat ttg cgc caa att gaa caa ctt ggt gcc aac gcc        96
   Ala Val Ser Glu His Leu Arg Gln Ile Glu Gln Leu Gly Ala Asn Ala
               20                  25                  30
   agt gca atc aaa acc gtc tca gaa ttg acc gca ctt gat ggt tta gtg        144
   Ser Ala Ile Lys Thr Val Ser Glu Leu Thr Ala Leu Asp Gly Leu Val
           35                  40                  45
   ctc ccg ggc ggt gaa agc acg acc att ggc aga tta atg cgt caa tat        192
   Leu Pro Gly Gly Glu Ser Thr Thr Ile Gly Arg Leu Met Arg Gln Tyr
       50                  55                  60
   ggg ttt att gag gca att caa gat gtt gcc aaa caa ggt aaa ggt att        240
   Gly Phe Ile Glu Ala Ile Gln Asp Val Ala Lys Gln Gly Lys Gly Ile
   65                  70                  75                  80
   ttc ggc acc tgt gcc ggc atg att tta ctc gca aag caa tta gaa aat        288
   Phe Gly Thr Cys Ala Gly Met Ile Leu Leu Ala Lys Gln Leu Glu Asn
                   85                  90                  95
   gat cct acg gtg cat tta ggt tta atg gac atc tgt gtg caa cgc aac        336
   Asp Pro Thr Val His Leu Gly Leu Met Asp Ile Cys Val Gln Arg Asn
               100                 105                 110
   gcc ttt ggg cga caa gtg gat agc ttt caa acc gcc ctt gaa att gaa        384
   Ala Phe Gly Arg Gln Val Asp Ser Phe Gln Thr Ala Leu Glu Ile Glu
           115                 120                 125
   ggc ttt gct aca acg ttt cct gca gtt ttt atc cgt gca cca cat att        432
   Gly Phe Ala Thr Thr Phe Pro Ala Val Phe Ile Arg Ala Pro His Ile
       130                 135                 140
   gct caa gtc aat cat gaa aaa gtg caa tgt cta gcg act ttt cag ggg        480
   Ala Gln Val Asn His Glu Lys Val Gln Cys Leu Ala Thr Phe Gln Gly
   145                 150                 155                 160
   cat gtt gtc ctc gcg aaa caa caa aat ttg ttg gct tgt gcc ttt cac        528
   His Val Val Leu Ala Lys Gln Gln Asn Leu Leu Ala Cys Ala Phe His
                   165                 170                 175
   cca gaa ctg acg aca gat ctg cgc gtc atg caa cac ttt tta gaa atg        576
   Pro Glu Leu Thr Thr Asp Leu Arg Val Met Gln His Phe Leu Glu Met
               180                 185                 190
   tgt tag                                                                582
   Cys
<210> 77
   <211> 193
   <212> PRT
   <213> Pasteurella multocida
<400> 77
   Met Lys Asp Tyr Ser His Leu His Ile Gly Val Leu Ala Leu Gln Gly
   1               5                   10                  15
   Ala Val Ser Glu His Leu Arg Gln Ile Glu Gln Leu Gly Ala Asn Ala
               20                  25                  30
   Ser Ala Ile Lys Thr Val Ser Glu Leu Thr Ala Leu Asp Gly Leu Val
           35                  40                  45
   Leu Pro Gly Gly Glu Ser Thr Thr Ile Gly Arg Leu Met Arg Gln Tyr
       50                  55                  60
   Gly Phe Ile Glu Ala Ile Gln Asp Val Ala Lys Gln Gly Lys Gly Ile
   65                  70                  75                  80
   Phe Gly Thr Cys Ala Gly Met Ile Leu Leu Ala Lys Gln Leu Glu Asn
                   85                  90                  95
   Asp Pro Thr Val His Leu Gly Leu Met Asp Ile Cys Val Gln Arg Asn
                       100                     105                     110
   Ala Phe Gly Arg Gln Val Asp Ser Phe Gln Thr Ala Leu Glu Ile Glu
               115                     120                     125
   Gly Phe Ala Thr Thr Phe Pro Ala Val Phe Ile Arg Ala Pro His Ile
       130                     135                     140
   Ala Gln Val Asn His Glu Lys Val Gln Cys Leu Ala Thr Phe Gln Gly
   145                     150                     155                     160
   His Val Val Leu Ala Lys Gln Gln Asn Leu Leu Ala Cys Ala Phe His
                   165                     170                     175
   Pro Glu Leu Thr Thr Asp Leu Arg Val Met Gln His Phe Leu Glu Met
               180                     185                     190
   Cys
<210> 78
   <211> 723
   <212> DNA
   <213> Arabidopsis thaliana (Mouse-ear cress)
<400> 78
   atg acc gtc gga gtt tta gct ttg caa ggt tct ttc aat gag cac atc      48
   Met Thr Val Gly Val Leu Ala Leu Gln Gly Ser Phe Asn Glu His Ile
   1               5                   10                  15
   gcg gct ctg cgg cgg ctc ggt gtc caa ggc gtc gag att agg aag gct      96
   Ala Ala Leu Arg Arg Leu Gly Val Gln Gly Val Glu Ile Arg Lys Ala
               20                  25                  30
   gac cag ctt ctc acc gtt tct tct ctt atc att cct ggc ggc gag agc      144
   Asp Gln Leu Leu Thr Val Ser Ser Leu Ile Ile Pro Gly Gly Glu Ser
           35                  40                  45
   acc acc atg gcc aaa ctc gcc gag tat cat aac ttg ttt ccg gct cta      192
   Thr Thr Met Ala Lys Leu Ala Glu Tyr His Asn Leu Phe Pro Ala Leu
       50                  55                  60
   cgt gag ttt gtt aag atg ggg aaa cct gtt tgg ggg aca tgc gca ggt      240
   Arg Glu Phe Val Lys Met Gly Lys Pro Val Trp Gly Thr Cys Ala Gly
   65                  70                  75                  80
   ctt ata ttc ttg gca gac aga gca gtt gag gga ggt cag gaa tta gtt      288
   Leu Ile Phe Leu Ala Asp Arg Ala Val Glu Gly Gly Gln Glu Leu Val
                   85                  90                  95
   ggt ggc ctt gat tgc acc gta cat agg aac ttc ttc ggt agc cag att      336
   Gly Gly Leu Asp Cys Thr Val His Arg Asn Phe Phe Gly Ser Gln Ile
               100                 105                 110
   caa agt ttt gaa gct gat atc tta gta cct caa cta aca tct caa gaa      384
   Gln Ser Phe Glu Ala Asp Ile Leu Val Pro Gln Leu Thr Ser Gln Glu
           115                 120                 125
   ggt ggg cca gag aca tac agg gga gtg ttc ata cgt gct cca gct gtt      432
   Gly Gly Pro Glu Thr Tyr Arg Gly Val Phe Ile Arg Ala Pro Ala Val
       130                 135                 140
   ctt gat gta ggt cct gat gtc gaa gtc ctg gcg gat tat ccc gtc cca      480
   Leu Asp Val Gly Pro Asp Val Glu Val Leu Ala Asp Tyr Pro Val Pro
   145                 150                 155                 160
   tca aac aag gaa gat gct ctt cct gaa aca aaa gtc att gtt gct gtg      528
   Ser Asn Lys Glu Asp Ala Leu Pro Glu Thr Lys Val Ile Val Ala Val
                   165                 170                 175
   aag caa gga aac ttg tta gca act gct ttt cat ccc gag ctt act gca      576
   Lys Gln Gly Asn Leu Leu Ala Thr Ala Phe His Pro Glu Leu Thr Ala
               180                 185                 190
   gac act cga tgg cac agt tat ttc ata aag atg acg aaa gag att gag      624
   Asp Thr Arg Trp His Ser Tyr Phe Ile Lys Met Thr Lys Glu Ile Glu
           195                 200                 205
   caa gga gct tct tca agc agt agt aag act att gta tct gtt gga gaa      672
   Gln Gly Ala Ser Ser Ser Ser Ser Lys Thr Ile Val Ser Val Gly Glu
       210                 215                 220
   aca agt gct ggt ccc gag cca gct aag cct gat ctt cct ata ttt caa      720
   Thr Ser Ala Gly Pro Glu Pro Ala Lys Pro Asp Leu Pro Ile Phe Gln
   225                 230                 235                 240
   taa                                                                  723
<210> 79
   <211> 240
   <212> PRT
   <213> Arabidopsis thaliana (Mouse-ear cress)
<400> 79
   Met Thr Val Gly Val Leu Ala Leu Gln Gly Ser Phe Asn Glu His Ile
   1               5                   10                  15
   Ala Ala Leu Arg Arg Leu Gly Val Gln Gly Val Glu Ile Arg Lys Ala
               20                  25                  30
   Asp Gln Leu Leu Thr Val Ser Ser Leu Ile Ile Pro Gly Gly Glu Ser
           35                  40                  45
   Thr Thr Met Ala Lys Leu Ala Glu Tyr His Asn Leu Phe Pro Ala Leu
       50                  55                  60
   Arg Glu Phe Val Lys Met Gly Lys Pro Val Trp Gly Thr Cys Ala Gly
   65                  70                  75                  80
   Leu Ile Phe Leu Ala Asp Arg Ala Val Glu Gly Gly Gln Glu Leu Val
                   85                  90                  95
   Gly Gly Leu Asp Cys Thr Val His Arg Asn Phe Phe Gly Ser Gln Ile
               100                 105                 110
   Gln Ser Phe Glu Ala Asp Ile Leu Val Pro Gln Leu Thr Ser Gln Glu
           115                 120                 125
   Gly Gly Pro Glu Thr Tyr Arg Gly Val Phe Ile Arg Ala Pro Ala Val
       130                 135                 140
   Leu Asp Val Gly Pro Asp Val Glu Val Leu Ala Asp Tyr Pro Val Pro
   145                 150                 155                 160
   Ser Asn Lys Glu Asp Ala Leu Pro Glu Thr Lys Val Ile Val Ala Val
                   165                 170                 175
   Lys Gln Gly Asn Leu Leu Ala Thr Ala Phe His Pro Glu Leu Thr Ala
               180                 185                 190
   Asp Thr Arg Trp His Ser Tyr Phe Ile Lys Met Thr Lys Glu Ile Glu
           195                 200                 205
   Gln Gly Ala Ser Ser Ser Ser Ser Lys Thr Ile Val Ser Val Gly Glu
       210                 215                 220
   Thr Ser Ala Gly Pro Glu Pro Ala Lys Pro Asp Leu Pro Ile Phe Gln
   225                 230                 235                 240
<210> 80
   <211> 1574
   <212> DNA
   <213> Cercospora nicotianae
<400> 80
   ggcaatcaat gcagcgtgca caactacgct gtgcttggtg cgccgccggt catcgattct     60
   ggagtcccga aaacgtgatc ggcgcagcat tcccgaatcc tgtctctctt catcctcaca    120
   attcctcttc cagcacgccg ccagccagat gcacgcggtc gtgacgatgt tggtgtgacg    180
   ggactgcctc atgcatcgcc cgcctggtcg atagtaggca tcacagaatg cgagcagaga    240
   acatgtgtcg aagaatcatg cccgttcagc atccgatcga gtgtgtagaa cccactttcc    300
   tcagctgtcc tattcctccg tctgcgcgtc atttgtgcat ctctcctcct ccaccaagac    360
   gccatcgaca atgacttcgc gccctatcgg accaaaccgc tgcgagtcca tctctgtagc    420
   gaccattttc gtgactcact cccgcggcca agcgagcagc attccgttct agtaccctca    480
   catcgcaccc gccaatgcac attcccggcg acacgaccac acc atg aca ggc          532
                                                   Met Thr Gly
                                                   1
   tcc cac tcc tcc cac tcc ctc acc gtc ggc gtg ctg gcc ctc caa ggc      580
   Ser His Ser Ser His Ser Leu Thr Val Gly Val Leu Ala Leu Gln Gly
       5                   10                  15
   gcc ttc atc gag cac atc acc ctc ctc cga caa gcc gcg ccg gca ctg      628
   Ala Phe Ile Glu His Ile Thr Leu Leu Arg Gln Ala Ala Pro Ala Leu
   20                  25                  30                  35
   act gcc ggg tac gga gtc cac ttc acc ttc att gag gtc agg acg ccc      676
   Thr Ala Gly Tyr Gly Val His Phe Thr Phe Ile Glu Val Arg Thr Pro
                   40                  45                  50
   gaa cag ctg gac cga tgc gac gct ctc atc ctg ccc gga ggc gag agc      724
   Glu Gln Leu Asp Arg Cys Asp Ala Leu Ile Leu Pro Gly Gly Glu Ser
               55                  60                  65
   acc gcc atc tcg ctc atc gcc gaa cgc tgc ggc ctg ctc gaa ccg ctg      772
   Thr Ala Ile Ser Leu Ile Ala Glu Arg Cys Gly Leu Leu Glu Pro Leu
           70                  75                  80
   cga aac ttt gtc aaa tgg caa cgt cgt ccc aca tgg gga aca tgc gcg      820
   Arg Asn Phe Val Lys Trp Gln Arg Arg Pro Thr Trp Gly Thr Cys Ala
       85                  90                  95
   ggg ctc att ttg ctg gct gag gaa gcg aac aag agc aag gcg aca ggg      868
   Gly Leu Ile Leu Leu Ala Glu Glu Ala Asn Lys Ser Lys Ala Thr Gly
   100                 105                 110                 115
   caa gag ttg atc gga ggt ctg gac gtg cgg gtt cag cgt aat tac ttt      916
   Gln Glu Leu Ile Gly Gly Leu Asp Val Arg Val Gln Arg Asn Tyr Phe
                   120                 125                 130
   ggc cga caa gtc gag tct ttc gaa gca gcg ctg caa ctg ccc ttc ctc      964
   Gly Arg Gln Val Glu Ser Phe Glu Ala Ala Leu Gln Leu Pro Phe Leu
               135                 140                 145
   gga ccc gat ccc ttc cac tcc gta ttc atc cgc gca cca gtg gta gag      1012
   Gly Pro Asp Pro Phe His Ser Val Phe Ile Arg Ala Pro Val Val Glu
           150                 155                 160
   aac att ctg gcg tcg tcc gcc aaa gat gtc acg acg gag att gta gag      1060
   Asn Ile Leu Ala Ser Ser Ala Lys Asp Val Thr Thr Glu Ile Val Glu
       165                 170                 175
   aag agt gcc ggc gaa agc aag gca gtt cga ccc agc atg ccc aac cga      1108
   Lys Ser Ala Gly Glu Ser Lys Ala Val Arg Pro Ser Met Pro Asn Arg
   180                 185                 190                 195
   gca gac acc atc tct gcc cca cag ata aag gcg acc tca gca ccg gta      1156
   Ala Asp Thr Ile Ser Ala Pro Gln Ile Lys Ala Thr Ser Ala Pro Val
                   200                 205                 210
   gag atc ctg ggg cga ctg ccc gga agg gca aag gcg atc aaa gac aag      1204
   Glu Ile Leu Gly Arg Leu Pro Gly Arg Ala Lys Ala Ile Lys Asp Lys
               215                 220                 225
   acg agc acg gcg gaa gag ctg gga gag gag ggc gat att gtc gct gtg      1252
   Thr Ser Thr Ala Glu Glu Leu Gly Glu Glu Gly Asp Ile Val Ala Val
           230                 235                 240
   aag cag ggc aac gtt ttt ggc aca tcc ttc cac ccc gag ttg acc ggc      1300
   Lys Gln Gly Asn Val Phe Gly Thr Ser Phe His Pro Glu Leu Thr Gly
       245                 250                 255
   gat gac aga ata cac gcc tgg tgg ttg agg gaa gtc atc aag agc aag      1348
   Asp Asp Arg Ile His Ala Trp Trp Leu Arg Glu Val Ile Lys Ser Lys
   260                 265                 270                 275
   cag gcc act tgaacaaatg cgggacaacg catgctcatg aacaaaatac aacgcgggag   1407
   Gln Ala Thr
   acgccaagtc tgtggacatg gtgaacccac agaacgatcc ctctgctgga atggactctt    1467
   tccttccaac ctgcctgcaa cccctgcctc gaaacaaggg acacccctcc tcctcctctc    1527
   acactgctca cccctggtac cggcatcgag ttcggcgtgt tcggcag                  1574
<210> 81
   <211> 278
   <212> PRT
   <213> Cercospora nicotianae
<400> 81
   Met Thr Gly Ser His Ser Ser His Ser Leu Thr Val Gly Val Leu Ala
   1               5                   10                  15
   Leu Gln Gly Ala Phe Ile Glu His Ile Thr Leu Leu Arg Gln Ala Ala
               20                  25                  30
   Pro Ala Leu Thr Ala Gly Tyr Gly Val His Phe Thr Phe Ile Glu Val
           35                  40                  45
   Arg Thr Pro Glu Gln Leu Asp Arg Cys Asp Ala Leu Ile Leu Pro Gly
       50                  55                  60
   Gly Glu Ser Thr Ala Ile Ser Leu Ile Ala Glu Arg Cys Gly Leu Leu
   65                  70                  75                  80
   Glu Pro Leu Arg Asn Phe Val Lys Trp Gln Arg Arg Pro Thr Trp Gly
                   85                  90                  95
   Thr Cys Ala Gly Leu Ile Leu Leu Ala Glu Glu Ala Asn Lys Ser Lys
               100                 105                 110
   Ala Thr Gly Gln Glu Leu Ile Gly Gly Leu Asp Val Arg Val Gln Arg
           115                 120                 125
   Asn Tyr Phe Gly Arg Gln Val Glu Ser Phe Glu Ala Ala Leu Gln Leu
       130                 135                 140
   Pro Phe Leu Gly Pro Asp Pro Phe His Ser Val Phe Ile Arg Ala Pro
   145                 150                 155                 160
   Val Val Glu Asn Ile Leu Ala Ser Ser Ala Lys Asp Val Thr Thr Glu
                   165                 170                 175
   Ile Val Glu Lys Ser Ala Gly Glu Ser Lys Ala Val Arg Pro Ser Met
               180                 185                 190
   Pro Asn Arg Ala Asp Thr Ile Ser Ala Pro Gln Ile Lys Ala Thr Ser
           195                 200                 205
   Ala Pro Val Glu Ile Leu Gly Arg Leu Pro Gly Arg Ala Lys Ala Ile
       210                 215                 220
   Lys Asp Lys Thr Ser Thr Ala Glu Glu Leu Gly Glu Glu Gly Asp Ile
   225                 230                 235                 240
   Val Ala Val Lys Gln Gly Asn Val Phe Gly Thr Ser Phe His Pro Glu
                   245                 250                 255
   Leu Thr Gly Asp Asp Arg Ile His Ala Trp Trp Leu Arg Glu Val Ile
               260                 265                 270
   Lys Ser Lys Gln Ala Thr
           275
<210> 82
   <211> 612
   <212> DNA
   <213> Thermoplasma acidophilum
<400> 82
   atg aac att gga gtt ctt ggc ttt cag gga gat gtg cag gaa cac atg      48
   Met Asn Ile Gly Val Leu Gly Phe Gln Gly Asp Val Gln Glu His Met
   1               5                   10                  15
   gat atg ctg aaa aaa tta tcc aga aag aac aga gac ctt aca tta acc      96
   Asp Met Leu Lys Lys Leu Ser Arg Lys Asn Arg Asp Leu Thr Leu Thr
               20                  25                  30
   cac gta aaa agg gtt atc gat ctg gaa cac gta gat gcg ctc ata ata     144
   His Val Lys Arg Val Ile Asp Leu Glu His Val Asp Ala Leu Ile Ile
           35                  40                  45
   cct gga gga gaa agt acg act ata tac aag ctt act ctg gaa tac ggc     192
   Pro Gly Gly Glu Ser Thr Thr Ile Tyr Lys Leu Thr Leu Glu Tyr Gly
       50                  55                  60
   ctt tac gac gcc ata gtg aag aga tct gcc gaa ggt atg ccg att atg     240
   Leu Tyr Asp Ala Ile Val Lys Arg Ser Ala Glu Gly Met Pro Ile Met
   65                  70                  75                  80
   gcc aca tgc gcc ggc ctg ata ctc gta tcg aag aat aca aat gat gaa     288
   Ala Thr Cys Ala Gly Leu Ile Leu Val Ser Lys Asn Thr Asn Asp Glu
                   85                  90                  95
   agg gtc aga ggt atg ggc cta ctg gat gtg acc ata aga agg aat gcc     336
   Arg Val Arg Gly Met Gly Leu Leu Asp Val Thr Ile Arg Arg Asn Ala
               100                 105                 110
   tat gga aga cag gtc atg tcc ttc gaa acg gac ata gaa ata aat gga     384
   Tyr Gly Arg Gln Val Met Ser Phe Glu Thr Asp Ile Glu Ile Asn Gly
           115                 120                 125
   atc ggc atg ttt ccg gcc gta ttc ata agg gct ccg gta ata gag gat     432
   Ile Gly Met Phe Pro Ala Val Phe Ile Arg Ala Pro Val Ile Glu Asp
       130                 135                 140
   tct gga aaa acc gag gtt ctt ggt acg ctg gat gga aag ccc gtt atc       480
   Ser Gly Lys Thr Glu Val Leu Gly Thr Leu Asp Gly Lys Pro Val Ile
   145                 150                 155                 160
   gtc aaa cag ggg aat gtg ata ggg atg aca ttt cat cca gag ctc acc       528
   Val Lys Gln Gly Asn Val Ile Gly Met Thr Phe His Pro Glu Leu Thr
                   165                 170                 175
   ggc gat aca agg ctg cat gaa tac ttc ata aac atg gtg agg ggg aga       576
   Gly Asp Thr Arg Leu His Glu Tyr Phe Ile Asn Met Val Arg Gly Arg
               180                 185                 190
   ggg ggg tac att tcc act gca gat gtg aaa agg tga                       612
   Gly Gly Tyr Ile Ser Thr Ala Asp Val Lys Arg
           195                 200
<210> 83
   <211> 203
   <212> PRT
   <213> Thermoplasma acidophilum
<400> 83
   Met Asn Ile Gly Val Leu Gly Phe Gln Gly Asp Val Gln Glu His Met
   1               5                   10                  15
   Asp Met Leu Lys Lys Leu Ser Arg Lys Asn Arg Asp Leu Thr Leu Thr
               20                  25                  30
   His Val Lys Arg Val Ile Asp Leu Glu His Val Asp Ala Leu Ile Ile
           35                  40                  45
   Pro Gly Gly Glu Ser Thr Thr Ile Tyr Lys Leu Thr Leu Glu Tyr Gly
       50                  55                  60
   Leu Tyr Asp Ala Ile Val Lys Arg Ser Ala Glu Gly Met Pro Ile Met
   65                  70                  75                  80
   Ala Thr Cys Ala Gly Leu Ile Leu Val Ser Lys Asn Thr Asn Asp Glu
                   85                  90                  95
   Arg Val Arg Gly Met Gly Leu Leu Asp Val Thr Ile Arg Arg Asn Ala
               100                 105                 110
   Tyr Gly Arg Gln Val Met Ser Phe Glu Thr Asp Ile Glu Ile Asn Gly
           115                 120                 125
   Ile Gly Met Phe Pro Ala Val Phe Ile Arg Ala Pro Val Ile Glu Asp
       130                 135                 140
   Ser Gly Lys Thr Glu Val Leu Gly Thr Leu Asp Gly Lys Pro Val Ile
   145                 150                 155                 160
   Val Lys Gln Gly Asn Val Ile Gly Met Thr Phe His Pro Glu Leu Thr
                   165                 170                 175
   Gly Asp Thr Arg Leu His Glu Tyr Phe Ile Asn Met Val Arg Gly Arg
               180                 185                 190
   Gly Gly Tyr Ile Ser Thr Ala Asp Val Lys Arg
           195                 200
<210> 84
   <211> 591
   <212> DNA
   <213> Bacillus cereus ATCC 10987
<400> 84
   atg gtg aaa atc ggt gta cta ggt ctt caa ggt gca gtt cgt gaa cat       48
   Met Val Lys Ile Gly Val Leu Gly Leu Gln Gly Ala Val Arg Glu His
   1               5                   10                  15
   gta aaa tca gtt gaa gca agt ggt gca gaa gct gtt gtt gta aag cgt       96
   Val Lys Ser Val Glu Ala Ser Gly Ala Glu Ala Val Val Val Lys Arg
               20                  25                  30
   ata gaa caa ctt gaa gag att gat ggt ctt att tta cca ggc ggt gaa      144
   Ile Glu Gln Leu Glu Glu Ile Asp Gly Leu Ile Leu Pro Gly Gly Glu
           35                  40                  45
   agt aca act atg cgc cgt ctt att gat aag tat gct ttc atg gag cca      192
   Ser Thr Thr Met Arg Arg Leu Ile Asp Lys Tyr Ala Phe Met Glu Pro
       50                  55                  60
   ctt cgt aca ttt gcg aag tct ggt aaa cca atg ttt ggt aca tgt gca      240
   Leu Arg Thr Phe Ala Lys Ser Gly Lys Pro Met Phe Gly Thr Cys Ala
   65                  70                  75                  80
   gga atg att ctt ctt gca aaa aca ctt att ggc tat gac gaa gca cat      288
   Gly Met Ile Leu Leu Ala Lys Thr Leu Ile Gly Tyr Asp Glu Ala His
                   85                  90                  95
   att ggt gct atg gat att aca gtt gag cgc aat gcg ttt gga cgt caa      336
   Ile Gly Ala Met Asp Ile Thr Val Glu Arg Asn Ala Phe Gly Arg Gln
               100                 105                 110
   aaa gat agc ttt gaa gct gca ctt tct att aaa ggt gtg gga gaa gat      384
   Lys Asp Ser Phe Glu Ala Ala Leu Ser Ile Lys Gly Val Gly Glu Asp
           115                 120                 125
   ttt gtt ggc gta ttt att cgt gcc ccg tat gtt gta aat gta gcg gat      432
   Phe Val Gly Val Phe Ile Arg Ala Pro Tyr Val Val Asn Val Ala Asp
       130                 135                 140
   aat gtt gag gta ctt tct aca cat ggt gat cga atg gta gcg gta agg      480
   Asn Val Glu Val Leu Ser Thr His Gly Asp Arg Met Val Ala Val Arg
   145                 150                 155                 160
   caa ggg ccg ttt tta gct gct tct ttc cat ccg gaa tta acg gat gat      528
   Gln Gly Pro Phe Leu Ala Ala Ser Phe His Pro Glu Leu Thr Asp Asp
                   165                 170                 175
   cat cgt gta aca gca tac ttt gta gaa atg gta aaa gaa gcg aaa atg      576
   His Arg Val Thr Ala Tyr Phe Val Glu Met Val Lys Glu Ala Lys Met
               180                 185                 190
   aaa aaa gtt gta taa                                                  591
   Lys Lys Val Val
           195
<210> 85
   <211> 196
   <212> PRT
   <213> Bacillus cereus ATCC 10987
<400> 85
   Met Val Lys Ile Gly Val Leu Gly Leu Gln Gly Ala Val Arg Glu His
   1               5                   10                  15
   Val Lys Ser Val Glu Ala Ser Gly Ala Glu Ala Val Val Val Lys Arg
               20                  25                  30
   Ile Glu Gln Leu Glu Glu Ile Asp Gly Leu Ile Leu Pro Gly Gly Glu
           35                  40                  45
   Ser Thr Thr Met Arg Arg Leu Ile Asp Lys Tyr Ala Phe Met Glu Pro
       50                  55                  60
   Leu Arg Thr Phe Ala Lys Ser Gly Lys Pro Met Phe Gly Thr Cys Ala
   65                  70                  75                  80
   Gly Met Ile Leu Leu Ala Lys Thr Leu Ile Gly Tyr Asp Glu Ala His
                   85                  90                  95
   Ile Gly Ala Met Asp Ile Thr Val Glu Arg Asn Ala Phe Gly Arg Gln
               100                 105                 110
   Lys Asp Ser Phe Glu Ala Ala Leu Ser Ile Lys Gly Val Gly Glu Asp
           115                 120                 125
   Phe Val Gly Val Phe Ile Arg Ala Pro Tyr Val Val Asn Val Ala Asp
       130                 135                 140
   Asn Val Glu Val Leu Ser Thr His Gly Asp Arg Met Val Ala Val Arg
   145                 150                 155                 160
   Gln Gly Pro Phe Leu Ala Ala Ser Phe His Pro Glu Leu Thr Asp Asp
                   165                 170                 175
   His Arg Val Thr Ala Tyr Phe Val Glu Met Val Lys Glu Ala Lys Met
               180                 185                 190
   Lys Lys Val Val
           195
<210> 86
   <211> 828
   <212> DNA
   <213> Ashbya gossypii (Yeast) (Eremothecium gossypii)
<400> 86
   atg aac gta gta gcc aac gac tat gca gag tcc att ttg ctc gta gtc       48
   Met Asn Val Val Ala Asn Asp Tyr Ala Glu Ser Ile Leu Leu Val Val
   1               5                   10                  15
   gag cga cag aat agc tct tac ctc aga aaa cgc aga ggc aga aaa aac      96
   Glu Arg Gln Asn Ser Ser Tyr Leu Arg Lys Arg Arg Gly Arg Lys Asn
               20                  25                  30
   gct gca ggc gtg tcg ttg tca ctt tac ctg cgt ata tat aga gct agc      144
   Ala Ala Gly Val Ser Leu Ser Leu Tyr Leu Arg Ile Tyr Arg Ala Ser
           35                  40                  45
   gcc ggc att aca aca tta agc caa ctt cgg aac agc gta cgc agt cag      192
   Ala Gly Ile Thr Thr Leu Ser Gln Leu Arg Asn Ser Val Arg Ser Gln
       50                  55                  60
   ttt gat ata atg agt aaa gta gtt gga gtc ctt gca ttg cag ggt tca      240
   Phe Asp Ile Met Ser Lys Val Val Gly Val Leu Ala Leu Gln Gly Ser
   65                  70                  75                  80
   ttt gca gag cac atc gac tgc cta gag gct tgc gtc aga gaa aat gga      288
   Phe Ala Glu His Ile Asp Cys Leu Glu Ala Cys Val Arg Glu Asn Gly
                   85                  90                  95
   cac aac gtc gag gtg atc gcg gta aag aca caa cag gaa cta gcg cgc      336
   His Asn Val Glu Val Ile Ala Val Lys Thr Gln Gln Glu Leu Ala Arg
               100                 105                 110
   tgc gat tcg ctc att att cca gga ggc gag tca acg gct att tcg cag      384
   Cys Asp Ser Leu Ile Ile Pro Gly Gly Glu Ser Thr Ala Ile Ser Gln
           115                 120                 125
   atc gca gaa cgc acc ggt ctg cat gag cac cta tac cag ttt gtg cgg      432
   Ile Ala Glu Arg Thr Gly Leu His Glu His Leu Tyr Gln Phe Val Arg
       130                 135                 140
   acg ccc ggc aaa tcg gcc tgg ggc acg tgc gca ggg ctc atc ttc ctg      480
   Thr Pro Gly Lys Ser Ala Trp Gly Thr Cys Ala Gly Leu Ile Phe Leu
   145                 150                 155                 160
   tcg aac cag gtc gcc aac cag gca gca ctg ctg aag ccg ctc ggt atc      528
   Ser Asn Gln Val Ala Asn Gln Ala Ala Leu Leu Lys Pro Leu Gly Ile
                   165                 170                 175
   ctg gac gtg act gtg gag cgg aat gcc ttc ggc cgc cag ctg cag tcc      576
   Leu Asp Val Thr Val Glu Arg Asn Ala Phe Gly Arg Gln Leu Gln Ser
               180                 185                 190
   ttc gag aag gac tgc gat ttt tcg tcc ttt tgg gat cac gac ggt ccc      624
   Phe Glu Lys Asp Cys Asp Phe Ser Ser Phe Trp Asp His Asp Gly Pro
           195                 200                 205
   ttc cca acc gtc ttc ata cgc gcg cca gtc att tcc aag atc aac agc      672
   Phe Pro Thr Val Phe Ile Arg Ala Pro Val Ile Ser Lys Ile Asn Ser
       210                 215                 220
   aag aac gtc gag gtc ttg tac acg ttg cag agg gac gac ggc tcc gag      720
   Lys Asn Val Glu Val Leu Tyr Thr Leu Gln Arg Asp Asp Gly Ser Glu
   225                 230                 235                 240
   caa atc gta gcc gtg cgg cag ggc agt atc ctg ggc acc tcc ttc cac      768
   Gln Ile Val Ala Val Arg Gln Gly Ser Ile Leu Gly Thr Ser Phe His
                   245                 250                 255
   cct gag cta ggt tct gac acc cgc ttc cac gac tgg ttc ctc cgt acc      816
   Pro Glu Leu Gly Ser Asp Thr Arg Phe His Asp Trp Phe Leu Arg Thr
               260                 265                 270
   ttc gtc ctg tag                                                      828
   Phe Val Leu
           275
<210> 87
   <211> 275
   <212> PRT
   <213> Ashbya gossypii (Yeast) (Eremothecium gossypii)
<400> 87
   Met Asn Val Val Ala Asn Asp Tyr Ala Glu Ser Ile Leu Leu Val Val
   1               5                   10                  15
   Glu Arg Gln Asn Ser Ser Tyr Leu Arg Lys Arg Arg Gly Arg Lys Asn
               20                  25                  30
   Ala Ala Gly Val Ser Leu Ser Leu Tyr Leu Arg Ile Tyr Arg Ala Ser
           35                  40                  45
   Ala Gly Ile Thr Thr Leu Ser Gln Leu Arg Asn Ser Val Arg Ser Gln
       50                  55                  60
   Phe Asp Ile Met Ser Lys Val Val Gly Val Leu Ala Leu Gln Gly Ser
   65                  70                  75                  80
   Phe Ala Glu His Ile Asp Cys Leu Glu Ala Cys Val Arg Glu Asn Gly
   85 90 95
   His Asn Val Glu Val Ile Ala Val Lys Thr Gln Gln Glu Leu Ala Arg
   100 105 110
   Cys Asp Ser Leu Ile Ile Pro Gly Gly Glu Ser Thr Ala Ile Ser Gln
   115 120 125
   Ile Ala Glu Arg Thr Gly Leu His Glu His Leu Tyr Gln Phe Val Arg
   130 135 140
   Thr Pro Gly Lys Ser Ala Trp Gly Thr Cys Ala Gly Leu Ile Phe Leu
   145 150 155 160
   Ser Asn Gln Val Ala Asn Gln Ala Ala Leu Leu Lys Pro Leu Gly Ile
   165 170 175
   Leu Asp Val Thr Val Glu Arg Asn Ala Phe Gly Arg Gln Leu Gln Ser
   180 185 190
   Phe Glu Lys Asp Cys Asp Phe Ser Ser Phe Trp Asp His Asp Gly Pro
   195 200 205
   Phe Pro Thr Val Phe Ile Arg Ala Pro Val Ile Ser Lys Ile Asn Ser
   210 215 220
   Lys Asn Val Glu Val Leu Tyr Thr Leu Gln Arg Asp Asp Gly Ser Glu
   225 230 235 240
   Gln Ile Val Ala Val Arg Gln Gly Ser Ile Leu Gly Thr Ser Phe His
   245 250 255
   Pro Glu Leu Gly Ser Asp Thr Arg Phe His Asp Trp Phe Leu Arg Thr
   260 265 270
   Phe Val Leu
   275
<210> 88
   <211> 576
   <212> DNA
   <213> Thermus thermophilus HB27
<400> 88
   atg agg ggc gtg gtt ggc gtt ttg gcc tta cag ggg gat ttc cgc gag 48
   Met Arg Gly Val Val Gly Val Leu Ala Leu Gln Gly Asp Phe Arg Glu
   1 5 10 15
   cac aag gag gcg ctt aag cgc ctg ggg ata gag gcc aag gag gtg cgg 96
   His Lys Glu Ala Leu Lys Arg Leu Gly Ile Glu Ala Lys Glu Val Arg
   20 25 30
   aag gtt aag gac ctc gag ggg cta aaa gcc ctc atc gtt ccg ggc ggc 144
   Lys Val Lys Asp Leu Glu Gly Leu Lys Ala Leu Ile Val Pro Gly Gly
   35 40 45
   gag tcc acc acc atc ggc aag ctc gcc cgg gag tac ggt ctg gag gag 192
   Glu Ser Thr Thr Ile Gly Lys Leu Ala Arg Glu Tyr Gly Leu Glu Glu
   50 55 60
   gcg gtg cgg agg cgg gtg gag gag ggc acc ctg gcc ctc ttc ggg acc 240
   Ala Val Arg Arg Arg Val Glu Glu Gly Thr Leu Ala Leu Phe Gly Thr
   65 70 75 80
   tgc gcc ggg gcc atc tgg ctt gcc cgg gag atc ctg ggc tac ccc gag 288
   Cys Ala Gly Ala Ile Trp Leu Ala Arg Glu Ile Leu Gly Tyr Pro Glu
   85 90 95
   cag ccc cgc ctc ggg gtc ttg gac gcc gcc gtg gag cgg aac gcc ttc 336
   Gln Pro Arg Leu Gly Val Leu Asp Ala Ala Val Glu Arg Asn Ala Phe
   100 105 110
   ggg cgg cag gtg gaa agc ttt gag gag gac ctg gag gtg gag ggc ctc 384
   Gly Arg Gln Val Glu Ser Phe Glu Glu Asp Leu Glu Val Glu Gly Leu
   115 120 125
   ggc ccc ttc cac ggc gtc ttc atc cgc gcc ccc gtc ttc cgc agg ctg 432
   Gly Pro Phe His Gly Val Phe Ile Arg Ala Pro Val Phe Arg Arg Leu
   130 135 140
   ggg gag ggg gtg gag gtc ctg gcc agg ctt ggg gac ctt ccc gtt ctg 480
   Gly Glu Gly Val Glu Val Leu Ala Arg Leu Gly Asp Leu Pro Val Leu
   145 150 155 160
   gtc cgc cag ggg aag gtc ctc gcc agc agc ttc cac ccc gag ctc acg 528
   Val Arg Gln Gly Lys Val Leu Ala Ser Ser Phe His Pro Glu Leu Thr
   165 170 175
   gag gac ccc cgc ctc cac cgc tac ttc ctg gag ctc gcc ggg gtt 573
   Glu Asp Pro Arg Leu His Arg Tyr Phe Leu Glu Leu Ala Gly Val
   180 185 190
   taa 576
<210> 89
   <211> 191
   <212> PRT
   <213> Thermus thermophilus HB27
<400> 89
   Met Arg Gly Val Val Gly Val Leu Ala Leu Gln Gly Asp Phe Arg Glu
   1               5                   10                  15
   His Lys Glu Ala Leu Lys Arg Leu Gly Ile Glu Ala Lys Glu Val Arg
               20                  25                  30
   Lys Val Lys Asp Leu Glu Gly Leu Lys Ala Leu Ile Val Pro Gly Gly
           35                  40                  45
   Glu Ser Thr Thr Ile Gly Lys Leu Ala Arg Glu Tyr Gly Leu Glu Glu
       50                  55                  60
   Ala Val Arg Arg Arg Val Glu Glu Gly Thr Leu Ala Leu Phe Gly Thr
   65                  70                  75                  80
   Cys Ala Gly Ala Ile Trp Leu Ala Arg Glu Ile Leu Gly Tyr Pro Glu
                   85                  90                  95
   Gln Pro Arg Leu Gly Val Leu Asp Ala Ala Val Glu Arg Asn Ala Phe
               100                 105                 110
   Gly Arg Gln Val Glu Ser Phe Glu Glu Asp Leu Glu Val Glu Gly Leu
           115                 120                 125
   Gly Pro Phe His Gly Val Phe Ile Arg Ala Pro Val Phe Arg Arg Leu
       130                 135                 140
   Gly Glu Gly Val Glu Val Leu Ala Arg Leu Gly Asp Leu Pro Val Leu
   145                 150                 155                 160
   Val Arg Gln Gly Lys Val Leu Ala Ser Ser Phe His Pro Glu Leu Thr
                   165                 170                 175
   Glu Asp Pro Arg Leu His Arg Tyr Phe Leu Glu Leu Ala Gly Val
               180                 185                 190
<210> 90
   <211> 1047
   <212> DNA
   <213> Oryza sativa (japonica cultivar-group)
<400> 90
   gagaagagga ggggagcagc agcagcagca gcagca atg gcg gtc gtc ggc gtc        54
                                           Met Ala Val Val Gly Val
                                           1               5
   ctc gcg ctg cag ggc tcc ttc aac gag cac ttg gcc gcg ctg agg agg       102
   Leu Ala Leu Gln Gly Ser Phe Asn Glu His Leu Ala Ala Leu Arg Arg
               10                  15                  20
   atc ggg gtg agg ggg gtg gag gtg cgg aag ccg gag cag ctg cag ggg       150
   Ile Gly Val Arg Gly Val Glu Val Arg Lys Pro Glu Gln Leu Gln Gly
           25                  30                  35
   ctc gac tcg ctc atc atc ccc gga ggc gag agc acc acc atg gcc aaa       198
   Leu Asp Ser Leu Ile Ile Pro Gly Gly Glu Ser Thr Thr Met Ala Lys
       40                  45                  50
   ctc gcc aac tac cac aac ctg ttt cct gca ctt cga gaa ttt gtt ggt       246
   Leu Ala Asn Tyr His Asn Leu Phe Pro Ala Leu Arg Glu Phe Val Gly
   55                  60                  65                  70
   aca gga agg cct gtc tgg gga act tgt gct gga ctc atc ttc cta gct       294
   Thr Gly Arg Pro Val Trp Gly Thr Cys Ala Gly Leu Ile Phe Leu Ala
                   75                  80                  85
   aac aag gca gta ggc caa aaa tcc gga ggt cag gag ctt att gga gga       342
   Asn Lys Ala Val Gly Gln Lys Ser Gly Gly Gln Glu Leu Ile Gly Gly
               90                  95                  100
   cta gat tgt act gtc cac cgg aac ttt ttt ggg agc cag ctt caa agc       390
   Leu Asp Cys Thr Val His Arg Asn Phe Phe Gly Ser Gln Leu Gln Ser
           105                 110                 115
   ttt gaa acg gaa ctt tca gtg cca atg ctt gca gag aag gaa gga ggg       438
   Phe Glu Thr Glu Leu Ser Val Pro Met Leu Ala Glu Lys Glu Gly Gly
       120                 125                 130
   agc gat aca tgc cgt ggc gta ttt ata cga gca cct gct atc ttg gat       486
   Ser Asp Thr Cys Arg Gly Val Phe Ile Arg Ala Pro Ala Ile Leu Asp
   135                 140                 145                 150
   gta ggt tca aat gtt gaa gta ctg gcg gat tgt cct gtt cca tcg gat       534
   Val Gly Ser Asn Val Glu Val Leu Ala Asp Cys Pro Val Pro Ser Asp
                   155                 160                 165
   aga ccc agt att aca ata gcg tct gga gag ggt gtt gag gaa gaa gtg       582
   Arg Pro Ser Ile Thr Ile Ala Ser Gly Glu Gly Val Glu Glu Glu Val
               170                 175                 180
   tac tcg aaa gat cgg gta att gtt gct gta agg caa ggg aac atc ctc       630
   Tyr Ser Lys Asp Arg Val Ile Val Ala Val Arg Gln Gly Asn Ile Leu
           185                 190                 195
   gct act gct ttt cac cca gaa ttg aca tca gac tct aga tgg cat cgg       678
   Ala Thr Ala Phe His Pro Glu Leu Thr Ser Asp Ser Arg Trp His Arg
       200                 205                 210
   ttc ttc ctg gac atg gat aaa gaa tct gat aca aaa gcc ttc tct gct       726
   Phe Phe Leu Asp Met Asp Lys Glu Ser Asp Thr Lys Ala Phe Ser Ala
   215                 220                 225                 230
   ctc tct ctc tca tca tct tca aga gac act caa gat ggg tca aag aat       774
   Leu Ser Leu Ser Ser Ser Ser Arg Asp Thr Gln Asp Gly Ser Lys Asn
                   235                 240                 245
   aag cct ctt gat cta ccc atc ttc gag tagctcatga aagaaaagaa             821
   Lys Pro Leu Asp Leu Pro Ile Phe Glu
               250                 255
   agactgttaa acattgaaga acagaagatg aagaagctaa caaaattttg agcattcagt     881
   tggtgacaat agagaaagtt gagtacgtgt gatgctcagt ccaaatgtgt tattgttgtc     941
   aaactgtacc aatcaaaata atgataatgc cgtcccaaac attgtgattt tgctacgaca    1001
   aagaatctga ttcagttgaa tatatgtcac aatttttttt cttccg                    1047
<210> 91
   <211> 255
   <212> PRT
   <213> Oryza sativa (japonica cultivar-group)
<400> 91
   Met Ala Val Val Gly Val Leu Ala Leu Gln Gly Ser Phe Asn Glu His
   1               5                   10                  15
   Leu Ala Ala Leu Arg Arg Ile Gly Val Arg Gly Val Glu Val Arg Lys
               20                  25                  30
   Pro Glu Gln Leu Gln Gly Leu Asp Ser Leu Ile Ile Pro Gly Gly Glu
           35                  40                  45
   Ser Thr Thr Met Ala Lys Leu Ala Asn Tyr His Asn Leu Phe Pro Ala
       50                  55                  60
   Leu Arg Glu Phe Val Gly Thr Gly Arg Pro Val Trp Gly Thr Cys Ala
   65                  70                  75                  80
   Gly Leu Ile Phe Leu Ala Asn Lys Ala Val Gly Gln Lys Ser Gly Gly
                   85                  90                  95
   Gln Glu Leu Ile Gly Gly Leu Asp Cys Thr Val His Arg Asn Phe Phe
               100                 105                 110
   Gly Ser Gln Leu Gln Ser Phe Glu Thr Glu Leu Ser Val Pro Met Leu
           115                 120                 125
   Ala Glu Lys Glu Gly Gly Ser Asp Thr Cys Arg Gly Val Phe Ile Arg
       130                 135                 140
   Ala Pro Ala Ile Leu Asp Val Gly Ser Asn Val Glu Val Leu Ala Asp
   145                 150                 155                 160
   Cys Pro Val Pro Ser Asp Arg Pro Ser Ile Thr Ile Ala Ser Gly Glu
                   165                 170                 175
   Gly Val Glu Glu Glu Val Tyr Ser Lys Asp Arg Val Ile Val Ala Val
               180                 185                 190
   Arg Gln Gly Asn Ile Leu Ala Thr Ala Phe His Pro Glu Leu Thr Ser
           195                 200                 205
   Asp Ser Arg Trp His Arg Phe Phe Leu Asp Met Asp Lys Glu Ser Asp
       210                 215                 220
   Thr Lys Ala Phe Ser Ala Leu Ser Leu Ser Ser Ser Ser Arg Asp Thr
   225                 230                 235                 240
   Gln Asp Gly Ser Lys Asn Lys Pro Leu Asp Leu Pro Ile Phe Glu
                   245                 250                 255
<210> 92
   <211> 594
   <212> DNA
   <213> Parachlamydia sp. UWE25
<400> 92
   atg ctg ata ggt ata tta gca tta cag gga gat ttc ttt aaa cat caa        48
   Met Leu Ile Gly Ile Leu Ala Leu Gln Gly Asp Phe Phe Lys His Gln
   1               5                   10                  15
   gaa atg ctt cat tct ctt ggt ata gaa acg atc caa gtt aaa act cga        96
   Glu Met Leu His Ser Leu Gly Ile Glu Thr Ile Gln Val Lys Thr Arg
               20                  25                  30
   aat gag tta gat ttt tgt gat gct ctt att att cct ggt ggg gaa tct        144
   Asn Glu Leu Asp Phe Cys Asp Ala Leu Ile Ile Pro Gly Gly Glu Ser
           35                  40                  45
   act gtg atg atg cga caa ctt gaa aca aca aat ctt aaa gag cta tta        192
   Thr Val Met Met Arg Gln Leu Glu Thr Thr Asn Leu Lys Glu Leu Leu
       50                  55                  60
   gtt cat ttt gcg atc cat aaa cct gtt ttt gga act tgt gct ggc ctt        240
   Val His Phe Ala Ile His Lys Pro Val Phe Gly Thr Cys Ala Gly Leu
   65                  70                  75                  80
   att tta atg tct tct cac gtt caa aat tct gca atg atg ccg ctt gga        288
   Ile Leu Met Ser Ser His Val Gln Asn Ser Ala Met Met Pro Leu Gly
                   85                  90                  95
   ctg tta cat att gct gtc gaa cga aat gcg ttt ggg cgg caa gtc gat        336
   Leu Leu His Ile Ala Val Glu Arg Asn Ala Phe Gly Arg Gln Val Asp
               100                 105                 110
   tct ttt caa gtg gat gtg tct gtt tat tta aaa cca gga gac gaa ata        384
   Ser Phe Gln Val Asp Val Ser Val Tyr Leu Lys Pro Gly Asp Glu Ile
           115                 120                 125
   tgt ttt cct gct ttt ttt att cga gct cca cgt att cga aca agt gaa        432
   Cys Phe Pro Ala Phe Phe Ile Arg Ala Pro Arg Ile Arg Thr Ser Glu
       130                 135                 140
   act ccc gtg caa att ctt gct tct tat gaa ggg gag cct att ttg gtt        480
   Thr Pro Val Gln Ile Leu Ala Ser Tyr Glu Gly Glu Pro Ile Leu Val
   145                 150                 155                 160
   cgg caa ggg cat cat tta gga gca tcg ttt cat ccg gag tta aca gtc        528
   Arg Gln Gly His His Leu Gly Ala Ser Phe His Pro Glu Leu Thr Val
                   165                 170                 175
   aac cct tct att cat ctt tat ttt ctt gaa atg gtc aaa gaa aac tta        576
   Asn Pro Ser Ile His Leu Tyr Phe Leu Glu Met Val Lys Glu Asn Leu
               180                 185                 190
   gaa aat cat aag aaa tag                                                594
   Glu Asn His Lys Lys
           195
<210> 93
   <211> 197
   <212> PRT
   <213> Parachlamydia sp. UWE25
<400> 93
   Met Leu Ile Gly Ile Leu Ala Leu Gln Gly Asp Phe Phe Lys His Gln
   1               5                   10                  15
   Glu Met Leu His Ser Leu Gly Ile Glu Thr Ile Gln Val Lys Thr Arg
               20                  25                  30
   Asn Glu Leu Asp Phe Cys Asp Ala Leu Ile Ile Pro Gly Gly Glu Ser
           35                  40                  45
   Thr Val Met Met Arg Gln Leu Glu Thr Thr Asn Leu Lys Glu Leu Leu
       50                  55                  60
   Val His Phe Ala Ile His Lys Pro Val Phe Gly Thr Cys Ala Gly Leu
   65                  70                  75                  80
   Ile Leu Met Ser Ser His Val Gln Asn Ser Ala Met Met Pro Leu Gly
                   85                  90                  95
   Leu Leu His Ile Ala Val Glu Arg Asn Ala Phe Gly Arg Gln Val Asp
               100                 105                 110
   Ser Phe Gln Val Asp Val Ser Val Tyr Leu Lys Pro Gly Asp Glu Ile
           115                 120                 125
   Cys Phe Pro Ala Phe Phe Ile Arg Ala Pro Arg Ile Arg Thr Ser Glu
       130                 135                 140
   Thr Pro Val Gln Ile Leu Ala Ser Tyr Glu Gly Glu Pro Ile Leu Val
   145                 150                 155                 160
   Arg Gln Gly His His Leu Gly Ala Ser Phe His Pro Glu Leu Thr Val
                   165                 170                 175
   Asn Pro Ser Ile His Leu Tyr Phe Leu Glu Met Val Lys Glu Asn Leu
               180                 185                 190
   Glu Asn His Lys Lys
           195
<210> 94
   <211> 564
   <212> DNA
   <213> Methanococcus maripaludis
<400> 94
   atg aaa ata atc ggg ata ctc ggc att cag ggc gac att gaa gaa cac       48
   Met Lys Ile Ile Gly Ile Leu Gly Ile Gln Gly Asp Ile Glu Glu His
   1               5                   10                  15
   gaa gat gca gtt aaa aaa ata aat tgc atc cct aaa cgg ata aga acg       96
   Glu Asp Ala Val Lys Lys Ile Asn Cys Ile Pro Lys Arg Ile Arg Thr
               20                  25                  30
   gta gat gat tta gaa gga ata gac gca tta ata att cca ggg gga gaa      144
   Val Asp Asp Leu Glu Gly Ile Asp Ala Leu Ile Ile Pro Gly Gly Glu
           35                  40                  45
   agt acc aca att gga aaa ttg atg gta agt tat gga ttt atc gat aaa      192
   Ser Thr Thr Ile Gly Lys Leu Met Val Ser Tyr Gly Phe Ile Asp Lys
       50                  55                  60
   att aga aat tta aaa atc ccg ata ctt gga act tgt gca gga atg gtt      240
   Ile Arg Asn Leu Lys Ile Pro Ile Leu Gly Thr Cys Ala Gly Met Val
   65                  70                  75                  80
   ctt tta tca aaa gga act gga aaa gag cag cca tta ctt gaa atg ttg      288
   Leu Leu Ser Lys Gly Thr Gly Lys Glu Gln Pro Leu Leu Glu Met Leu
                   85                  90                  95
   aat gtg acg ata aaa aga aat gca tac ggc agt caa aaa gat agt ttt      336
   Asn Val Thr Ile Lys Arg Asn Ala Tyr Gly Ser Gln Lys Asp Ser Phe
               100                 105                 110
   gaa aaa gaa ata gat tta ggc gga aaa aaa ata aat gct gta ttt att      384
   Glu Lys Glu Ile Asp Leu Gly Gly Lys Lys Ile Asn Ala Val Phe Ile
           115                 120                 125
   cga gca cca caa gtt ggg gag att ctc tca aaa gat gtt gaa atc att      432
   Arg Ala Pro Gln Val Gly Glu Ile Leu Ser Lys Asp Val Glu Ile Ile
       130                 135                 140
   tca aaa gac gat gaa aat att gtg gga ata aaa gaa gga aat ata atg      480
   Ser Lys Asp Asp Glu Asn Ile Val Gly Ile Lys Glu Gly Asn Ile Met
   145                 150                 155                 160
   gca ata tca ttt cac ccg gaa ctt tca gat gac ggg gtt att gca tat      528
   Ala Ile Ser Phe His Pro Glu Leu Ser Asp Asp Gly Val Ile Ala Tyr
                   165                 170                 175
   gaa tac ttt ttg aaa aat ttt gtg gaa aaa aga taa                      564
   Glu Tyr Phe Leu Lys Asn Phe Val Glu Lys Arg
               180                 185
<210> 95
   <211> 187
   <212> PRT
   <213> Methanococcus maripaludis
<400> 95
   Met Lys Ile Ile Gly Ile Leu Gly Ile Gln Gly Asp Ile Glu Glu His
   1               5                   10                  15
   Glu Asp Ala Val Lys Lys Ile Asn Cys Ile Pro Lys Arg Ile Arg Thr
               20                  25                  30
   Val Asp Asp Leu Glu Gly Ile Asp Ala Leu Ile Ile Pro Gly Gly Glu
           35                  40                  45
   Ser Thr Thr Ile Gly Lys Leu Met Val Ser Tyr Gly Phe Ile Asp Lys
       50                  55                  60
   Ile Arg Asn Leu Lys Ile Pro Ile Leu Gly Thr Cys Ala Gly Met Val
   65                  70                  75                  80
   Leu Leu Ser Lys Gly Thr Gly Lys Glu Gln Pro Leu Leu Glu Met Leu
                   85                  90                  95
   Asn Val Thr Ile Lys Arg Asn Ala Tyr Gly Ser Gln Lys Asp Ser Phe
               100                 105                 110
   Glu Lys Glu Ile Asp Leu Gly Gly Lys Lys Ile Asn Ala Val Phe Ile
           115                 120                 125
   Arg Ala Pro Gln Val Gly Glu Ile Leu Ser Lys Asp Val Glu Ile Ile
       130                 135                 140
   Ser Lys Asp Asp Glu Asn Ile Val Gly Ile Lys Glu Gly Asn Ile Met
   145                 150                 155                 160
   Ala Ile Ser Phe His Pro Glu Leu Ser Asp Asp Gly Val Ile Ala Tyr
                   165                 170                 175
   Glu Tyr Phe Leu Lys Asn Phe Val Glu Lys Arg
               180                 185
<210> 96
   <211> 25
   <212> DNA
   <213> Saccharomyces cerevisiae
<400> 96
   atgcacaaaa cccacagtac aatgt 25
<210> 97
   <211> 28
   <212> DNA
   <213> Saccharomyces cerevisiae
<400> 97
   ttaattagaa acaaactgtc tgataaac 28
<210> 98
   <211> 714
   <212> DNA
   <213> Brassica napus
<400> 98
   atg acc gtg gga gta tta gct tta caa ggc tct ttc aac gag cac atc      48
   Met Thr Val Gly Val Leu Ala Leu Gln Gly Ser Phe Asn Glu His Ile
   1               5                   10                  15
   gcg gct ctg cgg cgg ctc ggc gtc caa gga atc gag att agg aag gcg      96
   Ala Ala Leu Arg Arg Leu Gly Val Gln Gly Ile Glu Ile Arg Lys Ala
               20                  25                  30
   gaa cag cta ctc acc gtt tca tct ctc ata atc cct ggc ggc gag agc      144
   Glu Gln Leu Leu Thr Val Ser Ser Leu Ile Ile Pro Gly Gly Glu Ser
           35                  40                  45
   acc acc atg gcc aaa ctc gcc gag tac cac aac ctg ttt ccg gct cta      192
   Thr Thr Met Ala Lys Leu Ala Glu Tyr His Asn Leu Phe Pro Ala Leu
       50                  55                  60
   cgt gag ttt gtc aag acg ggg aaa cct gta tgg ggg aca tgc gct ggt      240
   Arg Glu Phe Val Lys Thr Gly Lys Pro Val Trp Gly Thr Cys Ala Gly
   65                  70                  75                  80
   ctt atc ttc ttg gca gac aga gcc gtt ggt cag aaa gag gga ggt caa      288
   Leu Ile Phe Leu Ala Asp Arg Ala Val Gly Gln Lys Glu Gly Gly Gln
                   85                  90                  95
   gaa cta gta ggt ggc ctt gac tgc acc gtg cat agg aac ttc ttt ggc      336
   Glu Leu Val Gly Gly Leu Asp Cys Thr Val His Arg Asn Phe Phe Gly
               100                 105                 110
   agc cag att caa agt ttt gaa gct gat atc tca gta cct cta cta aca      384
   Ser Gln Ile Gln Ser Phe Glu Ala Asp Ile Ser Val Pro Leu Leu Thr
           115                 120                 125
   tct aaa gaa ggt ggg ccg gag aca tac cga gga gtc ttc ata cgt gct      432
   Ser Lys Glu Gly Gly Pro Glu Thr Tyr Arg Gly Val Phe Ile Arg Ala
       130                 135                 140
   cca gct gtt ctc gat gtt ggc cct gat gtc gaa gtc tta gcg cat tat      480
   Pro Ala Val Leu Asp Val Gly Pro Asp Val Glu Val Leu Ala His Tyr
   145                 150                 155                 160
   ccc gtc cca tca aac aag gtc ttg tat tca agc tct act gtc caa atc      528
   Pro Val Pro Ser Asn Lys Val Leu Tyr Ser Ser Ser Thr Val Gln Ile
                   165                 170                 175
   caa gag gaa gat gct ctt cca gag acg aac gtc att gtt gct gta aag      576
   Gln Glu Glu Asp Ala Leu Pro Glu Thr Asn Val Ile Val Ala Val Lys
               180                 185                 190
   caa aga aac ttg tta gca act gcg ttt cat ccc gag tta acc gca gac      624
   Gln Arg Asn Leu Leu Ala Thr Ala Phe His Pro Glu Leu Thr Ala Asp
           195                 200                 205
   acg cgt tgg cac agt tat ttc atg aag atg gcg aaa gag atg gaa caa      672
   Thr Arg Trp His Ser Tyr Phe Met Lys Met Ala Lys Glu Met Glu Gln
       210                 215                 220
   gga gct tct tca agc ggt ggt gga act att gat tct gtc tag              714
   Gly Ala Ser Ser Ser Gly Gly Gly Thr Ile Asp Ser Val
   225                 230                 235
<210> 99
   <211> 237
   <212> PRT
   <213> Brassica napus
<400> 99
   Met Thr Val Gly Val Leu Ala Leu Gln Gly Ser Phe Asn Glu His Ile
   1               5                   10                  15
   Ala Ala Leu Arg Arg Leu Gly Val Gln Gly Ile Glu Ile Arg Lys Ala
               20                  25                  30
   Glu Gln Leu Leu Thr Val Ser Ser Leu Ile Ile Pro Gly Gly Glu Ser
           35                  40                  45
   Thr Thr Met Ala Lys Leu Ala Glu Tyr His Asn Leu Phe Pro Ala Leu
       50                  55                  60
   Arg Glu Phe Val Lys Thr Gly Lys Pro Val Trp Gly Thr Cys Ala Gly
   65                  70                  75                  80
   Leu Ile Phe Leu Ala Asp Arg Ala Val Gly Gln Lys Glu Gly Gly Gln
                   85                  90                  95
   Glu Leu Val Gly Gly Leu Asp Cys Thr Val His Arg Asn Phe Phe Gly
               100                 105                 110
   Ser Gln Ile Gln Ser Phe Glu Ala Asp Ile Ser Val Pro Leu Leu Thr
           115                 120                 125
   Ser Lys Glu Gly Gly Pro Glu Thr Tyr Arg Gly Val Phe Ile Arg Ala
       130                 135                 140
   Pro Ala Val Leu Asp Val Gly Pro Asp Val Glu Val Leu Ala His Tyr
   145                 150                 155                 160
   Pro Val Pro Ser Asn Lys Val Leu Tyr Ser Ser Ser Thr Val Gln Ile
                   165                 170                 175
   Gln Glu Glu Asp Ala Leu Pro Glu Thr Asn Val Ile Val Ala Val Lys
               180                 185                 190
   Gln Arg Asn Leu Leu Ala Thr Ala Phe His Pro Glu Leu Thr Ala Asp
           195                 200                 205
   Thr Arg Trp His Ser Tyr Phe Met Lys Met Ala Lys Glu Met Glu Gln
       210                 215                 220
   Gly Ala Ser Ser Ser Gly Gly Gly Thr Ile Asp Ser Val
   225                 230                 235
<210> 100
   <211> 765
   <212> DNA
   <213> Glycine max
<400> 100
   atg gcc gtc gtt ggc gtc ctc gcg ctg caa gga tct ttc aac gaa cac        48
   Met Ala Val Val Gly Val Leu Ala Leu Gln Gly Ser Phe Asn Glu His
   1               5                   10                  15
   ata gct gct ctt aga agg tta ggg gtg caa ggc gtg gag att cga aag         96
   Ile Ala Ala Leu Arg Arg Leu Gly Val Gln Gly Val Glu Ile Arg Lys
               20                  25                  30
   cca gag cag ctt aac aca att agt tcc ctc att atc cct ggt gga gaa        144
   Pro Glu Gln Leu Asn Thr Ile Ser Ser Leu Ile Ile Pro Gly Gly Glu
           35                  40                  45
   agc acc acc atg gct aag ctc gcc gag tat cac aac ctg ttt cct gct        192
   Ser Thr Thr Met Ala Lys Leu Ala Glu Tyr His Asn Leu Phe Pro Ala
       50                  55                  60
   ttg cga gag ttt gta caa atg gga aag cct gtt tgg gga acc tgt gca        240
   Leu Arg Glu Phe Val Gln Met Gly Lys Pro Val Trp Gly Thr Cys Ala
   65                  70                  75                  80
   ggg ctt ata ttc ttg gca aat aaa gct ata gga cag aag act ggt gga        288
   Gly Leu Ile Phe Leu Ala Asn Lys Ala Ile Gly Gln Lys Thr Gly Gly
                   85                  90                  95
   caa tat ttg gtt ggt gga ctt gat tgt aca gtg cat aga aat ttc ttt        336
   Gln Tyr Leu Val Gly Gly Leu Asp Cys Thr Val His Arg Asn Phe Phe
               100                 105                 110
   ggc agc cag att caa agc ttt gag gca gag ctt tca gtg cca gag ctc        384
   Gly Ser Gln Ile Gln Ser Phe Glu Ala Glu Leu Ser Val Pro Glu Leu
           115                 120                 125
   gtc tcc aaa gaa gga ggt cct gaa aca ttt cgt gga att ttt att cgt        432
   Val Ser Lys Glu Gly Gly Pro Glu Thr Phe Arg Gly Ile Phe Ile Arg
       130                 135                 140
   gcc cct gca att ctt gaa gca ggg cca gaa gtt caa gtg ctg gct gat        480
   Ala Pro Ala Ile Leu Glu Ala Gly Pro Glu Val Gln Val Leu Ala Asp
   145                 150                 155                 160
   tat ctt gta cct tct agc aga ttg ttg agt tct gat tcc tct att gaa        528
   Tyr Leu Val Pro Ser Ser Arg Leu Leu Ser Ser Asp Ser Ser Ile Glu
                   165                 170                 175
   gac aaa acg gag aat gct gag aaa gaa agt aaa gtt ata gtt gct gtg        576
   Asp Lys Thr Glu Asn Ala Glu Lys Glu Ser Lys Val Ile Val Ala Val
               180                 185                 190
   aga caa ggg aac ata tta gcc act gct ttc cat cct gaa ttg aca gcc        624
   Arg Gln Gly Asn Ile Leu Ala Thr Ala Phe His Pro Glu Leu Thr Ala
           195                 200                 205
   gat act cga tgg cat agt tat ttc gta aaa atg tca aat gaa att aga        672
   Asp Thr Arg Trp His Ser Tyr Phe Val Lys Met Ser Asn Glu Ile Arg
       210                 215                 220
   gaa gag gcc tct tcg agt agc ctt gtt cct gca caa gtc agt agt aca        720
   Glu Glu Ala Ser Ser Ser Ser Leu Val Pro Ala Gln Val Ser Ser Thr
   225                 230                 235                 240
   agt caa tat caa cag ccc cgg aat gac ctt cct atc tat cga tag            765
   Ser Gln Tyr Gln Gln Pro Arg Asn Asp Leu Pro Ile Tyr Arg
                   245                 250
<210> 101
   <211> 254
   <212> PRT
   <213> Glycine max
<400> 101
   Met Ala Val Val Gly Val Leu Ala Leu Gln Gly Ser Phe Asn Glu His
   1               5                   10                  15
   Ile Ala Ala Leu Arg Arg Leu Gly Val Gln Gly Val Glu Ile Arg Lys
               20                  25                  30
   Pro Glu Gln Leu Asn Thr Ile Ser Ser Leu Ile Ile Pro Gly Gly Glu
           35                  40                  45
   Ser Thr Thr Met Ala Lys Leu Ala Glu Tyr His Asn Leu Phe Pro Ala
       50                  55                  60
   Leu Arg Glu Phe Val Gln Met Gly Lys Pro Val Trp Gly Thr Cys Ala
   65                  70                  75                  80
   Gly Leu Ile Phe Leu Ala Asn Lys Ala Ile Gly Gln Lys Thr Gly Gly
                   85                  90                  95
   Gln Tyr Leu Val Gly Gly Leu Asp Cys Thr Val His Arg Asn Phe Phe
               100                 105                 110
   Gly Ser Gln Ile Gln Ser Phe Glu Ala Glu Leu Ser Val Pro Glu Leu
           115                 120                 125
   Val Ser Lys Glu Gly Gly Pro Glu Thr Phe Arg Gly Ile Phe Ile Arg
       130                 135                 140
   Ala Pro Ala Ile Leu Glu Ala Gly Pro Glu Val Gln Val Leu Ala Asp
   145                 150                 155                 160
   Tyr Leu Val Pro Ser Ser Arg Leu Leu Ser Ser Asp Ser Ser Ile Glu
                   165                 170                 175
   Asp Lys Thr Glu Asn Ala Glu Lys Glu Ser Lys Val Ile Val Ala Val
               180                 185                 190
   Arg Gln Gly Asn Ile Leu Ala Thr Ala Phe His Pro Glu Leu Thr Ala
           195                 200                 205
   Asp Thr Arg Trp His Ser Tyr Phe Val Lys Met Ser Asn Glu Ile Arg
       210                 215                 220
   Glu Glu Ala Ser Ser Ser Ser Leu Val Pro Ala Gln Val Ser Ser Thr
   225                 230                 235                 240
   Ser Gln Tyr Gln Gln Pro Arg Asn Asp Leu Pro Ile Tyr Arg
                   245                 250
<210> 102
   <211> 768
   <212> DNA
   <213> zea mays
<400> 102
   atg gcg gtg gtg ggc gtc ctc gcg ctg cag gga tcc tac aac gag cac      48
   Met Ala Val Val Gly Val Leu Ala Leu Gln Gly Ser Tyr Asn Glu His
   1               5                   10                  15
   atg gcc gcg ctg agg agg atc ggg gtg aag ggg gtg gag gtg cgc aaa      96
   Met Ala Ala Leu Arg Arg Ile Gly Val Lys Gly Val Glu Val Arg Lys
               20                  25                  30
   gca gag cag ctc ctc ggc atc gac tcg ctc atc atc ccc ggt ggc gag      144
   Ala Glu Gln Leu Leu Gly Ile Asp Ser Leu Ile Ile Pro Gly Gly Glu
           35                  40                  45
   agc acc acc atg gcc aag ctc gcc aac tac cac aac ctg ttc cct gca      192
   Ser Thr Thr Met Ala Lys Leu Ala Asn Tyr His Asn Leu Phe Pro Ala
       50                  55                  60
   ctt cga gag ttc gtc gga ggt gga aag cct gtc tgg gga acc tgt gct      240
   Leu Arg Glu Phe Val Gly Gly Gly Lys Pro Val Trp Gly Thr Cys Ala
   65                  70                  75                  80
   ggg ctc atc ttt ctt gca aac aaa gca gta ggg caa aaa aca ggg ggg      288
   Gly Leu Ile Phe Leu Ala Asn Lys Ala Val Gly Gln Lys Thr Gly Gly
                   85                  90                  95
   cag gaa ctt gtt gga gga tta gat tgt aca gtc cac cga aac ttt ttt      336
   Gln Glu Leu Val Gly Gly Leu Asp Cys Thr Val His Arg Asn Phe Phe
               100                 105                 110
   ggg agt cag ctt caa agc ttt gag aca gag ctt tcc gtg cca aag ctt      384
   Gly Ser Gln Leu Gln Ser Phe Glu Thr Glu Leu Ser Val Pro Lys Leu
           115                 120                 125
   tcg gag aag gaa gga ggg aat gat aca tgc cgc ggt gta ttt ata cgg      432
   Ser Glu Lys Glu Gly Gly Asn Asp Thr Cys Arg Gly Val Phe Ile Arg
       130                 135                 140
   gca cct gct ata ttg gaa gta ggt cca gat gtt gaa ata ttg gcg gat      480
   Ala Pro Ala Ile Leu Glu Val Gly Pro Asp Val Glu Ile Leu Ala Asp
   145                 150                 155                 160
   tgc cct gtt cct gtt gac aga ccc agc att aca ata tca ttt ggg gag      528
   Cys Pro Val Pro Val Asp Arg Pro Ser Ile Thr Ile Ser Phe Gly Glu
                   165                 170                 175
   ggt act gag gaa gaa gag tat tca aaa gat cgg gta att gtt gca gtg      576
   Gly Thr Glu Glu Glu Glu Tyr Ser Lys Asp Arg Val Ile Val Ala Val
               180                 185                 190
   cgg caa ggg aac atc ctc gca act gct ttc cac cca gaa ttg aca tca      624
   Arg Gln Gly Asn Ile Leu Ala Thr Ala Phe His Pro Glu Leu Thr Ser
           195                 200                 205
   gac tcc aga tgg cat cgt ttc ttc ttg gac atg gat aaa gaa tcc cca      672
   Asp Ser Arg Trp His Arg Phe Phe Leu Asp Met Asp Lys Glu Ser Pro
       210                 215                 220
   gca aag gcg ttt tct gcg ctc tcc ctg tcg tca tcg tca aga gac act      720
   Ala Lys Ala Phe Ser Ala Leu Ser Leu Ser Ser Ser Ser Arg Asp Thr
   225                 230                 235                 240
   gaa ggc ctg cca aag aat aag ccg ttt gat ctg ccc att ttt gag         765
   Glu Gly Leu Pro Lys Asn Lys Pro Phe Asp Leu Pro Ile Phe Glu
                   245                 250                 255
   taa                                                                 768
<210> 103
   <211> 255
   <212> PRT
   <213> Zea mays
<400> 103
   Met Ala Val Val Gly Val Leu Ala Leu Gln Gly Ser Tyr Asn Glu His
   1               5                   10                  15
   Met Ala Ala Leu Arg Arg Ile Gly Val Lys Gly Val Glu Val Arg Lys
               20                  25                  30
   Ala Glu Gln Leu Leu Gly Ile Asp Ser Leu Ile Ile Pro Gly Gly Glu
           35                  40                  45
   Ser Thr Thr Met Ala Lys Leu Ala Asn Tyr His Asn Leu Phe Pro Ala
       50                  55                  60
   Leu Arg Glu Phe Val Gly Gly Gly Lys Pro Val Trp Gly Thr Cys Ala
   65                  70                  75                  80
   Gly Leu Ile Phe Leu Ala Asn Lys Ala Val Gly Gln Lys Thr Gly Gly
                   85                  90                  95
   Gln Glu Leu Val Gly Gly Leu Asp Cys Thr Val His Arg Asn Phe Phe
               100                 105                 110
   Gly Ser Gln Leu Gln Ser Phe Glu Thr Glu Leu Ser Val Pro Lys Leu
           115                 120                 125
   Ser Glu Lys Glu Gly Gly Asn Asp Thr Cys Arg Gly Val Phe Ile Arg
       130                 135                 140
   Ala Pro Ala Ile Leu Glu Val Gly Pro Asp Val Glu Ile Leu Ala Asp
   145                 150                 155                 160
   Cys Pro Val Pro Val Asp Arg Pro Ser Ile Thr Ile Ser Phe Gly Glu
                   165                 170                 175
   Gly Thr Glu Glu Glu Glu Tyr Ser Lys Asp Arg Val Ile Val Ala Val
               180                 185                 190
   Arg Gln Gly Asn Ile Leu Ala Thr Ala Phe His Pro Glu Leu Thr Ser
           195                 200                 205
   Asp Ser Arg Trp His Arg Phe Phe Leu Asp Met Asp Lys Glu Ser Pro
       210                 215                 220
   Ala Lys Ala Phe Ser Ala Leu Ser Leu Ser Ser Ser Ser Arg Asp Thr
   225                 230                 235                 240
   Glu Gly Leu Pro Lys Asn Lys Pro Phe Asp Leu Pro Ile Phe Glu
                   245                 250                 255
<210> 104
   <211> 768
   <212> DNA
   <213> Hordeum vulgare
<400> 104
   atg gcg gtg gtc ggc gtt ctg gcg ctg cag ggc tcc tac aac gag cac      48
   Met Ala Val Val Gly Val Leu Ala Leu Gln Gly Ser Tyr Asn Glu His
   1               5                   10                  15
   atg tcc gcg ctg agg agg atc ggg gtg aag ggg gtg gag gtg cgc aag      96
   Met Ser Ala Leu Arg Arg Ile Gly Val Lys Gly Val Glu Val Arg Lys
               20                  25                  30
   ccg gag cag ctg cag ggc atc gac tcg ctc atc atc ccc ggc ggc gag      144
   Pro Glu Gln Leu Gln Gly Ile Asp Ser Leu Ile Ile Pro Gly Gly Glu
           35                  40                  45
   acc acc acc atg gcc aag ctc gcc aac tac cac aac ctc ttt cct gca      192
   Thr Thr Thr Met Ala Lys Leu Ala Asn Tyr His Asn Leu Phe Pro Ala
       50                  55                  60
   ctt cga gaa ttt gtc ggc aca gga aaa ccc gta tgg gga acc tgt gct      240
   Leu Arg Glu Phe Val Gly Thr Gly Lys Pro Val Trp Gly Thr Cys Ala
   65                  70                  75                  80
   ggg ctc atc ttc ctt gca aac aag gca gta ggg cag aaa aca gga ggc      288
   Gly Leu Ile Phe Leu Ala Asn Lys Ala Val Gly Gln Lys Thr Gly Gly
                   85                  90                  95
   caa gag ctt gtt ggt ggg cta gat tgt act gtc cac cgt aac ttt ttt      336
   Gln Glu Leu Val Gly Gly Leu Asp Cys Thr Val His Arg Asn Phe Phe
               100                 105                 110
   ggg agt cag ctt caa agc ttc gaa aca gaa ctt tca gtg cca atg ctt      384
   Gly Ser Gln Leu Gln Ser Phe Glu Thr Glu Leu Ser Val Pro Met Leu
           115                 120                 125
   gca gag aag gaa gga ggg agt aat aca tgt cgt ggc gta ttt ata cga      432
   Ala Glu Lys Glu Gly Gly Ser Asn Thr Cys Arg Gly Val Phe Ile Arg
       130                 135                 140
   gca cct gct atc cta gaa gta ggc cag gat gtt gaa gta ttg gcc gat      480
   Ala Pro Ala Ile Leu Glu Val Gly Gln Asp Val Glu Val Leu Ala Asp
   145                 150                 155                 160
   tgc cct gtt cct gct ggc aga ccc agc att aca ata aca tct gcc gag      528
   Cys Pro Val Pro Ala Gly Arg Pro Ser Ile Thr Ile Thr Ser Ala Glu
                   165                 170                 175
   ggt gtg gag gaa caa gtg tac tcc aaa gat cgg gta att gtt gca gta      576
   Gly Val Glu Glu Gln Val Tyr Ser Lys Asp Arg Val Ile Val Ala Val
               180                 185                 190
   cga caa ggg aac atc ctc gcc acc gca ttt cac cca gag cta aca tca      624
   Arg Gln Gly Asn Ile Leu Ala Thr Ala Phe His Pro Glu Leu Thr Ser
           195                 200                 205
   gac tct aga tgg cat caa ctc ttc ttg gac atg gac aaa gaa tct caa      672
   Asp Ser Arg Trp His Gln Leu Phe Leu Asp Met Asp Lys Glu Ser Gln
       210                 215                 220
   gca aag gcc ttg gcc gcg cta tcg cta tct gca tct tca aac aat gca      720
   Ala Lys Ala Leu Ala Ala Leu Ser Leu Ser Ala Ser Ser Asn Asn Ala
   225                 230                 235                 240
   gaa gtt ggg tcg aag aat aag gct cct gat cta ccc att ttt gag          765
   Glu Val Gly Ser Lys Asn Lys Ala Pro Asp Leu Pro Ile Phe Glu
                   245                 250                 255
   tag                                                                  768
<210> 105
   <211> 255
   <212> PRT
   <213> Hordeum vulgare
<400> 105
   Met Ala Val Val Gly Val Leu Ala Leu Gln Gly Ser Tyr Asn Glu His
   1               5                   10                  15
   Met Ser Ala Leu Arg Arg Ile Gly Val Lys Gly Val Glu Val Arg Lys
               20                  25                  30
   Pro Glu Gln Leu Gln Gly Ile Asp Ser Leu Ile Ile Pro Gly Gly Glu
           35                  40                  45
   Thr Thr Thr Met Ala Lys Leu Ala Asn Tyr His Asn Leu Phe Pro Ala
       50                  55                  60
   Leu Arg Glu Phe Val Gly Thr Gly Lys Pro Val Trp Gly Thr Cys Ala
   65                  70                  75                  80
   Gly Leu Ile Phe Leu Ala Asn Lys Ala Val Gly Gln Lys Thr Gly Gly
                   85                  90                  95
   Gln Glu Leu Val Gly Gly Leu Asp Cys Thr Val His Arg Asn Phe Phe
               100                 105                 110
   Gly Ser Gln Leu Gln Ser Phe Glu Thr Glu Leu Ser Val Pro Met Leu
           115                 120                 125
   Ala Glu Lys Glu Gly Gly Ser Asn Thr Cys Arg Gly Val Phe Ile Arg
       130                 135                 140
   Ala Pro Ala Ile Leu Glu Val Gly Gln Asp Val Glu Val Leu Ala Asp
   145                 150                 155                 160
   Cys Pro Val Pro Ala Gly Arg Pro Ser Ile Thr Ile Thr Ser Ala Glu
                   165                 170                 175
   Gly Val Glu Glu Gln Val Tyr Ser Lys Asp Arg Val Ile Val Ala Val
               180                 185                 190
   Arg Gln Gly Asn Ile Leu Ala Thr Ala Phe His Pro Glu Leu Thr Ser
           195                 200                 205
   Asp Ser Arg Trp His Gln Leu Phe Leu Asp Met Asp Lys Glu Ser Gln
       210                 215                 220
   Ala Lys Ala Leu Ala Ala Leu Ser Leu Ser Ala Ser Ser Asn Asn Ala
   225                 230                 235                 240
   Glu Val Gly Ser Lys Asn Lys Ala Pro Asp Leu Pro Ile Phe Glu
                   245                 250                 255
<210> 106
   <211> 1264
   <212> DNA
   <213> Saccharomyces cerevisiae
<400> 106
   ttttccaata cttgattaac ctctttttcg tttcttgtct ttattttaga tttgttttaa      60
   tatcgcctaa tttttccttc tttactttat atttttttta tttttcgcct aaagatttgt     120
   atcaattaat tagccaacaa aaacaaaaac aataaagtca tataagggtt gataattgat      180
   attg atg gca gct aat tct gta ggg aaa atg agt gaa aag tta aga atc       229
        Met Ala Ala Asn Ser Val Gly Lys Met Ser Glu Lys Leu Arg Ile
        1               5                   10                  15
   aag gtg gac gat gtt aaa atc aac ccc aag tat gtt tta tac ggt gtt        277
   Lys Val Asp Asp Val Lys Ile Asn Pro Lys Tyr Val Leu Tyr Gly Val
                   20                  25                  30
   agt aca cca aac aag cgc ctt tac aaa agg tat tcc gag ttt tgg aaa        325
   Ser Thr Pro Asn Lys Arg Leu Tyr Lys Arg Tyr Ser Glu Phe Trp Lys
               35                  40                  45
   ctg aag aca cga ttg gag aga gat gta gga agc acc atc cca tat gac        373
   Leu Lys Thr Arg Leu Glu Arg Asp Val Gly Ser Thr Ile Pro Tyr Asp
           50                  55                  60
   ttc cct gaa aag ccc ggt gta ttg gac agg agg tgg caa aga aga tat        421
   Phe Pro Glu Lys Pro Gly Val Leu Asp Arg Arg Trp Gln Arg Arg Tyr
       65                  70                  75
   gat gat ccg gaa atg atc gat gaa aga cgg atc gga cta gag agg ttc        469
   Asp Asp Pro Glu Met Ile Asp Glu Arg Arg Ile Gly Leu Glu Arg Phe
   80                  85                  90                  95
   ctc aat gaa ttg tat aac gat cgt ttt gat tct cga tgg aga gac aca        517
   Leu Asn Glu Leu Tyr Asn Asp Arg Phe Asp Ser Arg Trp Arg Asp Thr
                   100                 105                 110
   aaa ata gcg caa gac ttc ctg cag ttg tca aag cca aat gtt tct caa        565
   Lys Ile Ala Gln Asp Phe Leu Gln Leu Ser Lys Pro Asn Val Ser Gln
               115                 120                 125
   gaa aag tca cag cag cat cta gaa act gct gac gaa gtg gga tgg gat        613
   Glu Lys Ser Gln Gln His Leu Glu Thr Ala Asp Glu Val Gly Trp Asp
           130                 135                 140
   gag atg ata aga gat att aaa ttg gat tta gat aag gag agt gat ggc        661
   Glu Met Ile Arg Asp Ile Lys Leu Asp Leu Asp Lys Glu Ser Asp Gly
       145                 150                 155
   aca ccc agc gtg cgt gga gca cta agg gca cgt acg aag ctc cac aag        709
   Thr Pro Ser Val Arg Gly Ala Leu Arg Ala Arg Thr Lys Leu His Lys
   160                 165                 170                 175
   tta cga gag cga cta gaa cag gat gtg caa aag aag tct ctt cca agc        757
   Leu Arg Glu Arg Leu Glu Gln Asp Val Gln Lys Lys Ser Leu Pro Ser
                   180                 185                 190
   acg gaa gtg act cgt cgc gcc gct cta ttg agg tcc ttg ctc aag gaa        805
   Thr Glu Val Thr Arg Arg Ala Ala Leu Leu Arg Ser Leu Leu Lys Glu
               195                 200                 205
   tgc gat gac att ggt aca gca aac ata gct cag gac cgt gga cga ctt        853
   Cys Asp Asp Ile Gly Thr Ala Asn Ile Ala Gln Asp Arg Gly Arg Leu
           210                 215                 220
   ctg ggg gtt gcc acc agt gac aac tct tca acc acg gaa gtt caa gga        901
   Leu Gly Val Ala Thr Ser Asp Asn Ser Ser Thr Thr Glu Val Gln Gly
       225                 230                 235
   aga acg aat aac gat ttg caa cag ggg cag atg caa atg gtg cgc gat        949
   Arg Thr Asn Asn Asp Leu Gln Gln Gly Gln Met Gln Met Val Arg Asp
   240                 245                 250                 255
   caa gaa caa gag ttg gtt gca ctg cac cga att atc cag gca caa cgt        997
   Gln Glu Gln Glu Leu Val Ala Leu His Arg Ile Ile Gln Ala Gln Arg
               260                 265                 270
   gga ttg gcc tta gag atg aac gag gag ctg caa aca cag aat gag cta       1045
   Gly Leu Ala Leu Glu Met Asn Glu Glu Leu Gln Thr Gln Asn Glu Leu
           275                 280                 285
   ctt aca gca ctt gaa gat gac gtc gat aac act ggt agg agg tta cag       1093
   Leu Thr Ala Leu Glu Asp Asp Val Asp Asn Thr Gly Arg Arg Leu Gln
       290                 295                 300
   ata gcc aac aag aag gct aga cat ttt aac aac agt gct tgaattaatg        1142
   Ile Ala Asn Lys Lys Ala Arg His Phe Asn Asn Ser Ala
   305                 310                 315
   agttactatc cgggttacaa atcctgagag tatatttgta ctaaaaaaaa aaattgtaaa      1202
   tctagtaatt gaaaaatttt ggcgatgaga cgatatggta agagtaaagc aaaggaaccg     1262
   tc                                                                    1264
<210> 107
   <211> 316
   <212> PRT
   <213> Saccharomyces cerevisiae
<400> 107
   Met Ala Ala Asn Ser Val Gly Lys Met Ser Glu Lys Leu Arg Ile Lys
   1               5                   10                  15
   Val Asp Asp Val Lys Ile Asn Pro Lys Tyr Val Leu Tyr Gly Val Ser
               20                  25                  30
   Thr Pro Asn Lys Arg Leu Tyr Lys Arg Tyr Ser Glu Phe Trp Lys Leu
           35                  40                  45
   Lys Thr Arg Leu Glu Arg Asp Val Gly Ser Thr Ile Pro Tyr Asp Phe
       50                  55                  60
   Pro Glu Lys Pro Gly Val Leu Asp Arg Arg Trp Gln Arg Arg Tyr Asp
   65                  70                  75                  80
   Asp Pro Glu Met Ile Asp Glu Arg Arg Ile Gly Leu Glu Arg Phe Leu
                   85                  90                  95
   Asn Glu Leu Tyr Asn Asp Arg Phe Asp Ser Arg Trp Arg Asp Thr Lys
               100                 105                 110
   Ile Ala Gln Asp Phe Leu Gln Leu Ser Lys Pro Asn Val Ser Gln Glu
           115                 120                 125
   Lys Ser Gln Gln His Leu Glu Thr Ala Asp Glu Val Gly Trp Asp Glu
       130                 135                 140
   Met Ile Arg Asp Ile Lys Leu Asp Leu Asp Lys Glu Ser Asp Gly Thr
   145                 150                 155                 160
   Pro Ser Val Arg Gly Ala Leu Arg Ala Arg Thr Lys Leu His Lys Leu
                   165                 170                 175
   Arg Glu Arg Leu Glu Gln Asp Val Gln Lys Lys Ser Leu Pro Ser Thr
               180                 185                 190
   Glu Val Thr Arg Arg Ala Ala Leu Leu Arg Ser Leu Leu Lys Glu Cys
           195                 200                 205
   Asp Asp Ile Gly Thr Ala Asn Ile Ala Gln Asp Arg Gly Arg Leu Leu
       210                 215                 220
   Gly Val Ala Thr Ser Asp Asn Ser Ser Thr Thr Glu Val Gln Gly Arg
   225                 230                 235                 240
   Thr Asn Asn Asp Leu Gln Gln Gly Gln Met Gln Met Val Arg Asp Gln
                   245                 250                 255
   Glu Gln Glu Leu Val Ala Leu His Arg Ile Ile Gln Ala Gln Arg Gly
               260                 265                 270
   Leu Ala Leu Glu Met Asn Glu Glu Leu Gln Thr Gln Asn Glu Leu Leu
           275                 280                 285
   Thr Ala Leu Glu Asp Asp Val Asp Asn Thr Gly Arg Arg Leu Gln Ile
           290                 295                 300
   Ala Asn Lys Lys Ala Arg His Phe Asn Asn Ser Ala
   305                 310                 315
<210> 108
   <211> 975
   <212> DNA
   <213> oryza sativa
<400> 108
   atg gtc gaa gcc gaa gcc acg aaa ggc ccg cac cga gat cga ctc gac      48
   Met Val Glu Ala Glu Ala Thr Lys Gly Pro His Arg Asp Arg Leu Asp
   1               5                   10                  15
   gac gcc gcc atc agc cgt cgg cga tgg cga cgc gcg gct gtg gcc ggc      96
   Asp Ala Ala Ile Ser Arg Arg Arg Trp Arg Arg Ala Ala Val Ala Gly
               20                  25                  30
   ggg gga agc gga cga gct gac acc gcc gac acg cct cat gcc agc tct      144
   Gly Gly Ser Gly Arg Ala Asp Thr Ala Asp Thr Pro His Ala Ser Ser
           35                  40                  45
   gtc gtg ccg ctg ttg tgc tac gtc ctc cca agc ctg tct gac cct aag      192
   Val Val Pro Leu Leu Cys Tyr Val Leu Pro Ser Leu Ser Asp Pro Lys
       50                  55                  60
   ctc gcc cgc gtg gcc tct agc ttc ctc tcg acc tcc gac tcc gca aga      240
   Leu Ala Arg Val Ala Ser Ser Phe Leu Ser Thr Ser Asp Ser Ala Arg
   65                  70                  75                  80
   agg gca gcg ttg gcc ctc atc gtc gcc acg gcg tct tcc cca ttg gag      288
   Arg Ala Ala Leu Ala Leu Ile Val Ala Thr Ala Ser Ser Pro Leu Glu
                   85                  90                  95
   caa tgg atg aag cgg ttc gag gag gcg gag agg ctc gtg gcc gac gtc      336
   Gln Trp Met Lys Arg Phe Glu Glu Ala Glu Arg Leu Val Ala Asp Val
               100                 105                 110
   gtc gag agg atc gcg gag agg gag tcc gtc tcg ccg tcg ctg ccg cag      384
   Val Glu Arg Ile Ala Glu Arg Glu Ser Val Ser Pro Ser Leu Pro Gln
           115                 120                 125
   gag ctg cag cgg cga acc gcc gaa atc agg agg aaa gtc gcg att ctc      432
   Glu Leu Gln Arg Arg Thr Ala Glu Ile Arg Arg Lys Val Ala Ile Leu
       130                 135                 140
   gag acc agg ctt gac atg atg cag gaa gac ctt tct caa ctc cca aac      480
   Glu Thr Arg Leu Asp Met Met Gln Glu Asp Leu Ser Gln Leu Pro Asn
   145                 150                 155                 160
   aag caa cgc ata agc ctg aaa gag ttg aac aag cta gca gcc aag cac      528
   Lys Gln Arg Ile Ser Leu Lys Glu Leu Asn Lys Leu Ala Ala Lys His
                   165                 170                 175
   tcc act ctg agc tcc aag gtg aag gag gtt ggc gct ccg ttc acc cgg      576
   Ser Thr Leu Ser Ser Lys Val Lys Glu Val Gly Ala Pro Phe Thr Arg
               180                 185                 190
   aag cgc ttc tcc aat agg agc gac ctg ctt gga ccg gac gac aac cac      624
   Lys Arg Phe Ser Asn Arg Ser Asp Leu Leu Gly Pro Asp Asp Asn His
           195                 200                 205
   gca aag atc gat gta agc agc att gcc aat atg gac aac cgt gag atc      672
   Ala Lys Ile Asp Val Ser Ser Ile Ala Asn Met Asp Asn Arg Glu Ile
       210                 215                 220
   att gag ttg cag agg aac gtt att aaa gag caa gac gac gaa ttg gac      720
   Ile Glu Leu Gln Arg Asn Val Ile Lys Glu Gln Asp Asp Glu Leu Asp
   225                 230                 235                 240
   aag ctg gag gag acg ata gtc agc acc aag cac att gcg ctg gcg atc      768
   Lys Leu Glu Glu Thr Ile Val Ser Thr Lys His Ile Ala Leu Ala Ile
                   245                 250                 255
   aac gaa gag ttg gat ctg cac act agg ttg att gat gac tta gac gag      816
   Asn Glu Glu Leu Asp Leu His Thr Arg Leu Ile Asp Asp Leu Asp Glu
               260                 265                 270
   aaa aca gaa gag aca agc aac cag ctt cag cgt gcg cag aaa aag ttg      864
   Lys Thr Glu Glu Thr Ser Asn Gln Leu Gln Arg Ala Gln Lys Lys Leu
           275                 280                 285
   aaa tct gta aca aca cgc atg agg aaa agc gct tcc tgc tca tgc ctt      912
   Lys Ser Val Thr Thr Arg Met Arg Lys Ser Ala Ser Cys Ser Cys Leu
       290                 295                 300
   ctc ctg tcg gtt att gca gtt gta att ctt gta gct cta tta tgg gct      960
   Leu Leu Ser Val Ile Ala Val Val Ile Leu Val Ala Leu Leu Trp Ala
   305                 310                 315                 320
   ctc atc atg tac tag                                                     975
   Leu Ile Met Tyr
<210> 109
   <211> 324
   <212> PRT
   <213> Oryza sativa
<400> 109
   Met Val Glu Ala Glu Ala Thr Lys Gly Pro His Arg Asp Arg Leu Asp
   1               5                   10                  15
   Asp Ala Ala Ile Ser Arg Arg Arg Trp Arg Arg Ala Ala Val Ala Gly
               20                  25                  30
   Gly Gly Ser Gly Arg Ala Asp Thr Ala Asp Thr Pro His Ala Ser Ser
           35                  40                  45
   Val Val Pro Leu Leu Cys Tyr Val Leu Pro Ser Leu Ser Asp Pro Lys
       50                  55                  60
   Leu Ala Arg Val Ala Ser Ser Phe Leu Ser Thr Ser Asp Ser Ala Arg
   65                  70                  75                  80
   Arg Ala Ala Leu Ala Leu Ile Val Ala Thr Ala Ser Ser Pro Leu Glu
                   85                  90                  95
   Gln Trp Met Lys Arg Phe Glu Glu Ala Glu Arg Leu Val Ala Asp Val
               100                 105                 110
   Val Glu Arg Ile Ala Glu Arg Glu Ser Val Ser Pro Ser Leu Pro Gln
           115                 120                 125
   Glu Leu Gln Arg Arg Thr Ala Glu Ile Arg Arg Lys Val Ala Ile Leu
       130                 135                 140
   Glu Thr Arg Leu Asp Met Met Gln Glu Asp Leu Ser Gln Leu Pro Asn
   145                 150                 155                 160
   Lys Gln Arg Ile Ser Leu Lys Glu Leu Asn Lys Leu Ala Ala Lys His
                   165                 170                 175
   Ser Thr Leu Ser Ser Lys Val Lys Glu Val Gly Ala Pro Phe Thr Arg
               180                 185                 190
   Lys Arg Phe Ser Asn Arg Ser Asp Leu Leu Gly Pro Asp Asp Asn His
           195                 200                 205
   Ala Lys Ile Asp Val Ser Ser Ile Ala Asn Met Asp Asn Arg Glu Ile
       210                 215                 220
   Ile Glu Leu Gln Arg Asn Val Ile Lys Glu Gln Asp Asp Glu Leu Asp
   225                 230                 235                 240
   Lys Leu Glu Glu Thr Ile Val Ser Thr Lys His Ile Ala Leu Ala Ile
                   245                 250                 255
   Asn Glu Glu Leu Asp Leu His Thr Arg Leu Ile Asp Asp Leu Asp Glu
               260                 265                 270
   Lys Thr Glu Glu Thr Ser Asn Gln Leu Gln Arg Ala Gln Lys Lys Leu
           275                 280                 285
   Lys Ser Val Thr Thr Arg Met Arg Lys Ser Ala Ser Cys Ser Cys Leu
       290                 295                 300
   Leu Leu Ser Val Ile Ala Val Val Ile Leu Val Ala Leu Leu Trp Ala
   305                 310                 315                 320
   Leu Ile Met Tyr
<210> 110
   <211> 1160
   <212> DNA
   <213> Candida albicans
<400> 110
   atg cat gat ata gaa att ggt ggg tca acg tac tat caa att aac ata      48
   Met His Asp Ile Glu Ile Gly Gly Ser Thr Tyr Tyr Gln Ile Asn Ile
   1               5                   10                  15
   aaa cta cca ctt cgg tca ttc acg ata aag aaa cgg tac ctg gaa ttc      96
   Lys Leu Pro Leu Arg Ser Phe Thr Ile Lys Lys Arg Tyr Leu Glu Phe
               20                  25                  30
   cag caa ttg gtg ctg gac ttg agt cgt aat cta ggc att gat agt cga      144
   Gln Gln Leu Val Leu Asp Leu Ser Arg Asn Leu Gly Ile Asp Ser Arg
           35                  40                  45
   gat ttt cca tat gaa tta cct ggg aaa cgg atc aac tgg ctt aac aag      192
   Asp Phe Pro Tyr Glu Leu Pro Gly Lys Arg Ile Asn Trp Leu Asn Lys
       50                  55                  60
   acc agt att gtt gag gag aga aaa gtg gga ctt gca gaa ttt ctc aat      240
   Thr Ser Ile Val Glu Glu Arg Lys Val Gly Leu Ala Glu Phe Leu Asn
   65                  70                  75                  80
   aac ctc att caa gac tca aca ctt cag aat gaa cga gaa gtg ttg tcg      288
   Asn Leu Ile Gln Asp Ser Thr Leu Gln Asn Glu Arg Glu Val Leu Ser
                   85                  90                  95
   ttt ttg caa ttg ccg tct aat ttt aga ttc acc aag gat atg tta cag      336
   Phe Leu Gln Leu Pro Ser Asn Phe Arg Phe Thr Lys Asp Met Leu Gln
               100                 105                 110
   aat aat cga gca gac ttg gat tct gtg caa aat aac tgg tac gat gta      384
   Asn Asn Arg Ala Asp Leu Asp Ser Val Gln Asn Asn Trp Tyr Asp Val
           115                 120                 125
   tat cgt aag ttg aaa ctg gat ata ctc aac gaa tcg tct agc agc att      432
   Tyr Arg Lys Leu Lys Leu Asp Ile Leu Asn Glu Ser Ser Ser Ser Ile
       130                 135                 140
   agt gaa cag ata cat att cgt gat cgc att agt cgg gtc tac caa cca      480
   Ser Glu Gln Ile His Ile Arg Asp Arg Ile Ser Arg Val Tyr Gln Pro
   145                 150                 155                 160
   cgg att ctc gac ttg gtc agg gct att ggt aca gat aaa gaa gag gcc      528
   Arg Ile Leu Asp Leu Val Arg Ala Ile Gly Thr Asp Lys Glu Glu Ala
                   165                 170                 175
   cta aag aag aag cag ttg gtt tcc caa tta caa gag agt ata gat aat      576
   Leu Lys Lys Lys Gln Leu Val Ser Gln Leu Gln Glu Ser Ile Asp Asn
               180                 185                 190
   ttg tta gta cag gaa gtt ccc cga tca aag agg gtg ttg ggt gga gca      624
   Leu Leu Val Gln Glu Val Pro Arg Ser Lys Arg Val Leu Gly Gly Ala
           195                 200                 205
   gtt aag gaa acg cca gag aca tta cca tta aac aat aaa gaa ctt ctt      672
   Val Lys Glu Thr Pro Glu Thr Leu Pro Leu Asn Asn Lys Glu Leu Leu
       210                 215                 220
   caa cac caa gta caa att cat caa aac caa gac aaa gaa cta gac cag      720
   Gln His Gln Val Gln Ile His Gln Asn Gln Asp Lys Glu Leu Asp Gln
   225                 230                 235                 240
   ctt agg gtg tta att gcc cgg cag aaa cag att ggc gag cta att aat      768
   Leu Arg Val Leu Ile Ala Arg Gln Lys Gln Ile Gly Glu Leu Ile Asn
                   245                 250                 255
   gca gaa gta gag gaa cag aat gaa atg ttg gat agg ttt aat gaa gag      816
   Ala Glu Val Glu Glu Gln Asn Glu Met Leu Asp Arg Phe Asn Glu Glu
               260                 265                 270
   gtc gac tac acg tcc agc aaa atc aag caa gca aga cgc aga gct aag      864
   Val Asp Tyr Thr Ser Ser Lys Ile Lys Gln Ala Arg Arg Arg Ala Lys
           275                 280                 285
   aag ata tta tagtaatttg ttcgctactt cgatattatc tgccattgac gttattcttg      923
   Lys Ile Leu
       290
   caggttggcc caattgttcg tttgaaagtt tttcgaggtc ttcagcgtct aatgccctat      983
   ctgagctctc gccatcgagt ttccaaaacc cgccgatatt ttgaaagaat ctttgaatgc      1043
   caaaccgtcg tggcgggaac gatctgcctg cgttggccaa gttgaatatg ctagggtggt      1103
   actgtaaata gaagacagat ccaataaacg ttcctataaa tgcaaaaaaa aaaaaaa      1160
<210> 111
   <211> 291
   <212> PRT
   <213> Candida albicans
<400> 111
   Met His Asp Ile Glu Ile Gly Gly Ser Thr Tyr Tyr Gln Ile Asn Ile
   1               5                   10                  15
   Lys Leu Pro Leu Arg Ser Phe Thr Ile Lys Lys Arg Tyr Leu Glu Phe
               20                  25                  30
   Gln Gln Leu Val Leu Asp Leu Ser Arg Asn Leu Gly Ile Asp Ser Arg
           35                  40                  45
   Asp Phe Pro Tyr Glu Leu Pro Gly Lys Arg Ile Asn Trp Leu Asn Lys
       50                  55                  60
   Thr Ser Ile Val Glu Glu Arg Lys Val Gly Leu Ala Glu Phe Leu Asn
   65                  70                  75                  80
   Asn Leu Ile Gln Asp Ser Thr Leu Gln Asn Glu Arg Glu Val Leu Ser
                   85                  90                  95
   Phe Leu Gln Leu Pro Ser Asn Phe Arg Phe Thr Lys Asp Met Leu Gln
               100                 105                 110
   Asn Asn Arg Ala Asp Leu Asp Ser Val Gln Asn Asn Trp Tyr Asp Val
           115                 120                 125
   Tyr Arg Lys Leu Lys Leu Asp Ile Leu Asn Glu Ser Ser Ser Ser Ile
       130                 135                 140
   Ser Glu Gln Ile His Ile Arg Asp Arg Ile Ser Arg Val Tyr Gln Pro
   145                 150                 155                 160
   Arg Ile Leu Asp Leu Val Arg Ala Ile Gly Thr Asp Lys Glu Glu Ala
                   165                 170                 175
   Leu Lys Lys Lys Gln Leu Val Ser Gln Leu Gln Glu Ser Ile Asp Asn
               180                 185                 190
   Leu Leu Val Gln Glu Val Pro Arg Ser Lys Arg Val Leu Gly Gly Ala
           195                 200                 205
   Val Lys Glu Thr Pro Glu Thr Leu Pro Leu Asn Asn Lys Glu Leu Leu
       210                 215                 220
   Gln His Gln Val Gln Ile His Gln Asn Gln Asp Lys Glu Leu Asp Gln
   225                 230                 235                 240
   Leu Arg Val Leu Ile Ala Arg Gln Lys Gln Ile Gly Glu Leu Ile Asn
                   245                 250                 255
   Ala Glu Val Glu Glu Gln Asn Glu Met Leu Asp Arg Phe Asn Glu Glu
               260                 265                 270
   Val Asp Tyr Thr Ser Ser Lys Ile Lys Gln Ala Arg Arg Arg Ala Lys
           275                 280                 285
   Lys Ile Leu
       290
<210> 112
   <211> 1689
   <212> DNA
   <213> Neurospora crassa
<400> 112
   atg gcc ccc cca gcc gag atc tcc atc ccc aca acc tcc ata tcc acc      48
   Met Ala Pro Pro Ala Glu Ile Ser Ile Pro Thr Thr Ser Ile Ser Thr
   1               5                   10                  15
   ccc tct tcc gaa tcc ggt ggc tcc tca aaa ccc ttc aca ctc tat aac      96
   Pro Ser Ser Glu Ser Gly Gly Ser Ser Lys Pro Phe Thr Leu Tyr Asn
               20                  25                  30
   atc act ctc cga ctt ccc ctc cgc tcc ttt gtc gtc caa aag cgc tac      144
   Ile Thr Leu Arg Leu Pro Leu Arg Ser Phe Val Val Gln Lys Arg Tyr
           35                  40                  45
   tcc gac ttc ctc gct ctg cac caa gcc ctc acc tcc ctt gtc ggc tcc      192
   Ser Asp Phe Leu Ala Leu His Gln Ala Leu Thr Ser Leu Val Gly Ser
       50                  55                  60
   ccg ccc ccc gaa ccc ttg ccc gcc aag aac tgg ttc aaa tcc acc gtc      240
   Pro Pro Pro Glu Pro Leu Pro Ala Lys Asn Trp Phe Lys Ser Thr Val
   65                  70                  75                  80
   aac tct ccc gag ctg acg gaa aag cgc cgc gtc gct ctc gag cgc tac      288
   Asn Ser Pro Glu Leu Thr Glu Lys Arg Arg Val Ala Leu Glu Arg Tyr
                   85                  90                  95
   ctc cgc gcc atc gcc gag ccg ccc gat cgt cgg tgg cgt gat acg ccc      336
   Leu Arg Ala Ile Ala Glu Pro Pro Asp Arg Arg Trp Arg Asp Thr Pro
               100                 105                 110
   gtc tgg cgc gcg ttt ctg aac ctg ccc ggc ggg gct agc ggt gcc aat      384
   Val Trp Arg Ala Phe Leu Asn Leu Pro Gly Gly Ala Ser Gly Ala Asn
           115                 120                 125
   gcc gcc gct agt act gcg ggt agt ggc agc gga atc gag ggg aaa atc      432
   Ala Ala Ala Ser Thr Ala Gly Ser Gly Ser Gly Ile Glu Gly Lys Ile
       130                 135                 140
   ccc gct ata ggc ctg aaa gac gcg aac ctc gct gct gcc agt gac ccg      480
   Pro Ala Ile Gly Leu Lys Asp Ala Asn Leu Ala Ala Ala Ser Asp Pro
   145                 150                 155                 160
   ggc acg tgg ctg gat ttg cac cgc gag ctg aag ggc gcg ctg cac gag      528
   Gly Thr Trp Leu Asp Leu His Arg Glu Leu Lys Gly Ala Leu His Glu
                   165                 170                 175
   gcg cgc gtg gcg ctg ggg agg agg gat ggg gcg acg gag aat atg acg      576
   Ala Arg Val Ala Leu Gly Arg Arg Asp Gly Ala Thr Glu Asn Met Thr
               180                 185                 190
   aag ctg gag gcg ggc gcg gcg gcc aag agg gcg ctg gtt agg gcg ggc      624
   Lys Leu Glu Ala Gly Ala Ala Ala Lys Arg Ala Leu Val Arg Ala Gly
           195                 200                 205
   agc ttg ctg ggc gcg ttg cag gag ggc ttg ggg gtt ctg aag agt agt      672
   Ser Leu Leu Gly Ala Leu Gln Glu Gly Leu Gly Val Leu Lys Ser Ser
       210                 215                 220
   gga cgg gtc ggg gaa ggg gag ctc cgg aga cga agg gac ctg ctg gcg      720
   Gly Arg Val Gly Glu Gly Glu Leu Arg Arg Arg Arg Asp Leu Leu Ala
   225                 230                 235                 240
   gcc gcg agg gtg gag agg gat ggg ttg gat aag ctc agt tcg agc ttg      768
   Ala Ala Arg Val Glu Arg Asp Gly Leu Asp Lys Leu Ser Ser Ser Leu
                   245                 250                 255
   gcg cat gcg agc agg gag gcg gcg agg cag gct tcg gtt agt ggg ccg      816
   Ala His Ala Ser Arg Glu Ala Ala Arg Gln Ala Ser Val Ser Gly Pro
               260                 265                 270
   tcg ggg agt ggg agt agt agc ggg gag gcc ggg gag agg gcc aag ttg      864
   Ser Gly Ser Gly Ser Ser Ser Gly Glu Ala Gly Glu Arg Ala Lys Leu
           275                 280                 285
   ttt gct ggg tct tct ggt gct ggt gga gga tcg gtg aga gga ggg aga      912
   Phe Ala Gly Ser Ser Gly Ala Gly Gly Gly Ser Val Arg Gly Gly Arg
       290                 295                 300
   gta ttg ggt gcc ccg ttg ccg gag acg gaa agg act agg gag ttg gat      960
   Val Leu Gly Ala Pro Leu Pro Glu Thr Glu Arg Thr Arg Glu Leu Asp
   305                 310                 315                 320
   aat gag ggg gtg ctg cag ctg cag agg gat aca atg cgt gat cag gat     1008
   Asn Glu Gly Val Leu Gln Leu Gln Arg Asp Thr Met Arg Asp Gln Asp
                   325                 330                 335
   atg gag gtg gag gcg ctg gcg agg atc gtc agg agg cag aag gag atg     1056
   Met Glu Val Glu Ala Leu Ala Arg Ile Val Arg Arg Gln Lys Glu Met
               340                 345                 350
   gga ctg gct atc aac gat gag gtt gag cgg cag acg aac atg ctg gat     1104
   Gly Leu Ala Ile Asn Asp Glu Val Glu Arg Gln Thr Asn Met Leu Asp
           355                 360                 365
   aac ctc aac act aat gtt gat gta gtg gat aag aag ttg agg gtc gcc     1152
   Asn Leu Asn Thr Asn Val Asp Val Val Asp Lys Lys Leu Arg Val Ala
       370                 375                 380
   aag gga cgg gag gag gat gag gag aat aac gac gat gat agt ctc aac     1200
   Lys Gly Arg Glu Glu Asp Glu Glu Asn Asn Asp Asp Asp Ser Leu Asn
   385                 390                 395                 400
   agg atg atg ttt atc atg tca agc gag gaa ggt tcc gtg gcg gag gtt     1248
   Arg Met Met Phe Ile Met Ser Ser Glu Glu Gly Ser Val Ala Glu Val
                   405                 410                 415
   gtt gct ctt cct acc acg gtg gcg caa gga gac cag cac gaa gct atc     1296
   Val Ala Leu Pro Thr Thr Val Ala Gln Gly Asp Gln His Glu Ala Ile
               420                 425                 430
   cac aga ccc cga aat ggc cgc tta cga cta cga cgg gac caa tgg ctg     1344
   His Arg Pro Arg Asn Gly Arg Leu Arg Leu Arg Arg Asp Gln Trp Leu
           435                 440                 445
   tat gaa tta tca ttg gat gac gac gga cac gac gac cac agc agc acc     1392
   Tyr Glu Leu Ser Leu Asp Asp Asp Gly His Asp Asp His Ser Ser Thr
       450                 455                 460
   aaa gac gag aag aag agc agg aca gca tca caa caa cag caa caa ggg     1440
   Lys Asp Glu Lys Lys Ser Arg Thr Ala Ser Gln Gln Gln Gln Gln Gly
   465                 470                 475                 480
   gac gaa gga aag ggg aaa cga aat gaa gga ttg aga gca aag ggt agg     1488
   Asp Glu Gly Lys Gly Lys Arg Asn Glu Gly Leu Arg Ala Lys Gly Arg
                   485                 490                 495
   ccc tcg gga agc ggc ggc ggc ggc ggc gaa gaa ggt aac atg ttt gat        1536
   Pro Ser Gly Ser Gly Gly Gly Gly Gly Glu Glu Gly Asn Met Phe Asp
               500                 505                 510
   gct ttc ctt ttg ctt tgt gtc aag ggc gtt ctc gcc ggc gtc caa ggg        1584
   Ala Phe Leu Leu Leu Cys Val Lys Gly Val Leu Ala Gly Val Gln Gly
           515                 520                 525
   ttt tgg ttg ttg cag tgg gtg ttg ggg agg ttg tcg gat gtg ctc act        1632
   Phe Trp Leu Leu Gln Trp Val Leu Gly Arg Leu Ser Asp Val Leu Thr
       530                 535                 540
   tgc gtg gtg gag ttt ggc cta ctt ctt ttg gga caa cct tcg gag tca        1680
   Cys Val Val Glu Phe Gly Leu Leu Leu Leu Gly Gln Pro Ser Glu Ser
   545                 550                 555                 560
   ttt ggt tga                                                            1689
   Phe Gly
<210> 113
   <211> 562
   <212> PRT
   <213> Neurospora crassa
<400> 113
   Met Ala Pro Pro Ala Glu Ile Ser Ile Pro Thr Thr Ser Ile Ser Thr
   1               5                   10                  15
   Pro Ser Ser Glu Ser Gly Gly Ser Ser Lys Pro Phe Thr Leu Tyr Asn
               20                  25                  30
   Ile Thr Leu Arg Leu Pro Leu Arg Ser Phe Val Val Gln Lys Arg Tyr
           35                  40                  45
   Ser Asp Phe Leu Ala Leu His Gln Ala Leu Thr Ser Leu Val Gly Ser
       50                  55                  60
   Pro Pro Pro Glu Pro Leu Pro Ala Lys Asn Trp Phe Lys Ser Thr Val
   65                  70                  75                  80
   Asn Ser Pro Glu Leu Thr Glu Lys Arg Arg Val Ala Leu Glu Arg Tyr
                   85                  90                  95
   Leu Arg Ala Ile Ala Glu Pro Pro Asp Arg Arg Trp Arg Asp Thr Pro
               100                 105                 110
   Val Trp Arg Ala Phe Leu Asn Leu Pro Gly Gly Ala Ser Gly Ala Asn
           115                 120                 125
   Ala Ala Ala Ser Thr Ala Gly Ser Gly Ser Gly Ile Glu Gly Lys Ile
       130                 135                 140
   Pro Ala Ile Gly Leu Lys Asp Ala Asn Leu Ala Ala Ala Ser Asp Pro
   145                 150                 155                 160
   Gly Thr Trp Leu Asp Leu His Arg Glu Leu Lys Gly Ala Leu His Glu
                   165                 170                 175
   Ala Arg Val Ala Leu Gly Arg Arg Asp Gly Ala Thr Glu Asn Met Thr
               180                 185                 190
   Lys Leu Glu Ala Gly Ala Ala Ala Lys Arg Ala Leu Val Arg Ala Gly
           195                 200                 205
   Ser Leu Leu Gly Ala Leu Gln Glu Gly Leu Gly Val Leu Lys Ser Ser
       210                 215                 220
   Gly Arg Val Gly Glu Gly Glu Leu Arg Arg Arg Arg Asp Leu Leu Ala
   225                 230                 235                 240
   Ala Ala Arg Val Glu Arg Asp Gly Leu Asp Lys Leu Ser Ser Ser Leu
                   245                 250                 255
   Ala His Ala Ser Arg Glu Ala Ala Arg Gln Ala Ser Val Ser Gly Pro
               260                 265                 270
   Ser Gly Ser Gly Ser Ser Ser Gly Glu Ala Gly Glu Arg Ala Lys Leu
           275                 280                 285
   Phe Ala Gly Ser Ser Gly Ala Gly Gly Gly Ser Val Arg Gly Gly Arg
       290                 295                 300
   Val Leu Gly Ala Pro Leu Pro Glu Thr Glu Arg Thr Arg Glu Leu Asp
   305                 310                 315                 320
   Asn Glu Gly Val Leu Gln Leu Gln Arg Asp Thr Met Arg Asp Gln Asp
                   325                 330                 335
   Met Glu Val Glu Ala Leu Ala Arg Ile Val Arg Arg Gln Lys Glu Met
               340                 345                 350
   Gly Leu Ala Ile Asn Asp Glu Val Glu Arg Gln Thr Asn Met Leu Asp
           355                 360                 365
   Asn Leu Asn Thr Asn Val Asp Val Val Asp Lys Lys Leu Arg Val Ala
       370                 375                 380
   Lys Gly Arg Glu Glu Asp Glu Glu Asn Asn Asp Asp Asp Ser Leu Asn
   385                 390                 395                 400
   Arg Met Met Phe Ile Met Ser Ser Glu Glu Gly Ser Val Ala Glu Val
                   405                 410                 415
   Val Ala Leu Pro Thr Thr Val Ala Gln Gly Asp Gln His Glu Ala Ile
               420                 425                 430
   His Arg Pro Arg Asn Gly Arg Leu Arg Leu Arg Arg Asp Gln Trp Leu
           435                 440                 445
   Tyr Glu Leu Ser Leu Asp Asp Asp Gly His Asp Asp His Ser Ser Thr
       450                 455                 460
   Lys Asp Glu Lys Lys Ser Arg Thr Ala Ser Gln Gln Gln Gln Gln Gly
   465                 470                 475                 480
   Asp Glu Gly Lys Gly Lys Arg Asn Glu Gly Leu Arg Ala Lys Gly Arg
                   485                 490                 495
   Pro Ser Gly Ser Gly Gly Gly Gly Gly Glu Glu Gly Asn Met Phe Asp
               500                 505                 510
   Ala Phe Leu Leu Leu Cys Val Lys Gly Val Leu Ala Gly Val Gln Gly
           515                 520                 525
   Phe Trp Leu Leu Gln Trp Val Leu Gly Arg Leu Ser Asp Val Leu Thr
       530                 535                 540
   Cys Val Val Glu Phe Gly Leu Leu Leu Leu Gly Gln Pro Ser Glu Ser
   545                 550                 555                 560
   Phe Gly
<210> 114
   <211> 925
   <212> DNA
   <213> Phytophthora infestans (Potato late blight fungus)
<400> 114
   ccacgcgttc gcggacgcgt gggcggacgc gtgggcggac gcgtgggcgg acgcgtgggc      60
   tgtcaagcgg cgtctgcaga taccagccat gatgaagaag gagccgtcc atg gcg gca    118
                                                         Met Ala Ala
                                                         1
   gct agc ggc gac ccg ttc tac gtt ttc aag gat gaa ctg gag agc aaa      166
   Ala Ser Gly Asp Pro Phe Tyr Val Phe Lys Asp Glu Leu Glu Ser Lys
       5                   10                  15
   gtg tcg gcc gtg aat cag aaa cac gcc aaa tgg cgc gcc atc ttg aac      214
   Val Ser Ala Val Asn Gln Lys His Ala Lys Trp Arg Ala Ile Leu Asn
   20                  25                  30                  35
   gtc aaa gac tca ccc gcc gca aag gaa cta ccg gcg ctt aca cat cag      262
   Val Lys Asp Ser Pro Ala Ala Lys Glu Leu Pro Ala Leu Thr His Gln
                   40                  45                  50
   atc gag ggc gcc gtg gcg aca gcg gag aag tcg ctc aag ttt ttg gaa      310
   Ile Glu Gly Ala Val Ala Thr Ala Glu Lys Ser Leu Lys Phe Leu Glu
               55                  60                  65
   gag acc atc gtc atg gtg gaa gcc aat cga gca aaa ttc gag cac att      358
   Glu Thr Ile Val Met Val Glu Ala Asn Arg Ala Lys Phe Glu His Ile
           70                  75                  80
   gac gcg gcg gag atc gca agt cgg aaa gcg ttt gta gcc gcc act aga      406
   Asp Ala Ala Glu Ile Ala Ser Arg Lys Ala Phe Val Ala Ala Thr Arg
       85                  90                  95
   aag gaa ctc caa gct gtt tca acc gaa atc tca acc gac act gtg aag      454
   Lys Glu Leu Gln Ala Val Ser Thr Glu Ile Ser Thr Asp Thr Val Lys
   100                 105                 110                 115
   acc cga atc cgc aaa gaa gaa cgc aag ttg atg caa cca gcg aag tcg      502
   Thr Arg Ile Arg Lys Glu Glu Arg Lys Leu Met Gln Pro Ala Lys Ser
                   120                 125                 130
   tcg acg tct ttc agg tca aat ctc acg ggg caa gag cga aac gag cga      550
   Ser Thr Ser Phe Arg Ser Asn Leu Thr Gly Gln Glu Arg Asn Glu Arg
               135                 140                 145
   ttt ttg gag gat gaa aca cag cgg caa cag caa att atg cag gag cag      598
   Phe Leu Glu Asp Glu Thr Gln Arg Gln Gln Gln Ile Met Gln Glu Gln
           150                 155                 160
   aat gac agt ttg gca gga ctt cac tcg gat atc aca cgc ttg cat gga        646
   Asn Asp Ser Leu Ala Gly Leu His Ser Asp Ile Thr Arg Leu His Gly
       165                 170                 175
   gtc acc gtg gag atc tcg agc gaa gtc aaa cac cag aat aaa atg ctg        694
   Val Thr Val Glu Ile Ser Ser Glu Val Lys His Gln Asn Lys Met Leu
   180                 185                 190                 195
   gac gat ctg act gac gat gtg gac gaa gca caa gag cga atg aat ttt        742
   Asp Asp Leu Thr Asp Asp Val Asp Glu Ala Gln Glu Arg Met Asn Phe
                   200                 205                 210
   gtc atg gga cgt ttg agc aag ctc ctg aag aca aaa gac aaa tgt caa        790
   Val Met Gly Arg Leu Ser Lys Leu Leu Lys Thr Lys Asp Lys Cys Gln
               215                 220                 225
   ctt gga ctc atc ctc ttc cta gtg gcc gtg ctc gct gtc atg atc ttc        838
   Leu Gly Leu Ile Leu Phe Leu Val Ala Val Leu Ala Val Met Ile Phe
           230                 235                 240
   ctg gtc gtg tac aca taacgcggta ctatcttccg tagttgctag acgttaatat        893
   Leu Val Val Tyr Thr
       245
   gaagctctag ctagacgaat aactatgtac tg                                    925
<210> 115
   <211> 248
   <212> PRT
   <213> Phytophthora infestans (Potato late blight fungus).
<400> 115
   Met Ala Ala Ala Ser Gly Asp Pro Phe Tyr Val Phe Lys Asp Glu Leu
   1               5                   10                  15
   Glu Ser Lys Val Ser Ala Val Asn Gln Lys His Ala Lys Trp Arg Ala
               20                  25                  30
   Ile Leu Asn Val Lys Asp Ser Pro Ala Ala Lys Glu Leu Pro Ala Leu
           35                  40                  45
   Thr His Gln Ile Glu Gly Ala Val Ala Thr Ala Glu Lys Ser Leu Lys
       50                  55                  60
   Phe Leu Glu Glu Thr Ile Val Met Val Glu Ala Asn Arg Ala Lys Phe
   65                  70                  75                  80
   Glu His Ile Asp Ala Ala Glu Ile Ala Ser Arg Lys Ala Phe Val Ala
                   85                  90                  95
   Ala Thr Arg Lys Glu Leu Gln Ala Val Ser Thr Glu Ile Ser Thr Asp
               100                 105                 110
   Thr Val Lys Thr Arg Ile Arg Lys Glu Glu Arg Lys Leu Met Gln Pro
           115                 120                 125
   Ala Lys Ser Ser Thr Ser Phe Arg Ser Asn Leu Thr Gly Gln Glu Arg
       130                 135                 140
   Asn Glu Arg Phe Leu Glu Asp Glu Thr Gln Arg Gln Gln Gln Ile Met
   145                 150                 155                 160
   Gln Glu Gln Asn Asp Ser Leu Ala Gly Leu His Ser Asp Ile Thr Arg
                   165                 170                 175
   Leu His Gly Val Thr Val Glu Ile Ser Ser Glu Val Lys His Gln Asn
               180                 185                 190
   Lys Met Leu Asp Asp Leu Thr Asp Asp Val Asp Glu Ala Gln Glu Arg
           195                 200                 205
   Met Asn Phe Val Met Gly Arg Leu Ser Lys Leu Leu Lys Thr Lys Asp
       210                 215                 220
   Lys Cys Gln Leu Gly Leu Ile Leu Phe Leu Val Ala Val Leu Ala Val
   225                 230                 235                 240
   Met Ile Phe Leu Val Val Tyr Thr
                   245
<210> 116
   <211> 795
   <212> DNA
   <213> Neurospora crassa
<400> 116
   atg tcc tcc acg aac gag gag gac ccc ttc ctt gag gtc caa cag gac      48
   Met Ser Ser Thr Asn Glu Glu Asp Pro Phe Leu Glu Val Gln Gln Asp
   1               5                   10                  15
   gtc cta acc caa ctc caa tcc acc cgc tcc ctc ttc acc tcc tac cta      96
   Val Leu Thr Gln Leu Gln Ser Thr Arg Ser Leu Phe Thr Ser Tyr Leu
               20                  25                  30
   cgc atc cgc tcc ctc ttc acc tct tcc tcc tcc tct tcc acc gac tct      144
   Arg Ile Arg Ser Leu Phe Thr Ser Ser Ser Ser Ser Ser Thr Asp Ser
           35                  40                  45
   cct gag ctg atc gcg gcc cgc tcc gac ctc gaa tcc gcc ctc tcc tcc      192
   Pro Glu Leu Ile Ala Ala Arg Ser Asp Leu Glu Ser Ala Leu Ser Ser
       50                  55                  60
   ctc gcc gaa gac ctc gcc gac ctc gtc gag tcc gtc aag gcc atc gag      240
   Leu Ala Glu Asp Leu Ala Asp Leu Val Glu Ser Val Lys Ala Ile Glu
   65                  70                  75                  80
   cgc gac ccc acg caa tat ggc ctg tcg gcg cac gaa gtc acg cgg cgc      288
   Arg Asp Pro Thr Gln Tyr Gly Leu Ser Ala His Glu Val Thr Arg Arg
                   85                  90                  95
   aag cgc ctt gtg caa gat gtc ggg tcc gag gta gag aac atg cgg cag      336
   Lys Arg Leu Val Gln Asp Val Gly Ser Glu Val Glu Asn Met Arg Gln
               100                 105                 110
   gag ctc gca tcc aaa tcc gcc gtc tct gga aag ggt acc cag caa aag      384
   Glu Leu Ala Ser Lys Ser Ala Val Ser Gly Lys Gly Thr Gln Gln Lys
           115                 120                 125
   gac caa tta cca gac cca tca tct ttc gcc atc ccg gac ggt gaa aac      432
   Asp Gln Leu Pro Asp Pro Ser Ser Phe Ala Ile Pro Asp Gly Glu Asn
       130                 135                 140
   ggt gcc gct ggc gcc acc ggc gaa gac gac gat tac gca gcc gaa ttc      480
   Gly Ala Ala Gly Ala Thr Gly Glu Asp Asp Asp Tyr Ala Ala Glu Phe
   145                 150                 155                 160
   gag cac cag cag cag ata cag atg atg cgc gag cag gat cag cat ttg      528
   Glu His Gln Gln Gln Ile Gln Met Met Arg Glu Gln Asp Gln His Leu
                   165                 170                 175
   gat ggg gta ttc cag acg gtc ggc gtg ctg agg cgg cag gcg gac gac      576
   Asp Gly Val Phe Gln Thr Val Gly Val Leu Arg Arg Gln Ala Asp Asp
               180                 185                 190
   atg ggc cgt gag ttg gag gag cag agg gag atg ctg gag gtg gcg gac      624
   Met Gly Arg Glu Leu Glu Glu Gln Arg Glu Met Leu Glu Val Ala Asp
           195                 200                 205
   gat ttg gcg gac cgc gtg gga ggg agg ttg cag acg ggg atg cag aag      672
   Asp Leu Ala Asp Arg Val Gly Gly Arg Leu Gln Thr Gly Met Gln Lys
       210                 215                 220
   ttg aca tat gtg atg agg cac aac gag gac acg ctg agc agt tgt tgc      720
   Leu Thr Tyr Val Met Arg His Asn Glu Asp Thr Leu Ser Ser Cys Cys
   225                 230                 235                 240
   att gcg gtc ttg atc ttc cca cga gtt gtt gcc gcc atg gtc cag gtg      768
   Ile Ala Val Leu Ile Phe Pro Arg Val Val Ala Ala Met Val Gln Val
                   245                 250                 255
   aaa acg ggc atc ggt cag caa cat tga                                  795
   Lys Thr Gly Ile Gly Gln Gln His
               260
<210> 117
   <211> 264
   <212> PRT
   <213> Neurospora crassa
<400> 117
   Met Ser Ser Thr Asn Glu Glu Asp Pro Phe Leu Glu Val Gln Gln Asp
   1               5                   10                  15
   Val Leu Thr Gln Leu Gln Ser Thr Arg Ser Leu Phe Thr Ser Tyr Leu
               20                  25                  30
   Arg Ile Arg Ser Leu Phe Thr Ser Ser Ser Ser Ser Ser Thr Asp Ser
           35                  40                  45
   Pro Glu Leu Ile Ala Ala Arg Ser Asp Leu Glu Ser Ala Leu Ser Ser
       50                  55                  60
   Leu Ala Glu Asp Leu Ala Asp Leu Val Glu Ser Val Lys Ala Ile Glu
   65                  70                  75                  80
   Arg Asp Pro Thr Gln Tyr Gly Leu Ser Ala His Glu Val Thr Arg Arg
                   85                  90                  95
   Lys Arg Leu Val Gln Asp Val Gly Ser Glu Val Glu Asn Met Arg Gln
               100                 105                 110
   Glu Leu Ala Ser Lys Ser Ala Val Ser Gly Lys Gly Thr Gln Gln Lys
           115                 120                 125
   Asp Gln Leu Pro Asp Pro Ser Ser Phe Ala Ile Pro Asp Gly Glu Asn
       130                 135                 140
   Gly Ala Ala Gly Ala Thr Gly Glu Asp Asp Asp Tyr Ala Ala Glu Phe
   145                 150                 155                 160
   Glu His Gln Gln Gln Ile Gln Met Met Arg Glu Gln Asp Gln His Leu
                   165                 170                 175
   Asp Gly Val Phe Gln Thr Val Gly Val Leu Arg Arg Gln Ala Asp Asp
               180                 185                 190
   Met Gly Arg Glu Leu Glu Glu Gln Arg Glu Met Leu Glu Val Ala Asp
           195                 200                 205
   Asp Leu Ala Asp Arg Val Gly Gly Arg Leu Gln Thr Gly Met Gln Lys
       210                 215                 220
   Leu Thr Tyr Val Met Arg His Asn Glu Asp Thr Leu Ser Ser Cys Cys
   225                 230                 235                 240
   Ile Ala Val Leu Ile Phe Pro Arg Val Val Ala Ala Met Val Gln Val
                   245                 250                 255
   Lys Thr Gly Ile Gly Gln Gln His
               260
<210> 118
   <211> 1134
   <212> DNA
   <213> Arabidopsis thaliana (Mouse-ear cress)
<400> 118
   tcattcttca aataaattaa aatcttcgtt ggcgttgttg ttggttgcgt tacagatttt      60
   ggactaatca ttattttcgt gcctgcaaag tcagcacgac gatcgcgttt cgatcttcaa     120
   agtagaagaa gacccgccac aatcacaaat cgcggtgcat atagtctaaa gggtca         176
   atg gcc tct tct tcg gat cca tgg atg aga gag tac aat gag gct ttg       224
   Met Ala Ser Ser Ser Asp Pro Trp Met Arg Glu Tyr Asn Glu Ala Leu
   1               5                   10                  15
   aaa ctc tct gag gat att aat ggc atg atg tct gaa agg aat gcc tcc       272
   Lys Leu Ser Glu Asp Ile Asn Gly Met Met Ser Glu Arg Asn Ala Ser
               20                  25                  30
   ggg tta acc ggg cct gat gct caa cgt cgt gcc tct gca att cga aga       320
   Gly Leu Thr Gly Pro Asp Ala Gln Arg Arg Ala Ser Ala Ile Arg Arg
           35                  40                  45
   aag atc acc att ttg ggg act cga tta gac agt ctg caa tcc ctt ctt       368
   Lys Ile Thr Ile Leu Gly Thr Arg Leu Asp Ser Leu Gln Ser Leu Leu
       50                  55                  60
   gtc aag gtt cct ggc aag cag cat gtt tcg gag aaa gag atg aat cgt       416
   Val Lys Val Pro Gly Lys Gln His Val Ser Glu Lys Glu Met Asn Arg
   65                  70                  75                  80
   cgc aag gat atg gtt ggg aat ttg aga tca aaa aca aat cag gtg gcc       464
   Arg Lys Asp Met Val Gly Asn Leu Arg Ser Lys Thr Asn Gln Val Ala
                   85                  90                  95
   tct gct ttg aat atg tca aac ttt gca aac aga gac agc ttg ttt gga       512
   Ser Ala Leu Asn Met Ser Asn Phe Ala Asn Arg Asp Ser Leu Phe Gly
               100                 105                 110
   aca gat tta aag ccg gat gat gcg ata aat aga gtc tct ggc atg gac       560
   Thr Asp Leu Lys Pro Asp Asp Ala Ile Asn Arg Val Ser Gly Met Asp
           115                 120                 125
   aac caa gga att gtt gta ttt caa cgg caa gtt atg aga gaa caa gac       608
   Asn Gln Gly Ile Val Val Phe Gln Arg Gln Val Met Arg Glu Gln Asp
       130                 135                 140
   gag gga ctt gag aag ttg gag gaa aca gtc atg agt acc aaa cac att       656
   Glu Gly Leu Glu Lys Leu Glu Glu Thr Val Met Ser Thr Lys His Ile
   145                 150                 155                 160
   gct ctc gct gtt aac gag gag ctc acc ctg cag aca agg ctt att gat       704
   Ala Leu Ala Val Asn Glu Glu Leu Thr Leu Gln Thr Arg Leu Ile Asp
                   165                 170                 175
   gac tta gat tac gat gtg gat atc act gac tct cgc tta cgg cgt gtt       752
   Asp Leu Asp Tyr Asp Val Asp Ile Thr Asp Ser Arg Leu Arg Arg Val
               180                 185                 190
   caa aag agc ctt gcc ttg atg aac aag agc atg aaa agt ggt tgc tca       800
   Gln Lys Ser Leu Ala Leu Met Asn Lys Ser Met Lys Ser Gly Cys Ser
           195                 200                 205
   tgc atg tct atg ctc ttg tct gtg ctt gga atc gtt ggt ctt gct ctt       848
   Cys Met Ser Met Leu Leu Ser Val Leu Gly Ile Val Gly Leu Ala Leu
       210                 215                 220
   gta att tgg ctg ctg gtt aag tac ctg taataatgcc aatgtggtgg             895
   Val Ile Trp Leu Leu Val Lys Tyr Leu
   225                 230
   caacttgtga aagctcatcc ttttctctca gcctatcctc tgtgcttaat ggttgttttc     955
   tattccttct atcgattgat tcgtgtctgt gaggcaaaga agaataccac tgcgtgtaag    1015
   aaaccctcag aagtacataa tctgtattac cttcgtatca accacgaatt gtaaactaag    1075
   ttgacatttg tctatatatg gtatggctcc tacttggttc aataaagaga actagtggc     1134
<210> 119
   <211> 233
   <212> PRT
   <213> Arabidopsis thaliana (Mouse-ear cress)
<400> 119
   Met Ala Ser Ser Ser Asp Pro Trp Met Arg Glu Tyr Asn Glu Ala Leu
   1               5                   10                  15
   Lys Leu Ser Glu Asp Ile Asn Gly Met Met Ser Glu Arg Asn Ala Ser
               20                  25                  30
   Gly Leu Thr Gly Pro Asp Ala Gln Arg Arg Ala Ser Ala Ile Arg Arg
           35                  40                  45
   Lys Ile Thr Ile Leu Gly Thr Arg Leu Asp Ser Leu Gln Ser Leu Leu
       50                  55                  60
   Val Lys Val Pro Gly Lys Gln His Val Ser Glu Lys Glu Met Asn Arg
   65                  70                  75                  80
   Arg Lys Asp Met Val Gly Asn Leu Arg Ser Lys Thr Asn Gln Val Ala
                   85                  90                  95
   Ser Ala Leu Asn Met Ser Asn Phe Ala Asn Arg Asp Ser Leu Phe Gly
               100                 105                 110
   Thr Asp Leu Lys Pro Asp Asp Ala Ile Asn Arg Val Ser Gly Met Asp
           115                 120                 125
   Asn Gln Gly Ile Val Val Phe Gln Arg Gln Val Met Arg Glu Gln Asp
       130                 135                 140
   Glu Gly Leu Glu Lys Leu Glu Glu Thr Val Met Ser Thr Lys His Ile
   145                 150                 155                 160
   Ala Leu Ala Val Asn Glu Glu Leu Thr Leu Gln Thr Arg Leu Ile Asp
                   165                 170                 175
   Asp Leu Asp Tyr Asp Val Asp Ile Thr Asp Ser Arg Leu Arg Arg Val
               180                 185                 190
   Gln Lys Ser Leu Ala Leu Met Asn Lys Ser Met Lys Ser Gly Cys Ser
           195                 200                 205
   Cys Met Ser Met Leu Leu Ser Val Leu Gly Ile Val Gly Leu Ala Leu
       210                 215                 220
   Val Ile Trp Leu Leu Val Lys Tyr Leu
   225                 230
<210> 120
   <211> 1047
   <212> DNA
   <213> Ashbya gossypii (Yeast) (Eremothecium gossypii)
<400> 120
   atg gtc aag aag ctt aat gtc cat gtg acg ata tcc gac gcc agc gtg     48
   Met Val Lys Lys Leu Asn Val His Val Thr Ile Ser Asp Ala Ser Val
   1               5                   10                  15
   gtg aat aag tca tat gta cag tat act acg agg gtt agg gtg cag cac     96
   Val Asn Lys Ser Tyr Val Gln Tyr Thr Thr Arg Val Arg Val Gln His
               20                  25                  30
   ggg tcg gag tct gca gtg gaa tac aag tgc aga agg cgg ttc agc gag     144
   Gly Ser Glu Ser Ala Val Glu Tyr Lys Cys Arg Arg Arg Phe Ser Glu
           35                  40                  45
   ttt ctg cag ctg aag ctg gat ctg gag cgg gaa ttt gac gcg gag ata     192
   Phe Leu Gln Leu Lys Leu Asp Leu Glu Arg Glu Phe Asp Ala Glu Ile
       50                  55                  60
   cca tac gac ttc cct gcg cgc aag ttc aat cta tgg aac atg aag tcg     240
   Pro Tyr Asp Phe Pro Ala Arg Lys Phe Asn Leu Trp Asn Met Lys Ser
   65                  70                  75                  80
   cgg tcg tgc gac ccg gcg gtg gtg gac gag cgg cgg gag aga ctg acg     288
   Arg Ser Cys Asp Pro Ala Val Val Asp Glu Arg Arg Glu Arg Leu Thr
                   85                  90                  95
   agc ttt ttg acc gac ctg ctc aac gac tcg ttt gat gtg cgt tgg aag     336
   Ser Phe Leu Thr Asp Leu Leu Asn Asp Ser Phe Asp Val Arg Trp Lys
               100                 105                 110
   aca tcg ccg acg ctg tgc gcg ttt ctg aac atg ccg gac gac tgg tgg     384
   Thr Ser Pro Thr Leu Cys Ala Phe Leu Asn Met Pro Asp Asp Trp Trp
           115                 120                 125
   cag cag tcg gag cag cgg ggc tcg agc gcc gcg gag agt gag gcg gac     432
   Gln Gln Ser Glu Gln Arg Gly Ser Ser Ala Ala Glu Ser Glu Ala Asp
       130                 135                 140
   tcg gtg gag cag ctg cag gac gtg tcc aaa tgg ctg gag tcg att cgc     480
   Ser Val Glu Gln Leu Gln Asp Val Ser Lys Trp Leu Glu Ser Ile Arg
   145                 150                 155                 160
   gac gcc aag tcg cag ttc gag gac gca aac cgt aat ggc aac aac atc     528
   Asp Ala Lys Ser Gln Phe Glu Asp Ala Asn Arg Asn Gly Asn Asn Ile
                   165                 170                 175
   acg atg atg cgg atc cgg ctg aag ctg cag aag ctc gaa gag gcg ctg     576
   Thr Met Met Arg Ile Arg Leu Lys Leu Gln Lys Leu Glu Glu Ala Leu
               180                 185                 190
   gca gtg atc cag gag aat aag ctt gtg ggc gag ggc gag atc agc cgt     624
   Ala Val Ile Gln Glu Asn Lys Leu Val Gly Glu Gly Glu Ile Ser Arg
           195                 200                 205
   cgc tgg atc atc ttg aac gcg ttg aag gcg gac ctc aac aag cag tcg     672
   Arg Trp Ile Ile Leu Asn Ala Leu Lys Ala Asp Leu Asn Lys Gln Ser
       210                 215                 220
   ggc gcg ctg cgg ccg cgc agc aac gat aac gag tac atg cag cgt gag     720
   Gly Ala Leu Arg Pro Arg Ser Asn Asp Asn Glu Tyr Met Gln Arg Glu
   225                 230                 235                 240
   ctg ctg aag gag cag ctg ttg cca gcc aag tct gag ccg cac agg ccc     768
   Leu Leu Lys Glu Gln Leu Leu Pro Ala Lys Ser Glu Pro His Arg Pro
                   245                 250                 255
   gct gcc ggc cgg cgg aag ctc ggc gag act agc caa aca gtt ggc ctc     816
   Ala Ala Gly Arg Arg Lys Leu Gly Glu Thr Ser Gln Thr Val Gly Leu
               260                 265                 270
   aac aat cag cag ctg ctt cag ctc cac aaa gac agc atg aag gac cag     864
   Asn Asn Gln Gln Leu Leu Gln Leu His Lys Asp Ser Met Lys Asp Gln
           275                 280                 285
   gac ttc gag ctg gaa caa cta cgc agc ata gtc cag cgc cag aag att     912
   Asp Phe Glu Leu Glu Gln Leu Arg Ser Ile Val Gln Arg Gln Lys Ile
       290                 295                 300
   atg tca ctg aac atg aac cag gag ctc gcg atc cag aac gag atg cta     960
   Met Ser Leu Asn Met Asn Gln Glu Leu Ala Ile Gln Asn Glu Met Leu
   305                 310                 315                 320
   gat atg ttt gcg gac gac gtt aac gcc aca tcc aac aaa tta cgc atg     1008
   Asp Met Phe Ala Asp Asp Val Asn Ala Thr Ser Asn Lys Leu Arg Met
                   325                 330                 335
   gcc aac atc agc gcg aaa agg ttc aac gag aga aag taa                 1047
   Ala Asn Ile Ser Ala Lys Arg Phe Asn Glu Arg Lys
               340                 345
<210> 121
   <211> 348
   <212> PRT
   <213> Ashbya gossypii (Yeast) (Eremothecium gossypii)
<400> 121
   Met Val Lys Lys Leu Asn Val His Val Thr Ile Ser Asp Ala Ser Val
   1               5                   10                  15
   Val Asn Lys Ser Tyr Val Gln Tyr Thr Thr Arg Val Arg Val Gln His
               20                  25                  30
   Gly Ser Glu Ser Ala Val Glu Tyr Lys Cys Arg Arg Arg Phe Ser Glu
           35                  40                  45
   Phe Leu Gln Leu Lys Leu Asp Leu Glu Arg Glu Phe Asp Ala Glu Ile
       50                  55                  60
   Pro Tyr Asp Phe Pro Ala Arg Lys Phe Asn Leu Trp Asn Met Lys Ser
   65                  70                  75                  80
   Arg Ser Cys Asp Pro Ala Val Val Asp Glu Arg Arg Glu Arg Leu Thr
                   85                  90                  95
   Ser Phe Leu Thr Asp Leu Leu Asn Asp Ser Phe Asp Val Arg Trp Lys
               100                 105                 110
   Thr Ser Pro Thr Leu Cys Ala Phe Leu Asn Met Pro Asp Asp Trp Trp
           115                 120                 125
   Gln Gln Ser Glu Gln Arg Gly Ser Ser Ala Ala Glu Ser Glu Ala Asp
       130                 135                 140
   Ser Val Glu Gln Leu Gln Asp Val Ser Lys Trp Leu Glu Ser Ile Arg
   145                 150                 155                 160
   Asp Ala Lys Ser Gln Phe Glu Asp Ala Asn Arg Asn Gly Asn Asn Ile
                   165                 170                 175
   Thr Met Met Arg Ile Arg Leu Lys Leu Gln Lys Leu Glu Glu Ala Leu
               180                 185                 190
   Ala Val Ile Gln Glu Asn Lys Leu Val Gly Glu Gly Glu Ile Ser Arg
           195                 200                 205
   Arg Trp Ile Ile Leu Asn Ala Leu Lys Ala Asp Leu Asn Lys Gln Ser
       210                 215                 220
   Gly Ala Leu Arg Pro Arg Ser Asn Asp Asn Glu Tyr Met Gln Arg Glu
   225                 230                 235                 240
   Leu Leu Lys Glu Gln Leu Leu Pro Ala Lys Ser Glu Pro His Arg Pro
                   245                 250                 255
   Ala Ala Gly Arg Arg Lys Leu Gly Glu Thr Ser Gln Thr Val Gly Leu
               260                 265                 270
   Asn Asn Gln Gln Leu Leu Gln Leu His Lys Asp Ser Met Lys Asp Gln
           275                 280                 285
   Asp Phe Glu Leu Glu Gln Leu Arg Ser Ile Val Gln Arg Gln Lys Ile
       290                 295                 300
   Met Ser Leu Asn Met Asn Gln Glu Leu Ala Ile Gln Asn Glu Met Leu
   305                 310                 315                 320
   Asp Met Phe Ala Asp Asp Val Asn Ala Thr Ser Asn Lys Leu Arg Met
                   325                 330                 335
   Ala Asn Ile Ser Ala Lys Arg Phe Asn Glu Arg Lys
               340                 345
<210> 122
   <211> 25
   <212> DNA
   <213> Saccharomyces cerevisiae
<400> 122
   atggcagcta attctgtagg gaaaa 25
<210> 123
   <211> 26
   <212> DNA
   <213> Saccharomyces cerevisiae
<400> 123
   tcaagcactg ttgttaaaat gtctag 26
<210> 124
   <211> 348
   <212> DNA
   <213> Saccharomyces cerevisiae
<400> 124
   atg ggt agt ttt tgg gac gca ttc gca gta tac gac aag aaa aag cac        48
   Met Gly Ser Phe Trp Asp Ala Phe Ala Val Tyr Asp Lys Lys Lys His
   1               5                   10                  15
   gca gat cca agt gta tat gga gga aac cat aac aac aca gga gac agt        96
   Ala Asp Pro Ser Val Tyr Gly Gly Asn His Asn Asn Thr Gly Asp Ser
               20                  25                  30
   aaa acg cag gtt atg ttt tcg aaa gag tac cgt caa cct agg aca cat       144
   Lys Thr Gln Val Met Phe Ser Lys Glu Tyr Arg Gln Pro Arg Thr His
           35                  40                  45
   cag caa gag aac ttg cag agc atg aga aga tct tcc ata gga tca cag       192
   Gln Gln Glu Asn Leu Gln Ser Met Arg Arg Ser Ser Ile Gly Ser Gln
       50                  55                  60
   gac agt tcc gat gtt gag gac gtt aag gaa ggg aga tta ccc gca gaa       240
   Asp Ser Ser Asp Val Glu Asp Val Lys Glu Gly Arg Leu Pro Ala Glu
   65                  70                  75                  80
   gta gaa ata cca aag aat gtt gac atc tct aac atg tcg caa ggt gag       288
   Val Glu Ile Pro Lys Asn Val Asp Ile Ser Asn Met Ser Gln Gly Glu
                   85                  90                  95
   ttt tta aga ctt tac gaa agt ttg agg agg ggg gaa ccc gac aat aaa       336
   Phe Leu Arg Leu Tyr Glu Ser Leu Arg Arg Gly Glu Pro Asp Asn Lys
               100                 105                 110
   gta aat aga taa                                                       348
   Val Asn Arg
           115
<210> 125
   <211> 115
   <212> PRT
   <213> Saccharomyces cerevisiae
<400> 125
   Met Gly Ser Phe Trp Asp Ala Phe Ala Val Tyr Asp Lys Lys Lys His
   1               5                   10                  15
   Ala Asp Pro Ser Val Tyr Gly Gly Asn His Asn Asn Thr Gly Asp Ser
               20                  25                  30
   Lys Thr Gln Val Met Phe Ser Lys Glu Tyr Arg Gln Pro Arg Thr His
           35                  40                  45
   Gln Gln Glu Asn Leu Gln Ser Met Arg Arg Ser Ser Ile Gly Ser Gln
       50                  55                  60
   Asp Ser Ser Asp Val Glu Asp Val Lys Glu Gly Arg Leu Pro Ala Glu
   65                  70                  75                  80
   Val Glu Ile Pro Lys Asn Val Asp Ile Ser Asn Met Ser Gln Gly Glu
                   85                  90                  95
   Phe Leu Arg Leu Tyr Glu Ser Leu Arg Arg Gly Glu Pro Asp Asn Lys
               100                 105                 110
   Val Asn Arg
           115
<210> 126
   <211> 24
   <212> DNA
   <213> Saccharomyces cerevisiae (Baker's yeast)
<400> 126
   atgggtagtt tttgggacgc attc 24
<210> 127
   <211> 27
   <212> DNA
   <213> Saccharomyces cerevisiae (Baker's yeast)
<400> 127
   ttatctattt actttattgt cgggttc 27
<210> 128
   <211> 987
   <212> DNA
   <213> Saccharomyces cerevisiae
<400> 128
   atg gaa aaa aaa cat gtc act gtg caa ata caa agt gct ccc ccc tcc      48
   Met Glu Lys Lys His Val Thr Val Gln Ile Gln Ser Ala Pro Pro Ser
   1               5                   10                  15
   tat atc aaa ttg gaa gca aat gaa aaa ttc gta tat att aca agt aca      96
   Tyr Ile Lys Leu Glu Ala Asn Glu Lys Phe Val Tyr Ile Thr Ser Thr
               20                  25                  30
   atg aac ggc tta tct tat caa att gcg gct ata gtt tca tac cca gaa      144
   Met Asn Gly Leu Ser Tyr Gln Ile Ala Ala Ile Val Ser Tyr Pro Glu
           35                  40                  45
   aag aga aat tca tca act gca aat aaa gaa gat ggt aaa tta ctg tgc      192
   Lys Arg Asn Ser Ser Thr Ala Asn Lys Glu Asp Gly Lys Leu Leu Cys
       50                  55                  60
   aag gaa aat aaa cta gca ttg tta cta cac gga agt caa tct cac aag      240
   Lys Glu Asn Lys Leu Ala Leu Leu Leu His Gly Ser Gln Ser His Lys
   65                  70                  75                  80
   aac gct att tat caa act tta cta gca aaa agg ctg gcc gaa ttc gga      288
   Asn Ala Ile Tyr Gln Thr Leu Leu Ala Lys Arg Leu Ala Glu Phe Gly
                   85                  90                  95
   tat tgg gta cta aga ata gat ttt agg ggc caa ggt gat tcc tca gat      336
   Tyr Trp Val Leu Arg Ile Asp Phe Arg Gly Gln Gly Asp Ser Ser Asp
               100                 105                 110
   aac tgc gac cct ggc ctt ggt agg acg ctc gct cag gat ctt gaa gat      384
   Asn Cys Asp Pro Gly Leu Gly Arg Thr Leu Ala Gln Asp Leu Glu Asp
           115                 120                 125
   ttg agt aca gta tac caa aca gta tct gac agg tct ctt agg gtg caa      432
   Leu Ser Thr Val Tyr Gln Thr Val Ser Asp Arg Ser Leu Arg Val Gln
       130                 135                 140
   ttg tac aaa act agt aca ata tca ctg gac gtg gtt gtg gca cat tct      480
   Leu Tyr Lys Thr Ser Thr Ile Ser Leu Asp Val Val Val Ala His Ser
   145                 150                 155                 160
   aga gga tct ctt gcc atg ttc aaa ttc tgt cta aaa tta cat gca gct      528
   Arg Gly Ser Leu Ala Met Phe Lys Phe Cys Leu Lys Leu His Ala Ala
                   165                 170                 175
   gaa tct cca tta ccg tct cac ctg atc aat tgc gct gga aga tat gat      576
   Glu Ser Pro Leu Pro Ser His Leu Ile Asn Cys Ala Gly Arg Tyr Asp
               180                 185                 190
   ggg aga gga ctt att gaa cgc tgc aca cga ctg cac ccg cat tgg caa      624
   Gly Arg Gly Leu Ile Glu Arg Cys Thr Arg Leu His Pro His Trp Gln
           195                 200                 205
   gca gaa ggt ggg ttt tgg gcg aat ggt cca cga aat ggc gaa tac aaa      672
   Ala Glu Gly Gly Phe Trp Ala Asn Gly Pro Arg Asn Gly Glu Tyr Lys
       210                 215                 220
   gac ttt tgg ata cca tta agt gag act tat agt atc gct ggc gtt tgc      720
   Asp Phe Trp Ile Pro Leu Ser Glu Thr Tyr Ser Ile Ala Gly Val Cys
   225                 230                 235                 240
   gtt ccg gaa ttt gcc acg ata cca caa act tgt tca gta atg tcc tgc      768
   Val Pro Glu Phe Ala Thr Ile Pro Gln Thr Cys Ser Val Met Ser Cys
                   245                 250                 255
   tat ggc atg tgt gat cac ata gtg cca att agc gca gcc tca aat tat      816
   Tyr Gly Met Cys Asp His Ile Val Pro Ile Ser Ala Ala Ser Asn Tyr
               260                 265                 270
   gca agg ctt ttc gag ggc aga cat tca ttg aaa ctt att gaa aat gcg      864
   Ala Arg Leu Phe Glu Gly Arg His Ser Leu Lys Leu Ile Glu Asn Ala
           275                 280                 285
   gac cac aat tat tat ggc att gaa ggt gat ccc aac gcg cta ggc tta      912
   Asp His Asn Tyr Tyr Gly Ile Glu Gly Asp Pro Asn Ala Leu Gly Leu
       290                 295                 300
   ccg ata agg agg ggt aga gtc aac tac tca cca cta gta gtt gat cta       960
   Pro Ile Arg Arg Gly Arg Val Asn Tyr Ser Pro Leu Val Val Asp Leu
   305                 310                 315                 320
   att atg gaa tac ctg caa gat aca tag                                   987
   Ile Met Glu Tyr Leu Gln Asp Thr
                   325
<210> 129
   <211> 328
   <212> PRT
   <213> Saccharomyces cerevisiae
<400> 129
   Met Glu Lys Lys His Val Thr Val Gln Ile Gln Ser Ala Pro Pro Ser
   1               5                   10                  15
   Tyr Ile Lys Leu Glu Ala Asn Glu Lys Phe Val Tyr Ile Thr Ser Thr
               20                  25                  30
   Met Asn Gly Leu Ser Tyr Gln Ile Ala Ala Ile Val Ser Tyr Pro Glu
           35                  40                  45
   Lys Arg Asn Ser Ser Thr Ala Asn Lys Glu Asp Gly Lys Leu Leu Cys
       50                  55                  60
   Lys Glu Asn Lys Leu Ala Leu Leu Leu His Gly Ser Gln Ser His Lys
   65                  70                  75                  80
   Asn Ala Ile Tyr Gln Thr Leu Leu Ala Lys Arg Leu Ala Glu Phe Gly
                   85                  90                  95
   Tyr Trp Val Leu Arg Ile Asp Phe Arg Gly Gln Gly Asp Ser Ser Asp
               100                 105                 110
   Asn Cys Asp Pro Gly Leu Gly Arg Thr Leu Ala Gln Asp Leu Glu Asp
           115                 120                 125
   Leu Ser Thr Val Tyr Gln Thr Val Ser Asp Arg Ser Leu Arg Val Gln
       130                 135                 140
   Leu Tyr Lys Thr Ser Thr Ile Ser Leu Asp Val Val Val Ala His Ser
   145                 150                 155                 160
   Arg Gly Ser Leu Ala Met Phe Lys Phe Cys Leu Lys Leu His Ala Ala
                   165                 170                 175
   Glu Ser Pro Leu Pro Ser His Leu Ile Asn Cys Ala Gly Arg Tyr Asp
               180                 185                 190
   Gly Arg Gly Leu Ile Glu Arg Cys Thr Arg Leu His Pro His Trp Gln
           195                 200                 205
   Ala Glu Gly Gly Phe Trp Ala Asn Gly Pro Arg Asn Gly Glu Tyr Lys
       210                 215                 220
   Asp Phe Trp Ile Pro Leu Ser Glu Thr Tyr Ser Ile Ala Gly Val Cys
   225                 230                 235                 240
   Val Pro Glu Phe Ala Thr Ile Pro Gln Thr Cys Ser Val Met Ser Cys
                   245                 250                 255
   Tyr Gly Met Cys Asp His Ile Val Pro Ile Ser Ala Ala Ser Asn Tyr
               260                 265                 270
   Ala Arg Leu Phe Glu Gly Arg His Ser Leu Lys Leu Ile Glu Asn Ala
           275                 280                 285
   Asp His Asn Tyr Tyr Gly Ile Glu Gly Asp Pro Asn Ala Leu Gly Leu
       290                 295                 300
   Pro Ile Arg Arg Gly Arg Val Asn Tyr Ser Pro Leu Val Val Asp Leu
   305                 310                 315                 320
   Ile Met Glu Tyr Leu Gln Asp Thr
                   325
<210> 130
   <211> 25
   <212> DNA
   <213> Saccharomyces cerevisiae (Baker's yeast)
<400> 130
   atggaaaaaa aacatgtcac tgtgc 25
<210> 131
   <211> 25
   <212> DNA
   <213> Saccharomyces cerevisiae (Baker's yeast)
<400> 131
   ctatgtatct tgcaggtatt ccata 25
<210> 132
   <211> 989
   <212> DNA
   <213> Brassica napus
<220>
   <221> CDS
   <222> (63)..(830)
<400> 132
   tcatctgaca cacacacact ctctctctct ctctctctct ctctcatcac gacgccgccg      60
   ca atg acc gtg gga gta tta gct tta caa ggc tct ttc aac gag cac        107
      Met Thr Val Gly Val Leu Ala Leu Gln Gly Ser Phe Asn Glu His
      1               5                   10                  15
   atc gcg gct ctg cgg cgg cta ggc gtc caa gga atc gag att agg aag       155
   Ile Ala Ala Leu Arg Arg Leu Gly Val Gln Gly Ile Glu Ile Arg Lys
                   20                  25                  30
   gcg gag cag ctt ctc acc gtt tca tct ctc ata atc cct ggc ggc gag       203
   Ala Glu Gln Leu Leu Thr Val Ser Ser Leu Ile Ile Pro Gly Gly Glu
               35                  40                  45
   agc acc acc atg gcc aaa ctg gcc gag tac cac aac ctg ttc ccg gct       251
   Ser Thr Thr Met Ala Lys Leu Ala Glu Tyr His Asn Leu Phe Pro Ala
           50                  55                  60
   cta cgt gag ttt gtc aag acg ggg aaa cct gtt tgg ggg aca tgc gct       299
   Leu Arg Glu Phe Val Lys Thr Gly Lys Pro Val Trp Gly Thr Cys Ala
       65                  70                  75
   ggt ctt atc ttc ttg gca gac aga gca gtt ggt cag aaa gag gga ggt       347
   Gly Leu Ile Phe Leu Ala Asp Arg Ala Val Gly Gln Lys Glu Gly Gly
   80                  85                  90                  95
   caa gaa cta gtt ggt ggc ctt gac tgc acc gta cac agg aac ttc ttt       395
   Gln Glu Leu Val Gly Gly Leu Asp Cys Thr Val His Arg Asn Phe Phe
                   100                 105                 110
   ggc agc cag att caa agt ttt gaa gct gat atc tct gta cct att cta       443
   Gly Ser Gln Ile Gln Ser Phe Glu Ala Asp Ile Ser Val Pro Ile Leu
               115                 120                 125
   aca tct aaa gaa ggt ggg ccg gag aca tac cga gga gtc ttc ata cgc       491
   Thr Ser Lys Glu Gly Gly Pro Glu Thr Tyr Arg Gly Val Phe Ile Arg
           130                 135                 140
   gct cca gct gtt ctc gat gtt ggc cct gat gtc gag gtt tta gcg cat       539
   Ala Pro Ala Val Leu Asp Val Gly Pro Asp Val Glu Val Leu Ala His
       145                 150                 155
   tat ccc gtc cca tca aac aag gtc ttg tat tca agc tct act gtc caa       587
   Tyr Pro Val Pro Ser Asn Lys Val Leu Tyr Ser Ser Ser Thr Val Gln
   160                 165                 170                 175
   atc caa gag gaa gat gct ctt cta gag acg aac gtc att gtt gcg gtg       635
   Ile Gln Glu Glu Asp Ala Leu Leu Glu Thr Asn Val Ile Val Ala Val
                   180                 185                 190
   aag caa aga aac ttg tta gcg act gcg ttt cat ccc gag tta ccc gca       683
   Lys Gln Arg Asn Leu Leu Ala Thr Ala Phe His Pro Glu Leu Pro Ala
               195                 200                 205
   gac ccg cga tgg cac agt ttt ttc atg aaa atg gcg aaa gag atg gaa       731
   Asp Pro Arg Trp His Ser Phe Phe Met Lys Met Ala Lys Glu Met Glu
           210                 215                 220
   caa ggg gct tct tca agc agt ggt gga act ttt gtt ttt gtt ggg gaa       779
   Gln Gly Ala Ser Ser Ser Ser Gly Gly Thr Phe Val Phe Val Gly Glu
       225                 230                 235
   acc agc gtt ggt ccc ggg caa act aag cct gat ttt cct ata tat cgg       827
   Thr Ser Val Gly Pro Gly Gln Thr Lys Pro Asp Phe Pro Ile Tyr Arg
   240                 245                 250                 255
   taattaaaat ggggggaaga cactcacttc tcttgaaata aaatagaaaa gtgtcagatt      887
   ctttttgatg ttttggaaag aaaatgtcaa tctagtttgc atttgtcaca aaaaaaaaaa    947
   aaaaaaaaaa aaaaaaaaaa aaaaaaaaaa aaaaaaaaaa aa                       989
<210> 133
   <211> 255
   <212> PRT
   <213> Brassica napus
<400> 133
   Met Thr Val Gly Val Leu Ala Leu Gln Gly Ser Phe Asn Glu His Ile
   1               5                   10                  15
   Ala Ala Leu Arg Arg Leu Gly Val Gln Gly Ile Glu Ile Arg Lys Ala
               20                  25                  30
   Glu Gln Leu Leu Thr Val Ser Ser Leu Ile Ile Pro Gly Gly Glu Ser
           35                  40                  45
   Thr Thr Met Ala Lys Leu Ala Glu Tyr His Asn Leu Phe Pro Ala Leu
       50                  55                  60
   Arg Glu Phe Val Lys Thr Gly Lys Pro Val Trp Gly Thr Cys Ala Gly
   65                  70                  75                  80
   Leu Ile Phe Leu Ala Asp Arg Ala Val Gly Gln Lys Glu Gly Gly Gln
                   85                  90                  95
   Glu Leu Val Gly Gly Leu Asp Cys Thr Val His Arg Asn Phe Phe Gly
               100                 105                 110
   Ser Gln Ile Gln Ser Phe Glu Ala Asp Ile Ser Val Pro Ile Leu Thr
           115                 120                 125
   Ser Lys Glu Gly Gly Pro Glu Thr Tyr Arg Gly Val Phe Ile Arg Ala
       130                 135                 140
   Pro Ala Val Leu Asp Val Gly Pro Asp Val Glu Val Leu Ala His Tyr
   145                 150                 155                 160
   Pro Val Pro Ser Asn Lys Val Leu Tyr Ser Ser Ser Thr Val Gln Ile
                   165                 170                 175
   Gln Glu Glu Asp Ala Leu Leu Glu Thr Asn Val Ile Val Ala Val Lys
               180                 185                 190
   Gln Arg Asn Leu Leu Ala Thr Ala Phe His Pro Glu Leu Pro Ala Asp
           195                 200                 205
   Pro Arg Trp His Ser Phe Phe Met Lys Met Ala Lys Glu Met Glu Gln
       210                 215                 220
   Gly Ala Ser Ser Ser Ser Gly Gly Thr Phe Val Phe Val Gly Glu Thr
   225                 230                 235                 240
   Ser Val Gly Pro Gly Gln Thr Lys Pro Asp Phe Pro Ile Tyr Arg
                   245                 250                 255
<210> 134
   <211> 1042
   <212> DNA
   <213> Glycine max
<220>
   <221> CDS
   <222> (61)..(825)
<400> 134
   gttcaaaacc tttttcaacc acctcaaaac gctgctatct ctttctccac tctccccaac      60
   atg gcc gtc gtt ggc gtc ctc gcg ctg caa gga tct ttc aac gaa cac       108
   Met Ala Val Val Gly Val Leu Ala Leu Gln Gly Ser Phe Asn Glu His
   1               5                   10                  15
   ata gct gct ctt aga agg tta ggg gtg caa ggc gtg gag att cga aag       156
   Ile Ala Ala Leu Arg Arg Leu Gly Val Gln Gly Val Glu Ile Arg Lys
               20                  25                  30
   cca gag cag ctt aac aca att agt tcc ctc att atc cct ggt gga gaa       204
   Pro Glu Gln Leu Asn Thr Ile Ser Ser Leu Ile Ile Pro Gly Gly Glu
           35                  40                  45
   agc acc acc atg gct aag ctc gcc gag tat cac aac ctg ttt cct gct      252
   Ser Thr Thr Met Ala Lys Leu Ala Glu Tyr His Asn Leu Phe Pro Ala
       50                  55                  60
   ttg cga gag ttt gta caa atg gga aag cct gtt tgg gga acc tgt gca      300
   Leu Arg Glu Phe Val Gln Met Gly Lys Pro Val Trp Gly Thr Cys Ala
   65                  70                  75                  80
   ggg ctt ata ttc ttg gca aat aaa gct ata gga cag aag act ggt ggt      348
   Gly Leu Ile Phe Leu Ala Asn Lys Ala Ile Gly Gln Lys Thr Gly Gly
                   85                  90                  95
   caa tat ttg gtt ggt gga ctt gat tgt aca gtg cat aga aat ttc ttt      396
   Gln Tyr Leu Val Gly Gly Leu Asp Cys Thr Val His Arg Asn Phe Phe
               100                 105                 110
   ggc agc cag att caa agc ttt gag gca gag ctt tca gtg ccg gag ctt      444
   Gly Ser Gln Ile Gln Ser Phe Glu Ala Glu Leu Ser Val Pro Glu Leu
           115                 120                 125
   gtc tcc aag gaa gga ggt cct gaa aca ttt tgt gga att ttt att cgt      492
   Val Ser Lys Glu Gly Gly Pro Glu Thr Phe Cys Gly Ile Phe Ile Arg
       130                 135                 140
   gcc cct gca att ctt gaa gca ggg cca gaa gtt caa gtg ctg gct gat      540
   Ala Pro Ala Ile Leu Glu Ala Gly Pro Glu Val Gln Val Leu Ala Asp
   145                 150                 155                 160
   tat cct gta cct tct agc aga ttg ttg agt tct gat tcc tct att gaa      588
   Tyr Pro Val Pro Ser Ser Arg Leu Leu Ser Ser Asp Ser Ser Ile Glu
                   165                 170                 175
   gac caa acg gag aat gct gag aaa gaa agt aaa gtt ata gtt gct gtg      636
   Asp Gln Thr Glu Asn Ala Glu Lys Glu Ser Lys Val Ile Val Ala Val
               180                 185                 190
   aga caa ggg aac ata tta gcc act gct ttc cat cct gaa ttg aca gcc      684
   Arg Gln Gly Asn Ile Leu Ala Thr Ala Phe His Pro Glu Leu Thr Ala
           195                 200                 205
   gat act cga tgg cat agt tat ttc gta aaa atg tca aat gaa att aga      732
   Asp Thr Arg Trp His Ser Tyr Phe Val Lys Met Ser Asn Glu Ile Arg
       210                 215                 220
   gaa gag gcc tct tcg agt agc ctt gtt cct gca caa gtc agt agt aca      780
   Glu Glu Ala Ser Ser Ser Ser Leu Val Pro Ala Gln Val Ser Ser Thr
   225                 230                 235                 240
   agt caa tat caa cag ccc cgg aat gac ctt cct atc tat cga taggaccaga   832
   Ser Gln Tyr Gln Gln Pro Arg Asn Asp Leu Pro Ile Tyr Arg
                   245                 250
   atactcccca agcctttctt gaacaattgt ggatgatttt tttttctttc tatatttttc   892
   tcgaacattt tatcatataa ttgttggatc ttagaagata tagctagctg tttattattc    952
   ttttttctat ttggacaaac agtattgtat ttagactttg atgttttctg ttaagtagtc    1012
   atctatctgc cgaaaaaaaa aaaaaaaaaa                                     1042
<210> 135
   <211> 254
   <212> PRT
   <213> Glycine max
<400> 135
   Met Ala Val Val Gly Val Leu Ala Leu Gln Gly Ser Phe Asn Glu His
   1               5                   10                  15
   Ile Ala Ala Leu Arg Arg Leu Gly Val Gln Gly Val Glu Ile Arg Lys
               20                  25                  30
   Pro Glu Gln Leu Asn Thr Ile Ser Ser Leu Ile Ile Pro Gly Gly Glu
           35                  40                  45
   Ser Thr Thr Met Ala Lys Leu Ala Glu Tyr His Asn Leu Phe Pro Ala
       50                  55                  60
   Leu Arg Glu Phe Val Gln Met Gly Lys Pro Val Trp Gly Thr Cys Ala
   65                  70                  75                  80
   Gly Leu Ile Phe Leu Ala Asn Lys Ala Ile Gly Gln Lys Thr Gly Gly
                   85                  90                  95
   Gln Tyr Leu Val Gly Gly Leu Asp Cys Thr Val His Arg Asn Phe Phe
               100                 105                 110
   Gly Ser Gln Ile Gln Ser Phe Glu Ala Glu Leu Ser Val Pro Glu Leu
           115                 120                 125
   Val Ser Lys Glu Gly Gly Pro Glu Thr Phe Cys Gly Ile Phe Ile Arg
       130                 135                 140
   Ala Pro Ala Ile Leu Glu Ala Gly Pro Glu Val Gln Val Leu Ala Asp
   145                 150                 155                 160
   Tyr Pro Val Pro Ser Ser Arg Leu Leu Ser Ser Asp Ser Ser Ile Glu
                   165                 170                 175
   Asp Gln Thr Glu Asn Ala Glu Lys Glu Ser Lys Val Ile Val Ala Val
               180                 185                 190
   Arg Gln Gly Asn Ile Leu Ala Thr Ala Phe His Pro Glu Leu Thr Ala
           195                 200                 205
   Asp Thr Arg Trp His Ser Tyr Phe Val Lys Met Ser Asn Glu Ile Arg
       210                 215                 220
   Glu Glu Ala Ser Ser Ser Ser Leu Val Pro Ala Gln Val Ser Ser Thr
   225                 230                 235                 240
   Ser Gln Tyr Gln Gln Pro Arg Asn Asp Leu Pro Ile Tyr Arg
                   245                 250
<210> 136
   <211> 342
   <212> DNA
   <213> Saccharomyces cerevisiae
<220>
   <221> CDS
   <222> (1)..(342)
<400> 136
   atg agc att cta tca tcc aca caa tcc aca att tta cgt ata ccc tcc      48
   Met Ser Ile Leu Ser Ser Thr Gln Ser Thr Ile Leu Arg Ile Pro Ser
   1               5                   10                  15
   ggt cta att act ttt ctc ctc agc aag cta ttt ctt ttg ctc cgc gta      96
   Gly Leu Ile Thr Phe Leu Leu Ser Lys Leu Phe Leu Leu Leu Arg Val
               20                  25                  30
   gaa cct tct tca gcg tct atg tct ata tcg gag tcg gag tta tta ctc      144
   Glu Pro Ser Ser Ala Ser Met Ser Ile Ser Glu Ser Glu Leu Leu Leu
           35                  40                  45
   atg ggt aat att aac gac gaa tcc ccc aaa ccg gga aag tta gct tct      192
   Met Gly Asn Ile Asn Asp Glu Ser Pro Lys Pro Gly Lys Leu Ala Ser
       50                  55                  60
   gca cca cta gct tca ttg acc aat ctt gtt ttt tcc att gac gta aag      240
   Ala Pro Leu Ala Ser Leu Thr Asn Leu Val Phe Ser Ile Asp Val Lys
   65                  70                  75                  80
   ggc ctt act ctt ata gct acg act atg gag gat tgt ctt gtt tca ggc      288
   Gly Leu Thr Leu Ile Ala Thr Thr Met Glu Asp Cys Leu Val Ser Gly
                   85                  90                  95
   acg ttc atg tta gtg tca ata gta tac agc tgg aaa gaa aac tca agt      336
   Thr Phe Met Leu Val Ser Ile Val Tyr Ser Trp Lys Glu Asn Ser Ser
               100                 105                 110
   agt taa                                                              342
   Ser
<210> 137
   <211> 113
   <212> PRT
   <213> Saccharomyces cerevisiae
<400> 137
   Met Ser Ile Leu Ser Ser Thr Gln Ser Thr Ile Leu Arg Ile Pro Ser
   1               5                   10                  15
   Gly Leu Ile Thr Phe Leu Leu Ser Lys Leu Phe Leu Leu Leu Arg Val
               20                  25                  30
   Glu Pro Ser Ser Ala Ser Met Ser Ile Ser Glu Ser Glu Leu Leu Leu
           35                  40                  45
   Met Gly Asn Ile Asn Asp Glu Ser Pro Lys Pro Gly Lys Leu Ala Ser
       50                  55                  60
   Ala Pro Leu Ala Ser Leu Thr Asn Leu Val Phe Ser Ile Asp Val Lys
   65                  70                  75                  80
   Gly Leu Thr Leu Ile Ala Thr Thr Met Glu Asp Cys Leu Val Ser Gly
                   85                  90                  95
   Thr Phe Met Leu Val Ser Ile Val Tyr Ser Trp Lys Glu Asn Ser Ser
               100                 105                 110
   Ser
<210> 138
   <211> 25
   <212> DNA
   <213> Primer
<400> 138 25
   atgagcattc tatcatccac acaat 25
<210> 139
   <211> 26
   <212> DNA
   <213> Primer
<400> 139
   ttaactactt gagttttctt tccagc 26

## Description

The present description discloses a method for preparing a plant with faster growth and/or higher yield in comparison with a reference plant, which method comprises increasing in said plant or in one or more parts thereof the activity of SEQ ID NO: 2, in comparison with said reference organism, for example on the basis of increasing the amount of SEQ ID NO: 1, RNA and/or SEQ ID NO: 2, polypeptide, advantageously on the basis of increased expression of SEQ ID NO: 1. The description discloses a method for preparing plants, which grow faster or give higher yields, which method comprises an increased SEQ ID NO: 2, activity in said organisms, and a plant, whose SEQ ID NO: 2, activity is increased and the yield or biomass thereof. Furthermore, the description also discloses a SEQ ID NO: 2, polypeptide, a polynucleotide coding therefor and cells, plants, microorganisms and useful animals transformed therewith and methods for preparing fine chemicals by using the subject-matter disclosed by the present description.

Ever since useful plants were first cultivated, increasing the crop yield has, in addition to improving resistance to abiotic and biotic stress, been the most important goal when growing new plant varieties. Means as diverse as tilling, fertilizing, irrigation, cultivation or crop protection agents, to name but a few, are used for improving yields: Thus, cultivation successes in increasing the crop, for example by increasing the seed setting, and those in reducing the loss of crop, for example owing to bad weather, i.e. weather which is too dry, too wet, too hot or too cold, or due to infestation with pests such as, for example, insects, fungi or bacteria, complement one another. In view of the rapidly growing world population, a substantial increase in yield, without extending the economically arable areas, is absolutely necessary in order to provide sufficient food and, at the same time, protect other existing natural spaces.

The methods of classical genetics and cultivation for developing new varieties with better yields are increasingly supplemented by genetic methods. Thus, genes have been identified which are responsible for particular properties such as resistance to abiotic or biotic stress or growth rate control. Interesting genes or gene products thereof may be appropriately regulated in the desired useful plants, for example by mutation, (over)expression or reduction/inhibition of such genes or their products, in order to achieve the desired increased yield or higher tolerance to stress.

The same applies to microorganisms and useful animals, the breeding of which is primarily and especially concerned with likewise achieving a particular biomass or a particular weight more rapidly, in addition to higher resistance to biotic or abiotic stress.

One example of a strategy resulting in better or more rapid plant growth is to increase the photosynthetic capability of plants (US 6,239,332 and DE 19940270). This approach, however, is promising only if the photosynthetic performance of said plants is growth-limiting. Another approach is to modulate regulation of plant growth by influencing cell cycle control (WO 01/31041, CA 2263067, WO 00/56905, WO 00/37645). However, a change in the plant's architecture may be the undesired side effect of a massive intervention in the control of plant growth (WO 01/31041; CA 2263067). Other approaches may involve putative transcriptional regulators as for example claimed in WO 02/079403 or US 2003/013228. Such transcriptional regulators often occur in gene families, in which the family members might display significant cross talk and/or antagonistic control. In addition the function of transcription factors rely on the precise presence of their recognition sequences in the target organisms. This fact might complicate the transfer of result from model spezies to target organisms. Despite a few promising approaches, there is nevertheless still a great need of providing methods for preparing organisms with faster growth and higher yield, in particular plants and microorganisms, and of providing such organisms, in particular plants and microorganisms.

It is an object of the present invention to provide a method of this kind for increasing the yield and growth of plants.

We have found that this object is achieved by the inventive method described herein and the embodiments characterized in the claims.

Consequently, the description discloses a method for preparing a plant with increased growth rate, i.e. with faster growth and/or increased yield in comparison with a reference organism, which method comprises increasing in said plant or in one or more parts thereof the activity of SEQ ID NO: 2, in comparison with a reference organism, for example on the basis of increasing the amount of SEQ ID NO: 1, RNA and/or SEQ ID NO: 2, polypeptide.

Increased expression of SEQ ID NO: 1, in Arabidopsis thaliana has been found to lead to accelerated growth of the plants and to an increased final weight and an increased amount of seeds.

SEQ ID NO: 2 has been described as a protein of unconfirmed function, which might be involved in pyridoxine metabolism and the expression of which is induced during stationary phase. (GenBank Accession NO: PIR|S55081 for YMR095C) from Saccharomyces cerevisiae. Therefore a clear function is not mentioned in the annotation of the ORF. However, a Blastp comparison of the YMR095C (SEQ ID: 2) sequence under standard conditions revealed a significant homology to SEQ ID NO: 4, 6, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 99, 101, 103,105, 133 and 135.

A particular surprise was the finding that expression of SEQ ID NO: 1 of the evolutionarily distant yeast Saccharomyces cerevisiae increases growth in Arabidopsis thaliana. It may also be assumed that SEQ ID NO: 1 is a functionally conserved gene and that an increase in the activity of SEQ ID NO: 2 or of the specific homologs 4, 6, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 99, 101, 103, 105, 133 or 135 also leads to faster growth or increased yield in an organism in the same manner as has been observed according to the invention for SEQ ID NO: 2 and in Arabidopsis. Presumably, therefore, transgenic expression of other distant SEQ ID NO: 1 homologs in an organism also result in the observed faster growth and higher yield.

In a disclosure, the description discloses a method for preparing an organism, a cell, a tissue, e.g. a plant with increased growth rate, i.e. with faster growth and/or increased yield, which method comprises increasing in said organism or in one or more parts thereof the activity of SEQ ID NO: 2, for example on the basis of increasing the amount of SEQ ID NO: 1, RNA and/or SEQ ID NO: 2, polypeptide.

"Organism" here means any organism which is not a human being. Consequently, the term relates to prokaryotic and eukaryotic cells, microorganisms, higher and lower plants, including mosses and algae, and to nonhuman animals or cells. The organism may be unicellular or multicellular.

"Increased growth", "faster growth" or "increased growth rate" here means that the increase in weight, for example fresh weight, or in biomass per time unit is greater than that of a reference, in particular of the starting organism from which the non human organism disclosed herein is prepared. Faster growth preferably results in a higher final weight of said non human organism. Thus, for example, faster growth makes it possible to reach a particular developmental stage earlier or to prolong growth in a particular developmental stage. Preference is given to attaining a higher final weight.

The terms "wild type", "control" or "reference" are exchangeable and can be a cell or a part of organisms such as an organelle or a tissue, or an organism, in particular a microorganism or a plant, which was not modified or treated according to the herein described method. Accordingly, the cell or a part of organisms such as an organelle or a tissue, or an organism, in particular a microorganism or a plant used as wild type, control or reference corresponds to the cell, organism or part thereof as much as possible and is in any other property but in the result of the method disclosed herein as identical to the subject matter disclosed herein as possible. Thus, the wild type, control or reference is treated identically or as identical as possible, saying that only conditions or properties might be different which do not additionally influence the quality of the tested property.

Preferably, any comparison is carried out under analogous conditions. The term "analogous conditions" means that all conditions such as, for example, culture or growing conditions, assay conditions (such as buffer composition, temperature, substrates, pathogen strain, concentrations and the like) are kept identical between the experiments to be compared.

The "reference", "control", or "wild type" is preferably a subject, e.g. an organelle, a cell, a tissue, an organism, in particular a plant or a microorganism, which was not modified or treated according to the herein described method disclosed herein and is in any other property as similar to the subject matter disclosed herein as possible. The reference, control or wild type is in its genome, transcriptome, proteome or metabolome as similar as possible to the subject of the present invention. Preferably, the term "reference-" "control-" or "wild type-"-organelle, -cell, -tissue or-organism, in particular plant, relates to an organelle, cell, tissue or organism, in particular plant or microorganism, which is nearly genetically identical to the organelle, cell, tissue or organism, in particular plant, or a part thereof preferably 95%, are 98%, even are 99,00%, in particular 99,10%, 99,30%, 99,50%, 99,70%, 99,90%, 99,99%, 99, 999% or more. Most preferable the "reference", "control", or "wild type" is preferably a subject, e.g. an organelle, a cell, a tissue, an organism, which is genetically identical to the organism, cell organelle used according to the disclosed method except that nucleic acid molecules or the gene product encoded by them are changed according to the disclosed method.

Preferably, the reference, control or wild type differs form the subject of the present invention only in the cellular activity of the polypeptide disclosed herein, e.g. as result of an increase in the level of the nucleic acid molecule disclosed herein or an increase of the specific activity of the polypeptide disclosed herein, e.g. by or in the expression level or activity of an protein having an said activity and its biochemical or genetical causes.

In case, a control, reference or wild type differing from the subject of the present invention only by not being subject of the method of the invention can not be provided, a control, reference or wild type can be an organism in which the cause for the modulation of an activity conferring the increase of the yield or growth or expression of the nucleic acid molecule disclosed herein as described herein has been switched back or off, e.g. by knocking out the expression of the responsible gene product, e.g. by antisense inhibition, by inactivation of an activator or agonist, by activation of an inhibitor or antagonist, by inhibition through adding inhibitory antibodies, by adding active compounds as e.g. hormones, by introducing negative dominant mutants, etc. A gene production can for example be knocked out by introducing inactivating point mutations, which lead to an enzymatic activity inhibition or a destabilization or an inhibition of the ability to bind to cofactors etc.

Accordingly, preferred reference subject is the starting subject of the present method of the invention. Preferably, the reference and the subject matter of the invention are compared after standardization and normalization, e.g. to the amount of total RNA, DNA, or Protein or activity or expression of reference genes, like housekeeping genes, such as ubiquitin.

A series of mechanisms exists via which a modification in the polypeptide disclosed herein can directly or indirectly affect the yield. For example, the molecule number or the specific activity of the polypeptide disclosed herein or the nucleic acid molecule disclosed herein may be increased. The desired biomass increase can be achieved for example by increasing the copy number of the disclosed protein encoding gene. However, it is also possible to increase the expression of the gene which is naturally present in the organisms, for example by modifying the regulation of the gene, or by increasing the stability of the mRNA or of the gene product encoded by the nucleic acid molecule disclosed herein.

Accordingly, preferred reference subject is the starting subject of the present inventive method. Preferably, the reference and the inventive subject are compared after normalization, e.g. to the amount of total RNA, DNA, or protein or activity or expression of reference genes, like housekeeping genes or shown in the examples.

The increase, decrease or modulation can be constitutive, e.g. due to a stable expression, or transient, e.g. due to a transient transformation or temporary addition of a modulator as a agonist or antagonist or inducible, e.g. after transformation with a inducible construct carrying the disclosed sequences and adding the inducer.

The term "increase" or "decrease" of an activity in a cell, tissue, organism, e.g. plant or microorganism, means that the overall activity in said compartment is increased or decreased, e.g. as result of an increased or decreased expression of the gene product, the addition or reduction of an agonist or antagonist, the inhibition or activation of an enzyme, or a modulation of the specific activity of the gene product, for example as result of a mutation. A mutation in the catalytic centre of a disclosed enzyme can modulate the turn over rate of the enzyme, e.g. a knock out of an essential amino acid can lead to a reduced or completely knock out activity of the enzyme. The specific activity of an enzyme disclosed herein can be increased such that the turn over rate is increased or the binding of a co-factor is improved. Improving the stability of the encoding mRNA or the protein can also increase the activity of a gene product. The stimulation of the activity is also under the scope of the term "increased activity".
The specific activity of a disclosed protein or a protein encoded by a disclosed polynucleotide or inventive expression cassette can be tested as described in the examples. In particular, the expression of said protein in a cell, e.g. a plant cell or a microorganism and the detection of an increase in fresh weight, dry weight, seed number and/or seed weight in comparison to a control is an easy test.

Accordingly, the term "increase" or "decrease" means that the specific activity as well as the amount of a compound, e.g. of the disclosed protein, mRNA or DNA, can be increased or decreased.

The term "increase" also means, that a compound or an activity is introduced into a cell de novo or that the compound or the activity has not been detectable. Accordingly, in the following, the term "increasing" also comprises the term "generating" or "stimulating".

In general, an activity of a gene product in an organism, in particular in a plant cell, a plant, or a plant tissue or a part thereof can be increased by increasing the amount of the specific encoding mRNA or the corresponding protein in said organism or part thereof. "Amount of protein or mRNA" is understood as meaning the molecule number of disclosed polypeptide or mRNA molecules in an organism, a tissue, a cell or a cell compartment. "Increase" in the amount of the disclosed protein means the quantitative increase of the molecule number of said protein in an organism, a tissue, a cell or a cell compartment or part thereof - for example by one of the methods described herein below - in comparison to a wild type, control or reference.

The increase in molecule number amounts preferably to at least 1%, preferably to more than 10%, more preferably to 30% or more, especially preferably to 50%, 100% or more, very especially preferably to 500%, most preferably to 1000% or more. However, a de novo expression is also regarded as subject disclosed herein.

A modification, i.e. an increase or decrease, can be caused by endogenous or exogenous factors. For example, an increase in activity in an organism or a part thereof can be caused by adding a gene product or a precursor or an activator or an agonist to the media or nutrition or can be caused by introducing said subjects into an organism, transient or stable.

Accordingly, in one disclosure, the method disclosed herein comprises one or more of the following steps
a) stabilizing the disclosed protein;
b) stabilizing the disclosed protein encoding mRNA;
c) increasing the specific activity of the disclosed protein;
d) expressing or increasing the expression of a homologous or artificial transcription factor for disclosed protein expression;
e) stimulating the disclosed protein activity through exogenous inducing factors;
f) expressing a transgenic disclosed protein encoding gene; and/or
g) increasing the copy number of the disclosed protein encoding gene.
h) increasing the expression of the gene encoding the disclosed protein by for example manipulation of the endogenous regulation of the gene through side directed mutagenesis or other techniques.

In general, the amount of mRNA or polypeptide in a cell or a compartment of an organism correlates with the activity of the encoded protein or enzyme in said volume. Said correlation is not always linear, the activity in the volume is dependent on the stability of the molecules or the presence of activating or inhibiting co-factors. Further, product and educt inhibitions of enzymes are well known. However, the activity of the disclosed polypeptide may be increased via increasing the expression of the encoding gene, in particular of a nucleic acid molecule comprising the sequence of the disclosed polynucleotide, leading regulary to an increase in amount of disclosed polypeptide.

The increase in fresh weight, dry weight, seed weight and/or seed amount may be achieved by increasing the endogenous level of the disclosed protein. The endogenous level of the disclosed protein can for example be increased by modifying the transcriptional or translational regulation of the polypeptide. Regulatory sequences are operatively linked to the coding region of an endogenous protein and control its transcription and translation or the stability or decay of the encoding mRNA or the expressed protein. In order to modify and control the expression, promoter, UTRs, splicing sites, processing signals, polyadenylation sites, terminators, enhancers, post transcriptional or posttranslational modification sites can be changed or amended. For example, the expression level of the endogenous protein can be modulated by replacing the endogenous promoter with a stronger transgenic promoter or by replacing the endogenous 3'UTR with a 3'UTR which provides more stablitiy without amending the coding region. Further, the transcriptional regulation can be modulated by introduction of a artifical transcription factor as described in the examples. Alternative promoters, terminators and UTR are described below.

In one disclosure with regard to homologs of SEQ ID NO: 2, in the method disclosed herein the activity of a polypeptide is increased comprising or consisting of the following consensus sequence:

whereby 20 or less, for example 15 or 10, for example 9, 8, 7, or 6, 5 or 4, even 3, even 2, even 1, 0 of the amino acids positions indicated by a capital letter can be replaced by an x.

In one disclosure not more than 5, for example 4, even 3 or 2, one or non amino acid position indicated by a capital letter are/is replaced by an x.

In one disclosure 20 or less, for example 15 or 10, for example 9, 8, 7, or 6, 5 or 4, even 3, even 2, even 1, 0 amino acids are inserted into the consensus sequence.

In one disclosure 20 or less, for example 15 or 10, for example 9, 8, 7, or 6, 5 or 4, even 3, even 2, even 1, 0 amino acids represented by a x are deleted from the consensus sequence.

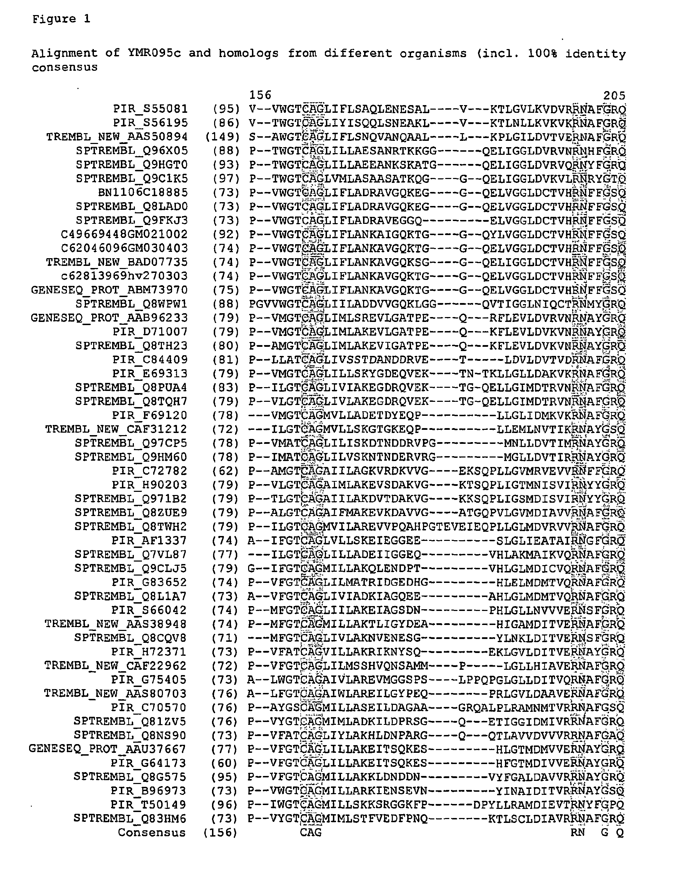

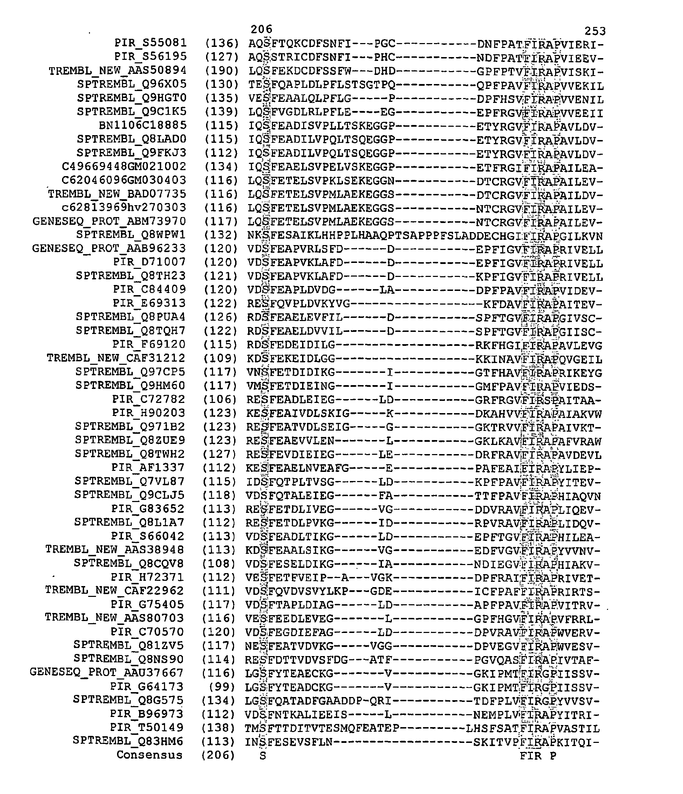

The consensus sequence was derived from a multiple alignment of the sequences of Aeropyrum pernix, Arabidopsis thaliana (Mouse-ear cress), Archaeoglobus fulgidus, Ashbya gossypii (Yeast) (Eremothecium gossypii), Bacillus cereus ATCC 10987, Bacillus circulans, Bacillus halodurans, Bacillus subtilis, Bifidobacterium longum, Brassica napus, Cercospora nicotianae, Clostridium acetobutylicum, Clostridium acetobutylicum, Corynebacterium glutamicum (Brevibacterium flavum), Deinococcus radiodurans, Emericella nidulans (Aspergillus nidulans), glycine max, Haemophilus ducreyi, Haemophilus influenzae, Halobacterium sp. NRC-1, Hordeum vulgare, Listeria monocytogenes, Methanobacterium thermoautotrophicum, Methanococcus maripaludis, Methanopyrus kandleri, Methanosarcina acetivorans, Methanosarcina mazei (Methanosarcina frisia), Mycobacterium tuberculosis, Neurospora crassa, Oryza sativa (japonica cultivar-group), Parachlamydia sp. UWE25, Pasteurella multocida, Pyrobaculum aerophilum, Pyrococcus abyssi, Pyrococcus furiosus, Pyrococcus horikoshii, Saccharomyces cerevisiae, Schizosaccharomyces pombe, Staphylococcus epidermidis, Streptococcus pneumoniae, Streptomyces avermitilis, Suberites domuncula (Sponge), Sulfolobus solfataricus, Sulfolobus tokodaii, Thermoplasma acidophilum, Thermoplasma volcanium, Thermotoga maritima, Thermus thermophilus HB27, Tropheryma whipplei (strain TW08/27) (Whipple's bacillus), Zea mays as shown in Figure 1. X indicates any given amino acid. Those amino acids are spezified in the consensus which are conserved in at least 80% of the aligned protein sequences (80% consensus).

In one disclosure with regard to homologs of SEQ ID NO: 2, in the method disclosed herein the activity of a polypeptide is increased comprising or consisting of the following consensus sequence based on the alignment of plant homologous sequences: whereby 20 or less, for example 15 or 10 or less, for example 7, preferred 4 or 3, 2, even 1, 0 of the amino acids positions indicated by a capital letter can be replaced by an x. For example, not more than one amino acid position indicated by a capital letter is replaced by an x.

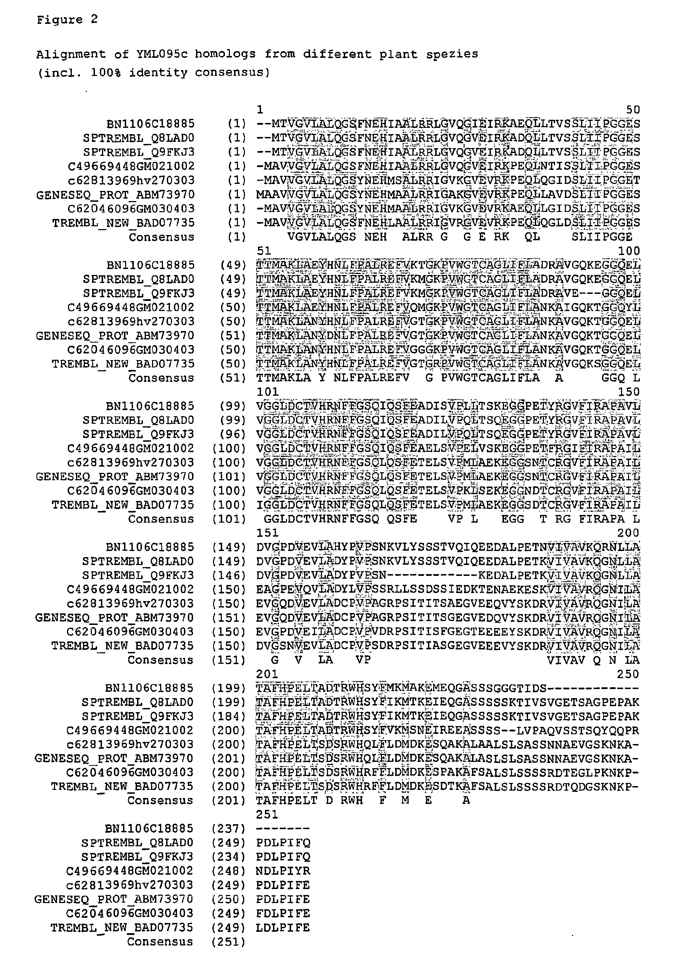

The consensus sequence was derived from a multiple alignment of the plant sequences of Arabidopsis thaliana, Canola, soybean, barley, rice, corn as shown in Figure 2. X indicates any given amino acid. In this case those amino acids are specified which are conserved in nearly 100% of the aligned plant protein sequences (100% Consensus).

Accordingly, in one disclosure, in the method disclosed herein the activity of a polypeptide comprising one or both said core consensus sequence is increased whereby 10 or less, for example 7, preferred 4 or 3, 2, even 1, 0 of the amino acids positions indicated by a capital letter can be replaced by an x. For example, not more than one amino acid position indicated by a capital letter is replaced by an x.

Core consensus sequence of homologs of SEQ ID NO: 2 of all organisms represent the essential part of the consensus sequence as follows:
(P/S)GGE(S/T)T
or
(G/A)(T/S)CAGX(I/V)
or
(V/A/I/C)XRNX(F/Y)GXQXXS(F/S)
or
FIR(A/S/G)P
or
FHPE(UM/E)

Accordingly, in one disclosure, in the method disclosed herein the activity of a polypeptide comprising one or more of said core consensus sequence(s) is increased.

Core consensus sequence of homologs of SEQ ID NO : 2 of plants represent the essential part of the consensus sequence as follows:

or
GGQXLXGGLDCTVHRNFFGSQXQSFE
or
EGGXXTXRGXFIRAPA
or
VIVAVXQXNXLATAFHPELTXDXRWH

Accordingly, in one disclosure, in the method disclosed herein the activity of a polypeptide comprising one or more of said core consensus sequence(s) of plant homologs is increased.

The multiple alignment was performed with the Software GenoMax Version 3.4, Informat™, Invitrogen™ life science software, U.S. Main Office, 7305 Executive Way, Frederick, MD 21704,USA with the following settings:
Gap opening penalty: 10,0; Gap extension penalty: 0,05; Gap separation penalty range: 8; % identity for alignment delay: 40; Residue substitution matrix: blosum; Hydrophilic residues: G P S N D Q E K R; Transition weighting: 0,5; Consensus calculation options: Residue fraction for consensus: 0,5.

Under term "consensus sequence" the above consensus sequences, core sequences, plant consensus sequence, plant core consensus sequence in all described variations are understood.

Accordingly, in one disclosure, in the method disclosed herein the activity of a polypeptide comprising one or both said core consensus sequence is increased, whereby 10 or less, for example 7, 4 or 3, 2, even 1, 0 of the amino acids positions indicated by a capital letter can be replaced by a x. For example, not more than one amino acid position indicated by a capital letter is replaced by an x.

Reference organism preferably means the starting organism (wild type) prior to carrying out the method of the invention or a control organism.

If the organism is a plant and a line of origin cannot be determined as reference, the variety which has been approved by the European or German plant variety office at the time of application and which has the highest genetic homology to the plant to be studied may be accepted as reference for determining an increased SEQ ID NO: 2, activity. Consequently, a plant variety which has already been approved at the time of application is then likewise a suitable reference or source for a reference organelle, a reference cell, a reference tissue or a reference organ. The genetic homology may be determined via methods which are well known to the skilled worker, for example via fingerprint analyses, for example as described in Roldan-Ruiz, Theor. Appl. Genet., 2001, 1138-1150. A plant or a variety which has increased SEQ ID NO: 2, activity and increased yield or faster growth, compared to the, if possible, genetically identical plant, as described herein, may consequently be regarded as a plant disclosed herein. Where appropriate, the specific SEQ ID NO: 2, activity may be replaced by the amount of SEQ ID NO: 1, mRNA or SEQ ID NO: 2, protein, as described herein. Similar methods for determining the genetic relationship of animals and microorganisms are sufficiently known to the skilled worker, in particular to sytematists.

Where appropriate, the organisms and, in particular, the strains mentioned in the examples serve as reference organisms. In particular, the plant strains mentioned there serve as reference organisms for the particular plant species in the rare cases, a reference described above cannot be provided.

The line of origin, which has been used for carrying out the method disclosed herein is a preferred reference.

Various strains or varieties of a species may have different amounts or activities of SEQ ID NO: 2,. The amounts or activities of SEQ ID NO: 2, in a cell compartment, cell organelle, cell, tissue, in organs or in the whole plant may be found to differ between different strains or varieties. However, owing to the observation on which the invention is based, it may be assumed that the increase, in particular in a total extract of the organism, preferably of the plant, in comparison with the respective starting strain or the respective starting variety or with the abovementioned reference, results in faster growth and/or higher yield. However, it is also conceivable that even the increased activity, for example due to overexpression, in specific organs may cause the desired effect, i.e. faster growth and higher yield.

In the following, the term "increasing" comprises the generating as well as the stimulating of a property.

In order to determine the "increase in amount", "increase in expression", "increase in activity" or "increase in mass", this property is compared to that of a reference or starting organism, but normalized to a defined value. For example, expression between the transgenic non human organism and the reference (wild type) is compared, normalizing, for example, to the amount of total RNA, total DNA or protein or to the activity or amount of mRNA of a particular gene (or gene product), for example of a housekeeping gene. Increasing the mass or yield likewise involves comparison of the modified and starting organisms, but with normalization to the individual plant or to the yield per hectare, etc.

The SEQ ID NO: 2, activity is preferably at least 5%, more preferably 10%, even more preferably 20%, 30%, 50% or 100%, higher than that of the reference organism. Most preferably, the activity is 200%, 500% or 1 000% or more, higher than in the reference organism.

Owing to the higher SEQ ID NO: 2, activity, in particular owing to a from 5% to 1 000% increase in SEQ ID NO: 2, activity, preferably owing to a from 10% to 100% increase, growth is preferably 5%, preferably 10%, 20% or 30%, faster. More preferably, growth is faster by 50%, 100%, 200% or 500% or more, in comparison with a reference organism. Preference is also given to increasing the SEQ ID NO: 2, activity by 10%, 20%, 30% or from 50% to 100% and to a faster growth of 10%, 20%, 30% or 50%.

Owing to the higher SEQ ID NO: 2, activity, in particular owing to a 5% to 1 000% increase in SEQ ID NO: 2, activity, preferably owing to a 10% to 100% increase, yield is preferably 5%, preferably 10%, 20% or 30%, higher. More preferably, yield is higher by 50%, 100%, 200% or 500% or more, in comparison with a reference organism. Preference is also given to increasing the SEQ ID NO: 2, activity by 10%, 20%, 30% or from 50% to 100% and to a higher yield of 10%, 20%, 30% or 50%.

"Accelerated growth", "faster growth" or "increased growth rate" in plants means faster "plant growth", i.e. that the increase in fresh weight in the vegetative phase is greater than that of a reference plant, in particular of the starting plant from which the plant of the invention has been prepared. Preferably, the final weight of said plant is also higher than that of the reference plant.

For microorganisms or cells, faster growth refers to higher production of biomass.

"Final weight" means a weight typically reached at the end of a particular phase or the produced biomass of an organism. For plants, "increased final weight" preferably means the higher fresh weight reached at the end of growth phase, in comparison with the fresh weight of a reference organism. More specifically, the higher final weight may be due to a higher yield, as discussed below. For microorganisms or cells, "increased final weight" means the amount of biomass produced by said microorganisms or cells in the exponential phase.

The term "yield" means according to the invention that the biomass or biomaterial suitable for further processing has increased. The term "further processing" refers both to industrial processing and to instant usage for feeding. If the method refers to a plant, this includes plant cells and tissue, organs and parts of plants in all of their physical forms such as seeds, leaves, fibers, roots, stems, embryos, calli, harvest material, wood, or plant tissue, reproductive tissue and cell cultures which are derived from the actual plant and/or may be used for producing a plant of the invention. Preference is given to any parts or organs of plants, such as leaf, stalk, shoot, flower, root, tubers, fruits, bark, seed, wood, etc. or the whole plant. Seeds comprise any seed parts such as seed covers, epidermal and seed cells or embryonic tissue. Particular preference is given to the agricultural or harvested products, in particular fruits, seeds, tubers, fruits, roots, bark or leaves or parts thereof.

Thus, Arabidopsis plants having increased SEQ ID NO: 2, expression not only reach a defined weight significantly earlier than the reference plants but also attained a higher maximum fresh weight, dry weight, seed weight and/or higher yield.

Thus, for example, the fresh weight of Arabidopsis thaliana having increased SEQ ID NO: 2, expression increased by 15% to 53% compared to the wild type in screening experiments (experiment 1.1 oder 1.2) and by 26%- 56% in confirmation experiments (confirmation loop 1 or 2) compared to wild type plants, grown in the same experiment under identical conditions. Details can be taken from Table 1.

If the method disclosed herein relates to a microorganism, the term "yield" means both the biomass produced by said microorganism, for example the fermentation broth, and the cells themselves. If said microorganism produces a particular product suitable for further processing or for direct application, for example the fine chemicals described below, the method disclosed herein for example increases production of said product per microorganism or per unit time. "Increasing the amount", "increasing expression", "increasing the activity" or "increasing the mass" means in each case increasing the particular property compared to the wild type or to a reference, taking into account the same growth conditions. The wild type or reference may be a cell compartment, a cell organelle, a cell, a tissue, an organ or a nonhuman organism, preferably a plant, which has not been subjected to the method disclosed herein but which is otherwise incubated under as identical conditions as possible and which is then compared to a product prepared according to the invention, with respect to the features mentioned herein.

An "increase" may also refer to a cell compartment, a cell organelle, a cell, a tissue, an organ or a non human organism, preferably a plant, as reference which has been modified, altered and/or manipulated in such a way that it is possible to measure in it an increased SEQ ID NO: 2, activity (product of the amount of SEQ ID NO: 2, and the relative activity thereof) or amount of SEQ ID NO: 2, (amount per compartment, organelle, cell, tissue, organ and/or nonhuman organism).

The increase may also be affected by endogenous or exogenous factors, for example by adding SEQ ID NO: 2, or a precursor or an activator thereof to nutrients or animal feed. The increase may also be carried out by increasing endogenous or transgenic expression of a gene coding for SEQ ID NO: 2, or for a precursor or activator or by increasing the stability of the abovementioned factors. The phenotypic action of a factor, in particular its SEQ ID NO: 2, activity, may be determined, for example in Arabidopsis, by constitutive expression, as described in the examples. SEQ ID NO: 2, activity here means an activity as described below.

Preference is given to increasing the SEQ ID NO: 2, activity in a cell, and more preference is given to the activity having increased in one or more tissues or one or more organs. Normally, the increase in a nonhuman organism entails an increase in one or more tissues or one or more organs, and this in turn often entails the increase in a cell, unless a protein is secreted. A higher SEQ ID NO: 2, activity in a cell may be caused, for example, by a higher activity in one of the cellular compartments as listed below.

"Increasing the amount", "increasing expression", "increasing the activity" or "increasing the mass" means in each case increasing in a constitutive or inducible, stable or transient manner. For example, the increase may also be increased in a cell or a tissue only at a particular time, in comparison with the reference, for example only in a particular developmental stage or only in a particular phase of the cell cycle.
The term "increase" also refers to an increase due to different amounts, which may be caused by the response to different inducing reagents such as, for example, hormones or biotic or abiotic signals. However, the activity may also be increased by SEQ ID NO: 2, polypeptide interacting with exogenous or endogenous modulators which act either in an inhibiting or activating manner.

"SEQ ID NO: 2, activity" of a polypeptide here preferably means that increased expression or activity of said polypeptide results in higher fresh weight, dry weight, seed weight and/or yield, and this particularly preferably results in a plurality of said features, even more preferably in all of said features. Furhter disclosed are polypeptides comprising the polypeptide consensus or consensus core sequence defined above, or encoded by a nucleic acid molecule comprising a nucleic acid molecule selected from the group consisting of:
(a) nucleic acid molecule encoding a SEQ ID NO: 2, polypeptide or encoding, for example at least the mature form of the, polypeptide which is depicted in SEQ ID NO: 2,;
(b) nucleic acid molecule comprising, for example at least the mature, polynucleotide of the coding sequence according to SEQ ID NO: 1,;
(c) nucleic acid molecule whose sequence is derivable from a polypeptide sequence encoded by a nucleic acid molecule according to (a) or (b), due to the degeneracy of the genetic code;
(d) nucleic acid molecule encoding an SEQ ID NO: 2, polypeptide whose sequence is at least 20%, for example 35%, for example 45%, for example 60%, for example 70%, 80%, 90%, 95%, 97%, 98% and 99%, identical to the amino acid sequence of the polypeptide encoded by the nucleic acid molecule according to (a) to (c);
(e) nucleic acid molecule encoding an SEQ ID NO: 2, polypeptide that is derived from an SEQ ID NO: 2, polypeptide encoded by a nucleic acid molecule according to (a) to (d) by substitution, deletion and/or addition of one or more amino acids of the amino acid sequence of the polypeptide encoded by the nucleic acid molecules (a) to (d);
(f) nucleic acid molecule encoding a fragment or an epitope or a consensus motive of the SEQ ID NO: 2, polypeptide encoded by any of the nucleic acid molecules according to (a) to (e);
(g) nucleic acid molecule comprising a polynucleotide which comprises the sequence of a nucleic acid molecule obtained by amplification of a for example microbial or plant cDNA bank using the primers in SEQ ID NO: 96 and 97, or of a for example microbial or plant genomic bank using the primers in SEQ ID NO: 96 and 97,;
(h) nucleic acid molecule encoding an SEQ ID NO: 2, polypeptide which has been isolated with the aid of monoclonal antibodies against a polypeptide encoded by any of the nucleic acid molecules according to (a) to (g); and
(i) nucleic acid molecule which is obtainable by screening an appropriate library under stringent conditions using a probe comprising any of the sequences according to (a) to (h) or a fragment of at least 15 nt, for example 20 nt, 30 nt, 50 nt, 100 nt, 200 nt or 500 nt, of the nucleic acid shown in (a) to (h) and which encodes a SEQ ID NO: 2, polypeptide;
(j) nucleic acid molecule encoding a growth or yield increasing polypeptide comprising the sequence shown in SEQ ID NO: 2, , whereby 20 or less, for example 15 or 10, of the amino acid positions indicated can be replaced by an X and/or whereby 20 or less, for example 15 or 10, of the amino acid are inserted into or deleted from the shown sequence or shown in SEQ ID NO: 2, 107, 125 or 129, whereby 10 or less, for example 7, of the amino acid positions indicated can be replaced by an X and/or whereby 10 or less, for example 7, of the amino acid are inserted into or deleted from the shown sequence;
and that its increased activity in a nonhuman organism, in comparison with a reference organism, preferably in a plant, results in faster growth and/or increased yield in comparison with a reference organism, as described above. The polynucleotide is for example of plant origin or originates from a prokaryotic or eukaryotic microorganism, for example Saccharomyces sp. The plant preferably grows faster or stronger and/or has a higher yield, as defined below.

"Increasing the SEQ ID NO: 2, activity" in a cell compartment, a cell organelle, a cell, a tissue, an organ or a nonhuman organism, preferably a plant, preferably means "increasing the absolute SEQ ID NO: 2, activity", i.e. independently of whether this is due to more protein or more active protein in a cell compartment, a cell organelle, a cell, a tissue, an organ or a nonhuman organism in a cell compartment, a cell organelle, a cell, a tissue, an organ or a nonhuman organism.

The specific activity may be increased, for example, by mutating the polypeptide, the consequence of which is higher turnover or better binding of cofactors, for example. Increasing the stability of the polypeptide increases, for example, the activity per unit, for example per volume or per cell, i.e. a loss of activity with time, due to degradation of said polypeptide, is prevented. An in-vitro assay for determining the specific activity of SEQ ID NO: 2, is not yet known to the skilled worker.

The specific activity of a polypeptide may be determined as described in the examples below. For example, it is possible to express a potential SEQ ID NO: 1, in a model organism and to compare the growth curve with that of a reference under identical conditions. Preferably, an increase in growth can already be detected at the cellular level, but it may be necessary to observe a full vegetative period. Preference may be given here to using a plant expression and assay system for this purpose. Thus it was surprisingly found that constitutive expression of the yeast proteins SEQ ID NO: 2, in plants also results in faster growth.

The term "increasing" means both that a substance or an activity, here SEQ ID NO: 2, RNA or SEQ ID NO: 2, DNA or SEQ ID NO: 2, protein or SEQ ID NO: 2, activity, for example, is introduced to a particular environment for the first time or has previously not been detectable in said environment, for example by transgenic expression of a SEQ ID NO: 1, nucleic acid in an SEQ ID NO: 2, deficient nonhuman organism, and that the activity or the amount of substance in a particular environment is increased in comparison with the original state, for example by transgenic coexpression of a SEQ ID NO: 1, gene in an SEQ ID NO: 2, expressing organism or by uptake of SEQ ID NO: 2, from the environment. The term "increasing" thus also comprises de-novo expression.

The "dry weight" of a plant cell, a plant cell organelle, a plant tissue, a plant or a part thereof means the weight of the organic material of the plant cell, the plant cell organelle, the plant tissue, the plant or the part thereof less the amount of water included in the plant cell, the plant cell organelle, the plant tissue, the plant or the part thereof.

In one embodiment of the invention the dry weight of a plant cell, a plant cell organelle, a plant tissue, a plant or a part thereof (over)expressing a nucleic acid having the sequence of SEQ ID NO: 1, is increased by 1%, 2,5%, 5%, 10%, 15%, 20%, 25%, 30%, 50% or more in comparison to wildtype plant.

In another embodiment of the invention the dry weight of a plant cell, a plant cell organelle, a plant tissue, a plant or a part thereof (over)expressing a nucleic acid having the sequence of SEQ ID NO: 1, is increased by 1%, 2,5%, 5%, 10%, 15%, 20%, 25%, 30%, 50% or more in comparison to the dry weight of a variety selected from the group consisting of
(a) G. hirsutum IPK Accession Number GOS 6 (D 120), GOS 7 (ST 446), GOS 10 (D 1635), GOS 17 (D 4302), or GOS 21 (D 5553), or G. areysianum Deflers, or G. incanum (Schwartz) Hillc., or G. raimondii Ulbr., or G. stocksii Masters, or G. thurberi Tod., or G. tomentosum Nutt. or G. triphyllum Hochr., or Gossypium arboreum IPK Accession Number GOS 13 (D 1634), GOS 16 (D 4240), GOS 18 (D 4505), GOS 19 (D 4506), GOS 20 (D 4750), or GOS 12 (D 1329), or Gossypium barbadense, or Gossypium herbaceum; and
(b) Brassica napus variety Mika, Brassica napus variety Digger, Brassica napus variety Artus, Brassica napus variety Terra, Brassica napus variety Smart, Brassica napus variety Olivine, Brassica napus variety Libretto, Brassica napus variety Wotan, Brassica napus variety Panther, Brassica napus variety Express, Brassica napus variety Oase, Brassica napus variety Elan, Brassica napus variety Ability, Brassica napus variety Mohican; and
(c) Linum usitatissimum variety Librina, Linum usitatissimum variety Flanders, Linum usitatissimum variety Scorpion, Linum usitatissimum variety Livia, Linum usitatissimum variety Lola, Linum usitatissimum variety Taurus, Linum usitatissimum variety Golda, Linum usitatissimum variety Lirima, and
(d) Zea mays variety Articat, Zea mays variety NK Dilitop, Zea mays variety Total, Zea mays variety Oldham, Zea mays variety Adenzo, Zea mays variety NK Lugan, Zea mays variety Liberal, Zea mays variety Peso; and
(e) Glycine max variety Oligata, Glycine max variety Lotus, Glycine max variety Primus,Glycine max variety Alma Ata, Glycine max variety OAC Vision, Glycine max variety Jutro; and
(f) Helianthus annus variety Helena, Helianthus annus variety Flavia, Helianthus annus variety Rigasol, Helianthus annus variety Flores, Helianthus annus variety Jazzy, Helianthus annus variety Pegaso, Helianthus annus variety Heliaroc, Helianthus annus variety Salut RM; and
(g) Camelina sativa variety Dolly, Camelina sativa variety Sonny, Camelina sativa variety Ligena, Camelina sativa variety Calinka; and
(h) Sinapis alba variety Martigena, Sinapis alba variety Silenda, Sinapis alba variety Sirola, Sinapis alba variety Sito, Sinapis alba variety Semper, Sinapis alba variety Seco; and
(i) Carthamus tinctorius variety Sabina, Carthamus tinctorius variety HUS-305, Carthamus tinctorius variety landrace, Carthamus tinctorius variety Thori-78, Carthamus tinctorius variety CR-34, Carthamus tinctorius variety CR-81; and
(j) Brassica juncea variety Vittasso, Brassica juncea variety Muscon M-973, Brassica juncea variety RAPD, Brassica juncea variety Co.J.86, Brassica juncea variety IAC 1-2, Brassica juncea variety Pacific Gold; and
(k) Cocos nucifera L. varietes Maypan, Ceylon Tall, Indian Tall, Jamaica Tall, Malayan Tall, Java Tall, Laguna, KingCRIC 60, CRIC 65, CRISL 98, Moorock tall, Plus palm tall, San Ramon, Typica, Nana or Aurantiaca; and
(l) Triticum aestivum L. variety Altos, Bundessortenamt file number 2646, Triticum aestivum L. variety Bussard, Bundessortenamt file number 1641, or Triticum aestivum L. variety Centrum, Bundessortenamt file number 2710; and
(m) Beta vulgaris variety Dieck 13, CPVO file number 19991828, Beta vulgaris variety FD 007, CPCO file number 20000506, or Beta vulgaris variety HI 0169, CPVO file number 20010315; and
(n) Hordeum vulgare variety Dorothea, CPVO file number 20031457, Hordeum vulgare variety Colibri, CPVO file number 20040122, Hordeum vulgare variety Brazil, CPVO file number 20010274, or Hordeum vulgare variety Christina, CPVO file number 20030277; and
(o) Secale cereale variety Esprit, CPVO file number 19950246, Secale cereale variety Resonanz, CPVO file number 20040651, or Secale cereale variety Ursus, CPVO file number 19970714; and
(p) Oryza sativa variety Gemini, CPVO file number 20010284, Oryza sativa variety Tanaro, CPVO file number 20020177, or Oryza sativa variety Zeus, CPVO file number 19980388; and
(q) Solanum tuberosum L. varieties Linda, Nicola, Solara, Agria, Sieglinde, or Russet Burbank; and
(r) Arachis hypogaea subsp. fastigiata cultivar Valencia; and
(s) Arachis hypogaea subsp. hypogaea cultivar Virginia variety 'Holland Jumbo', 'Virginia A23-7', or 'Florida 416'; and
(t) Arachis hypogaea subsp. hirsuta cultivar Peruvian runner variety 'Southeastern Runner 56-15', 'Dixie Runner', or'Early Runner'; and
(u) Arachis hypogaea subsp. vulgaris cultivar Spanish variety 'Dixie Spanish', 'Improved Spanish 2B', or 'GFA Spanish'.

According to the knowledge of the skilled worker, the amount of RNA or polypeptide in a cell, a compartment, etc. regularly correlates to the activity of a protein in a volume. This correlation is not always linear, for example the activity also depends on the stability of the molecules or on the presence of activating or inhibiting cofactors. Likewise, product and reactant inhibitions are known. The invention on which the present application is based shows a dependency between the amount of SEQ ID NO: 2, RNA and the increase in the amount of biomaterial, in particular fresh weight, number of leaves and yield. Normally, increased expression of a gene results in an increase of the amount of the mRNA of said gene and of encoded polypeptide, as is also shown here in the examples. Consequently, an increased activity within an organelle, a cell, a tissue, an organ or a plant can be expected when the amount of SEQ ID NO: 2, is increased there. The same may also be expected when the amount of SEQ ID NO: 2, is increased in a different way.

The amount of SEQ ID NO: 2, mRNA or SEQ ID NO: 2, protein in the plant or in the parts mentioned, for example organ, cell, tissue or organelle, is therefore increased. The amount may also be increased by, for example, de-novo or enhanced expression in the cells of the nonhuman organisms, by increased stability, reduced degradation or (increased) uptake from the outside.

In one disclosure, the method disclosed herein relates to faster growth and/or higher yield of a plant. Consequently, in a disclosure, the method disclosed herein comprises increasing the activity of a SEQ ID NO: 2, polypeptide encoded by a polynucleotide which comprises any of the abovementioned nucleic acid molecules (a) to (i) in a plant. For example, the polynucleotide encompasses any of the abovementioned nucleic acids molecules (a) to (c). Even more preference is given to increasing the activity of a polypeptide encoded by a polynucleotide which comprises any of the sequences depicted in SEQ ID NO: 1, or which comprises a nucleic acid coding for a polypeptide depicted in SEQ ID NO: 2,

Homologs are described below. Thus, a particularly preferred homolog at the amino acid level is at least 80%, even more preferably 90%, and most preferably 95%, 96%, 97%, 98% or 99%, identical to a polypeptide encoded according to SEQ ID NO: 1, or depicted in SEQ ID NO: 2, with preference again being given to a homolog of an amino acid sequence encoded according to SEQ ID NO: 1, or an amino acid sequence depicted in SEQ ID NO: 2, . If the present disclosure relates to a plant or to a method for increasing growth or yield in a plant, the SEQ ID NO : 2, activity in the plant is increased compared to the reference organism by 5% or more, more preferably by 10%, even more preferably by 20%, 30%, 50% or 100%. Most preferably, the activity is increased compared to the reference organism by 200%, 500% or 1 000% or more.

Owing to the higher SEQ ID NO: 2, activity, in particular owing to a from 5% to 1 000% increase in SEQ ID NO : 2, activity, preferably owing to a from 10% to 100% increase, growth of the plant is preferably 5%, preferably 10%, 20% or 30%, faster. More preferably, growth is faster by 50%, 100%, 200% or 500% or more, in comparison with a reference organism. Preference is also given to increasing the SEQ ID NO : 2, activity by 10%, 20%, 30% or from 50% to 1 000% and to a faster growth of 10%, 20%, 30% or from 50% to 200%.

Owing to the higher SEQ ID NO: 2, activity, in particular owing to a from 5% to 1 000% increase in SEQ ID NO: 2, activity, preferably owing to a from 10% to 100% increase, yield of the plant is preferably 5%, preferably 10%, 20% or 30%, higher. More preferably, yield is higher by 50%, 100%, 200% or 500% or more, in comparison with a reference organism. Preference is also given to increasing the SEQ ID NO: 2, activity by 10%, 20%, 30% or from 50% to 100% and to a higher yield of 10%, 20%, 30% or 50%.

In another disclosure, the method disclosed herein relates to faster growth and/or higher yield or a higher biomass in microorganisms. Surprisingly, expression of the SEQ ID NO: 1, of the yeast Saccharomyces cerevisiae, leads to faster growth in Arabidopsis and may lead to a higher yield. Owing to the highly conserved nature of SEQ ID NO: 2, , the increased activity of SEQ ID NO: 2, in microorganisms or animals can likewise be expected to result in faster growth, i.e. in a higher rate of division or higher growth rate or due to larger cells. Consequently, the method disclosed herein comprises increasing in a microorganism, an animal or a cell the activity of an SEQ ID NO: 2, polypeptide encoded by a polynucleotide which comprises any of the abovementioned nucleic acids (a) to (i). For example, the polynucleotide comprises any of the abovementioned nucleic acids (a) to (c) or any of said homologs thereof. Preferred homologs are described below. For example, a particularly preferred homolog is at least 80%, even more preferably 90%, and most preferably 95%, 96%, 97%, 98%, or 99%, identical at the amino acid level to a polypeptide according to SEQ ID NO: 2, .

In one disclosure, the nucleic acid molecule encodes a SEQ ID NO: 2, polypeptide comprising the sequence shown in SEQ ID NO: 1, , whereby 20 or less, for example 15 or 10, of the amino acid positions indicated can be replaced by an X and/or whereby 20 or less, for example 15 or 10, of the amino acid are inserted into the shown sequence, whereby 10 or less, for example 7, of the amino acid positions indicated can be replaced by an X and/or whereby 10 or less, for example 7, of the amino acid are inserted into or absent from the shown sequence.

In one disclosure, the nucleic acid molecule encodes a polypeptide SEQ ID NO: 2 comprising or consisting of a polypeptide comprising the consensus or consensus core sequence from different organisms defined above, and as shown in Figure 1, whereby 20 or less, for example 15 or 10, for example 9, 8, 7, or 6, 5 or 4, even 3, even 2, even 1, 0 of the amino acids positions indicated by a capital letter in Figure 1 can be replaced by an x and/or not more than 5, for example 4, even 3 or 2, one or non amino acid position indicated by a capital letter in Figure 1 are/is replaced by an x and/or 20 or less, for example 15 or 10, for example 9, 8, 7, or 6, 5 or 4, even 3, even 2, even 1, 0 amino acids are inserted into or absent from the consensus sequence.

In another disclosure, the nucleic acid molecule encodes a polypeptide SEQ ID NO: 2 comprising or consisting of a polypeptide comprising the consensus sequence from different plant species defined above, e.g. as shown in Figure 2, whereby 20 or less, for example 15 or 10, for example 9, 8, 7, or 6, 5 or 4, even 3, even 2, even 1, 0 of the amino acids positions indicated by a capital letter in Figure 2 can be replaced by an x and/or not more than 5, for example 4, even 3 or 2, one or non amino acid position indicated by a capital letter in Figure 2 are/is replaced by an x and/or 20 or less, for example 15 or 10, for example 9, 8, 7, or 6, 5 or 4, even 3, even 2, even 1, 0 amino acids are inserted into or absent from the consensus sequence.

Owing to the homology of SEQ ID NO: 2 to a protein involved in the biosynthesis of vitamin B6, however, it may be assumed that it is a corresponding protein which is directly or indirectly involved in the metabolism of vitamin B6. Thus it would be possible to determine increased activity of the SEQ ID NO: 2 protein in a cell, an organelle, a compartment, a tissue, an organ or a nonhuman organism, in particular a plant, by measuring vitamin B6 biosynthetic activity.

Apart from that, the SEQ ID NO: 2, activity may be determined indirectly via measuring the amount of SEQ ID NO: 2, RNA or SEQ ID NO: 2, protein. Thus, a quantitative Northern blot or quantitative PCR of the disclosed polynucleotides described herein may determine the amount of mRNA, for example in a cell or in a total extract, and a Western blot may be used to compare the amount of the protein, for example in a cell or a total extract, to that in a reference. Methods of this kind are known to the skilled worker and have been extensively described, for example also in Sambrook, Molecular Cloning: A Laboratory Manual, Cold Spring Harbor Laboratory, Cold Spring Harbor Laboratory Press, Cold Spring Harbor, NY, 1989 or in Current Protocols, 1989 and updates, John Wiley & Sons, N. Y., or in other sources cited below.

A suitable non human organism (host organism) for preparation in the method disclosed herein is in principle any nonhuman organism for which faster growth is useful and desirable, such as, for example, microorganisms such as yeasts, fungi or bacteria, monocotyledonous or dicotyledonous plants, mosses, algae, and also useful animals, as listed below.

The term "plants", as used herein, may include higher plants, lower plants, mosses and algae; however, in a preferred embodiment of the method of the invention, the term "plants" relates to higher plants.

Advantageously, the method disclosed herein uses plants which belong to the useful plants, as listed below. Apart from production of animal feed or food, the plants prepared according to the invention may in particular also be used for the preparation of fine chemicals.

In one disclosure, the method disclosed herein comprises increasing the activity of the SEQ ID NO: 2, polypeptide by increasing the activity of at least one polypeptide in said organism or in one or more parts thereof, which is encoded by a nucleic acid molecule comprising a nucleic acid molecule selected from the group consisting of:
(aa) nucleic acid molecule encoding a SEQ ID NO: 2, polypeptide or encoding, for example at least the mature, form of the polypeptide which is depicted in SEQ ID NO: 2,;
(bb) nucleic acid molecule comprising, for example at least the mature, polynucleotide of the coding sequence according to SEQ ID NO: 1,;
(cc) nucleic acid molecule whose sequence is derivable from a polypeptide sequence encoded by a nucleic acid molecule according to (aa) or (bb), due to the degeneracy of the genetic code;
(dd) nucleic acid molecule encoding a polypeptide whose sequence is at least 20%, for example 35%, more for example 45%, even more for example 60%, even more for example 70%, 80%, 90%, 95%, 97%, 98% and 99%, identical to the amino acid sequence of the polypeptide encoded by the nucleic acid molecule according to (aa) to (cc);
(ee) nucleic acid molecule encoding a polypeptide which is derived from a SEQ ID NO: 2, polypeptide encoded by a nucleic acid molecule according to (aa) to (dd,) for example (aa) to (cc), by substitution, deletion and/or addition of one or more amino acids of the amino acid sequence of the polypeptide encoded by the nucleic acid molecules (aa) to (dd), for example (aa) to (cc);
(ff) nucleic acid molecule encoding a fragment or an epitope of the SEQ ID NO: 2, polypeptide encoded by any of the nucleic acid molecules according to (aa) to (ee), for example (aa) to (cc);
(gg) nucleic acid molecule comprising a polynucleotide which comprises the sequence of a nucleic acid molecule obtained by amplification of a for example microbial or plant cDNA bank using the primers in SEQ ID NO: 96 and 97, or of a for example microbial or plant genomic bank using the primers in SEQ ID NO: 96 and 97,;
(hh) nucleic acid molecule encoding a SEQ ID NO: 2, polypeptide which is isolated with the aid of monoclonal antibodies against a polypeptide encoded by any of the nucleic acid molecules according to (aa) to (gg), for example (aa) to (cc) and
(ii) nucleic acid molecule which is obtainable by screening an appropriate library under stringent conditions using a probe comprising any of the sequences according to (aa) to (hh), for example (aa) to (cc), or a fragment of at least 15 nt, for example 20 nt, 30 nt, 50 nt, 100 nt, 200 nt or 500 nt, of the nucleic acid characterized in (aa) to (hh), for example (aa) to (cc), and which encodes a SEQ ID NO: 2, polypeptide; or
(jj) nucleic acid molecule encoding a SEQ ID NO: 2, polypeptide comprising the sequence shown in SEQ ID NO: 1, , whereby 20 or less, for example 15 or 10, of the amino acid positions indicated can be replaced by an X and/or whereby 20 or less, for example 15 or 10, of the amino acid are inserted into the shown sequence,or whereby 10 or less, for example 7, of the amino acid positions indicated can be replaced by an X and/or whereby 10 or less, for example 7, of the amino acid are inserted into or deleted from the shown sequence;
or which comprises a complementary sequence thereof.

In one disclosure, the activity of the SEQ ID NO: 2, protein is increased by
(a) increasing the expression of a SEQ ID NO: 2, polypeptide;
(b) increasing the stability of SEQ ID NO: 2, RNA or of the SEQ ID NO: 2, protein, for example of a polypeptide or polynucleotide as described in (a);
(c) increasing the specific activity of the SEQ ID NO: 2, protein, for example of a polypeptide as described in (a) or encoded by a polynucleotide described in (a);
(d) expressing a natural or artificial transcription factor capable of increasing expression of an endogenous SEQ ID NO: 2, gene function, for example comprising the sequence of a polynucleotide described in (a); or
(e) adding an exogenous factor which increases or induces SEQ ID NO: 2, activity or SEQ ID NO: 2, expression to the food or the medium, for example of a polynucleotide or polynucleotide described in (a).

In one embodiment, the method of the invention comprises increasing the activity of SEQ ID NO: 2, polypeptide by introducing a polynucleotide into into a plant, or into one or more parts thereof, which polynucleotide codes for a SEQ ID NO: 2, polypeptide encoded by a nucleic acid molecule comprising a nucleic acid molecule selected from the group consisting of:
(a) nucleic acid molecule encoding an SEQ ID NO: 2, polypeptide ;
(b) nucleic acid molecule comprising a polynucleotide of the coding sequence according to SEQ ID NO: 1, ;
(c) nucleic acid molecule whose sequence is derivable from a polypeptide sequence encoded by a nucleic acid molecule according to (a) or (b), due to the degeneracy of the genetic code;
(d) nucleic acid molecule encoding a polypeptide whose sequence is at least 80%, 90%, 95%, 97%, 98% and 99%, identical to the amino acid sequence of the polypeptide of SEQ ID NO: 2;
or which comprises a complementary sequence thereof.

The organism is for example a microorganism or, more preferably a plant.

The term "coding" sequence or "to code" means according to the invention both the codogenic sequence and the complementary sequence or a reference to these, i.e. both DNA and RNA sequences are regarded as coding. For example, a structural gene encodes an mRNA via transcription and a protein via translation, and a coding MRNA is translated into a protein. Both molecules contain the information leading to the sequence of the coded polypeptide, i.e. they encode the latter. Posttranscriptional and posttranslational modifications of RNA and polypeptide are sufficiently known to the skilled worker and are likewise included.

An "organism or one or more parts thereof" means a cell, a cell compartment, an organelle, a tissue or an organ of an organism or a nonhuman organism.

According to the invention, "plant or one or more parts thereof" means a cell, a cell compartment, an organelle, a tissue, an organ or a plant.

The terms "nucleic acid", "nucleic acid molecule" and "polynucleotide" and also "polypeptide" and "protein" are used herein synonymously.

In the method of the invention, "nucleic acids" or "polynucleotides" mean DNA or RNA sequences which may be single- or double-stranded or may have, where appropriate, synthetic, non-natural or modified nucleotide bases which can be incorporated into DNA or RNA.

Consequently, the present description also discloses a polynucleotide, which comprises a nucleic acid molecule selected from the group consisting of:
(a) nucleic acid molecule encoding, for example at least the mature form of, the polypeptide as depicted in SEQ ID NO: 2, or comprising, at least the mature form of, the polynucleotide depicted in SEQ ID NO: 1, ;
(b) nucleic acid molecule whose sequence is derivable from a polypeptide sequence encoded by a nucleic acid molecule according to (a) due to the degeneracy of the genetic code;
(c) nucleic acid molecule encoding a SEQ ID NO: 2, polypeptide whose sequence is at least 30%, for example 35%, more for example 45%, even more for example 60%, even more for example 70%, 80%, 90%, 95%, 97%, 98% and 99%, identical to the amino acid sequence of the polypeptide encoded by the sequence depicted in SEQ ID NO: 1, or comprising the sequence depicted in SEQ ID NO:1, ;
(d) nucleic acid molecule encoding a polypeptide that is derived from an SEQ ID NO: 2, polypeptide encoded by a polynucleotide according to (a) to (c) by substitution, deletion and/or addition of one or more amino acids of the amino acid sequence of the polypeptide encoded by the nucleic acid molecules (a) to (c) and encoding SEQ ID NO: 2,;
(e) nucleic acid molecule encoding a fragment or an epitope of the SEQ ID NO: 2, polypeptide encoded by any of the nucleic acid moleculesaccording to (a) to (d), for example (a) to (c) and encoding a protein having SEQ ID NO: 2, activity;
(f) nucleic acid molecule comprising a polynucleotide which comprises the sequence of a nucleic acid molecule obtained by amplification of a plant cDNA bank using the primers in SEQ ID NO: 96 and 97, or of a for example microbial or plant genomic bank using the primers in SEQ ID NO: 96 and 97,;
(g) nucleic acid molecule encoding a SEQ ID NO: 2, polypeptide which has been isolated with the aid of monoclonal antibodies against a polypeptide encoded by any of the nucleic acid molecules according to (a) to (f), for example (a) to (c) and encoding a protein having SEQ ID NO: 2, activity;
(h) nucleic acid molecule which is obtainable by screening an appropriate library under stringent conditions using a probe comprising any of the sequences according to (a) to (g) or a fragment of at least 15 nt, for example 20 nt, 30 nt, 50 nt, 100 nt, 200 nt or 500 nt, of the nucleic acid characterized in (a) to (g), for example (a) to (c) and which encodes a SEQ ID NO: 2, polypeptide,
(i) nucleic acid molecule encoding a SEQ ID NO: 2, polypeptide comprising the sequence shown in SEQ ID NO: 1, , whereby 20 or less, for example 15 or 10, of the amino acid positions indicated can be replaced by an X and/or whereby 20 or less, for example 15 or 10, of the amino acid are inserted into the shown sequence, or whereby 10 or less, for example 7, of the amino acid positions indicated can be replaced by an X and/or whereby 10 or less, for example 7, of the amino acid are inserted into or absent from the shown sequence;
or the complementary strand thereof, said polynucleotide or said nucleic acid molecule according to (a) to (i) not comprising the sequence depicted in SEQ ID NO: 1, .

For example, the polynucleotide disclosed herein differs from the herein shown previously published polynucleotides by at least one nucleotide, e.g. from SEQ ID NO: NO 1, 3, 5, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94. For example, the polypeptide encoded differs from the previously published polypeptides by at least one amino acid, e.g. from SEQ ID NO: 2, 4, 6, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95.

SEQ ID NO: 1 and 2 describe the polypeptide (SEQ ID NO: 2) and the nuleic acid sequence (SEQ ID NO: 1) for the locus YMR095C of Saccharomyces cerevisiae, as for example disclosed under Accession PIR|S55081 for the YMR095c protein and accession GENESEQ_DNA|AAA14857 for the YMR095C nucleic acid sequence.

In one disclosure, the description furthermore discloses a polynucleotide encoding a polypeptide, e.g. derived from plants, which comprises a nucleic acid molecule encoding a polypeptide comprising any one of SEQ ID NO: 52, 78, 90, 98, 100, 102, 104, 108, 118, 132 or 134 or selected from the group consisting of:
(a) nucleic acid molecule encoding for example at least the mature form of the polypeptide as depicted in SEQ ID NO: 53, 79, 91, 99, 101, 103, 105, 109, 119, 133 or 135 or comprising, for example at least the mature form of the polynucleotide depicted in SEQ ID NO: 52, 78, 90, 98, 100, 102, 104, 108, 118, 132 or 134;
(b) nucleic acid molecule whose sequence is derivable from a polypeptide sequence encoded by a nucleic acid molecule according to (a) due to the degeneracy of the genetic code;
(c) nucleic acid molecule encoding a polypeptide whose sequence is at least 55%, for example 60%, more for example 70%, even more for example 80%, even more preferably 90%, most preferably 95%, 96%, 97%, 98% or 99% identical to the amino acid sequence of the polypeptide encoded by the sequence depicted in SEQ ID NO: 52, 78, 90, 98, 100, 102, 104, 108, 118, 132 or 134; or comprising the sequence depicted in SEQ ID NO: 52, 78, 90, 98, 100, 102, 104, 108, 118, 132 or 134;
(d) nucleic acid molecule encoding a polypeptide whose sequence is at least 90%, for example 95%, 96%, 97%, 98% or 99%, identical to the amino acid sequence of the polypeptide encoded by the sequence depicted in SEQ ID NO: 53, 79, 91, 99, 101, 103, 105, 109, 119, 133 or 135 or comprising the sequence depicted in SEQ ID NO: 53, 79, 91, 99, 101, 103, 105, 109, 119, 133 or 135;
(e) nucleic acid molecule encoding a polypeptide whose sequence is at least 65%, more for example 70%, even more for example 80%, even more for example 90%, most for example 95%, 96%, 97%, 98% or 99%, identical to the amino acid sequence of the polypeptide encoded by the sequence depicted in SEQ ID NO: 53, 79, 91, 99, 101, 103, 105, 109, 119, 133 or 135 or comprising the sequence depicted in SEQ ID NO: 53, 79, 91, 99, 101, 103, 105, 109, 119, 133 or 135;
(f) nucleic acid molecule encoding a polypeptide whose sequence is at least 55%, more for example 70%, even more for example 80%, even more for example 90%, most for example 95%, 96%, 97%, 98% or 99%, identical to the amino acid sequence of the polypeptide encoded by the sequence depicted in SEQ ID NO: 53, 79, 91, 99, 101, 103, 105, 109, 119, 133 or 135 or comprising the sequence depicted in SEQ ID NO: 53, 79, 91, 99, 101, 103, 105, 109, 119, 133 or 135;
(g) nucleic acid molecule encoding a polypeptide whose sequence is at least 35%, more for example 50%, 60% or 70%, even more for example 80%, even more for example 90%, most for example 95%, 96%, 97%, 98% or 99%, identical to the amino acid sequence of the polypeptide encoded by the sequence depicted in SEQ ID NO: 53, 79, 91, 99, 101, 103, 105, 109, 119, 133 or 135 or comprising the sequence depicted in SEQ ID NO: 53, 79, 91, 99, 101, 103, 105, 109, 119, 133 or 135;
(h) nucleic acid molecule encoding a polypeptide whose sequence is at least 55%, for example 60%, more for example 70%, even more for example 80%, even more for example 90%, most for example 95%, 96%, 97%, 98% or 99% identical to the amino acid sequence of the polypeptide encoded by the sequence depicted in SEQ ID NO: 53, 79, 91, 99, 101, 103, 105, 109, 119, 133 or 135 or comprising the sequence depicted in SEQ ID NO: 53, 79, 91, 99, 101, 103, 105, 109, 119, 133 or 135;
(i) nucleic acid molecule encoding a polypeptide whose sequence is at least 90%, for example 95%, 96%, 97%, 98% or 99%, identical to the amino acid sequence of the polypeptide encoded by the sequence depicted in SEQ ID NO: 53, 79, 91, 99, 101, 103, 105, 109, 119, 133 or 135 or comprising the sequence depicted in SEQ ID NO: 53, 79, 91, 99, 101, 103, 105, 109, 119, 133 or 135;
(j) nucleic acid molecule encoding a polypeptide that is derived from a polypeptide encoded by a polynucleotide according to (a) to (i) by substitution, deletion and/or addition of one or more amino acids of the amino acid sequence of the polypeptide encoded by the nucleic acid molecules (a) to (i);
(k) nucleic acid molecule encoding a fragment or an epitope of the polypeptide encoded by any of the nucleic acid molecules according to (a) to (j);
(l) nucleic acid molecule comprising a polynucleotide which comprises the sequence of a nucleic acid molecule obtained by amplification of a for example microbial or plant cDNA library using the primers in SEQ ID NO: 96 and 97, or of a for example microbial or plant genomic library;
(m) nucleic acid molecule encoding a polypeptide SEQ ID NO: 2, which has been isolated with the aid of monoclonal antibodies against a polypeptide encoded by any of the nucleic acid molecules according to (a) to (I);
(n) nucleic acid molecule which is obtainable by screening an appropriate library under stringent conditions using a probe comprising any of the sequences according to (a) to (m) or a fragment of at least 15 nt, for example 20 nt, 30 nt, 50 nt, 100 nt, 200 nt or 500 nt, of the nucleic acid characterized in (a) to (m) and which encodes an polypeptide;
(o) nucleic acid molecule encoding a polypeptide comprising the sequence shown in SEQ ID No: 2, , whereby 20 or less, for example 15 or 10, of the amino acid positions indicated can be replaced by an X and/or whereby 20 or less, for example 15 or 10, of the amino acid are inserted into or absent from the shown sequence or shown in SEQ ID NO: 2, , whereby 10 or less, for example 7, of the amino acid positions indicated can be replaced by an X and/or whereby 10 or less, for example 7, of the amino acid are inserted into or absent from the shown sequence;
or the complementary strand thereof, for example said polynucleotide or said nucleic acid molecule according to (a) to (o) not comprising the sequence depicted in SEQ ID NO: 1, or the sequence complementary thereto.
The polynucleotide may be DNA or RNA.

In principle, any nucleic acids coding for polypeptides with SEQ ID NO: 2, activity may be used in the method disclosed herein. In case of preparing plants with higher biomass or higher yield, advantageously, said nucleic acids are from plants such as algae, mosses or higher plants.

In the method disclosed herein, a nucleic acid sequence is advantageously selected from the group consisting of the sequence SEQ ID NO: 52, 78, 90, 98, 100, 102, 104, 132, 134 or the above-described derivatives or homologs thereof coding for polypeptides which still have SEQ ID NO: 2, biological activity. These sequences are cloned individually or in combination, including with other genes, into expression constructs.

Nucleic acid sequences of a particular donor organism, which code for polypeptides with SEQ ID NO: 2, activity, are usually generally accessible. Particular mention must be made here of general gene databases such as the EMBL database (Stoesser G. et al., Nucleic Acids Res. 2001, Vol. 29, 17-21), the GenBank database (Benson D.A. et al., Nucleic Acids Res. 2000, Vol. 28, 15-18), or the PIR database (Barker W. C. et al., Nucleic Acids Res. 1999, Vol. 27, 39-43). It is furthermore possible to use organism-specific gene databases such as, for example, advantageously the SGD database (Cherry J. M. et al., Nucleic Acids Res. 1998, Vol. 26, 73-80) or the MIPS database (Mewes H.W. et al., Nucleic Acids Res. 1999, Vol. 27, 44-48) for yeast, the GenProtEC database (http://web.bham.ac.uk/bcm4ght6/res.html) for *E. coli*, and the TAIR database (Huala, E. et al., Nucleic Acids Res. 2001 Vol. 29(1), 102-5) or the MIPS database for Arabidopsis.

Advantageously, SEQ ID NO: 2, used in the method and the non human organism employed are from the same origin or from an origin which is genetically as close as possible, for example from the same or a very closely related type or species. However, a synthetic SEQ ID NO: 2, may also be used in a nonhuman organism.

It might be advantageously to use a gene encoding a protein disclosed herein which is not derived from the nonhuman organism, in which method should be carried out to avoid the problem of cosuppression which sometimes occurs when genes are overexpressed in the organism from which they are derived.

The term "gene" means in accordance with the invention a nucleic acid sequence which comprises a codogenic gene section and regulatory elements. "Codogenic gene sections" mean in accordance with the invention a continuous nucleic acid sequence ("open reading frame, abbreviated ORF). Said ORF may contain no, one or more introns which are linked via suitable splice sites to the exons present in the ORF. An ORF and its regulatory elements encode, for example, structural genes which are translated into enzymes, transporters, ion channels, etc., for example, or non-structural genes such as regulatory genes such as the Rho or Sigma protein, for example. However, genes may also be encoded which are not translated into proteins. For expression in a nonhuman organism, a codogenic gene section is expressed together with particular regulatory elements such as promoter, terminator, UTR, etc., for example. The regulatory elements may be of homologous or heterologous origin. Gene, codogenic gene section (ORFs), regulatory sequence are covered by the terms nucleic acid and polynucleotide hereinbelow.

The term "expression" means transcription and/or translation of a codogenic gene section or gene. The resulting product is usually an mRNA or a protein. However, expressed products also include RNAs such as, for example, regulatory RNAs or ribozymes. Expression may be systemic or local, for example restricted to particular cell types, tissues or organs. Expression includes processes in the area of transcription which relate especially to transcription of rRNA, tRNA and mRNA, to RNA transport and to processing of the transcript. In the area of protein biosynthesis, especially ribosome biogenesis, translation, translational control and aminoacyl-tRNA synthetases are included. Functions in the area of protein processing relate especially to folding and stabilizing, to targeting, sorting and translocation and to protein modification, assembly of protein complexes and proteolytic degradation of proteins.

The expression products of the codogenic gene sections (ORFs) and of their regulatory elements can be characterized by their function. Examples of these functions are those in the areas metabolism, energy, transcription, protein synthesis, protein processing, cellular transport and transport mechanisms, cellular communication and signal transduction, cell rescue, cellular defense and cell virulence, regulation of the cellular environment and interaction of the cell with its environment, cell fate, transposable elements, viral proteins and plasmid proteins, control of cellular organization, subcellular location, regulation of protein activity, proteins with binding function or cofactor requirement and facilitated transport. Genes with identical functions are grouped together in "functional gene families". According to the invention, expression of SEQ ID NO: 1, results in an increased growth rate.

A polynucleotide usually includes an untranslated sequence, located at the 3' and 5' ends of the coding gene region, for expression: for example, from 500 to 100 nucleotides of the sequence upstream of the 5' end of the coding region and/or, for example, from 200 to 20 nucleotides of the sequence downstream of the 3' end of the coding gene region. An "isolated" nucleic acid molecule is removed from other nucleic acid molecules present in the natural source of the nucleic acid. An "isolated" nucleic acid for example has no sequences which naturally flank the nucleic acid in the genomic DNA of the organism from which said nucleic acid originates (e.g. sequences located at the 5' and 3' ends of said nucleic acid). The isolated nucleic acid molecule SEQ ID NO: 1, may contain, for example, 5 kb, 4 kb, 3 kb, 2 kb, 1 kb, 0.5 0.1 kb or 0 kb of nucleotide sequences which naturally flank the nucleic acid molecule in the genomic DNA of the cell from which the nucleic acid originates.

The nucleic acid molecules used in the present method, for example a nucleic acid molecule having a nucleotide sequence of the nucleic acid molecules used in the method of the invention or of a part thereof, may be isolated using molecular-biological standard techniques and the sequence information provided herein. It is also possible to identify, for example, a homologous sequence or homologous, conserved sequence regions at the DNA or amino acid level with the aid of comparative algorithms. These sequence regions may be used as hybridization probes by means of standard hybridization techniques, as described, for example, in Sambrook, Molecular Cloning: A Laboratory Manual, Cold Spring Harbor Laboratory, Cold Spring Harbor Laboratory Press, Cold Spring Harbor, NY, 1989, to isolate further nucleic acid sequences useful in the method. In addition, a nucleic acid molecule comprising a complete sequence of SEQ ID NO: 1, or SEQ ID NO: 3, 5, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92,94, 98, 100, 102 or 104 , 132 or 134 or of the other nucleic acid molecules used in the method disclosed herein or a part thereof can be isolated by polymerase chain reaction (PCR) and prepared according to known methods. It is possible to amplify a nucleic acid disclosed herein according to standard PCR amplification techniques using cDNA prepared by means of reverse transcription or, alternatively, genomic DNA as template and suitable oligonucleotide primers. The nucleic acid amplified in this way may be cloned into a suitable vector and characterized by means of DNA sequence analysis.

Examples of homologs of the nucleic acid molecules used in the method disclosed herein are allelic variants which are at least 30%, for example 40%, more for example 50%, 60%, 70%, 80% or 90% and even more for example 95%, 96%, 97%, 98%, 99% or more, identical to any of the nucleotide sequences depicted in SEQ ID NO: 3, 5, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92,94, 98, 100, 102 or 104,132 or 134. Allelic variants include in particular functional variants which can be obtained by deletion, insertion or substitution of nucleotides from/into/in the sequence depicted in SEQ ID NO: 3, 5, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92,94, 98, 100, 102 or 104,132 or 134 , but with the idea of retaining or increasing the SEQ ID NO: 2, activity of the synthetized proteins derived therefrom. Proteins which still possess the biological or enzymic activity of SEQ ID NO: 2, also include those whose activity is essentially not reduced, i.e. proteins having 5%, for example 20%, particularly for example 30%, very particularly for example 40% or more of the original biological activity, compared to the protein encoded by SEQ ID NO: 4, 6, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 99, 101, 103 or 105133 or 135.

For example, however, the homologous activity is increased compared to heterologous expression of SEQ ID NO: 1, in the particular nonhuman organism.

Homologs of SEQ ID NO: 1, 3, 5, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92,94, 98, 100, 102 or 104 or 132, 134 of the nucleic acid molecules used in the method disclosed herein also mean, for example, prokaryotic or eukaryotic, i.e. for example bacterial, animal, fungal and plant homologs, truncated sequences, single-stranded DNA or RNA of the coding and noncoding DNA sequence.

Homologs of SEQ ID NO: 1, 3, 5, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92,94, 98, 100, 102 or 104 or 132, 134 of the nucleic acid molecules used in the method disclosed herein also include derivatives such as, for example, variants of the coding sequence or of the regulatory sequences, such as, for example, promoter, UTR, enhancer, splice signals, processing signals, polyadenylation signals, etc. The derivatives of the nucleotide sequences indicated may be modified by one or more nucleotide substitutions, by insertion(s) and/or deletion(s), without disturbing functionality or activity, however. It is furthermore possible that the activity of the derivatives is increased by modification of their sequence or that said derivatives are completely replaced with more active elements, even those from heterologous organisms.

In order to determine the percentage homology (= identity) of two amino acid sequences (e.g. any of the sequences of SEQ ID NO: 2, 4, 6, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 99, 101, 103 or 105 133, 135 or of two nucleic acids (e.g. any of the sequences of SEQ ID NO: 1, 3, 5, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92,94, 98, 100, 102 or 104 or 132, 134 ), the sequences are compared to one another, for example by aligning said sequences or by analyzing both sequences with the aid of computer programs. Gaps may be introduced in the sequence of one protein or one nucleic acid to produce optimal alignment with the other protein or the other nucleic acid. The amino acid residues or nucleotides at the corresponding amino acid positions or nucleotide positions are then compared. When a position in one sequence is occupied by the same amino acid residue or the same nucleotide as the corresponding position in the other sequence, then the molecules are identical at this position (i.e. amino acid or nucleic acid "homology", is used herein, is equivalent to amino acid or nucleic acid "identity"). The percentage homology between the two sequences is a function of the number of identical positions shared by the sequences (i.e. % homology = number of identical positions/total number of positions × 100). The terms homology and identity are thus used synonymously herein.

"Identity" between two proteins or nucleic acid sequences means identity over the entire length, in particular the identity carried out as described in the examples.

The NCBI standard settings were used for the blastp comparison of the amino acid sequences, i.e. using the following parameters: "composition based statics" and "low complexity filter, "Expect":10, "Word Size":3, "Matrix": Blosum62 and "Gap cost": Existence :11 Extension: 1.

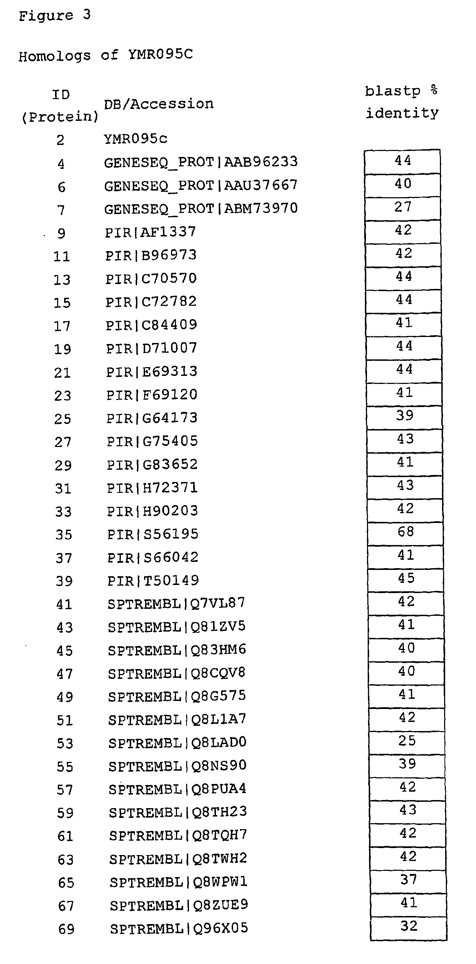

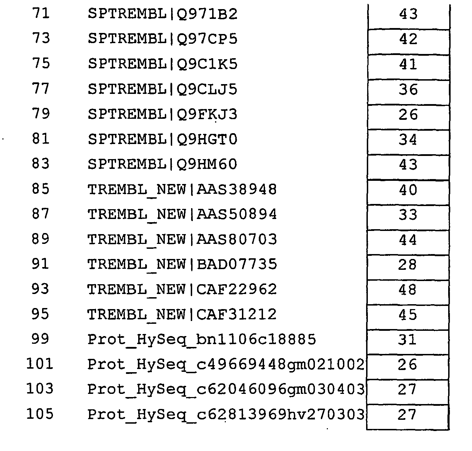

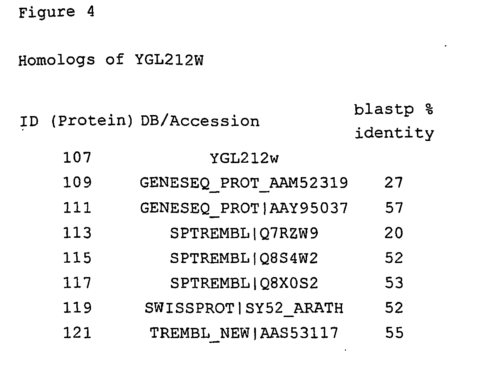

The identity of various amino acid sequences to the amino acid sequence of SEQ ID NO: 2 and 107 is indicated below by way of example for SEQ ID NO 2 in Figure 3 and for SEQ ID NO: 107 in Figure 4.

However, for the determination of the percentage homology (=identity) of two or more amino acids or of two or more nucleotide sequences several other computer software programs have been developed. The homology of two or more sequences can be calculated with for example the software fasta, which presently has been used in the version fasta 3 (W. R. Pearson and D. J. Lipman (1988), Improved Tools for Biological Sequence Comparison.PNAS 85:2444- 2448; W. R. Pearson (1990) Rapid and Sensitive Sequence Comparison with FASTP and FASTA, Methods in Enzymology 183:63 - 98; W. R. Pearson and D. J. Lipman (1988) Improved Tools for Biological Sequence Comparison.PNAS 85:2444- 2448; W. R. Pearson (1990); Rapid and Sensitive Sequence Comparison with FASTP and FASTAMethods in Enzymology 183:63 - 98). Another useful program for the calculation of homologies of different sequences is the standard blast program, which is included in the Biomax pedant software (Biomax, Munich, Federal Republic of Germany). This leads unfortunately sometimes to suboptimal results since blast does not always include complete sequences of the subject and the querry. Nevertheless as this program is very efficient it can be used for the comparison of a huge number of sequences. The following settings are typically used for such a comparisons of sequences:
-p Program Name [String]; -d Database [String]; default = nr; -i Query File [File In]; default = stdin; -e Expectation value (E) [Real]; default = 10.0; -m alignment view options: 0 = pairwise; 1 = query-anchored showing identities; 2 = query-anchored no identities; 3 = flat query-anchored, show identities; 4 = flat query-anchored, no identities; 5 = query-anchored no identities and blunt ends; 6 = flat query-anchored, no identities and blunt ends; 7 = XML Blast output; 8 = tabular; 9 tabular with comment lines [Integer]; default = 0; -o BLAST report Output File [File Out] Optional; default = stdout; -F Filter query sequence (DUST with blastn, SEG with others) [String]; default = T; -G Cost to open a gap (zero invokes default behavior) [Integer]; default = 0; -E Cost to extend a gap (zero invokes default behavior) [Integer]; default = 0; -X X dropoff value for gapped alignment (in bits) (zero invokes default behavior); blastn 30, megablast 20, tblastx 0, all others 15 [Integer]; default = 0; -I Show Gl's in deflines [T/F]; default = F; - q Penalty for a nucleotide mismatch (blastn only) [Integer]; default = -3; -r Reward for a nucleotide match (blastn only) [Integer]; default = 1; -v Number of database sequences to show one-line descriptions for (V) [Integer]; default = 500; -b Number of database sequence to show alignments for (B) [Integer]; default = 250; -f Threshold for extending hits, default if zero; blastp 11, blastn 0, blastx 12, tblastn 13; tblastx 13, megablast 0 [Integer]; default = 0; -g Perfom gapped alignment (not available with tblastx) [T/F]; default = T; -Q Query Genetic code to use [Integer]; default = 1; -D DB Genetic code (for tblast[nx] only) [Integer]; default = 1; -a Number of processors to use [Integer]; default = 1; -O SeqAlign file [File Out] Optional; -J Believe the query defline [T/F]; default = F; -M Matrix [String]; default = BLOSUM62; -W Word size, default if zero (blastn 11, megablast 28, all others 3) [Integer]; default = 0; -z Effective length of the database (use zero for the real size) [Real]; default = 0; -K Number of best hits from a region to keep (off by default, if used a value of 100 is recommended) [Integer]; default = 0; -P 0 for multiple hit, 1 for single hit [Integer]; default = 0; -Y Effective length of the search space (use zero for the real size) [Real]; default = 0; -S Query strands to search against database (for blast[nx], and tblastx); 3 is both, 1 is top, 2 is bottom [Integer]; default = 3; -T Produce HTML output [T/F]; default = F; -I Restrict search of database to list of Gl's [String] Optional; -U Use lower case filtering of FASTA sequence [T/F] Optional; default = F; -y X dropoff value for ungapped extensions in bits (0.0 invokes default behavior); blastn 20, megablast 10, all others 7 [Real]; default = 0.0; -Z X dropoff value for final gapped alignment in bits (0.0 invokes default behavior); blastn/megablast 50, tblastx 0, all others 25 [Integer]; default = 0; -R PSI-TBLASTN checkpoint file [File In] Optional; -n MegaBlast search [T/F]; default = F; -L Location on query sequence [String] Optional; -A Multiple Hits window size, default if zero (blastn/megablast 0, all others 40 [Integer]; default = 0; -w Frame shift penalty (OOF algorithm for blastx) [Integer]; default = 0; -t Length of the largest intron allowed in tblastn for linking HSPs (0 disables linking) [Integer]; default = 0.

Results of high quality are reached by using the algorithm of Needleman and Wunsch or Smith and Waterman. Therefore programs based on said algorithms are preferred. Advantageously the comparisons of sequences can be done with the program PileUp (J. Mol. Evolution., 25, 351-360, 1987, Higgins et al., CABIOS, 5 1989: 151-153) or preferably with the programs Gap and BestFit, which are respectively based on the algorithms of Needleman and Wunsch [J. Mol. Biol. 48; 443-453 (1970)] and Smith and Waterman [Adv. Appl. Math. 2; 482-489 (1981)]. Both programs are part of the GCG software-package [Genetics Computer Group, 575 Science Drive, Madison, Wisconsin, USA 53711 (1991); Altschul et al. (1997) Nucleic Acids Res. 25:3389 et SEQ]. Therefore preferably the calculations to determine the perentages of sequence homology are done with the program Gap over the whole range of the sequences. The following standard adjustments for the comparison of nucleic acid sequences can be used: gap weight: 50, length weight: 3, average match: 10.000, average mismatch: 0.000.

Nucleic acid molecules advantageous to the method disclosed herein may be isolated on the basis of their homology to the nucleic acids disclosed herein and used in the method disclosed herein by using the sequences or a part thereof as hybridization probe according to standard hybridization techniques under stringent hybridization conditions, as described also, for example, in US 2002/0023281. It is possible here to use, for example, isolated nucleic acid molecules which are at least 10, for example at least 15, nucleotides in length and hybridize under stringent conditions with the nucleic acid molecules which comprise a nucleotide sequence of SEQ ID NO: 1, 3, 5, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92,94, 98, 100, 102 or 104or 132, 134. The term "hybridizes under for example stringent conditions", as used herein, is intended to describe hybridization and washing conditions under which nucleotide sequences, which are at least 20% identical to one another hybridize with one another. The term "hybridizes under stringed conditions", as used herein, is intended to describe hybridization and washing conditions under which nucleotide sequences which are 30%, but for example 50% or more, identical to one another hybridize with one another. For example, the conditions are such that sequences which are 60%, more for example 75% and even more for example at least approximately 85% or more, identical to one another usually remain hybridized to one another. The identity of two polynucleic acids or amino acids may be determined as described herein. These stringent conditions are known to the skilled worker and can be found in Current Protocols in Molecular Biology, John Wiley & Sons, N. Y. (1989), 6.3.1-6.3.6., or in Sambrook, Molecular Cloning: A Laboratory Manual, Cold Spring Harbor Laboratory, Cold Spring Harbor Laboratory Press, Cold Spring Harbor, NY, 1989. A preferred, nonlimiting example of stringent hybridization conditions is hybridizations in 6 × sodium chloride/sodium citrate (SSC) at about 45°C, followed by one or more washing steps in 0.2 × SSC, 0.1 % SDS at from 50 to 65°C. It is known to the skilled worker that these hybridization conditions differ depending on the type of nucleic acids, in particular according to the AT or GC content, or on the presence of organic solvents, with respect to temperature, duration of washing and salt concentration of the hybridization solutions and the washing solution. Under "standard hybridization conditions", for example, the temperature differs between 42°C and 58°C in aqueous buffer with a concentration of from 0.1 to 5 × SSC (pH 7.2), depending on the type of nucleic acid. If an organic solvent is present in the abovementioned buffer, for example 50% formamide, the temperature under standard conditions is about 42°C. The hybridization conditions for DNA:DNA hybrids, for example, are 0.1 × SSC and 20°C to 45°C, for example between 30°C and 45°C. The hybridization conditions for DNA:RNA hybrids, for example, are for example 0.1 × SSC and from 30°C to 55°C, for example between 45°C and 55°C. The hybridization temperatures mentioned above are determined, for example, for a nucleic acid of about 100 bp (= base pairs) in length and with a G + C content of 50% in the absence of formamide. The skilled worker knows how to determine the required hybridization conditions on the basis of textbooks such as the one mentioned above or the following textbooks: Sambrook, "Molecular Cloning", Cold Spring Harbor Laboratory, 1989; Hames and Higgins (eds.) 1985, "Nucleic Acids Hybridization: A Practical Approach", IRL Press at Oxford University Press, Oxford; Brown (eds.) 1991, "Essential Molecular Biology: A Practical Approach", IRL Press at Oxford University Press, Oxford or "Current Protocols in Molecular Biology", John Wiley & Sons, N. Y. (1989).

Some examples of conditions for DNA hybridization (Southern blot assays) and wash step are shown hereinbelow:
(1) Hybridization conditions can be selected, for example, from the following conditions:
   a) 4X SSC at 65°C,
   b) 6X SSC at 45°C,
   c) 6X SSC, 100 mg/ml denatured fragmented fish sperm DNA at 68°C,
   d) 6X SSC, 0.5% SDS, 100 mg/ml denatured salmon sperm DNA at 68°C,
   e) 6X SSC, 0.5% SDS, 100 mg/ml denatured fragmented salmon sperm DNA, 50% formamide at 42°C,
   f) 50% formamide, 4X SSC at 42°C,
   g) 50% (vol/vol) formamide, 0.1% bovine serum albumin, 0.1% Ficoll, 0.1% polyvinylpyrrolidone, 50 mM sodium phosphate buffer pH 6.5, 750 mM NaCl, 75 mM sodium citrate at 42°C,
   h) 2X or 4X SSC at 50°C (low-stringency condition), or
   i) 30 to 40% formamide, 2X or 4X SSC at 42°C (low-stringency condition).
(2) Wash steps can be selected, for example, from the following conditions:
   a) 0.015 M NaCl/0.0015 M sodium citrate/0.1% SDS at 50°C.
   b) 0.1X SSC at 65°C.
   c) 0.1X SSC, 0.5 % SDS at 68°C.
   d) 0.1X SSC, 0.5% SDS, 50% formamide at 42°C.
   e) 0.2X SSC, 0.1 % SDS at 42°C.
   f) 2X SSC at 65°C (low-stringency condition).

Furthermore, it is possible to identify, by comparing protein sequences homologous to SEQ ID NO: 2, or proteins from various organisms, conserved regions from which then in turn degenerated primers can be derived. These degenerated primers may then be used further by means of PCR for amplification of fragments of new homologous genes from other organisms. These fragments may then be used as hybridization probes for isolating the complete gene sequence. Alternatively, the missing 5' and 3' sequences may be isolated by means of RACE-PCR. In this respect, reference is expressly made to the disclosures in US 2002/0023281 and to the abovementioned literature on molecular-biological methods, in particular Sambrook, "Molecular Cloning" and "Current Protocols in Molecular Biology", John Wiley & Sons.

An isolated nucleic acid molecule coding for a protein used in the method disclosed herein, which protein is homologous in particular to a protein sequence of SEQ ID NO: 2, may be generated, for example, by introducing one or more nucleotide substitutions, additions or deletions into a nucleotide sequence of SEQ ID NO: 1, so that one or more amino acid substitutions, additions or deletions are introduced into the encoded protein. Mutations may be introduced in any of the sequences of the nucleic acid molecules used in the method by means of standard techniques such as site-specific mutagenesis and PCR-mediated mutagenesis. Preference is given to generating conservative amino acid substitutions on one or more of the predicted nonessential amino acid residues. In a "conservative amino acid substitution" the amino acid residue is replaced by an amino acid residue having a similar side chain. Families of amino acid residues with similar side chains have been defined in the art. These families comprise amino acids with basic side chains (e.g. lysine, arginine, histidine), acidic side chains (e.g. aspartic acid, glutamic acid), uncharged polar side chains (e.g. glycine, asparagine, glutamine, serine, threonine, tyrosine, cysteine), nonpolar side chains (e.g. alanine, valine, leucine, isoleucine, proline, phenylalanine, methionine, tryptophan), beta-branched side chains (e.g. threonine, valine, isoleucine) and aromatic side chains (e.g. tyrosine, phenylalanine, tryptophan, histidine). A predicted nonessential amino acid residue is thus for example replaced by another amino acid residue from the same side chain family. Preference is given to carrying out "conservative" substitutions in which the replaced amino acid has a property similar to that of the original amino acid, for example a substitution of Asp for Glu, Asn for Gln, Ile for Val, Ile for Leu, Thr for Ser.

In another disclosure, the mutations may alternatively be introduced randomly across all or part of the coding sequence, for example by saturation mutagenesis, and the resulting mutants may be screened for the activity described herein in order to identify mutants which lead, for example, to plants with an increased growth rate, for example faster growth and/or higher yield. After mutagenesis, the encoded protein may be recombinantly expressed, and the activity of said protein may be determined using the assays described herein, for example.

The nucleic acid molecules used in the method disclosed herein code for proteins or parts thereof. Said proteins or the individual protein or parts thereof for example comprises one of the consensus sequences or core consensus sequences shown above, e.g. an amino acid sequence as shown in Figure 1 or 2, whereby 20 or less, for example 15 or 10, for example 9, 8, 7, or 6, 5 or 4, even 3, even 2, even 1, 0 of the amino acids positions indicated by a capital letter in Figure 1 or 2 can be replaced by an x and/or not more than 5, for example 4, even 3 or 2, one or non amino acid position indicated by a capital letter in Figure 1 or 2 are/is replaced by an x and/or 20 or less, for example 15 or 10, for example 9, 8, 7, or 6, 5 or 4, even 3, even 2, even 1, 0 amino acids are inserted into or absent from the consensus sequence or, which is sufficiently homologous to an amino acid sequence of the sequence SEQ ID NO: 2, 4, 6, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 99, 101, 103 or 105,133 or 135, so that said protein or said part thereof retains SEQ ID NO: 2 activity.

For example, the nucleic acid molecule-encoded protein or part thereof has its essential biological activity which causes, inter alia, the target organism, for example the target plant, to exhibit a higher growth rate or faster growth and thus higher biomass production and an increased yield. Conserved regions of a protein may be determined by sequence comparisons of various homologs or derivatives of a protein or of various members of a protein family. Moreover, computer programs which predict the structure of a protein, owing to its sequence and other properties, are known to the skilled worker. Antibody binding studies and studies on the sensitivity or hypersensitivity of protein domains with regard to protease digestion may likewise be used to study the structure of a polypeptide or its location in a particular environment, for example in a cell. Further methods of this kind for characterizing of proteins are known to the skilled worker and are disclosed in the literature described herein, for example also in US 2002/0023281.

For example, the used part of a protein or a domain is highly conserved among the sequences described herein, for example among the plant sequences, or animal sequences, for example among all sequences.

"Essential biological activity" of the proteins or polypeptides used means, as discussed above, that said proteins or polypeptides possess the biological activity of SEQ ID NO: 2, . The "biological activity of SEQ ID NO: 2, " means that expression of the polypeptide in a nonhuman organism results in accelerated growth or in an increase of the yield by 5% or more, compared to a nonhuman organism which does not express said polypeptide, or expresses it to a lesser extent. More preference is given to an acceleration by 10%, even more preference to 20%, most preference to 50%, 100% or 200% or more. A test system for determining the biological activity of a sequence homologous to SEQ ID NO: 2, , which may be studied, is the phenotype of expression in Arabidopsis thaliana or, where appropriate, also (over)expression in the organism from which the homologous nucleic acid is derived.

The cellular activity or function of SEQ ID NO: 2, and its homologs is, as described above, not yet known and, consequently, an in-vitro assay system is likewise not available yet. Presumably, however, it is possible for the skilled worker to measure a specific SEQ ID NO: 2, activity of a protein or polypeptide by (over)expressing said protein or polypeptide in a cell, preferably in a deficient cell, and comparing it with the phenotype of a deficient cell.

Proteins which may be used advantageously in the method are derived from plant organisms such as algae or mosses or, especially, from higher plants.

Consequently, one embodiment of the method of the invention comprises introducing a polynucleotide into a plant, or one or more parts thereof, which polynucleotide codes for a SEQ ID NO: 2, polypeptide. The polynucleotide preferably comprises a polynucleotide characterized by the claims, in particular a polynucleotide encoding a protein with the sequence according to SEQ ID NO: 2, or encoding a polypeptide encoded by a nucleic acid molecule characterized by the claims, in particular according to SEQ ID NO: 1, or comprising any of these sequences so that a transgenic plant with faster growth, higher yield and/or higher tolerance to stress is obtained. Disclosed are plants expressing any of the plant sequences mentioned herein or their plant homologs.. As mentioned, however, yeast SEQ ID NO: 1, nucleic acid also exhibits SEQ ID NO: 2, activity in plants.

In one disclosure, the present description discloses a polypeptide encoded by the nucleic acid molecule disclosed herein, for example conferring above-mentioned activity.

The present description also discloses a method for the production of a polypeptide disclosed herein, the polypeptide being expressed in a transgenic plant cell according to the invention.

In one disclosure, the nucleic acid molecule used in the method for the production of the polypeptide is derived from a microorganism, with an eukaryotic organism as host cell. In one disclosure the polypeptide is produced in a plant cell or plant with a nucleic acid molecule derived from a prokaryote or a fungus or an alga or another microorganismus but not from plant.

The skilled worker knows that protein and DNA expressed in different organisms differ in many respects and properties, e.g. methylation, degradation and post-translational modification as for example glucosylation, phosphorylation, acetylation, myristoylation, ADP-ribosylation, farnesylation, carboxylation, sulfation, ubiquination, etc. though having the same coding sequence. For example, the cellular expression control of the corresponding protein differs accordingly in the control mechanisms controlling the activity and expression of an endogenous protein or another eukaryotic protein.

The polypeptide disclosed herein is for example produced by recombinant DNA techniques. For example, a nucleic acid molecule encoding the protein is cloned into an vector (as described above), the vector is introduced into a host cell (as described above) and said polypeptide is expressed in the host cell. Said polypeptide can then be isolated from the cells by an appropriate purification scheme using standard protein purification techniques. Alternative to recombinant expression, the polypeptide or peptide disclosed herein can be synthesized chemically using standard peptide synthesis techniques. Moreover, native polypeptide can be isolated from cells (e.g., endothelial cells), for example using the antibody disclosed herein as described, which can be produced by standard techniques utilizing the polypeptide disclosed herein or fragment thereof.

In one disclosure, the present description discloses a polypeptide comprising or consisting of a polypeptide sequence shown in SEQ ID NO: 2 or a homolog thereof of 50%, 70%, 80%, 85%, 90%, 95%, 97%, 99% or 99,5% or more but not being, for example not consisting of the sequence shown in SEQ ID NO: 2.

In one disclosure, the protein disclosed herein does not comprise the sequence shown in Seq ID NO: 2.

In one disclosure, the present description discloses a polypeptide having the amino acid sequence encoded by a nucleic acid molecule disclosed herein or obtainable by a method disclosed herein. Said polypeptide confers for example the aforementioned activity, in particular, the polypeptide confers the increase of the yield or growth as described herein in a cell or an organism or a part thereof after increasing the cellular activity, e.g. by increasing the expression or the specific activity of the polypeptide. In one disclosure, said polypeptide distinguishes over the sequence depicted in SEQ ID No: 2, 4, 6, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, by one or more amino acids. In another disclosure, said polypeptide disclosed herein does not consist of the sequence shown in SEQ ID NO: 2, 4, 6, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95,. In one disclosure, said polypeptide does not consist of the sequence encoded by the nucleic acid molecules shown in SEQ ID NO: 1, 3, 5, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94,. In one disclosure, the polypeptide disclosed herein orginates from a non-plant cell, in particular from a microorganism, and was expressed in a plant cell. The terms "protein" and "polypeptide" used in this application are interchangeable. "Polypeptide" refers to a polymer of amino acids (amino acid sequence) and does not refer to a specific length of the molecule. Thus peptides and oligopeptides are included within the definition of polypeptide. This term does also refer to or include post-translational modifications of the polypeptide, for example, glycosylations, acetylations, phosphorylations and the like. Included within the definition are, for example, polypeptides containing one or more analogs of an amino acid (including, for example, unnatural amino acids, etc.), polypeptides with substituted linkages, as well as other modifications known in the art, both naturally occurring and non-naturally occurring.

For example, the polypeptide is isolated. An "isolated" or "purified" protein or polynucleiotide or biologically active portion thereof is substantially free of cellular material when produced by recombinant DNA techniques or chemical precursors or other chemicals when chemically synthesized.

The language "substantially free of cellular material" includes preparations of the polypeptide disclosed herein in which the protein is separated from cellular components of the cells in which it is naturally or recombinantly produced. In one disclosure, the language "substantially free of cellular material" includes preparations having less than about 30% (by dry weight) of "contaminating protein", more for example less than about 20% of "contaminating protein", still more for example less than about 10% of "contaminating protein", and most for example less than about 5% "contaminating protein". The term "Contaminating protein" relates to polypeptides, which are not polypeptides disclosed herein. When the polypeptide disclosed herein or biologically active portion thereof is recombinantly produced, it is also for example substantially free of culture medium, i.e., culture medium represents less than about 20%, more for example less than about 10%, and most for example less than about 5% of the volume of the protein preparation. The language "substantially free of chemical precursors or other chemicals" includes preparations in which the polypeptide disclosed herein is separated from chemical precursors or other chemicals which are involved in the synthesis of the protein.

A polypeptide disclosed herein can participate in the method of the present invention.

Further, the polypeptide can have an amino acid sequence which is encoded by a nucleotide sequence which hybridizes, for example hybridizes under stringent conditions as described above, to a nucleotide sequence of the polynucleotide disclosed herein. Accordingly, the polypeptide has an amino acid sequence which is encoded by a nucleotide sequence that is at least about 35%, 50%, or 60% for example at least about 70%, more for example at least about 80%, 90%, 95%, and even more for example at least about 96%, 97%, 98%, 99% or more homologous to one of the amino acid sequences of the polypeptide disclosed herein and shown herein. The preferred polypeptide disclosed herein for example possesses at least one of the activities described herein. A preferred polypeptide disclosed herein includes an amino acid sequence encoded by a nucleotide sequence which hybridizes, for example hybridizes under stringent conditions, as defined above.

The description also provides chimeric or fusion proteins.

As used herein, a "chimeric protein" or "fusion protein" comprises an polypeptide operatively linked to a polypeptide which does not confer above-mentioned activity.

Within the fusion protein, the term "operatively linked" is intended to indicate that the polypeptide disclosed herein and a non-invention polypeptide are fused to each other so that both sequences fulfil the proposed function addicted to the sequence used. The non-invention polypeptide can be fused to the N-terminus or C-terminus of the polypeptide disclosed herein. For example, in one disclosure the fusion protein is a GST-LMRP fusion protein in which the sequences of the polypeptide disclosed herein are fused to the C-terminus of the GST sequences. Such fusion proteins can facilitate the purification of recombinant polypeptides disclosed herein.

In another disclosure, the fusion protein is a polypeptide disclosed herein containing a heterologous signal sequence at its N-terminus. In certain host cells (e.g., mammalian host cells), expression and/or secretion can be increased through use of a heterologous signal sequence. Targeting sequences, are required for targeting the gene product into specific cell compartment (for a review, see Kermode, Crit. Rev. Plant Sci. 15, 4 (1996) 285-423 and references cited therein), for example into the vacuole, the nucleus, all types of plastids, such as amyloplasts, chloroplasts, chromoplasts, the extracellular space, the mitochondria, the endoplasmic reticulum, elaioplasts, peroxisomes, glycosomes, and other compartments of cells or extracellular. Sequences, which must be mentioned in this context are, in particular, the signal-peptide- or transit-peptide-encoding sequences which are known per se. For example, plastid-transit-peptide-encoding sequences enable the targeting of the expression product into the plastids of a plant cellTargeting sequences are also known for eukaryotic and to a lower extent for prokaryotic organisms and can advantageously be operable linked with the nucleic acid molecule disclosed herein to achieve an expression in one of said compartments or extracellular.

For example, a chimeric or fusion protein disclosed herein is produced by standard recombinant DNA techniques. For example, DNA fragments coding for the different polypeptide sequences are ligated together in-frame in accordance with conventional techniques, for example by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. The fusion gene can be synthesized by conventional techniques including automated DNA synthesizers. Alternatively, PCR amplification of gene fragments can be carried out using anchor primers, which give rise to complementary overhangs between two consecutive gene fragments which can subsequently be annealed and reamplified to generate a chimeric gene sequence (see, for example, Current Protocols in Molecular Biology, eds. Ausubel et al. John Wiley & Sons: 1992). Moreover, many expression vectors are commercially available that already encode a fusion moiety (e.g., a GST polypeptide). The polynucleotide disclosed herein can be cloned into such an expression vector such that the fusion moiety is linked in-frame to the encoded protein.

Furthermore, folding simulations and computer redesign of structural motifs of the protein disclosed herein can be performed using appropriate computer programs (Olszewski, Proteins 25 (1996), 286-299; Hoffman, Comput. Appl. Biosci. 11 (1995),675-679). Computer modeling of protein folding can be used for the conformational and energetic analysis of detailed peptide and protein models (Monge, J. Mol. Biol. 247 (1995), 995-1012; Renouf, Adv. Exp. Med. Biol. 376 (1995), 37-45). The appropriate programs can be used for the identification of interactive sites the polypeptide disclosed herein and its substrates or binding factors or other interacting proteins by computer assistant searches for complementary peptide sequences (Fassina, Immunomethods (1994), 114-120). Further appropriate computer systems for the design of protein and peptides are described in the prior art, for example in Berry, Biochem. Soc. Trans. 22 (1994), 1033-1036; Wodak, Ann. N. Y. Acad. Sci. 501 (1987), 1-13; Pabo, Biochemistry 25 (1986), 5987-5991. The results obtained from the above-described computer analysis can be used for, e.g., the preparation of peptidomimetics of the protein disclosed herein or fragments thereof. Such pseudopeptide analogues of the, natural amino acid sequence of the protein may very efficiently mimic the parent protein (Benkirane, J. Biol. Chem. 271 (1996), 33218-33224). For example, incorporation of easily available achiral Q-amino acid residues into a protein disclosed herein or a fragment thereof results in the substitution of amide bonds by polymethylene units of an aliphatic chain, thereby providing a convenient strategy for constructing a peptidomimetic (Banerjee, Biopolymers 39 (1996), 769-777).

Superactive peptidomimetic analogues of small peptide hormones in other systems are described in the prior art (Zhang, Biochem. Biophys. Res. Commun. 224(1996), 327-331). Appropriate peptidomimetics of the protein disclosed herein can also be identified by the synthesis of peptidomimetic combinatorial libraries through successive amide alkylation and testing the resulting compounds, e.g., for their binding and immunological properties. Methods for the generation and use of peptidomimetic combinatorial libraries are described in the prior art, for example in Ostresh, Methods in Enzymology 267 (1996), 220-234 and Dorner, Bioorg. Med. Chem. 4 (1996), 709-715.

Furthermore, a three-dimensional and/or crystallographic structure of the protein disclosed herein can be used for the design of peptidomimetic inhibitors of the biological activity of the protein disclosed herein (Rose, Biochemistry 35 (1996), 12933-12944; Rutenber, Bioorg. Med. Chem. 4 (1996), 1545-1558).

Furthermore, a three-dimensional and/or crystallographic structure of the protein disclosed herein and the identification of interactive sites the polypeptide disclosed herein and its substrates or binding factors can be used for design of mutants with modulated binding or turn over activities. For example, the active center of the polypeptide disclosed herein can be modelled and amino acid residues participating in the catalytic reaction can be modulated to increase or decrease the binding of the substrate to inactivate the polypeptide. The identification of the active center and the amino acids involved in the catalytic reaction facilitates the screening for mutants having an increased activity. In particular, the information about the conservative amino acids in the consensus sequences can help to modulate the activity.

"Transgenic" or "recombinant" means in accordance with the invention, for example with regard to a nucleic acid sequence, to an expression cassette (= gene construct) or to a vector comprising the nucleic acid sequence disclosed herein or to a plant transformed with the nucleic acid molecule sequences, expression cassette or vector disclosed herein, all those constructions produced by genetic methods, in which
a) the nucleic acid sequence used in the method of the invention or
b) a genetic control or regulatory sequence functionally linked to a nucleic acid sequence used in the method of the invention, for example a promotor, or
c) (a) and (b)
are not present in their natural, genetic environment or have been modified by genetic methods, said modification possibly being, by way of example, a substitution, addition, deletion, inversion or insertion of one or more nucleotide residues. Natural genetic environment means the natural genomic or chromosomal locus in the source organism or the presence in a genomic library. In the case of a genomic library, the natural, genetic environment of the nucleic acid sequence is for example at least partially still retained. The environment flanks the nucleic acid sequence at least on one side and its sequence is from 0 or more bp, for example 50 bp, more for example from 100 to 500 bp, particularly for example 1 000 bp or more, in length, although sequences of 5 000 bp or more have also been described. A naturally occurring expression cassette, for example the naturally occurring combination of the natural promoter of the vacuolar morphogenesis protein VAM7 nucleic acid sequence, becomes a transgenic expression cassette when altered by nonnatural, synthetic ("artificial") methods such as, for example, mutagenesis. Corresponding methods are described, for example, in US 5,565,350 or WO 00/15815.

The regulatory functions of a natural as well as artificial expression cassette may be altered indirectly or in trans by changing factors which regulate said expression cassette. This includes, in particular, homologous, heterologous and artificial transcription factors influencing regulation.

Cloning vectors as described in detail in the prior art and also herein may be used for transformation. Vectors and methods suitable for transformation of plants have been published or cited in, for example: Plant Molecular Biology and Biotechnology (CRC Press, Boca Raton, Florida), chapter 6/7, pp. 71-119 (1993); F.F. White, Vectors for Gene Transfer in Higher Plants; in: Transgenic Plants, vol. 1, Engineering and Utilization, eds: Kung and R. Wu, Academic Press, 1993, 15-38; B. Jenes et al., Techniques for Gene Transfer, in: Transgenic Plants, vol. 1, Engineering and Utilization, eds: Kung and R. Wu, Academic Press (1993), 128-143; Potrykus, Annu. Rev. Plant Physiol. Plant Molec. Biol. 42 (1991), 205-225.

The transformation of microorganisms and higher eukaryotes is described in numerous textbooks, for example in Sambrook, Molecular Cloning, 1989, Cold Spring Harbor Laboratory and in "Current Protocols in Molecular Biology", John Wiley & Sons, N. Y. (1989).

It is possible to express homologous or heterologous nucleic acids, i.e. the acceptor and donor organisms belong to the same species, where appropriate to the same variety, or to different species, where appropriate varieties. However, transgenic also means that the nucleic acids disclosed herein are located at their natural location in the genome of an organism but that the sequence has been altered compared to the natural sequence and/or the regulatory sequences of the natural sequences have been altered. Transgenic preferably means expression of the nucleic acids disclosed herein at a nonnatural site in the genome, i.e. homologous or, preferably, heterologous expression of said nucleic acids occurs.

The term "regulatory sequences" also includes those sequences which control constitutive expression of a nucleotide sequence in many host cell species and those which control direct expression of the nucleotide sequence only in particular host cells under particular conditions. The skilled worker appreciates that the design of the expression vector may depend on factors such as selection of the host cell to be transformed, degree of expression of the desired protein, etc. Transcription may be increased, for example, by using strong transcription signals such as promoter and/or enhancer or mRNA stabilizers, for example by particular 5' and/or 3'UTRs. Thus, for example, signals leading to a higher rate of transcription or to a more stable mRNA may be substituted for endogenous signals. In addition, however, it is also possible to enhance translation by improving, for example, ribosome binding or mRNA stability.
In principle, those promoters may be used which are able to stimulate transcription of genes in organisms such as microorganisms, plants or animals. Suitable promoters which are functional in said organisms are well known. They may be constitutive or inducible promoters. Suitable promoters may enable development- and/or tissue-specific expression in multicellular eukaryotes, and it is thus possible to use advantageously leaf-, root-, flower-, seed-, guard cell- or fruit-specific promoters in plants. Further regulatory sequences are described above and below.

The term "transgenic", used according to the invention, also refers to the progeny of a transgenic nonhuman organism, for example a plant, for example the T₁, T₂, T₃ and subsequent plant generations or the BC₁, BC₂, BC₃ and subsequent plant generations. Thus, the transgenic plants of the invention may be grown and crossed with themselves or with other individuals in order to obtain further transgenic plants of the invention. It is also possible to obtain transgenic plants by vegetative propagation of transgenic plant cells.
The amount of expression of a gene is regulated at the transcriptional or translational level or with respect to the stability and degradation of a gene product.

Regulatory sequences are usually arranged upstream (5'), within and/or downstream (3') with respect to a particular nucleic acid or a particular codogenic gene section. They control in particular transcription and/or translation and also transcript stability of the codogenic gene section, where appropriate in cooperation with further functional systems intrinsic to the cell, such as the protein biosynthesis apparatus of the cell. Thus it is possible to influence promoter, UTR, splice sites, polyadenylation signals, terminators, enhancers, processing signals, posttranscriptional and/or posttranslational modifications, etc. according to the knowledge of the skilled worker in order to increase expression of an endogenous protein without influencing the sequence of said protein itself.

Furthermore, transcriptional regulation may be specifically altered by introducing an artificial transcription factor, as described below and in the examples.

Regulatory sequences are disclosed, for example, in Goeddel: Gene Expression Technology: Methods in Enzymology 185, Academic Press, San Diego, CA (1990), or in Gruber; Methods in Plant Molecular Biology and Biotechnolgy, CRC Press, Boca Raton, Florida, eds.: Glick and Thompson, chapter 7, 89-108, including the references therein.

Generally it is possible, for example by means of promoter analyses, to identify endogenous transcription factors involved in transcriptional regulation of an endogenous SEQ ID NO: 1, gene. Increased activity of positive regulators or else reduced activity of negative regulators may increase transcription of an endogenous SEQ ID NO: 1, gene.

Furthermore, methods for altering expression of genes by means of artificial transcription factors are known to the skilled worker.

Thus, for example, an alteration in expressing a gene, in particular a gene expressing SEQ ID NO: 2, , may be achieved by modifying or synthesizing particular specific DNA-binding factors such as, for example, zinc-finger transcription factors. These factors bind to particular genomic regions of an endogenous target gene, preferably to the regulatory sequences, and may cause activation or repression of said gene. The use of such a method make it possible to activate or reduce expression of the endogenous gene, avoiding a recombinant manipulation of the sequence of said gene. Corresponding methods are described, for example, in Dreier B [(2001) J. Biol. Chem. 276(31): 29466-78 and (2000) J. Mol. Biol. 303(4): 489-502], Beerli RR (1998) Proc. Natl. Acad. Sci. USA 95(25): 14628-14633; (2000) Proc. Natl. Acad. Sci. USA 97(4): 1495-1500 and (2000) J. Biol. Chem. 275(42): 32617-32627), Segal DJ and Barbas CF (2000) Curr. Opin. Chem. Biol. 4(1): 34-39, Kang JS and Kim JS (2000) J. Biol. Chem. 275(12): 8742-8748, Kim JS, (1997) Proc. Natl. Acad. Sci. USA 94(8): 3616-3620, Klug A (1999) J. Mol. Biol. 293(2): 215-218, Tsai SY, (1998) Adv. Drug Deliv. Rev. 30(1-3): 23-31], Mapp AK (2000) Proc. Natl. Acad. Sci. USA 97(8): 3930-3935, Sharrocks AD (1997) Int. J. Biochem. Cell Biol. 29(12): 1371-1387 and Zhang L (2000) J. Biol. Chem. 275(43): 33850-33860.

Examples of applying the method for modification of gene expression in plants are described, for example, in WO 01/52620, Ordiz MI, (2002) Proc. Natl. Acad. Sci. USA, 99(20):13290-13295) or Guan (2002) Proc. Natl. Acad. Sci. USA, 99(20): 13296-13301) and in the examples mentioned below.

In one disclosure, the method disclosed herein comprises increasing the gene copy number of the polynucleotide used in the method disclosed herein and characterized herein in the plant.

Advantageously, the method described herein increases the number and size of leaves, the number of fruits and/or the size of fruits of a plant whose SEQ ID NO: 2, activity is increased, fruit meaning any harvested products of a plant, such as, for example, seeds, tubers, leaves, flowers, bark, fruits and roots.

The plant prepared in the method of the invention preferably has a fresh weight which is increased by 5%, more preferably by 10%, even more preferably by more than 15%, 20% or 30%. Even more preference is given to an increase in yield by 50% or more, for example by 75%, 100% or 200% or more.

The yield of the plant prepared in the method of the invention is preferably increased by at least 5%, more preferably by more than 10%, even more preferably by more than 15%, 20% or 30%. Even more preference is given to an increase in yield by more than 50% or more, for example by 75%, 100% or 200% or more.

In a further disclosure, the plant prepared in the method disclosed herein is more tolerant to abiotic or biotic stress.

In a disclosure, the description also discloses a method for preparing fine chemicals. The method comprises providing a cell, a tissue or an organism having increased SEQ ID NO: 2, activity and culturing said cell, said tissue or said organism under conditions which allow production of the desired fine chemicals in said cell, said tissue or said organism. Preference is given to providing in the method a plant of the invention, a microorganism disclosed herein or a useful animal disclosed herein.

As described above, increasing the activity of SEQ ID NO: 2, in a nonhuman organism, in particular in plants, results in an increase in the yield and in faster growth. By now, however, many organisms are used for producing fine chemicals. The production of fine chemicals nowadays is unimaginable without microorganisms which produce inexpensive and specific, even complex molecules whose chemical synthesis comprises many process stages and purification steps. Thus, fine chemicals such as vitamins and amino acids are industrially produced on a large scale in the same way as complex pharmaceutical active compounds such as, for example, growth factors, antibodies, etc., and the term fine chemicals is intended to also include these active compounds hereinbelow. Plants are likewise already used for producing various fine chemicals such as, for example, polymers, e.g. polyhydroxyalkanoids, vitamins, amino acids, sugars, fatty acids, in particular polyunsaturated fatty acids, etc. Even useful animals are already used for producing fine chemicals. Thus, production of antibodies and other pharmaceutical active compounds in the milk of goats or cows has already been described.

In a particularly preferred disclosure, the method disclosed herein consequently discloses a method in which the SEQ ID NO: 2, activity in a nonhuman organism, for example a plant or a microorganism, is increased and one or more metabolic pathways are modulated in such a way that the yield and/or efficiency of production of one or more fine chemicals is increased.

The terms production or productivity are known to the skilled worker and comprise increasing the concentration of desired products (e.g. fatty acids, carotenoids, (poly)saccharides, vitamins, isoprenoids, lipids, fatty acid (esters), and/or polymers such as polyhydroxyalkanoids and/or their metabolic products or other desired fine chemicals as described herein) within a particular time and a particular volume (e.g. kilogram/hour/liter).

The term "fine chemical" is known in the art and includes molecules which are produced by a nonhuman organism and are used in various branches of industry such as, for example, but not restricted to, the pharmaceutical industry, the agricultural industry and the cosmetics industry. These compounds comprise organic acids such as tartaric acid, itaconic acid and diaminopimelic acid, polymers or macromolecules such as, for example, polypeptides, e.g. enzymes, antibodies, growth factors or fragments thereof, nucleic acids, including polynucleic acids, both proteinogenic and nonproteinogenic amino acids, purine and pyrimidine bases, nucleosides and nucleotides (as described, for example, in Kuninaka, A. (1996) Nucleotides and related compounds, pp. 561-612, in Biotechnology vol. 6, Rehm et al., eds VCH: Weinheim and the references therein), lipids, saturated and unsaturated fatty acids (e.g. arachidonic acid), diols (e.g. propanediol and butanediol), carbohydrates (e.g. pentoses, hexoses, hyaluronic acid and trehalose), aromatic compounds (e.g. aromatic amine, vanillin and indigo), isoprenoids, prostagladins, triacylglycerol, cholesterol, polyhydroxyalkanoids, vitamins and cofactors (as described in Ullmann's Encyclopedia of Industrial Chemistry, vol. A27, "Vitamins", pp. 443-613 (1996) VCH: Weinheim and the references therein; and Ong, A.S., Niki, E. and Packer, L. (1995) "Nutrition, Lipids, Health and Disease" Proceedings of the UNESCO/Confederation of Scientific and Technological Associations in Malaysia and the Society for Free Radical Research - Asia, held on Sept. 1-3, 1994 in Penang, Malaysia, AOCS Press (1995)), enzymes and all other chemicals described by Gutcho (1983) in Chemicals by Fermentation, Noyes Data Corporation, ISBN: 0818805086 and the references indicated therein. The term "fine chemicals", as used herein, thus also includes pharmaceutical compounds which can be produced in organisms, for example antibodies, growth factors, etc. or fragments thereof.

The term "amino acid" is known in the art. Amino acids comprise the fundamental structural units of all proteins and are thus essential for normal cell functions. Proteinogenic amino acids, of which there are 20 types, serve as structural units for proteins in which they are linked together by peptide bonds, whereas the nonproteinogenic amino acids (hundreds of which are known) usually do not occur in proteins (see Ullmann's Encyclopedia of Industrial Chemistry, vol. A2, pp. 57-97 VCH: Weinheim (1985)). Amino acids can exist in the D or L configuration, although L-amino acids are usually the only type found in naturally occurring proteins. Biosynthetic and degradation pathways of each of the 20 proteinogenic amino acids are well characterized both in prokaryotic and eukaryotic cells (see, for example, Stryer, L. Biochemistry, 3rd edition, pp. 578-590 (1988)). Apart from their function in protein biosynthesis, these amino acids are interesting chemicals as such, and it has been found that many have various applications in the human food, animal feed, chemical, cosmetic, agricultural and pharmaceutical industries. Lysine is an important amino acid not only for human nutrition but also for monogastric animals such as poultry and pigs. Glutamate is most frequently used as a flavor additive (monosodium glutamate, MSG) and elsewhere in the food industry, as are aspartate, phenylalanine, glycine and cysteine. Glycine, L-methionine and tryptophan are all used in the pharmaceutical industry. Glutamine, valine, leucine, isoleucine, histidine, arginine, proline, serine and alanine are used in the pharmaceutical industry and the cosmetics industry. Threonine, tryptophan and D-/L-methionine are widely used animal feed additives (Leuchtenberger, W. (1996) Amino acids - technical production and use, pp. 466-502 in Rehm et al., (eds) Biotechnology vol. 6, chapter 14a, VCH: Weinheim). It has been found that these amino acids are moreover suitable as precursors for synthesizing synthetic amino acids and proteins, such as N-acetylcysteine, S-carboxymethyl-L-cysteine, (S)-5-hydroxytryptophan and other substances described in Ullmann's Encyclopedia of Industrial Chemistry, vol. A2, pp. 57-97, VCH, Weinheim, 1985.

The term "vitamin" is known in the art and comprises nutrients which are required for normal functioning of an organism but cannot be synthesized by this organism itself. The group of vitamins may include cofactors and nutraceutical compounds.

The term "cofactor" comprises nonproteinaceous compounds necessary for the appearance of a normal enzymic activity. These compounds may be organic or inorganic; the cofactor molecules disclosed herein are for example organic.

The term "nutraceutical" comprises food additives which are health-promoting in plants and animals, especially humans. Examples of such molecules are vitamins, antioxidants and likewise certain lipids (e.g. polyunsaturated fatty acids).

Vitamins, cofactors and nutraceuticals consequently comprise a group of molecules which cannot be synthesized by higher animals which therefore have to take them in, although they are readily synthesized by other organisms such as bacteria. These molecules are either bioactive molecules per se or precursors of bioactive substances which serve as electron carriers or intermediate products in a number of metabolic pathways. Besides their nutritional value, these compounds also have a substantial industrial value as colorants, antioxidants and catalysts or other processing auxiliaries. For an overview of the structure, activity and industrial applications of these compounds, see, for example, Ullmann's Encyclopedia of Industrial Chemistry, "Vitamins", vol. A27, pp. 443-613, VCH: Weinheim, 1996. Polyunsaturated fatty acids are described in particular in: Simopoulos 1999, Am. J. Clin. Nutr., 70 (3 Suppl):560-569, Takahata et al., Biosc. Biotechnol. Biochem, 1998, 62 (11):2079-2085, Willich and Winther, 1995, Deutsche Medizinische Wochenschrift, 120 (7):229 ff and the references therein.

The term "purine" or "pyrimidine" comprises nitrogen-containing bases which form part of nucleic acids, coenzymes and nucleotides. The term "nucleotide" comprises the fundamental structural units of nucleic acid molecules, which comprise a nitrogen-containing base, a pentose sugar (the sugar is ribose in the case of RNA and D-deoxyribose in the case of DNA) and phosphoric acid. The term "nucleoside" comprises molecules which serve as precursors of nucleotides but have, in contrast to the nucleotides, no phosphoric acid unit. It is possible to inhibit RNA and DNA synthesis by inhibiting the biosynthesis of these molecules or their mobilization to form nucleic acid molecules; targeted inhibition of this activity in cancer cells allows the ability of tumor cells to divide and replicate to be inhibited. Moreover, there are nucleotides which do not form nucleic acid molecules but serve as energy stores (i.e. AMP) or as coenzymes (i.e. FAD and NAD). However, purine and pyrimidine bases, nucleosides and nucleotides also have other possible uses: as intermediate products in the biosynthesis of various fine chemicals (e.g. thiamine, S-adenosylmethionine, folates or riboflavin), as energy carriers for the cell (e.g. ATP or GTP) and for chemicals themselves; they are ordinarily used as flavor enhancers (e.g. IMP or GMP) or for many medical applications (see, for example, Kuninaka, A., (1996) "Nucleotides and Related Compounds in Biotechnology" vol. 6, Rehm et al., eds. VCH: Weinheim, pp. 561-612). Enzymes involved in purine, pyrimidine, nucleoside or nucleotide metabolism are also increasingly serving as targets against which chemicals are being developed for crop protection, including fungicides, herbicides and insecticides.

A cell contains different carbon sources which are also included in the term "fine chemicals", for example sugars such as glucose, fructose, mannose, galactose, ribose, sorbose, ribulose, lactose, maltose, sucrose or raffinose, starch or cellulose, alcohols (e.g. methanol or ethanol), alkanes, fatty acids, in particular polyunsaturated fatty acids and organic acids such as acetic acid or lactic acid. Sugars may be transported by a multiplicity of mechanisms via the cell membrane into the cell. The ability of cells to grow and to divide rapidly in culture depends to a high degree on the extent of the ability of said cells to absorb and utilize energy-rich molecules such as glucose and other sugars. Trehalose consists of two glucose molecules linked together by an α,α-1,1-linkage. It is ordinarily used in the food industry as sweetener, as additive for dried or frozen foods and in beverages. However, it is also used in the pharmaceutical industry, the cosmetics industry and the biotechnology industry (see, for example, Nishimoto et al., (1998) US-Patent NO 5 759 610; Singer, M.A. and Lindquist, S. Trends Biotech. 16 (1998) 460-467; Paiva, C.L.A. and Panek, A.D. Biotech Ann. Rev. 2 (1996) 293-314; and Shiosaka, M. J. Japan 172 (1997) 97-102). Trehalose is used by enzymes of many microorganisms and is naturally released into the surrounding medium from which it can be isolated by methods known in the art.

The biosynthesis of said molecules in organisms has been comprehensively characterized, for example in Ullmann's Encyclopedia of Industrial Chemistry, VCH: Weinheim, 1996, e.g. chapter "Vitamins", vol. A27, pp. 443-613, Michal, G. (1999) Biochemical Pathways: An Atlas of Biochemistry and Molecular Biology, John Wiley & Sons; Ong, A.S., Niki, E. and Packer, L. (1995) "Nutrition, Lipids, Health and Disease" Proceedings of the UNESCO/Confederation of Scientific and Technological Associations in Malaysia and the Society for free Radical Research - Asia, held on Sept. 1-3, 1994 in Penang, Malaysia, AOCS Press, Champaign, IL X, 374 S).

Consequently, disclosed herein is a method for increasing oil production of a plant.

Plants may be used advantageously, for example, for the production of fatty acids. For example, storage lipids in the seeds of higher plants are synthesized from fatty acids which mainly have from 16 to 18 carbons. Said fatty acids are located in the seed oils of various plant species. An increase in SEQ ID NO: 2, in Arabidopsis has already shown that seed production is increased by approx. 30%. The production of said oils in plants may be increased, for example, by expressing polynucleotides characterized herein. Vegetable oils may then be used, for example, as fuel or as material for various products such as, for example, plastics, drugs, etc. Polyunsaturated fatty acids may be used particularly advantageously in nutrition and feeding.

In one disclosure, said method disclosed herein comprises preparing fine chemicals by transforming the nonhuman organism with one or more further polynucleotides whose gene products are part of one of the abovementioned metabolic pathways or whose gene products are involved in the regulation of one of these metabolic pathways so that the nonhuman organism produces the desired fine chemicals or the production of a desired fine chemical is increased. Advantageously, coexpression of the genes used in the method together with the increase in SEQ ID NO: 2, activity advantageously achieves an increase in production of said fine chemicals. Genes which serve the production of said fine chemicals are known to the skilled worker and have been described in the literature in many different ways.

The biosynthesis of said fine chemicals, for example of fatty acids, carotenoids, polysaccharides, vitamins, isoprenoids, lipids, fatty esters or polyhydroxyalkanoids and the abovementioned metabolic products, in plants often takes place in special metabolic pathways of particular cell organelles. Consequently, polynucleotides whose gene products play a part in these biosynthetic pathways and which are consequently located in said special organelles include sequences which code for corresponding signal peptides.

Further polynucleotides may be introduced into the host cell, preferably into a plant cell, with the gene constructs, expression cassettes, vectors, etc. described herein. Expression cassettes, gene constructs, vectors, etc. of this kind may be introduced by simultaneous transformation of a plurality of individual expression cassettes, gene constructs, vectors, etc. or, preferably, by combining a plurality of genes, ORFs or expression cassettes in one construct. It is also possible to use a plurality of vectors with in each case a plurality of expression cassettes for transformation and introduce them into the host cell.

Consequently, the gene constructs, expression cassettes, vectors, etc. described above for the method of the invention may mediate also the increase or reduction in further genes, in addition to the increase in SEQ ID NO: 1, expression.

It is therefore advantageous to introduce into the host organisms and express therein regulator genes such as genes for inducers, repressors or enzymes which, due to their activity, intervene in the regulation of one or more genes of a biosynthetic pathway. These genes may be of heterologous or homologous origin. Furthermore, it is possible additionally to introduce biosynthesis genes for producing fine chemicals so that the production of said fine chemicals is particularly effective due to the accelerated growth.

For this purpose, the aforementioned nucleic acids may be used for transformation of plants, for example with the aid of Agrobacterium, after they have been cloned into expression cassettes of the invention, for example in combination with nucleic acid molecules encoding other polypeptides. The genes encoding "other polypeptides" or "regulators" may also be introduced into the desired nonhuman organisms in independent transformations. This may take place before or after increasing the SEQ ID NO: 2, activity in said nonhuman organism. Cotransformation with a second expression construct or vector and subsequent selection for the appropriate marker is also possible.

In one embodiment, the invention relates to a gene construct, an expression cassette or a vector which comprises one or more of the nucleic acid molecules or polynucleotides as defined by the claims. Cassettes, constructs or vectors are preferably suitable for use in the method and comprise, for example, the abovementioned SEQ ID NO: 2, activity-encoding polynucleotides, preferably functionally linked to one or more regulatory signals for mediating or increasing gene expression in plants.

Said homologs, derivatives or analogs which are functionally linked to one or more regulatory signals or regulatory sequences, advantageously for increasing gene expression, are included.

The regulatory sequences are intended to make possible targeted expression of the genes and synthesis of the encoded proteins. The term "regulatory sequence" is defined above and includes, for example, include the described terminator, processing signals, posttranscriptional, posttranslational modifications, promoter, enhancer, UTR, splice sites, polyadenylation signals and other expression control elements known to the skilled worker and mentioned herein.

Depending on the host organism, for example, this may mean that the gene is expressed and/or overexpressed only after induction or that it is expressed and/or overexpressed immediately. Examples of these regulatory sequences are sequences to which inducers or repressors bind and thus regulate expression of the nucleic acid. In addition to these new regulatory sequences or instead of these sequences, the natural regulation of said sequences may still be present upstream of the actual structural genes and, where appropriate, may have been genetically modified so that natural regulation has been switched off and expression of the genes has been increased. However, the expression cassette (= expression construct = gene construct) may also have a simpler structure, i.e. no additional regulatory signals are inserted upstream of the nucleic acid sequence or derivatives thereof and the natural promoter with its regulation is not deleted. Instead, the natural regulatory sequence is mutated so that regulation no longer takes place and/or gene expression is increased. These modified promoters may also be put in the form of partial sequences (= promoter with parts of the nucleic acid sequences disclosed herein) alone upstream of the natural gene to increase the activity. Moreover, the gene construct may advantageously also comprise one or more "enhancer" sequences functionally linked to the promoter, which make increased expression of the nucleic acid sequence possible. Additional advantageous sequences such as further regulatory elements or terminators may also be inserted at the 3' end of the DNA sequences. The nucleic acid sequence(s) disclosed herein coding preferably for an SEQ ID NO: 2, activity may be present in one or more copies in the expression cassette (= gene construct). One or more copies of the genes may be present in the expression cassette. This gene construct or the gene constructs may be expressed together in the host organism. It is possible for the gene construct or gene constructs to be inserted in one or more vectors and be present in free form in the cell or else be inserted in the genome. In the case of plants, integration into the plastid genome or into the cell genome may have taken place. Cloning vectors as are comprehensively described in the prior art and here may be used for transformation.

Preference is given to introducing the nucleic acid sequences used in the method into an expression cassette which enables the nucleic acids to be expressed in a plant.

The expression cassettes may in principle be used directly for introduction into the plant or else be introduced into a vector.

In another disclosure, the description also discloses the complementary sequences of said polynucleotide disclosed herein and to an antisense polynucleic acid. An antisense nucleic acid molecule comprises, for example, a nucleotide sequence which is complementary to the "sense" nucleic acid molecule encoding a protein, for example complementary to the coding strand of a double-stranded cDNA molecule or complementary to an mRNA sequence. The term antisense molecule should also encompass RNA interference molecules specifically also RNAi hairpin molecules with or without spacer or linker sequences between the complementary sequences.

Consequently, an antisense nucleic acid molecule is capable of forming hydrogen bonds with a sense nucleic acid molecule. The antisense nucleic acid molecule may be complementary to any of the coding strands depicted here or only to a part thereof. An antisense oligonucleotide may, for example, be 5, 10, 15, 20, 25, 30, 35, 40, 45 or 50, nucleotides in length. An antisense nucleic acid molecule may be prepared by chemical synthesis and enzymic ligation according to methods known to the skilled worker. An antisense nucleic acid molecule may be chemically synthesized using naturally occurring nucleotides or nucleotides modified in various ways so as to increase the biological stability of the molecules or to enhance the physical stability of the duplex forming between the antisense nucleic acid and the sense nucleic acid; it is possible to use, for example, phosphorothioate derivatives and acridine-substituted nucleotides. Alternatively, it is possible to prepare antisense nucleic acid molecules biologically by using expression vectors into which polynucleotides have been cloned whose orientation is antisense. The antisense nucleic acid molecule may also be an "α-anomeric" nucleic acid molecule. An "α-anomeric" nucleic acid molecule forms specific double-stranded hybrids with complementary RNAs, in which the strands run parallel to one another, in contrast to ordinary β-units. The antisense nucleic acid molecule may comprise 2-0-methylribonucleotides or chimeric RNA-DNA analogs. The antisense nucleic acid molecule may also be a ribozyme. Ribozymes are catalytic RNA molecules having a ribonuclease activity and are capable of cleaving single-stranded nucleic acids to which they have a complementary region, such as mRNA, for example.

In another disclosure, the description discloses the polypeptide encoded by the polynucleotide disclosed herein and a polyclonal or monoclonal antibody, for example a monoclonal antibody, directed against said polypeptide.

"Antibodies" mean, for example, polyclonal, monoclonal, human or humanized or recombinant antibodies or fragments thereof, single-chain antibodies or else synthetic antibodies. Antibodies disclosed herein or fragments thereof mean in principle all the immunoglobulin classes such as IgM, IgG, IgD, IgE, IgA or their subclasses such as the IgG subclasses, or mixtures thereof. Suitable are IgG and its subclasses such as, for example, IgG1, IgG2, IgG2a, IgG2b, IgG3 and IgGM. Particularly suitableare the IgG subtypes IgG1 and IgG2b. Fragments which may be mentioned are any truncated or modified antibody fragments having one or two binding sites complementary to the antigen, such as antibody moieties having a binding site which corresponds to the antibody and is composed of a light chain and a heavy chain, such as Fv, Fab or F(ab')2 fragments or single-strand fragments. Suitable are truncated double-strand fragments such as Fv, Fab or F(ab')2. These fragments may be obtained, for example, either enzymatically, by cleaving off the Fc moiety of the antibodies using enzymes such as papain or pepsin, by means of chemical oxidation or by means of genetic manipulation of the antibody genes. Genetically manipulated nontruncated fragments may also be advantageously used. The antibodies or fragments may be used alone or in mixtures. Antibodies may also be part of a fusion protein.

In other disclosures, the present description discloses a method for preparing a vector, which comprises inserting the polynucleotide disclosed herein or the expression cassette into a vector, and to a vector comprising the polynucleotide disclosed herein.

Preferably the polynucleotide is functionally linked to regulatory sequences which allow expression in a prokaryotic or eukaryotic host.

The term "vector", as used herein, refers to a nucleic acid molecule capable of transporting another nucleic acid to which it is bound. An example of a type of vector is a "plasmid", i.e. a circular double-stranded DNA loop. Another type of vector is a viral vector, it being possible here to ligate additional DNA segments into the viral genome. Particular vectors such as, for example, vectors having an origin of replication may replicate autonomously in a host cell into which they have been introduced. Other preferred vectors are advantageously integrated into the genome of a host cell into which they have been introduced and thereby are replicated together with the host genome. Moreover, particular vectors can control expression of genes to which they are functionally linked. These vectors are referred to herein as "expression vectors". As mentioned above, they may replicate autonomously or be integrated into the host genome. Expression vectors suitable for DNA recombination techniques are usually in the form of plasmids. "Plasmid" and "vector" may be used synonymously in the present description. Consequently, the invention also comprises phages, viruses, for example SV40, CMV or TMV, transposons, IS elements, phasmids, phagemids, cosmids, linear or circular DNA and other expression vectors known to the skilled worker.

The recombinant expression vectors used advantageously in the method comprise the nucleic acids disclosed herein or the expression vector of the invention in a form suitable for expression of the nucleic acids used in a host cell, meaning that the recombinant expression vectors comprise one or more regulatory sequences which are selected on the basis of the host cells to be used for expression and which is functionally linked to the nucleic acid sequence to be expressed.

In a recombinant expression vector, "functionally linked" means that the nucleotide sequence of interest is bound to the regulatory sequence(s) in such a way that expression of said nucleotide sequence is possible and that they are bound to one another so that both sequences fulfil the predicted function attributed to the sequence (e.g. in an in-vitro transcription/translation system or in a host cell when introducing the vector into said host cell).

The recombinant expression vectors used may be designed especially for expression in plants. For example, genes encoding SEQ ID NO: 1, may be expressed in bacterial cells, insect cells, e.g. by using baculovirus expression vectors, yeast cells and other fungal cells [e.g. according to Romanos, (1992), Yeast 8:423-488; van den Hondel, C.A.M.J.J., (1991), in J.W. Bennet & L.L. Lasure, eds, pp. 396-428: Academic Press: San Diego; and van den Hondel, C.A.M.J.J., (1991) in: Applied Molecular Genetics of Fungi, Peberdy, J.F, ed., pp. 1-28, Cambridge University Press: Cambridge, in algae, e.g. according to Falciatore, 1999, Marine Biotechnology. 1 , 3:239-251, in ciliates, e.g. in Holotrichia, Peritrichia, Spirotrichia, Suctoria, Tetrahymena, Paramecium, Colpidium, Glaucoma, Platyophrya, Potomacus, Desaturaseudocohnilembus, Euplotes, Engelmaniella, Stylonychia, or in the genus Stylonychia lemnae, using vectors according to a transformation method as described in WO 98/01572, and preferably in cells of multicellular plants [see Schmidt, R., (1988) Plant Cell Rep.: 583-586; Plant Molecular Biology and Biotechnology, C Press, Boca Raton, Florida, chapter 6/7, pp. 71-119 (1993); F.F. White, B. Jenes, Transgenic Plants, vol. 1, Engineering and Utilization, eds: Kung and R. Wu, Academic Press (1993), 128-43; Potrykus, Annu. Rev. Plant Physiol. Plant Molec. Biol. 42 (1991), 205-225, and the references in the documents mentioned here. Suitable host cells are also discussed in Goeddel, Gene Expression Technology: Methods in Enzymology 185, Academic Press, San Diego, CA (1990). Alternatively, the recombinant expression vector may be transcribed and translated in vitro using, for example, T7-promoter regulatory sequences and T7 polymerase.

A plant expression cassette or a corresponding vector preferably comprises regulatory sequences which are capable of controlling gene expression in plant cells and are functionally linked to the ORF so that each sequence its function.

The expression cassette is preferably linked to a suitable promoter which carries out gene expression at the right time and in a cell- or tissue-specific manner. Consequently, advantageous regulatory sequences for the novel method are present in the plant promoters CaMV/35S [Franck, Cell 21 (1980) 285-294, US 5,352,605], PRP1 [Ward, Plant. Mol. Biol. 22 (1993)], SSU, PGEL1, OCS [Leisner, (1988) Proc Natl Acad Sci USA 85:2553], lib4, usp, mas [Comai (1990) Plant Mol Biol 15:373], STLS1, ScBV [Schenk(1999) Plant Mol Biol 39:1221, B33, SAD1 and SAD2 (flax promoter, [Jain, (1999) Crop Science, 39:1696) and nos [Shaw (1984) Nucleic Acids Res. 12:7831]. The various ubiquitin promoters of Arabidopsis [Callis (1990) J Biol Chem 265:12486; Holtorf (1995) Plant Mol Biol 29:637], Pinus, maize [(Ubi1 and Ubi2), US 5,510,474; US 6,020,190 and US 6,054,574] or parsley [Kawalleck (1993) Plant Molecular Biology, 21:673] or phaseolin promoters may be used advantageously. Inducible promoters such as the promoters described in EP-A-0 388 186 (benzylsulfonamide-inducible), Gatz, (1992) Plant J. 2:397 (tetracycline-inducible), EP-A-0 335 528 (abscisic acid-inducible) or WO 93/21334 (ethanol- or cyclohexanol-inducible) are likewise advantageous in this connection. Further suitable plant promoters are the promoter of cytosolic FBPase or the potato ST-LSI promoter (Stockhaus, 1989, EMBO J. 8, 2445), the Glycine max phosphoribosyl-pyrophosphate amidotransferase promoter (GenBank accession NO U87999) or the node-specific promoter described in EP-A-0 249 676. Promoters which make expression possible in specific tissues or show a preferential expression in certain tissues may also be suitable. Also advantageous are seed-specific promoters such as the USP promoter but also other promoters such as the LeB4, DC3, SAD1, phaseolin or napin promoter. Leaf-specific promoters as described in DE-A 19644478 or light-regulated promoters such as, for example, the petE promoter are also available for expression of genes in plants. Further advantageous promoters are seed-specific promoters which may be used for monocotyledonous or dicotyledonous plants and are described in US 5,608,152 (oil seed rape napin promoter), WO 98/45461 (Arabidopsis oleosin promoter), US 5,504,200 (Phaseolus vulgaris phaseolin promoter), WO 91/13980 (Brassica Bce4 promoter) and von Baeumlein, 1992, Plant J., 2:233 (Legume LeB4 promoter), these promoters being suitable for dicotyledons. Examples of promoters suitable for monocotyledons are the following: barley lpt-2- or lpt-1 promoter (WO 95/15389 and WO 95/23230), barley hordein promoter, the corn ubiquitin promoter and other suitable promoters described in WO 99/16890.

In order to express heterologous sequences strongly in as many tissues as possible, in particular also in leaves, preference is given to using, in addition to various of the abovementioned and promoters, plant promoters of actin or ubiquitin genes, such as, for example, the rice actin1 promoter. Another example of constitutive plant promoters are the sugar beet V-ATPase promoters (WO 01/14572).

It is possible in principle to use all natural promoters with their regulatory sequences, such as those mentioned above, for the novel method. It is likewise possible and advantageous to use synthetic promoters additionally or alone, particularly if they mediate constitutive expression. Examples of synthetic constitutive promoters are the Super promoter (WO 95/14098) and promoters derived from G boxes (WO 94/12015).

Plant genes can also be expressed via a chemically inducible promoter (see a review in Gatz 1997, Annu. Rev. Plant Physiol. Plant Mol. Biol., 48:89-108). Chemically inducible promoters are particularly suitable when it is desired to express genes in a time-specific manner. Examples of such promoters are a salicylic acid-inducible promoter (WO 95/19443), a tetracycline-inducible promoter (Gatz et al. (1992) Plant J. 2, 397-404), an ethanol-inducible promoter and EP-A 0 388 186, EP-A 0 335 528, WO 97/06268. Expression specifically in gymnosperms or angiosperms are also possible in principle.

Promoters responding to biotic or abiotic stress conditions are also suitable promoters, for example in plants the pathogen-induced PRP1 gene promoter (Ward, Plant. Mol. Biol. 22 (1993) 361), the tomato heat-inducible hsp80 promoter (US 5,187,267), the potato cold-inducible alpha-amylase promoter (WO 96/12814) or the wound-inducible pinll promoter (EP-A-0 375 091).

Preferred polyadenylation signals are sufficiently known to the skilled worker, for example for plants those derived from Agrobacterium tumefaciens t-DNA, such as gene 3, known as octopine synthase (ocs gene) of the Ti plasmid pTiACH5 (Gielen, EM-BO J. 3 (1984) 835), the nos gene or functional equivalents thereof. Other known terminators which are functionally active in plants are also suitable.

Further regulatory sequences which are expedient where appropriate also include sequences which control transport and/or location of the expression products (targeting). In this connection, mention should be made particularly of the signal peptide- or transit peptide-encoding sequences known per se. For example, it is possible with the aid of plastid transit peptide-encoding sequences to guide the expression product into the plastids of a plant cell. Consequently, preference is given to using for functional linkage in plant gene expression cassettes in particular targeting sequences which are required for guiding the gene product to its appropriate cell compartment (see a review in Kermode, Crit. Rev. Plant Sci. 15, 4 (1996) 285 and references therein), for example into the vacuole, the nucleus, any kind of plastids such as amyloplasts, chloroplasts, chromoplasts, the extracellular space, the mitochondria, the endoplasmic reticulum, oil bodies, peroxisomes and other compartments of plant cells. Thus, in particular peroxisome-targeting signals have been described, for example in Olsen LJ, Plant Mol Biol 1998, 38:163-189).

Accordingly the gene construct, the vector, the expression cassette, etc. are advantageously constructed in such a way that a promoter is followed by a suitable cleavage site for insertion of the nucleic acid to be expressed, for example in a polylinker, and a terminator is then located, where appropriate, downstream of the polylinker or the insert. This sequence may be repeated several times, for example three, four or five times, so that multiple genes are combined in one construct and can be introduced in this way into the transgenic plant for expression. Advantageously, each nucleic acid sequence has its own promoter and, where appropriate, its own terminator. In the case of microorganisms capable of processing a polycistronic RNA, it is also possible to insert a plurality of nucleic acid sequences downstream of a promoter and, where appropriate, upstream of a terminator. It is advantageously possible to use in the expression cassette different promoters. A different terminator sequence may be used advantageously for each gene.

The plant expression cassette preferably contains further functionally linked sequences such as translation enhancers, for example the overdrive sequence comprising the 5'untranslated leader sequence of tobacco mosaic virus, which increases the protein/RNA ratio (Gallie, 1987, Nucl. Acids Research 15:8693).

The vectors, cassettes, nucleic acid molecules, etc. to be introduced can be introduced into prokaryotic or eukaryotic cells via conventional transformation or transfection techniques.

The terms "transformation" and "transfection", conjugation and transduction, as used herein, are intended to include a multiplicity of methods known in the prior art for introducing foreign nucleic acid (e.g. DNA) into a host cell, including calcium phosphate or calcium chloride coprecipitation, DEAE-dextran-mediated transfection, lipofection, natural competence, chemically mediated transfer, electroporation or particle bombardment. Methods suitable for transforming or transfecting host cells, including plant cells, can be found in Sambrook et al. (Molecular Cloning: A Laboratory Manual., 2nd edition, Cold Spring Harbor Laboratory, Cold Spring Harbor Laboratory Press, Cold Spring Harbor, NY, 1989) and other laboratory manuals such as Methods in Molecular Biology, 1995, vol. 44, Agrobacterium protocols, eds: Gartland and Davey, Humana Press, Totowa, New Jersey.

Thus it is possible for the nucleic acids, gene constructs, expression cassettes, vectors, etc. used in the method to be integrated either in the plastidial genome or preferably in the nuclear genome of the host cell, after introduction into a plant cell or plant. Integration into the genome may be random or may be carried out via recombination in such a way that the introduced copy replaces the native gene, thereby modulating production of the desired compound by the cell, or by using a gene in trans so that said gene is functionally linked to a functional expression unit which comprises at least one sequence guaranteeing expression of a gene and at least one sequence guaranteeing polyadenylation of a functionally transcribed gene. Where appropriate, the nucleic acids are transferred into the plants via multiexpression cassettes or constructs for multiparallel expression of genes. The nucleic acid sequence may be introduced into the plant without further, different nucleic acid sequences.

As described above, the transfer of foreign genes into the genome of a plant is referred to as transformation. In this case, the methods described for transformation and regeneration of plants from plant tissues or plant cells are utilized for transient or stable transformation. Suitable methods are protoplast transformation by polyethylene glycolinduced DNA uptake, the biolistic method using the gene gun - the "particle bombardment" method, electroporation, incubation of dry embryos in DNA-containing solution, microinjection and Agrobacterium-mediated gene transfer. Said methods are described, for example, in B. Jenes, Techniques for Gene Transfer, in: Transgenic Plants, Vol. 1, Engineering and Utilization, edited by S.D. Kung and R. Wu, Academic Press (1993) 128-143 and in Potrykus Annu. Rev. Plant Physiol. Plant Molec. Biol. 42 (1991) 205-225).

The construct to be expressed is preferably cloned into a vector which is suitable for transforming *Agrobacterium tumefaciens,* for example as described herein, for example pBin19 (Bevan, Nucl. Acids Res. 12 (1984) 8711). Agrobacteria transformed with such a vector may then be used in the known manner for transforming plants, in particular crop plants, such as, for example, tobacco plants, by, for example, bathing wounded leaves or pieces of leaf in a solution of agrobacteria and then cultivating said leaves or pieces of leaf in suitable media. The transformation of plants with Agrobacterium tumefaciens is described, for example, by Höfgen, Nucl. Acid Res. (1988) 16, 9877 or is disclosed, inter alia, in F.F. White, Vectors for Gene Transfer in Higher Plants; in Transgenic Plants, Vol. 1, Engineering and Utilization, edited by S.D. Kung and R. Wu, Academic Press, 1993, pp. 15-38.

The nucleic acids, gene constructs, expression cassettes, vectors, etc. used in the method are checked, where appropriate, and then used for transforming the plants. For this purpose, it may be required first to obtain the constructs, plasmids, vectors, etc. from an intermediate host. For example, the constructs can be isolated as plasmids from bacterial hosts, following a conventional plasmid isolation. Numerous methods for transforming plants are known. Since stable integration of heterologous DNA into the genome of plants is advantageous according to the invention, T-DNA-mediated transformation, in particular, has proved to be expedient and may be carried out in a manner known per se. For example, the plasmid construct generated according to what has been said above may be transformed into competent agrobacteria by means of electroporation or heat shock. In principle, the distinction to be made here is between the formation of cointegrated vectors on the one hand and the transformation with binary vectors. In the first alternative, the vector constructs comprising the codogenic gene section do not contain any T-DNA sequences, rather the cointegrated vectors are formed in the agrobacteria by homologous recombination of the vector construct with T-DNA. T-DNA is present in agrobacteria in the form of Ti or Ri plasmids in which the oncogenes have conveniently been replaced by exogenous DNA. When using binary vectors, these may be transferred by means of bacterial conjugation or direct transfer to agrobacteria. Said agrobacteria conveniently already comprise the vector carrying the vir genes (frequently referred to as helper Ti(Ri) plasmid). Expediently, one or more markers may be used, on the basis of which the selection of transformed agrobacteria and transformed plant cells is possible. A multiplicity of markers is known to the skilled worker.

It is known about stable or transient integration of nucleic acids that, depending on the expression vector used and transfection technique used, only a small proportion of the cells takes up the foreign DNA and, if desired, integrates it in their genome. For identification and selection of these integrants, usually a gene which encodes a selectable marker (e.g. antibiotic resistance) is introduced together with the gene of interest into the host cells.

Marker genes are advantageously used for selection for successful introduction of the nucleic acids disclosed herein into a host organism, in particular into a plant. These marker genes make it possible to identify successful introduction of the nucleic acids disclosed herein by a number of different principles, for example by visual recognition with the aid of fluorescence, luminescence or in the wavelength range of light which is visible to humans, via a herbicide or antibiotic resistance, via "nutritional" (auxotrophic) markers or antinutritional markers, by enzyme assays or via phytohormones. Examples of such markers which may be mentioned here are GFP (= Green fluorescent Protein); the luciferin/luciferase system; β-galactosidase with its colored substrates, e.g. X-Gal; herbicide resistances to, for example, imidazolinone, glyphosate, phosphothricin or sulfonylurea; antibiotic resistances to, for example, bleomycin, hygromycin, streptomycin, kanamycin, tetracycline, chloramphenicol, ampicillin, gentamycin, geneticin (G418), spectinomycin or blasticidin, to mention only a few; nutritional markers such as utilization of mannose or xylose or antinutritional markers such as 2-deoxyglucose resistance. This list represents a small section of possible markers. Markers of this kind are well known to the skilled worker.

Different markers are preferred, depending on organism and selection method. Preferred selectable markers include in plants those which confer resistance to a herbicide such as glyphosphate or glufosinate. Further suitable markers are, for example, markers which encode genes which are involved in biosynthetic pathways of, for example, sugars or amino acids, such as β-galactosidase, ura3 or ilv2. Markers encoding genes such as luciferase, gfp or other fluorescence genes are likewise suitable. These markers can be used in mutants in which said genes are not functional because, for example, they have been deleted by means of conventional methods. Furthermore, markers may be introduced into a host cell on the same vector as that coding for SEQ ID NO: 2, polypeptides or another of the disclosed nucleic acid molecules described herein, or they may be introduced on a separate vector.

Since the marker genes, especially the antibiotic and herbicide resistance gene, are normally no longer required or are unwanted in the transgenic host cell after successful introduction of the nucleic acids, techniques making it possible to delete these marker genes are advantageously used in the method of the invention for introducing the nucleic acids. One such method is "cotransformation". Cotransformation involves using simultaneously two vectors for transformation, one vector harboring the nucleic acids disclosed herein and the second one harboring the marker gene(s). A large proportion of the transformants acquires or contains both vectors in the case of plants (up to 40% of the transformants and more). It is then possible to remove the marker genes from the transformed plant by crossing. A further method uses marker genes integrated into a transposon for the transformation together with the desired nucleic acids ("Ac/Ds technology). In some cases (approx. 10%), after successful transformation, the transposon jumps out of the genome of the host cell and is lost. In a further number of cases, the transposon jumps into another site. In these cases, it is necessary to outcross the marker gene again. Microbiological techniques enabling or facilitating detection of such events have been developed. A further advantageous method uses "recombination systems" which have the advantage that it is possible to dispense with outcrossing. The best-known system of this kind is the "Cre/lox" system. Cre1 is a recombinase which deletes the sequences located between the loxP sequence. If the marker gene is integrated between the loxP sequence, it is deleted by means of Cre1 recombinase after successful transformation. Further recombinase systems are the HIN/HIX, FLP/FRT and the REP/STB system (Tribble et al., J.Biol. Chem., 275, 2000: 22255 - 22267; Velmurugan et al., J. Cell Biol., 149, 2000: 553 - 566). Targeted integration of the nucleic acid sequences disclosed herein into the plant genome is also possible in principle, but less preferred up until now because of the large amount of work involved. These methods are, of course, also applicable to microorganisms such as yeasts, fungi or bacteria.

Agrobacteria transformed with an expression vector of the invention may likewise be used in a known manner for transforming plants such as test plants such as Arabidopsis or crop plants such as, for example, cereals, corn, oats, rye, barley, wheat, soybean, rice, cotton, sugar beet, canola, sunflower, flax, hemp, potato, tobacco, tomato, carrot, paprika, oilseed rape, tapioca, cassava, arrowroot, tagetes, alfalfa, lettuce and the various tree, nut and grape species, oil-containing crop plants such as soybean, peanut, castor oil plant, sunflower, corn, cotton, flax, oilseed rape, coconut, oil palm, safflower (Carthamus tinctorius) or cocoa bean or the other plants mentioned below, for example by bathing wounded leaves or pieces of leaf in a solution of agrobacteria and then cultivating said leaves or pieces of leaf in suitable media.

The genetically modified plant cells may be regenerated by any methods known to the skilled worker. Appropriate methods can be found in the abovementioned publications by S.D. Kung and R. Wu, Potrykus or Höfgen and Willmitzer.

If desired, the plasmid constructs may be checked again with regard to identity and/or integrity by means of PCR or Southern blot analysis, prior to their transformation into agrobacteria. It is normally desired that the codogenic gene sections with the linked regulatory sequences in the plasmid constructs are flanked on one or both sides by T-DNA. This is particularly useful when bacteria of the species Agrobacterium tumefaciens or Agrobacterium rhizogenes are used for transformation. The transformed agrobacteria may be cultured in a manner known per se and are thus available for convenient transformation of the plants. The plants or parts of plants to be transformed are grown and provided in a conventional manner. The agrobacteria may act on the plants or parts of plants in different ways. Thus it is possible, for example, to use a culture of morphogenic plant cells or tissues. Following T-DNA transfer, the bacteria are usually eliminated by antibiotics and regeneration of plant tissue is induced. For this purpose, particular use is made of suitable plant hormones in order to promote the formation of shoots, after initial callus formation. Preference is given to carrying out in planta transformation. For this purpose, it is possible to expose plant seeds, for example, to the agrobacteria or to inoculate plant meristems with agrobacteria. It has proved particularly expedient to expose the whole plant or at least the flower primordia to a suspension of transformed agrobacteria. The former is then grown further until seeds of the treated plant are obtained (Clough and Bent, Plant J. (1998) 16, 735). To select transformed plants, the plant material obtained from the transformation is usually subjected to selective conditions so that transformed plants can be distinguished from untransformed plants. For example, the seeds obtained in the manner described above can be sown anew and, after growing, subjected to a suitable spray selection. Another possibility is to grow the seeds, if necessary after sterilization, on agar plates, using a suitable selecting agent, in such a way that only the transformed seeds are able to grow to plants.

The invention furthermore relates to a plant cell which has been stably or transiently transformed or transfected with the vector of the invention or with the polynucleotide as defined by the claims.

In another embodiment, the invention also relates to a plant cell which contains the polynucleotide as defined by the claims or the vector of the invention. In a preferred embodiment, the invention relates in particular to a plant tissue or to a plant having an increased amount of SEQ ID NO: 2, and/or containing the plant cell of the invention. In one embodiment, the invention also relates to a plant compartment, a plant organelle, a plant cell, a plant tissue or a plant having an increased SEQ ID NO: 2, activity or an increased amount of SEQ ID NO: 2, polypeptide.

Host cells which are suitable in principle for taking up the nucleic acid disclosed herein, the gene product disclosed herein or the vector of the invention are cells of any prokaryotic or eukaryotic organisms. Organisms or host organisms suitable for the nucleic acid disclosed herein, the expression cassette or the vector are in principle any organisms for which faster growth and higher yield are desirable, with preference being given, as mentioned, to crop plants.

Further disclosed are transgenic organisms transformed with the expression cassette or vector of the invention and to cells, cell cultures, tissues, parts or propagation material derived from such organisms.

The terms "host organism", "host cell", "recombinant (host) organism", "recombinant (host) cell", "transgenic (host) organism" and "transgenic (host) cell" are used interchangeably herein. These terms relate, of course, not only to the particular host organism or to the particular target cell but also to the progeny or potential progeny of said organisms or cells. Since certain modifications may occur in subsequent generations, owing to mutation or environmental effects, these progeny are not necessarily identical to the parental cell but are still included within the scope of the term as used herein.

Examples which should be mentioned here are microorganisms such as fungi, for example the genus Mortierella, Saprolegnia or Pythium, bacteria such as, for example, the genus Escherichia, yeasts such as, for example, the genus Saccharomyces, cyanobacteria, ciliates, algae or protozoa such as, for example, dinoflagellates such as Crypthecodinium.

The increased growth rate of the microorganisms is particularly advantageous in combination with the synthesis of products of value, for example in the method disclosed herein for preparing fine chemicals. An advantageous disclosure is thus, for example, microorganisms which (naturally) synthesize relatively large amounts of vitamins, sugars, polymers, oils, etc. Examples which may be mentioned here are fungi such as, for example, Mortierella alpina, Pythium insidiosum, yeasts such as, for example, Saccharomyces cerevisiae and the microorganisms of the genus Saccharomyces, cyanobacteria, ciliates, algae or protozoa such as, for example, dinoflagellates such as Crypthecodinium.

Utilizable host cells are furthermore mentioned in: Goeddel, Gene Expression Technology: Methods in Enzymology 185, Academic Press, San Diego, CA (1990). Usable expression strains, for example those having relatively low protease activity, are described in: Gottesman, S., Gene Expression Technology: Methods in Enzymology 185, Academic Press, San Diego, California (1990) 119-128.

Proteins are usually expressed in prokaryotes by using vectors which contain constitutive or inducible promoters controlling expression of fusion or nonfusion proteins. Typical fusion expression vectors are, inter alia, pGEX (Pharmacia Biotech Inc; Smith, D.B., and Johnson, K.S. (1988) Gene 67:31-40), pMAL (New England Biolabs, Beverly, MA) and pRIT5 (Pharmacia, Piscataway, NJ). Examples of suitable inducible nonfusion *E. coli* expression vectors are inter alia, pTrc (Amann et al. (1988) Gene 69:301-315) and pET 11 d [Studier, Gene Expression Technology: Methods in Enzymology 185, Academic Press, San Diego, California (1990) 60].

Other vectors suitable in prokaryotic organisms are known to the skilled worker and are, for example, in E. coli pLG338, pACYC184, the pBR series such as pBR322, the pUC series such as pUC18 or pUC19, the M113mp series, pKC30, pRep4, pHS1, pHS2, pPLc236, pMBL24, pLG200, pUR290, pIN-III¹¹³-B1, lambda gt11 or pBdCl, in Streptomyces pIJ101, pIJ364, pIJ702 or pIJ361, in Bacillus pUB110, pC194 or pBD214, in Corynebacterium pSA77 or pAJ667.

However, preference is given to eukaryotic expression systems. In a further disclosure, the expression vector is a yeast expression vector. Examples of vectors for expression in the yeast *S*. *cerevisiae* include pYeDesaturasec1 (Baldari (1987) Embo J. 6:229), pMFa (Kurjan (1982) Cell 30:933), pJRY88 (Schultz (1987) Gene 54:113), 2 micron, pAG-1, YEp6, YEp13, pEMBLYe23 and pYES2 (Invitrogen Corporation, San Diego, CA). Vectors and methods for constructing vectors suitable for use in other fungi such as filamentous fungi include those described in detail in: van den Hondel, C.A.M.J.J. (1991) in: Applied Molecular Genetics of fungi, J.F. Peberdy, ed., pp. 1-28, Cambridge University Press: Cambridge; or in: J.W. Bennet, ed., p. 396: Academic Press: San Diego]. Examples of vectors in fungi are pALS1, pIL2 or pBB116 or in plants pLGV23, pGHlac⁺, pBIN19, pAK2004 or pDH51.

Alternatively, a product of value, for example the fine chemicals mentioned, may be expressed in insect cells using baculovirus expression vectors. Baculovirus vectors available for expression of proteins in cultured insect cells (e.g. Sf9 cells) include the pAc series (Smith (1983) Mol. Cell Biol.. 3:2156) and the pVL series (Lucklow (1989) Virology 170:31).

The abovementioned vectors offer only a small overview over possible suitable vectors. Further plasmids are known to the skilled worker and are described, for example, in: Cloning Vectors (eds Pouwels, P.H., et al., Elsevier, Amsterdam-New York-Oxford, 1985, ISBN 0 444 904018). For further expression systems suitable for prokaryotic and eukaryotic cells, see in chapters 16 and 17 of Sambrook, Molecular Cloning: A Laboratory Manual, 2nd edition, Cold Spring Harbor Laboratory Press, Cold Spring Harbor, NY, 1989 or Current Protocols in Molecular Biology, John Wiley & Sons, N. Y. (1989).

The microorganism has for example been transiently or stably transformed with a polynucleotide which comprises a nucleic acid molecule described above which is suitable for the method as disclosed herein.

In another advantageous disclosure disclosed herein, it is possible to express, for example, a product of value or the fine chemicals also in unicellular plant cells (such as algae), see Falciatore, 1999, Marine Biotechnology 1 (3): 239 and references therein, and in plant cells of higher plants (e.g. spermatophytes such as crops) so that said plants have higher SEQ ID NO: 2, activity and, consequently, a higher growth rate. Examples of plant expression vectors include those described in detail above or those from Becker, (1992), Plant Mol. Biol. 20:1195 and Bevan, (1984), Nucl. Acids Res. 12:8711; Vectors for Gene Transfer in Higher Plants; in: Transgenic Plants, vol. 1, Engineering and Utilization, eds: Kung and R. Wu, Academic Press, 1993, p. 15. A relatively recent review of Agrobacterium binary vectors can be found in Hellens, 2000, Trends in Plant Science, Vol. 5, 446.

Host organisms which are advantageously used are bacteria, fungi, yeasts or plants, preferably crop plants or parts thereof. Preference is given to using plants, particularly preferably plants, and special mention may be made of agricultural useful plants such as cereals and grasses, e.g. Triticum spp., Zea mais, Hordeum vulgare, oats, Secale cereale, Oryza sativa, Pennisetum glaucum, Sorghum bicolor, Triticale, Agrostis spp., Cenchrus ciliaris, Dactylis glomerata, Festuca arundinacea, Lolium spp., Medicago spp., Alfalfa and Saccharum spp., legumes and oil seed crops, e.g. Brassica juncea, Brassica napus, Brassica nigra, Sinapes alba, Glycine max, Arachis hypogaea, canola, castor oil plant, coconut, oil palm, cocoa bean, date palm, Gossypium hirsutum, Cicer arietinum, Helianthus annuus, Lens culinaris, Linum usitatissimum, Sinapis alba, Trifolium repens, Carthamus tinctorius and Vicia narbonensis, hemp, vegetables, lettuce and fruits, e.g. bananas, grapes, Lycopersicon esculentum, asparagus, cabbage, watermelons, kiwis, Solanum tuberosum, Solanum lypersicum, carrots, paprika, tapioca, manioc, Beta vulgaris, cassava and chicory, arrowroot, nut and grape species, trees, e.g. Coffea species, Citrus spp., Eucalyptus spp., Picea spp., Pinus spp. and Populus spp., tobacco, medicinal plants and trees and flowers, e.g. Tagetes.

If plants are selected as donor organism, said plant may in principle have any phylogenetic relationship to the receptor plant. Thus donor plant and receptor plant may belong to the same family, genus, species, variety or line, which results in increasing homology between the nucleic acids to be integrated and corresponding parts of the genome of the receptor plant.

The donor organism may be a fungi, preferably Saccharomycetaceae, in particular the genus Saccharomyces particularly preferred Saccharomyces cerevisiae.

Preferred receptor plants are particularly plants which can be appropriately transformed. These include mono- and dicotyledonous plants. In particular mention should be made of the agricultural useful plants such as cereals and grasses, e.g. Triticum spp., Zea mais, Hordeum vulgare, oats, Secale cereale, Oryza sativa, Pennisetum glaucum, Sorghum bicolor, Triticale, Agrostis spp., Cenchrus ciliaris, Dactylis glomerata, Festuca arundinacea, Lolium spp., Medicago spp. and Saccharum spp., legumes and oil seed crops, e.g. Brassica juncea, Brassica napus, Glycine max, Arachis hypogaea, Gossypium hirsutum, Cicer arietinum, Helianthus annuus, Lens culinaris, Linum usitatissimum, Sinapis alba, Trifolium repens und Vicia narbonensis, vegetables and fruits, e.g. bananas, grapes, Lycopersicon esculentum, asparagus, cabbage, water-melons, kiwis, Solanum tuberosum, Beta vulgaris, cassava and chicory, trees, e.g. Coffea species, Citrus spp., Eucalyptus spp., Picea spp., Pinus spp. and Populus spp., medicinal plants and trees, and flowers. According to a particular embodiment, the present invention relates to transgenic plants of the genus Arabidopsis, e.g. Arabidopsis thaliana and of the genus Oryza.

After transformation, plants are first regenerated as described above and then cultivated and grown as usual.

The plant compartments, plant organelles, plant cells, plant tissues or plants of the invention is preferably produced according to the method of the invention or contains the gene construct described herein or the described vector as defined by the claims.

The present invention also relates to transgenic plant material derivable from an inventive population of transgenic plants. Said material includes plant cells and certain tissues, organs and parts of plants in any phenotypic forms thereof, such as seeds, leaves, anthers, fibers, roots, root hairs, stalks, embryos, kalli, cotyledons, petioles, harvested material, plant tissue, reproductive tissue and cell cultures, which has been derived from the actual transgenic plant and/or may be used for producing the transgenic plant.

Preference is given to any plant parts or plant organs such as leaf, stem, shoot, flower, root, tubers, fruits, bark, wood, seeds, etc. or the entire plant. Seeds include in this connection all seed parts such as seed covers, epidermal and seed cells, endosperm or embryonic tissue. Particular preference is given to harvested products, in particular fruits, seeds, tubers, fruits, roots, bark or leaves or parts thereof.
In the method of the invention, transgenic plants also mean plant cells, plant tissues or plant organs to be regarded as agricultural product.

The biomaterial produced in the method, in particular of plants which have been modified by the method of the invention, may be marketed directly.

The invention likewise relates in one embodiment to propagation material of a plant prepared according to the method of the invention. Propagation material means any material which may serve for seeding or growing plants, even if it may have, for example, another function, e.g. as food.

"Growth" also means, for example, culturing the transgenic plant cells, plant tissues or plant organs on a nutrient medium or the whole plant on or in a substrate, for example in hydroculture or on a field.

The present description also discloses the use of the polynucleotide used in the method of the invention and characterized herein, of the gene construct, of the vector, of the plant cell or of the plant or of the plant tissue or of the plant material for preparing a plant with increased yield.

In one disclosure, the description discloses the use of an SEQ ID NO: 2, polypeptide or of the polynucleotide or polypeptide disclosed herein for increasing the yield and/or increasing growth of a nonhuman organism compared to a starting organism.

A further disclosure disclosed herein is the use of the products obtained by means of said methods, for example biomaterial, in particular plant materials as mentioned, in food products, animal feed products, nutrients, cosmetics or pharmaceuticals. It is also possible to isolate commercially utilizable substances such as fine chemicals from the plants or parts of plants obtained by means of the method disclosed herein.

The examples and figures below which should not be regarded as limiting further illustrate the present invention.

In a further disclosure, the present description discloses a method for the generation of a microorganism, comprising the introduction, into the microorganism or parts thereof, of the expression construct of the invention, or the vector of the invention or the polynucleotide disclosed herein.

The fine chemicals obtained in the method are suitable as starting material for the synthesis of further products of value. For example, they can be used in combination with each other or alone for the production of pharmaceuticals, foodstuffs, animal feeds or cosmetics. Accordingly, the present description discloses a method for the production of a pharmaceuticals, food stuff, animal feeds, nutrients or cosmetics comprising the steps of the method as disclosed herein, including the isolation of the fine chemicals, in particular amino acid composition produced e.g. methionine produced if desired and formulating the product with a pharmaceutical acceptable carrier or formulating the product in a form acceptable for an application in agriculture. A further disclosure is the use of the fine chemicals produced in the method or of the transgenic organisms in animal feeds, foodstuffs, medicines, food supplements, cosmetics or pharmaceuticals.

It is advantageous to use in the method disclosed herein transgenic microorganisms such as fungi such as the genus Claviceps or Aspergillus or Gram-positive bacteria such as the genera Bacillus, Corynebacterium, Micrococcus, Brevibacterium, Rhodococcus, Nocardia, Caseobacter or Arthrobacter or Gram-negative bacteria such as the genera Escherichia, Flavobacterium or Salmonella or yeasts such as the genera Rhodotorula, Hansenula or Candida. Particularly advantageous organisms are selected from the group of genera Corynebacterium, Brevibacterium, Escherichia, Bacillus, Rhodotorula, Hansenula, Candida, Claviceps or Flavobacterium. It is very particularly advantageous to use in the method disclosed herein microorganisms selected from the group of genera and species consisting of Hansenula anomala, Candida utilis, Claviceps purpurea, Bacillus circulans, Bacillus subtilis, Bacillus sp., Brevibacterium albidum, Brevibacterium album, Brevibacterium cerinum, Brevibacterium flavum, Brevibacterium glutamigenes, Brevibacterium iodinum, Brevibacterium ketoglutamicum, Brevibacterium lactofermentum, Brevibacterium linens, Brevibacterium roseum, Brevibacterium saccharolyticum, Brevibacterium sp., Corynebacterium acetoacidophilum, Corynebacterium acetoglutamicum, Corynebacterium ammoniagenes, Corynebacterium glutamicum (= Micrococcus glutamicum), Corynebacterium melassecola, Corynebacterium sp. or Escherichia coli, specifically Escherichia coli K12 and its described strains.

The method disclosed herein is, when the host organisms are microorganisms, advantageously carried out at a temperature between 0°C and 95°C, for example between 10°C and 85°C, particularly for example between 15°C and 75°C, very particularly for example between 15°C and 45°C. The pH is advantageously kept at between pH 4 and 12, for example between pH 6 and 9, particularly for example between pH 7 and 8, during this. The method disclosed herein can be operated batchwise, semibatchwise or continuously. A summary of known cultivation methods is to be found in the textbook by Chmiel (Bioprozeßtechnik 1. Einführung in die Bioverfahrenstechnik (Gustav Fischer Verlag, Stuttgart, 1991)) or in the textbook by Storhas (Bioreaktoren und periphere Einrichtungen (Vieweg Verlag, Braunschweig/Wiesbaden, 1994)). The culture medium to be used must meet the requirements of the respective strains in a suitable manner. Descriptions of culture media for various microorganisms are present in the handbook "Manual of Methods for General Bacteriology" of the American Society for Bacteriology (Washington D. C., USA, 1981). These media, which can be employed as disclosed herein include, as described above, usually one or more carbon sources, nitrogen sources, inorganic salts, vitamins and/or trace elements. Preferred carbon sources are sugars such as mono-, di- or polysaccharides. Examples of very good carbon sources are glucose, fructose, mannose, galactose, ribose, sorbose, ribulose, lactose, maltose, sucrose, raffinose, starch or cellulose. Sugars can also be added to the media via complex compounds such as molasses, or other byproducts of sugar refining. It may also be advantageous to add mixtures of various carbon sources. Other possible carbon sources are oils and fats such as, for example, soybean oil, sunflower oil, peanut oil and/or coconut fat, fatty acids such as, for example, palmitic acid, stearic acid and/or linoleic acid, alcohols and/or polyalcohols such as, for example, glycerol, methanol and/or ethanol and/or organic acids such as, for example, acetic acid and/or lactic acid. Nitrogen sources are usually organic or inorganic nitrogen compounds or materials, which contain these compounds. Examples of nitrogen sources include ammonia in liquid or gaseous form or ammonium salts such as ammonium sulfate, ammonium chloride, ammonium phosphate, ammonium carbonate or ammonium nitrate, nitrates, urea, amino acids or complex nitrogen sources such as corn steep liquor, soybean meal, soybean protein, yeast extract, meat extract and others. The nitrogen sources may be used singly or as a mixture. Inorganic salt compounds, which may be present in the media include the chloride, phosphorus or sulfate salts of calcium, magnesium, sodium, cobalt, molybdenum, potassium, manganese, zinc, copper and iron. For preparing sulfur-containing fine chemicals, in particular amino acids, e.g. methionine, it is possible to use as sulfur source inorganic sulfur-containing compounds such as, for example, sulfates, sulfites, dithionites, tetrathionates, thiosulfates, sulfides or else organic sulfur compounds such as mercaptans and thiols. It is possible to use as phosphorus source phosphoric acid, potassium dihydrogenphosphate or dipotassium hydrogenphosphate or the corresponding sodium-containing salts. Chelating agents can be added to the medium in order to keep the metal ions in solution. Particularly suitable chelating agents include dihydroxyphenols such as catechol or protocatechuate, or organic acids such as citric acid. The fermentation media employed as disclosed herein for cultivating microorganisms normally also contain other growth factors such as vitamins or growth promoters, which include, for example, biotin, riboflavin, thiamine, folic acid, nicotinic acid, pantothenate and pyridoxine. Growth factors and salts are often derived from complex media components such as yeast extract, molasses, corn steep liquor and the like. Suitable precursors can moreover be added to the culture medium. The exact composition of the media compounds depends greatly on the particular experiment and is chosen individually for each specific case. Information about media optimization is obtainable from the textbook "Applied Microbiol. Physiology, A Practical Approach" (editors P.M. Rhodes, P.F. Stanbury, IRL Press (1997) pp. 53-73, ISBN 0 19 963577 3). Growth media can also be purchased from commercial suppliers such as Standard 1 (Merck) or BHI (Brain heart infusion, DIFCO) and the like. All media components are sterilized either by heat (1.5 bar and 121°C for 20 min) or by sterilizing filtration. The components can be sterilized either together or, if necessary, separately. All media components can be present at the start of the cultivation or optionally be added continuously or batchwise. The temperature of the culture is normally between 15°C and 45°C, for example at 25°C to 40°C, and can be kept constant or changed during the experiment. The pH of the medium should be in the range from 5 to 8.5, for example around 7. The pH for the cultivation can be controlled during the cultivation by adding basic compounds such as sodium hydroxide, potassium hydroxide, ammonia or aqueous ammonia or acidic compounds such as phosphoric acid or sulfuric acid. Foaming can be controlled by employing antifoams such as, for example, fatty acid polyglycol esters. The stability of plasmids can be maintained by adding to the medium suitable substances having a selective effect, for example antibiotics. Aerobic conditions are maintained by introducing oxygen or oxygen-containing gas mixtures such as, for example, ambient air into the culture. The temperature of the culture is normally from 20°C to 45°C and for example from 25°C to 40°C. The culture is continued until formation of the desired product is at a maximum. This aim is normally achieved within 10 hours to 160 hours. The fermentation broths obtained in this way, containing in particular fine chemicals, normally have a dry matter content of from 7.5 to 25% by weight. Sugar-limited fermentation is additionally advantageous, at least at the end, but especially over at least 30% of the fermentation time. This means that the concentration of utilizable sugar in the fermentation medium is kept at, or reduced to, ≥ 0 to 3 g/l during this time. The fermentation broth is then processed further. Depending on requirements, the biomass can be removed entirely or partly by separation methods, such as, for example, centrifugation, filtration, decantation or a combination of these methods, from the fermentation broth or left completely in it. The fermentation broth can then be thickened or concentrated by known methods, such as, for example, with the aid of a rotary evaporator, thin-film evaporator, falling film evaporator, by reverse osmosis or by nanofiltration. This concentrated fermentation broth can then be worked up by freeze-drying, spray drying, spray granulation or by other methodes.

However, it is also possible to purify the fine chemicals produced further. For this purpose, the product-containing composition is subjected to a chromatography on a suitable resin, in which case the desired product or the impurities are retained wholly or partly on the chromatography resin. These chromatography steps can be repeated if necessary, using the same or different chromatography resins. The skilled worker is familiar with the choice of suitable chromatography resins and their most effective use. The purified product can be concentrated by filtration or ultrafiltration and stored at a temperature at which the stability of the product is a maximum.

The identity and purity of the isolated compound(s) can be determined by prior art techniques. These include high performance liquid chromatography (HPLC), spectroscopic methods, mass spectrometry (MS), staining methods, thin-layer chromatography, NIRS, enzyme assay or microbiological assays. These analytical methods are summarized in: Patek et al. (1994) Appl. Environ. Microbiol. 60:133-140; Malakhova et al. (1996) Biotekhnologiya 11 27-32; and Schmidt et al. (1998) Bioprocess Engineer. 19:67-70. Ulmann's Encyclopedia of Industrial Chemistry (1996) Vol. A27, VCH: Weinheim, pp. 89-90, pp. 521-540, pp. 540-547, pp. 559-566, 575-581 and pp. 581-587; Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, Vol. 17.

In yet another aspect, the invention also relates to harvestable parts and to propagation material of the transgenic plants according to the invention which either contain transgenic plant cells expressing a nucleic acid molecule as defined by the claims or which contains cells which show an increased cellular activity of the polypeptide as defined by the claims, e.g. an increased expression level or higher activity of the protein.

Harvestable parts can be in principle any useful parts of a plant, for example, flowers, pollen, seedlings, tubers, leaves, stems, fruit, seeds, roots etc. Propagation material includes, for example, seeds, fruits, cuttings, seedlings, tubers, rootstocks etc.

The description furthermore discloses the use of the transgenic non-human organisms as disclosed herein and of the cells, cell cultures, parts - such as, for example, roots, leaves and the like as mentioned above in the case of transgenic plant organisms - derived from them, and to transgenic propagation material such as seeds or fruits and the like as mentioned above, for the production of foodstuffs or feeding stuffs, pharmaceuticals or fine chemicals.

Accordingly in another disclosure, the present description discloses the use of the non-human organism, e.g. the microorganism, the plant, plant cell or plant tissue, the vector, disclosed herein for making fatty acids, carotenoids, isoprenoids, vitamins, lipids, wax esters, polysaccharides and/or polyhydroxyalkanoates, and/or its metabolism products, in particular, steroid hormones, cholesterol, prostaglandin, triacylglycerols, bile acids and/or ketone bodies producing cells, tissues and/or plants. There are a number of mechanisms by which the yield, production, and/or efficiency of production of fatty acids, carotenoids, isoprenoids, vitamins, wax esters, lipids, polysaccharides and/or polyhydroxyalkanoates, and/or its metabolism products, in particular, steroid hormones, cholesterol, triacylglycerols, prostaglandin, bile acids and/or ketone bodies or further of above defined fine chemicals incorporating such an altered protein can be affected. In the case of plants, by e.g. increasing the expression of acetyl-CoA which is the basis for many products, e.g., fatty acids, carotenoids, isoprenoids, vitamines, lipids, polysaccharides, wax esters, and/or polyhydroxyalkanoates, and/or its metabolism products, in particular, prostaglandin, steroid hormones, cholesterol, triacylglycerols, bile acids and/or ketone bodies in a cell, it may be possible to increase the amount of the produced said compounds thus permitting greater ease of harvesting and purification or in case of plants more efficient partitioning. Further, one or more of said metabolism products, increased amounts of the cofactors, precursor molecules, and intermediate compounds for the appropriate biosynthetic pathways maybe required. Therefore, by increasing the number and/or activity of transporter proteins involved in the import of nutrients, such as carbon sources (i.e., sugars), nitrogen sources (i.e., amino acids, ammonium salts), phosphate, and sulfur, it may be possible to improve the production of acetyl CoA and its metabolism products as mentioned above, due to the removal of any nutrient supply limitations on the biosynthetic process. In particular, it may be possible to increase the yield, production, and/or efficiency of production of said compounds, e.g. fatty acids, carotenoids, isoprenoids, vitamins, was esters, lipids, polysaccharides, and/or polyhydroxyalkanoates, and/or its metabolism products, in particular, steroid hormones, cholesterol, prostaglandin, triacylglycerols, bile acids and/or ketone bodies molecules etc. in plants.

Furthermore described is a method for the recombinant production of pharmaceuticals or fine chemicals in non-human host organisms, wherein a host organism is transformed with one of the above-described expression constructs comprising one or more structural genes which encode the desired fine chemical or catalyze the biosynthesis of the desired fine chemical, the transformed host organism is cultured, and the desired fine chemical is isolated from the culture medium. This method can be applied widely to fine chemicals such as enzymes, vitamins, amino acids, sugars, fatty acids, and natural and synthetic flavorings, aroma substances and colorants or compositions comprising these. Especially preferred is the additional production of amino acids, tocopherols and tocotrienols and carotenoids or compositions comprising said compounds. The transformed host organisms are cultured and the products are recovered from the host organisms or the culture medium by methods known to the skilled worker or the organism itself servers as food or feed supplement. The production of pharmaceuticals such as, for example, antibodies or vaccines, is described by Hood EE, Jilka JM. Curr Opin Biotechnol. 1999 Aug; 10(4):382-6; Ma JK, Vine ND. Curr Top Microbiol Immunol. 1999; 236:275-92.

In one disclosure, the present description discloses a method for the identification of a gene product conferring an increase in growth or yield in an organism, comprising the following steps:
a) contacting e.g. hybridising, the nucleic acid molecules of a sample, e.g. cells, tissues, plants or microorganisms or a nucleic acid library , which can contain a candidate gene encoding a gene product conferring an in yield or growth as described above after expression, with the polynucleotide disclosed herein;
b) identifying the nucleic acid molecules, which hybridize under relaxed stringent conditions with the polynucleotide disclosed herein and, optionally, isolating the full length cDNA clone or complete genomic clone;
c) introducing the candidate nucleic acid molecules in host cells, for example in a plant cell or a microorganism;
d) expressing the identified nucleic acid molecules in the host cells;
e) deriving, a transgenic organism and assaying the growth rate or yield in the host cells; and
f) identifying the nucleic acid molecule and its gene product which expression confers an increase after expression compared to the wild type.

Relaxed hybridisation conditions are: After standard hybridisation procedures washing steps can be performed at low to medium stringency conditions usually with washing conditions of 40°-55°C and salt conditions between 2xSSC and 0,2x SSC with 0,1% SDS in comparison to stringent washing conditions as e.g. 60°-68°C with 0,1xSSC and 0,1% SDS. Further examples can be found in the references listed above for the stringend hybridization conditions. Usually washing steps are repeated with increasing stringency and length until a useful signal to noise ratio is detected and depend on many factors as the target, e.g. its purity, GC-content, size etc, the probe, e.g.its length, is it a RNA or a DNA probe, salt conditions, washing or hybridisation temperature, washing or hybridisation time etc.

In another disclosure, the present description discloses a method for the identification of a gene product conferring an increase in yield or growth in an organism, comprising the following steps:
a) identifiying nucleic acid molecules of an organism; which can contain a candidate gene encoding a gene product conferring an increase in growth rate and/or yield after expression, which are at least 20%, for example 25%, more for example 30%, even are 35%. 40% or 50%, even are 60%, 70% or 80%, are 90% or 95% or more homology to the nucleic acid molecule disclosed herein, for example via homology search in a data bank;
b) introducing the candidate nucleic acid molecules in host cells, for example in a plant cells or microorganisms, appropriate for producing feed or food stuff or fine chemicals;
c) expressing the identified nucleic acid molecules in the host cells;
d) deriving the organism and assaying the yield or growth of the organism;
e) and identifying the nucleic acid molecule and its gene product which expression confers an increase in the yield or growth of the host cell after expression compared to the wild type.

The nucleic acid molecules identified can then be used in the same way as the polynucleotide disclosed herein.

Furthermore, in one disclosure, the present description discloses a method for the identification of a compound stimulating growth or yield to said plant comprising:
a) contacting cells which express the polypeptide disclosed herein or its mRNA with a candidate compound under cell cultivation conditions;
b) assaying an increase in expression of said polypeptide or said mRNA;
c) comparing the expression level to a standard response made in the absence of said candidate compound; whereby, an increased expression over the standard indicates that the compound is stimulating yield or growth.

Furthermore, in one disclosure, the present description discloses a method for the screening for agonists of the activity of the polypeptide disclosed herein:
a) contacting cells, tissues , plants or microorganisms which express the polypeptide disclosed herein with a candidate compound or a sample comprising a plurality of compounds under conditions which permit the expression the polypeptide disclosed herein;
b) assaying the growth, yield or the polypeptide expression level in the cell, tissue, plant or microorganism or the media the cell, tissue, plant or microorganisms is cultured or maintained in; and
c) identifying an agonist or antagonist by comparing the measured growth or yield or polypeptide expression level with a standard growth, yield or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.

Furthermore, in one disclosure, the present description discloses process for the identification of a compound conferring increased growth and/or yield production in a plant or microorganism, comprising the steps:
a) culturing a cell or tissue or microorganism or maintaining a plant expressing the polypeptide disclosed herein or a nucleic acid molecule encoding said polypeptide and a readout system capable of interacting with the polypeptide under suitable conditions which permit the interaction of the polypeptide with said readout system in the presence of a compound or a sample comprising a plurality of compounds and capable of providing a detectable signal in response to the binding of a compound to said polypeptide under conditions which permit the expression of said readout system and the polypeptide disclosed herein; and
b) identifying if the compound is an effective agonist by detecting the presence or absence or increase of a signal produced by said readout system.

Said compound may be chemically synthesized or microbiologically produced and/or comprised in, for example, samples, e.g., cell extracts from, e.g., plants, animals or microorganisms, e.g. pathogens. Furthermore, said compound(s) may be known in the art but hitherto not known to be capable of suppressing or activating the polypeptide disclosed herein. The reaction mixture may be a cell free extract or may comprise a cell or tissue culture. Suitable set ups for the method disclosed herein are known to the person skilled in the art and are, for example, generally described in Alberts et al., Molecular Biology of the Cell, third edition (1994), in particular Chapter 17. The compounds may be, e.g., added to the reaction mixture, culture medium, injected into the cell or sprayed onto the plant.

If a sample containing a compound is identified in the method disclosed herein, then it is either possible to isolate the compound from the original sample identified as containing the compound capable of activating or increasing, or one can further subdivide the original sample, for example, if it consists of a plurality of different compounds, so as to reduce the number of different substances per sample and repeat the method with the subdivisions of the original sample. Depending on the complexity of the samples, the steps described above can be performed several times, for example until the sample identified according to the method disclosed herein only comprises a limited number of or only one substance(s). For example said sample comprises substances of similar chemical and/or physical properties, and most for example said substances are identical. For example, the compound identified according to the above described method or its derivative is further formulated in a form suitable for the application in plant breeding or plant cell and tissue culture.

The compounds which can be tested and identified according to a method disclosed herein may be expression libraries, e.g., cDNA expression libraries, peptides, proteins, nucleic acids, antibodies, small organic compounds, hormones, peptidomimetics, PNAs or the like (Milner, Nature Medicine 1 (1995), 879-880; Hupp, Cell 83 (1995), 237-245; Gibbs, Cell 79 (1994), 193-198 and references cited supra). Said compounds can also be functional derivatives or analogues of known inhibitors or activators. Methods for the preparation of chemical derivatives and analogues are well known to those skilled in the art and are described in, for example, Beilstein, Handbook of Organic Chemistry, Springer edition New York Inc., 175 Fifth Avenue, New York, N.Y. 10010 U.S.A. and Organic Synthesis, Wiley, New York, USA. Furthermore, said derivatives and analogues can be tested for their effects according to methods known in the art. Furthermore, peptidomimetics and/or computer aided design of appropriate derivatives and analogues can be used, for example, according to the methods described above. The cell or tissue that may be employed in the method disclosed herein for example is a host cell, plant cell or plant tissue of the invention described in the embodiments hereinbefore.

Thus, in a further disclosure the description discloses a compound obtained or identified according to the method for identifying an agonist disclosed herein said compound being an agonist of the polypeptide disclosed herein.

Accordingly, in one disclosure, the present description further discloses a compound identified by the method for identifying a compound disclosed herein.

Said compound is, for example, a homologous of the polypeptide disclosed herein. Homologues of the polypeptid disclosed herein can be generated by mutagenesis, e.g., discrete point mutation or truncation of the polypeptide disclosed by the present description. As used herein, the term "homologue" refers to a variant form of the protein, which acts as an agonist of the activity of the polypeptide disclosed herein. An agonist of said protein can retain substantially the same, or a subset, of the biological activities of the polypeptide disclosed herein. In particular, said agonist confers the increase of the expression level of the polypeptide disclosed herein and/or the expression of said agonist in an organisms or part thereof confers the increase in growth and/or yield.

In one disclosure, the description discloses an antibody specifically recognizing the compound or agonist disclosed herein.

The description discloses a diagnostic composition comprising at least one of the aforementioned polynucleotide, nucleic acid molecules, vectors, proteins, antibodies or compounds disclosed herein and optionally suitable means for detection.

The diagnostic composition disclosed herein is suitable for the isolation of mRNA from a cell and contacting the mRNA so obtained with a probe comprising a nucleic acid probe as described above under hybridizing conditions, detecting the presence of mRNA hybridized to the probe, and thereby detecting the expression of the protein in the cell. Further methods of detecting the presence of a protein disclosed bythe present description comprise immunotechniques well known in the art, for example enzyme linked immunosorbent assay.

Furthermore, it is useful to use the nucleic acid molecules disclosed herein as molecular markers or primer in association mapping or plant breeding expecially marker assisted breeding. In a preferred disclosure the nucleic acid molecules disclosed herein can be used in association mapping or plant breeding especially marker assisted breeding for traits directly or indirectly related to plant growth or yield. For example the nucleic acid disclosed herein might colocalize with a quantitative trait locus for growth and yield. In this case the cosegregation of different variants of the nucleic acid disclosed herein with differences in growth or yield might allow advanced breeding for these traits by testing the offspring of crosses for the presence or absence of favourable or unfavourable variants of the nucleic acid disclosed herein. Suitable means for detection are well known to a person skilled in the arm, e.g. buffers and solutions for hydridization assays, e.g. the aforementioned solutions and buffers, further and means for Southern-, Western-, Northern- etc. -blots, as e.g. described in Sambrook et al. are known.

In another disclosure, the present description discloses a kit comprising the nucleic acid molecule, the vector, the host cell, the polypeptide, the antisense nucleic acid, the antibody, plant cell, the plant or plant tissue, the harvestable part, the propagation material and/or the compound or agonist identified disclosed bythe method disclosed herein.

The compounds of the kit disclosed herein may be packaged in containers such as vials, optionally with/in buffers and/or solution. If appropriate, one or more of said components might be packaged in one and the same container. Additionally or alternatively, one or more of said components might be adsorbed to a solid support as, e.g. a nitrocellulose filter, a glas plate, a chip, or a nylon membrane or to the well of a micro titerplate. The kit can be used for any of the herein described methods and embodiments, e.g. for the production of the host cells, transgenic plants, pharmaceutical compositions, detection of homologous sequences, identification of antagonists or agonists, as food or feed or as a supplement thereof, as supplement for the treating of plants, etc.

Further, the kit can comprise instructions for the use of the kit for any of said embodiments, in particular for the use for producing organisms or part thereof.

In one disclosure said kit comprises further a nucleic acid molecule encoding one or more of the aforementioned protein, and/or an antibody, a vector, a host cell, an antisense nucleic acid, a plant cell or plant tissue or a plant.

In a further disclosure, the present description discloses a method for the production of a agricultural composition providing the nucleic acid molecule, the vector or the polypeptide disclosed herein or comprising the steps of the method disclosed herein for the identification of said compound, agonist or antagonist; and formulating the nucleic acid molecule, the vector of the invention or the agonist, or compound identified according to the methods or processes disclosed herein or with use of the subject matters disclosed herein in a form applicable as plant agricultural composition.

In another disclosure, the present description discloses a method for the production of an agricultural composition conferring increased growth or yield of a plant comprising the steps of the method for disclosed herein; and formulating the compound identified in a form acceptable as agricultural composition.

Under "acceptable as agricultural composition" is understood, that such a composition is in agreement with the laws regulating the content of fungicides, plant nutrients, herbizides, etc. For example such a composition is without any harm for the protected plants and the animals (humans included) fed therewith.

The present description also pertains to several disclosures relating to further uses and methods. The polynucleotide, polypeptide, protein homologues, fusion proteins, primers, vectors, host cells, described herein can be used in one or more of the following methods: identification of plants useful pro amino acid production as mentioned and related organisms; mapping of genomes; identification and localization of sequences of interest; evolutionary studies; determination of regions required for function; modulation of an activity.

Advantageously, inhibitor of the polypeptide disclosed herein, identified in an analogous way to the identification of agonist, can be used as herbicides. The inhibition of the polypeptide disclosed herein can reduce the growth of plants. For example, the application of the inhibitor on a field is inhibiting the growth of plants not desired if useful plants which are over-expressing the polypeptide disclosed herein can survive.

Accordingly, the polynucleotides disclosed herein have a variety of uses. First, they may be used to identify an organism or a close relative thereof. Also, they may be used to identify the presence thereof or a relative thereof in a mixed population of microorganisms or plants. By probing the extracted genomic DNA of a culture of a unique or mixed population of plants under stringent conditions with a probe spanning a region of the gene disclosed herein which is unique to this, one can ascertain whether a unique organism is present in a mixed population.

Further, the polynucleotide disclosed herein may be sufficiently homologous to the sequences of related species such that these nucleic acid molecules may serve as markers for the construction of a genomic map in related organisms.

The polynucleotide disclosed herein are also useful for evolutionary and protein structural studies. By comparing the sequences of to those encoding similar enzymes from other organisms, the evolutionary relatedness of the organisms can be assessed. Similarly, such a comparison permits an assessment of which regions of the sequence are conserved and which are not, which may aid in determining those regions of the protein which are essential for the functioning of the enzyme. This type of determination is of value for protein engineering studies and may give an indication of what the protein can tolerate in terms of mutagenesis without losing function.

Further, the polynucleotide disclosed herein, the polypeptide disclosed herein, the nucleic acid construct of the invention, the disclosed organisms, the disclosed host cell, the plant, plant tissue, plant cell, or the part thereof of the invention, the vector of the invention, the antagonist or the agonist identified with the method disclosed herein, the antibody disclosed herein, the antisense molecule disclosed herein or the nucleic acid molecule identified with the method disclosed herein, can be used for the preparation of an agricultural composition.

Furthermore, the polynucleotide disclosed herein, the polypeptide disclosed herein, the nucleic acid construct of the invention, the disclosed organisms, the disclosed host cell, the plant, plant tissue, plant cell, or the part thereof of the invention, the vector of the invention, antagonist or the agonist identified with the method disclosed herein, the antibody disclosed herein, the antisense or RNAi molecule disclosed herein or the nucleic acid molecule identified with the method disclosed herein, can be used for the identification and production of compounds capapble of conferring a modulation of yield or growth levels in an organism or parts thereof, for example to identify and produce compounds conferring an increase of growth and yield levels or rates in an organism or parts thereof, if said identified compound is applied to the organism or part thereof, i.e. as part of its food, or in the growing or culture media.

These and other embodiments are disclosed and encompassed by the description and examples of the present invention. Further literature concerning any one of the methods, uses and compounds to be employed in accordance with the present invention may be retrieved from public libraries, using for example electronic devices. For example the public database "Medline" may be utilized which is available on the Internet, for example under hftp://www.ncbi.nlm.nih.gov/PubMed/medline.html. Further databases and addresses, such as hftp://www.ncbi.nlm.nih.gov/, hftp://www.infobiogen.fr/, hftp://www.fmi.ch/biology/research-tools.html, hftp://www.tigr.org/, are known to the person skilled in the art and can also be obtained using, e.g., hftp://www.lycos.com. An overview of patent information in biotechnology and a survey of relevant sources of patent information useful for retrospective searching and for current awareness is given in Berks, TIBTECH 12 (1994), 352-364.

### Examples:

### Example 1:

### Amplification and cloning of the yeast ORFs YMR095C,.

Unless stated otherwise, standard methods according to Sambrook et al., Molecular Cloning: A laboratory manual, Cold Spring Harbor 1989, Cold Spring Harbor Laboratory Press, are used. PCR amplification of ORFs YMR095C, was carried out according to the protocol of Pfu Turbo DNA polymerase (Stratagene). The composition was as follows: 1x PCR buffer [20 mM Tris-HCl (pH 8.8), 2 mM MgSO4, 10 mM KCl, 10 mM (NH4)2SO4, 0.1% Triton X-100, 0.1 mg/ml BSA], 0.2 mM d-thio-dNTP and dNTP (1:125), 100 ng of genomic DNA of Saccharomyces cerevisiae (strain S288C; Research Genetics, Inc., now Invitrogen), 50 pmol of forward primer, 50 pmol of reverse primer, 2.5 u of Pfu Turbo DNA polymerase. The amplification cycles were as follows:
1 cycle of 3 min at 95°C, followed by 36 cycles of in each case 1 min at 95°C, 45 s at 50°C and 210 s at 72°C, followed by 1 cycle of 8 min at 72°C, then 4°C.

The following primer sequences were chosen for amplification of the Saccharomyces cerevisiae genes according to SEQ ID NO: 1,:
forward primer for YMR095C (SEQ ID NO: 96):
   5'- atgcacaaaa cccacagtac aatgt -3'
reverse primer for YMR095C (SEQ ID NO: 97):
   5'- ttaattagaa acaaactgtc tgataaac -3'

The amplicons were subsequently purified via QIAquick columns according to a standard protocol (Qiagen).

Restriction of the vector DNA (30 ng) was carried out with EcoRI and Smal according to the standard protocol, the EcoRI cleavage site was filled in according to the standard protocol (MBI-Fermentas) and the reaction was stopped by adding high-salt buffer. The cleaved vector fragments were purified via Nucleobond columns according to standard protocol (Machery-Nagel). A binary vector was used which contained a selection cassette (promoter, selection marker for example the bar gene or the AHAS gene, terminator) and an expression cassette comprising a constitutive promoter such as the super-promoter (ocs3mas) (Ni et al., The Plant Journal 1995, 7, 661-676), a cloning cassette and a terminator sequence between the T-DNA border sequences. Other than in the cloning cassette, the binary vector had no EcoRI and Smal cleavage sites. Binary vectors which may be used are known to the skilled worker, and a review on binary vectors and their use can be found in Hellens, R., Mullineaux, P. and Klee H., (2000) "A guide to Agrobacterium binary vectors", Trends in Plant Science, Vol. 5 NO 10, 446-451. Depending on the vector used, cloning may advantageously also be carried out using other restriction enzymes. Corresponding advantageous cleavage sites may be attached to the ORF by using corresponding primers for PCR amplification.

Approx. 30 ng of prepared vector and a defined amount of prepared amplicon were mixed and ligated by adding ligase.
The ligated vectors were transformed in the same reaction vessel by adding competent E. coli cells (DH5alpha strain) and incubating at 1°C for 20 min, followed by a heat shock at 42°C for 90 s and cooling to 4°C. This was followed by addition of complete medium (SOC) and incubation at 37°C for 45 min. The entire mixture was then plated out on an agar plate containing antibiotics (selected depending on the binary vector used) and incubated at 37°C overnight.

Successful cloning was checked by amplification with the aid of primers which bind upstream and downstream of the restriction cleavage site and thus make amplification of the insertion possible. The amplification was carried out according to the Taq DNA polymerase protocol (Gibco-BRL). The composition was as follows: 1x PCR buffer [20 mM Tris-HCL (pH 8.4), 1.5 mM MgCl2, 50 mM KCl, 0.2 mM dNTP, 5 pmol of forward primer, 5 pmol of reverse primer, 0.625 u of Taq DNA polymerase].

The amplification cycles were as follows: 1 cycle of 5 min at 94°C, followed by 35 cycles of in each case 15 s at 94°C, 15 s at 66°C and 5 min at 72°C, followed by 1 cycle of 10 min at 72°C, then 4°C.

Several colonies were checked further by restriction digests and sequencing and only one colony for which a PCR product of the expected size had been identified in the correct orientation was used further.

One aliquot of this positive colony was transferred to a reaction vessel filled with complete medium (LB) and incubated at 37°C overnight. The LB medium contained an antibiotic for selection of the clone, which was selected according to the binary vector used and the resistance gene contained therein.
Plasmid preparation was carried out according to the guidelines of the Qiaprep standard protocol (Qiagen).

Example 2:

### General plant transformation

Plant transformation via transfections with Agrobacterium and regeneration of the plants may be carried out according to standard methods, for example as described herein or in Gelvin, Stanton B.; Schilperoort, Robert A, "Plant Molecular Biology Manual", 2nd Ed. - Dordrecht: Kluwer Academic Publ., 1995. - in Sect., Ringbuc Zentrale Signatur: BT11-P ISBN 0-7923-2731-4; Glick, Bernard R.; Thompson, John E., "Methods in Plant Molecular Biology and Biotechnology", Boca Raton : CRC Press, 1993. - 360 S., ISBN 0-8493-5164-2.

Oil seed rape may be transformed by means of cotyledon transformation, for example according to Moloney et al., Plant cell Report 8 (1989), 238-242; De Block et al., Plant Physiol. 91 (1989, 694-701).

Soybeans may be transformed, for example, according to the methods described in EP 0424 047, US 5,322,783 or in EP 0397 687, US 5,376,543, US 5,169,770.

Alternatively, DNA uptake may be achieved and a plant may be transformed also by particle bombardment, polyethylene glycol mediation or via the "silicon carbide fiber" technique, rather than by Agrobacterium-mediated plant transformation, see, for example, Freeling and Walbot "The maize handbook" (1993) ISBN 3-540-97826-7, Springer Verlag New York.

### Example 3:

### Preparation of plants overexpressing ORFs YMR095C,.

The respective plasmid constructs were transformed by means of electroporation into the agrobacterial strain pGV3101 containing the pMP90 plasmid, and the colonies were plated out on TB medium (QBiogen, Germany) containing the selection markers kanamycin, gentamycin and rifampicin and incubated at 28°C for 2 days. The antibiotics or selection agents are to be selected according to the plasmid used and to the compatible agrobacterial strain. A review on binary plasmids and agrobacteria strains can be found in Hellens, R., Mullineaux, P. and Klee H., (2000) "A guide to Agrobacterium binary vectors", Trends in Plant Science, Vol 5 NO 10, 446-451.

A colony was picked from the agar plate with the aid of a toothpick and taken up in 3 ml of TB medium containing the abovementioned antibiotics.

The preculture grew in a shaker incubator at 28°C and 120 rpm for 48 h. 400 ml of LB medium containing the appropriate antibiotics were used for the main culture. The preculture was transferred into the main culture which grew at 28°C and 120 rpm for 18 h. After centrifugation at 4000 rpm, the pellet was resuspended in infiltration medium (M & S medium with 10% sucrose). Dishes (Piki Saat 80, green, provided with a screen bottom, 30 x 20 x 4.5 cm, from Wiesauplast, Kunststofftechnik, Germany) were half-filled with a GS 90 substrate (standard soil, Werkverband E.V., Germany). The dishes were watered overnight with 0.05% Previcur solution (Previcur N, Aventis Crop-Science). Transformation of Arabidopsis was carried out following Bechtold N. and Pelletier G. (1998) In planta Agrobacterium-mediated transformation of adult Arabidopsis thaliana plants by vacuum infiltration. Methods in Molecular Biology. 82:259-66 and Clough and Bent Clough, JC and Bent, AF. 1998 Floral dip: a simplified method for Agrobacterium-mediated transformation of Aràbidopsis thaliana, Plant J. 16:735-743.

Arabidopsis thaliana, C24 seeds (Nottingham Arabidopsis Stock Centre, UK; NASC Stock N906) were scattered over the dish, approx. 1000 seeds per dish. The dishes were covered with a hood and placed in the stratification facility (8 h 110 µE, 5°C; 16 h dark 6°C). After 5 days, the dishes were placed into the short-day phytotron (8 h 130 µE, 22°C; 16 H dark 20°C), where they remained for 10 days, until the first true leaves had formed. The seedlings are transferred into pots containing the same substrate (Teku pots, 10 cm Ø, LC series, manufactured by Pöppelmann GmbH&Co, Germany). Nine plants were pricked out into each pot. The pots were then returned into the short-day phytotron for the plants to continue growing. After 10 days, the plants were transferred into a greenhouse cabinet, 16 h 340 µE 22°C and 8 h dark 20°C, where they grew for a further 10 days.

Seven-week-old Arabidopsis plants which had just started flowering were immersed for 10 sec into the above-described agrobacterial suspension which had previously been treated with 10 µl of Silwett L77 (Crompton S.A., Osi Specialties, Switzerland). The method is described in Bechtold N. and Pelletier G. (1998). The plants were subsequently placed into a humid chamber for 18 h and the pots were subsequently returned to the greenhouse for the plants to continue growing. The plants remained there for another 10 weeks until the seeds were harvested.

Depending on the resistance marker used for selecting the transformed plants, the harvested seeds were sown in a greenhouse and subjected to spray selection or else, after sterilization, cultivated on agar plates with the appropriate selecting agent. In case of BASTA®-resistance, plantlets were sprayed four times at an interval of 2 to 3 days with 0.02 % BASTA®. After approx. 10-14 days, the transformed resistant plants differed distinctly from the dead wild-type seedlings and could be pricked out into 6-cm pots. Transformed plants were allowed to set seeds. The seeds of the transgenic *A. thaliana* plants were stored in a freezer (at -20°C).

### Example 4

### Analysis of lines overexpressing SEQ ID NO: 1, by determination of fresh weight

A line overexpressing SEQ ID NO: 1, RNA was selected. For this purpose, total RNA was extracted from three-week-old Arabidopsis plants transgenic for SEQ ID NO: 1,. For hybridization, 20 µg of RNA were electrophoretically fractionated, blotted to Hybond N membrane (Amersham Biosciences Europe GmbH, Freiburg, Germany) according to the manufacturer's instructions and hybridized with an YMR095C, -specific probe. Rothi-Hybri-Quick buffer (Roth, Karlsruhe, Germany) was used for hybridization and the probe was labeled using the Rediprime II DNA Labeling System (Amersham Biosciences Europe GmbH Freiburg, Germany) according to the manufacturer's instructions. The DNA fragment for these probes were prepared by means of a standard PCR of Arabidopsis genomic DNA and the primers:
5'- atgcacaaaa cccacagtac aatgt -3'(SEQ ID NO: 96)
and
5'- ttaattagaa acaaactgtc tgataaac -3'(SEQ ID NO: 97),
respectively.

For analysis, the plants were cultivated in a phytotron from Swalöf Weibull (Sweden) under the following conditions. After stratification, the test plants were cultured in a 16 h light/8 h dark rhythm at 20°C, a humidity of 60% and a CO₂ concentration of 400 ppm for 22-23 days. The light sources used were Powerstar HQI-T 250 W/D Daylight lamps from Osram, which generate light of a color spectrum similar to that of the sun with a light intensity of 220 µE/m²/s⁻¹.

On days 24 after sowing, which correspond to approximately day 17 after germination, in each case approximately 40 individual plants of both the wild type (WT) and the YMR095C, -overexpressing line, (lines 3318, 5194, 3325, 4803 and 9001 respectively) were studied. The fresh weight of aboveground parts of transgenic lines and wildtype (WT) Arabidopsis plants was determined immediately thereafter, using a precision balance. The differences between the results for the wild-type plants and the heaviest transgenic line were tested for significance by means of a T test for each line.

The result is depicted in table 1.

**Table 1: Overview over the increase of biomass of transgenic Arabidopsis plants over-expressing one yeast gene in comparison to the MC24 wild type. The biomass analysis was performed in different experiments (1.1 or 1.2) and then confirmed in confirmation loops (1 or 2).**

| Line # | Gene name | Experiment 1 (Weight mg) | p value t-test | Experiment 2 Confirmation loop (Weight mg) | p value t-test |
|---|---|---|---|---|---|
| | | | p=0,003 | | |
| | | 317 mg ± 87 | | 424,75 mg ± 110,28 | p=0,01 |
| 3318 | YMR095C | 15% increase Experiment 1.2 | | 38% increase Confirmation loop 1 | |
| | | 186 mg ± 47 Experiment 1.1 | | 307,15 mg ± 96,36 Confirmation loop1 | |
| WT | - | 276 mg ± 88 Experiment 1.2 | | 212,5 mg ± 48 Confirmation loop2 | |

### Literature:

Gibson, (1996) A novel method for real time quantitative RT-PCR. Genome Res. 6, 995-1001
Lie, (1998) Advances in quantitative PCR technology: 5'nuclease assays

### Example 5

### Overexpression of SEQ ID NO: 1, in tobacco and canola

For transformation of canola (Brassica napus), cotyledonary petioles and hypocotyls of seedlings at an age of from 5 to 6 days were used as explants for the tissue culture and transformed as described, inter alia, in Babic et al. (1998, Plant Cell Rep 17: 183-188). The commercial variety Westar is the standard variety for transformation but other varieties may also be utilized. The sequence encoding the SEQ ID NO: 2, activity is cloned into the expression cassette of a binary vector containing a selection cassette according to molecular standard methods. Exemplary clonings are described elsewhere in the examples and are known to the skilled worker.
The agrobacterial strain *Agrobacterium tumefaciens* LBA4404 containing, which is transformed with the binary vector, is used for transformation. A multiplicity of binary vectors for plant transformation have already been described (inter alia, An, G. in Agrobacterium Protocols. Methods in Molecular Biology vol. 44, pp. 47-62, Gartland KMA and Davey MR eds. Humana Press, Totowa, New Jersey). Many binary vectors derive from the binary vector pBIN19 which has been described by Bevan (Nucleic Acid Research. 1984. 12:8711-8721) and which comprises an expression cassette for plants which is flanked by the left and right border of the *Agrobacterium tumefaciens* Ti plasmid. A plant expression cassette comprises at least two components, a selection marker gene and a suitable promoter capable of regulating the transcription of cDNA or genomic DNA in plant cells in the desired manner. A multiplicity of selection marker genes such as antibiotic resistance or herbicide resistance genes may be used, such as, for example, a mutated Arabidopsis gene which encodes a mutated herbicide-resistant AHAS enzyme (US 5,767,366 and US 6,225,105). Similarly, it is also possible to use different promoters for expressing the gene with SEQ ID NO: 2, activity. For example, either a constitutive expression as is mediated by the 34S promoter (GenBank Accession NO: M59930 and X16673) or else seed-specific expression may be desired.

Canola seeds are sterilized in 70% ethanol for two minutes and then in 30% chlorox containing a drop of Tween-20 for 10 minutes, followed by three washing steps in sterile water.

The seeds are incubated in vitro on semi-concentrated MS medium without hormones, containing 1% sucrose, 0.7% phytagar at 23°C and in a 16/8 h day/night rhythm for 5 days for germination. The cotyledonary petiole explants were separated together with the cotyledons from seedlings and inoculated with the agrobacteria by dipping the site of the cutting into the bacterial suspension. The explants were then incubated on MSBAP-3 medium containing 3 mg/l BAP, 3% sucrose and 0.7% phytagar at 23°C and 16 h of light for two days. After two days of cocultivation with the agrobacteria, the explants are transferred to MSBAP-3 medium containing 3 mg/l BAP, cefotaxime, carbenicillin or timentin (300 mg/l) for 7 days and then to MSBAP-3 medium containing cefotaxime, carbenicillin or timentin and selecting agent until shoot regeneration. When the shoots are 5 - 10 mm in length, they are cut off and transferred to "shoot elongation medium" (MSBAP-0.5, containing 0.5 mg/l BAP). Shoots of approx. 2 cm in length are then transferred to root medium (MS0) for induction of roots.

Material of primary transgenic plants is studied by means of PCR in order to verify incorporation of the T-DNA into the genome. Positive results are then confirmed by means of Southern blot analysis.

Confirmed transgenic plants are then tested for faster growth and higher yield.

### Sterile culture of tobacco plants

Tobacco plants cultivated under aseptic conditions are propagated in vitro by placing stem pieces of approx. 1-2 cm in length and with, in each case, one internodium on sterile medium. (Murashige and Skoog medium containing 2% sucrose and 0.7% agar-agar)(Murashige, T. and Skoog, F. (1962) Physiol. Plant. 15:473-497). The plants grow at 23°C, 200 µE and with a 16 h/8 h light/dark rhythm.

After about 5-6 weeks of growth, leaves of said plants are cut into approx. 1 cm² pieces under sterile conditions.

### Bacterial culture

An agrobacterial colony transformed with the construct for expressing an SEQ ID NO: 2, activity is picked from an agar plate with the aid of a sterile plastic tip which is then transferred into approx. 20 ml of liquid YEB medium (Sambrook et al., Molecular Cloning: A laboratory manual, Cold Spring Harbor 1989, Cold Spring Harbor Laboratory Press) containing the relevant antibiotics. The volume of said YEB medium is chosen as a function of the number of transformants. Normally, 20 ml of bacterial culture are sufficient in order to produce approx. 80 transgenic tobacco plants. The bacterial culture is grown on a shaker at 200 rpm and 28°C for 1 day.
On the following day, the bacterial culture is removed by centrifugation at 4000 rpm and taken up in liquid Murashige and Skoog medium.

### Transformation

The leaf pieces are briefly dipped into the bacterial suspension and cultured on Murashige and Skoog medium (2% sucrose and 0.7% agar-agar) in the dark for 2 days. The explants are transferred to MS medium containing antibiotics and corresponding hormones, as described in the method of Rocha-Sosa (Rocha-Sosa, M., Sonnewald, U., Frommer, W., Stratmann, M., Schell, J. and Willmitzer, L. 1998, EMBO J. 8: 23-29).

Transgenic lines can then be analyzed for expression of the SEQ ID NO: 2, transgene by means of Northern blot analysis. It is then possible to determine the increase in fresh weight and in the yield of seeds of selected lines in comparison with the wild type.

### Example 6

Design and expression of a synthetic transcription factor binding close to the endogenous SEQ ID NO: 2, homolog and activating the transcription thereof.

The endogenous ORF for SEQ ID NO: 1, or a homologous ORF in other plant species may also be activated by introducing a synthetic specific activator. For this purpose, a gene for a chimeric zinc finger protein which binds to a specific region in the regulatory region of the SEQ ID NO: 1, ORF or of its homologs in other plants is constructed. The artificial zinc finger protein comprises a specific DNA-binding domain and an activation domain such as, for example, the Herpes simplex virus VP16 domain. Expression of this chimeric activator in plants then results in specific expression of the target gene, here, for example, SEQ ID NO. 118, the Arabidopsis homolog of YGL212w, or SEQ ID 102 a maize homolog for SEQ ID 1 (YMR095C) or of other homologs of SEQ ID NO: 1, in other plant species. The experimental details may be carried out as described in WO 01/52620 or Ordiz MI, (Proc. Natl. Acad. Sci. USA, 2002, Vol. 99, Issue 20, 13290) or Guan, (Proc. Natl. Acad. Sci. USA, 2002, Vol. 99, Issue 20, 13296).

### Example 7

### Identification of a line in which a strong promoter is integrated upstream of SEQ ID NO: 1, homologs in plants and thus activates expression

It is furthermore possible for strong ectopic expression of the desired ORF to integrate a strong promoter upstream of said ORF. For this purpose, a population of transgenic Arabidopsis plants was generated into which a vector containing the bidirectional mas promoter (Velten, 1984, EMBO J, 3, 2723) at the left T-DNA border was integrated. Said promoter enabled, via its 2' promoter, transcription from the T-DNA via the left border into the adjacent genomic DNA. The genomic DNA was then isolated from the individual plants and pooled according to a specific plan. The method of this reverse screening for T-DNA integrations at a particular locus has been described in detail by Krysan et al.,(Krysan, 1999, The Plant Cell, Vol 11, 2283) and references therein. Lines in which the T-DNA had integrated upstream of the plant homologs of SEQ ID NO: 1, ORF were identified. Enhanced expression of the plant homologs of SEQ ID NO: 1, in these lines, compared to the wild type, were detected by means of Northern blot analysis.

### Example 8

### Identification of homologous genes in other plant species

Homologous sequences of other plants were identified by means of special database search tools such as, in particular, the BLAST algorithm (Basic Local Alignment Search Tool, Altschul, 1990, J. Mol. Biol., 215, 403 and Altschul, 1997, Nucl. Acid Res., 25, 3389). The blastn and blastp comparisons were carried out in the standard manner using the BLOSUM-62 scoring matrix (Henikoff, 1992, Proc. Natl. Acad. Sci. USA, 89, 10915). The NCBI GenBank database as well as three libraries of expressed sequence tags (ESTs) of *Brassica napus* cv. "AC Excel", "Quantum" and "Cresor" (canola) and *Oryza sativa* cv. Nippon-Barre (Japonica rice) were studied. The search identified amino acid sequences and their respective nucleic acid sequences from various organisms, which are homologous to SEQ ID NO: 2,.

### Example 9

### Engineering plants

### Example 9a

### Engineering ryegrass plants

Seeds of several different ryegrass varieties can be used as explant sources for transformation, including the commercial variety Gunne available from Svalof Weibull seed company or the variety Affinity. Seeds are surface-sterilized sequentially with 1% Tween-20 for 1 minute, 100% bleach for 60 minutes, 3 rinses with 5 minutes each with de-ionized and distilled H₂O, and then germinated for 3-4 days on moist, sterile filter paper in the dark. Seedlings are further sterilized for 1 minute with 1% Tween-20, 5 minutes with 75% bleach, and rinsed 3 times with ddH2O, 5 min each.

Surface-sterilized seeds are placed on the callus induction medium containing Murashige and Skoog basal salts and vitamins, 20 g/l sucrose, 150 mg/l asparagine, 500 mg/l casein hydrolysate, 3 g/l Phytagel, 10 mg/l BAP, and 5 mg/l dicamba. Plates are incubated in the dark at 25°C for 4 weeks for seed germination and embryogenic callus induction.

After 4 weeks on the callus induction medium, the shoots and roots of the seedlings are trimmed away, the callus is transferred to fresh media, is maintained in culture for another 4 weeks, and is then transferred to MSO medium in light for 2 weeks. Several pieces of callus (11-17 weeks old) are either strained through a 10 mesh sieve and put onto callus induction medium, or are cultured in 100 ml of liquid ryegrass callus induction media (same medium as for callus induction with agar) in a 250 ml flask. The flask is wrapped in foil and shaken at 175 rpm in the dark at 23°C for 1 week. Sieving the liquid culture with a 40-mesh sieve is collected the cells. The fraction collected on the sieve is plated and is cultured on solid ryegrass callus induction medium for 1 week in the dark at 25°C. The callus is then transferred to and is cultured on MS medium containing 1% sucrose for 2 weeks.

Transformation can be accomplished with either Agrobacterium or with particle bombardment methods. An expression vector is created containing a constitutive plant promoter and the cDNA of the gene in a pUC vector. The plasmid DNA is prepared from E. coli cells using with Qiagen kit according to manufacturer's instruction. Approximately 2 g of embryogenic callus is spread in the center of a sterile filter paper in a Petri dish. An aliquot of liquid MSO with 10 g/l sucrose is added to the filter paper. Gold particles (1.0 µm in size) are coated with plasmid DNA according to method of Sanford et al., 1993 and are delivered to the embryogenic callus with the following parameters: 500 µg particles and 2 µg DNA per shot, 1300 psi and a target distance of 8.5 cm from stopping plate to plate of callus and 1 shot per plate of callus.

After the bombardment, calli are transferred back to the fresh callus development medium and maintained in the dark at room temperature for a 1-week period. The callus is then transferred to growth conditions in the light at 25 °C to initiate embryo differentiation with the appropriate selection agent, e.g. 250 nM Arsenal, 5 mg/l PPT or 50 mg/L Kanamycin. Shoots resistant to the selection agent are appearing and once rooted are transferred to soil.

Samples of the primary transgenic plants (Tₒ) are analyzed by PCR to confirm the presence of T-DNA. These results are confirmed by Southern hybridization in which DNA is electrophoresed on a 1% agarose gel and transferred to a positively charged nylon membrane (Roche Diagnostics). The PCR DIG Probe Synthesis Kit (Roche Diagnostics) is used to prepare a digoxigenin-labelled probe by PCR, and used as recommended by the manufacturer.

Transgenic Tₒ ryegrass plants are propagated vegetatively by excising tillers. The transplanted tillers are maintained in the greenhouse for 2 months until well established. The shoots are defoliated and allowed to grow for 2 weeks.

### Example 9b

### Engineering soybean plants

Soybean can be transformed according to the following modification of the method described in the Texas A&M patent US 5,164,310. Several commercial soybean varieties are amenable to transformation by this method. The cultivar Jack (available from the Illinois Seed Foundation) is commonly used for transformation. Seeds are sterilized by immersion in 70% (v/v) ethanol for 6 min and in 25 % commercial bleach (NaOCl) supplemented with 0.1 % (v/v) Tween for 20 min, followed by rinsing 4 times with sterile double distilled water. Removing the radicle, hypocotyl and one cotyledon from each seedling propagates seven-day seedlings. Then, the epicotyl with one cotyledon is transferred to fresh germination media in petri dishes and incubated at 25 °C under a 16-hr photoperiod (approx. 100 µE-m-2s-1) for three weeks. Axillary nodes (approx. 4 mm in length) are cut from 3-4 week-old plants. Axillary nodes are excised and incubated in Agrobacterium LBA4404 culture.

Many different binary vector systems have been described for plant transformation (e.g. An, G. in Agrobacterium Protocols. Methods in Molecular Biology vol 44, pp 47-62, Gartland KMA and MR Davey eds. Humana Press, Totowa, New Jersey). Many are based on the vector pBIN19 described by Bevan (Nucleic Acid Research. 1984. 12:8711-8721) that includes a plant gene expression cassette flanked by the left and right border sequences from the Ti plasmid of Agrobacterium tumefaciens. A plant gene expression cassette consists of at least two genes - a selection marker gene and a plant promoter regulating the transcription of the cDNA or genomic DNA of the trait gene. Various selection marker genes can be used as described above, including the Arabidopsis gene encoding a mutated acetohydroxy acid synthase (AHAS) enzyme (US patents 5,767,366 and US 6,225,105). Similarly, various promoters can be used to regulate the trait gene to provide constitutive, developmental, tissue or environmental regulation of gene transcription as described above. In this example, the 34S promoter (GenBank Accession numbers M59930 and X16673) is used to provide constitutive expression of the trait gene.

After the co-cultivation treatment, the explants are washed and transferred to selection media supplemented with 500 mg/L timentin. Shoots are excised and placed on a shoot elongation medium. Shoots longer than 1 cm are placed on rooting medium for two to four weeks prior to transplanting to soil.

The primary transgenic plants (Tₒ) are analyzed by PCR to confirm the presence of T-DNA. These results are confirmed by Southern hybridization in which DNA is electrophoresed on a 1 % agarose gel and transferred to a positively charged nylon membrane (Roche Diagnostics). The PCR DIG Probe Synthesis Kit (Roche Diagnostics) is used to prepare a digoxigenin-labelled probe by PCR, and is used as recommended by the manufacturer.

### Example 9c

### Engineering corn plants

Transformation of maize (Zea Mays L.) is performed with a modification of the method described by Ishida et al. (1996. Nature Biotech 14745-50). Transformation is genotype-dependent in corn and only specific genotypes are amenable to transformation and regeneration. The inbred line A188 (University of Minnesota) or hybrids with A188 as a parent are good sources of donor material for transformation (Fromm et al. 1990 Biotech 8:833-839), but other genotypes can be used successfully as well. Ears are harvested from corn plants at approximately 11 days after pollination (DAP) when the length of immature embryos is about 1 to 1.2 mm. Immature embryos are co-cultivated with Agrobacterium tumefaciens that carry "super binary" vectors and transgenic plants are recovered through organogenesis. The super binary vector system of Japan Tobacco is described in WO patents WO94/00977 and WO95/06722. Vectors can be constructed as described. Various selection marker genes can be used including the maize gene encoding a mutated acetohydroxy acid synthase (AHAS) enzyme (US 6,025,541). Similarly, various promoters can be used to regulate the trait gene to provide constitutive, developmental, tissue or environmental regulation of gene transcription. In this example, the 34S promoter (GenBank Accession numbers M59930 and X16673) is used to provide constitutive expression of the trait gene.

Excised embryos are grown on callus induction medium, then maize regeneration medium, containing imidazolinone as a selection agent. The Petri plates are incubated in the light at 25 °C for 2-3 weeks, or until shoots develop. The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the imidazolinone herbicides and which are PCR positive for the transgenes.

The T1 generation of single locus insertions of the T-DNA can segregate for the transgene in a 3:1 ratio. Those progeny containing one or two copies of the transgene are tolerant of the imidazolinone herbicide. Homozygous T2 plants can exhibited similar phenotypes as the T1 plants. Hybrid plants (F1 progeny) of homozygous transgenic plants and non-transgenic plants can also exhibited increased similar phenotyps.

### Example 9d

### Engineering wheat plants

Transformation of wheat is performed with the method described by Ishida et al. (1996 Nature Biotech. 14745-50. The cultivar Bobwhite (available from CYMMIT, Mexico) is commonly used in transformation. Immature embryos are co-cultivated with Agrobacterium tumefaciens that carry "super binary" vectors, and transgenic plants are recovered through organogenesis. The super binary vector system of Japan Tobacco is described in WO patents WO94/00977 and WO95/06722. Vectors were constructed as described. Various selection marker genes can be used including the maize gene encoding a mutated acetohydroxy acid synthase (AHAS) enzyme (US 6,025,541). Similarly, various promoters can be used to regulate the trait gene to provide constitutive, developmental, tissue or environmental regulation of gene transcription. In this example, the 34S promoter (GenBank Accession numbers M59930 and X16673) can be used to provide constitutive expression of the trait gene.

After incubation with Agrobacterium, the embryos are grown on callus induction medium, then regeneration medium, containing imidazolinone as a selection agent. The Petri plates are incubated in the light at 25 °C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the imidazolinone herbicides and which are PCR positive for the transgenes.

The T1 generation of single locus insertions of the T-DNA can segregate for the transgene in a 3:1 ratio. Those progeny containing one or two copies of the transgene are tolerant of the imidazolinone herbicide. Homozygous T2 plants exhibited similar phenotypes.

### Example 9e

### Engineering Rapeseed/Canola plants

Cotyledonary petioles and hypocotyls of 5-6 day-old young seedlings are used as explants for tissue culture and transformed according to Babic et al. (1998, Plant Cell Rep 17: 183-188. The commercial cultivar Westar (Agriculture Canada) is the standard variety used for transformation, but other varieties can be used.

Agrobacterium tumefaciens LBA4404 containing a binary vector are used for canola transformation. Many different binary vector systems have been described for plant transformation (e.g. An, G. in Agrobacterium Protocols. Methods in Molecular Biology vol 44, pp 47-62, Gartland KMA and MR Davey eds. Humana Press, Totowa, New Jersey. Many are based on the vector pBIN19 described by Bevan (Nucleic Acid Research. 1984. 12:8711-8721) that includes a plant gene expression cassette flanked by the left and right border sequences from the Ti plasmid of Agrobacterium tumefaciens. A plant gene expression cassette consists of at least two genes - a selection marker gene and a plant promoter regulating the transcription of the cDNA or genomic DNA of the trait gene. Various selection marker genes can be used including the Arabidopsis gene encoding a mutated acetohydroxy acid synthase (AHAS) enzyme (US 5,767,366 and US 6,225,105). Similarly, various promoters can be used to regulate the trait gene to provide constitutive, developmental, tissue or environmental regulation of gene transcription. In this example, the 34S promoter (GenBank Accession numbers M59930 and X16673) can be used to provide constitutive expression of the trait gene.

Canola seeds are surface-sterilized in 70% ethanol for 2 min., and then in 30% Clorox with a drop of Tween-20 for 10 min, followed by three rinses with sterilized distilled water. Seeds are then germinated in vitro 5 days on half strength MS medium without hormones, 1% sucrose, 0.7% Phytagar at 23oC, 16 hr. light. The cotyledon petiole explants with the cotyledon attached are excised from the in vitro seedlings, and are inoculated with Agrobacterium by dipping the cut end of the petiole explant into the bacterial suspension. The explants are then cultured for 2 days on MSBAP-3 medium containing 3 mg/l BAP, 3 % sucrose, 0.7 % Phytagar at 23 °C, 16 hr light. After two days of co-cultivation with Agrobacterium, the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 - 10 mm in length, they are cut and transferred to shoot elongation medium (MSBAP-0.5, containing 0.5 mg/l BAP). Shoots of about 2 cm in length are transferred to the rooting medium (MS0) for root induction.

Samples of the primary transgenic plants (To) are analyzed by PCR to confirm the presence of T-DNA. These results are confirmed by Southern hybridization in which DNA is electrophoresed on a 1 % agarose gel and are transferred to a positively charged nylon membrane (Roche Diagnostics). The PCR DIG Probe Synthesis Kit (Roche Diagnostics) is used to prepare a digoxigenin-labelled probe by PCR, and used as recommended by the manufacturer.

### Example 9f

### Engineering alfalfa plants

A regenerating clone of alfalfa (Medicago sativa) is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112. Alternatively, the RA3 variety (University of Wisconsin) has been selected for use in tissue culture (Walker et al., 1978 Am J Bot 65:654-659).

Petiole explants are cocultivated with an overnight culture of Agrobacterium tumefaciens C58C1 pMP90 (McKersie et al., 1999 Plant Physiol 119: 839-847) or LBA4404 containing a binary vector. Many different binary vector systems have been described for plant transformation (e.g. An, G. in Agrobacterium Protocols. Methods in Molecular Biology vol 44, pp 47-62, Gartland KMA and MR Davey eds. Humana Press, Totowa, New Jersey). Many are based on the vector pBIN19 described by Bevan (Nucleic Acid Research. 1984. 12:8711-8721) that includes a plant gene expression cassette flanked by the left and right border sequences from the Ti plasmid of Agrobacterium tumefaciens. A plant gene expression cassette consists of at least two genes - a selection marker gene and a plant promoter regulating the transcription of the cDNA or genomic DNA of the trait gene. Various selection marker genes can be used including the Arabidopsis gene encoding a mutated acetohydroxy acid synthase (AHAS) enzyme (US 5,767,366 and US 6,225,105). Similarly, various promoters can be used to regulate the trait gene that provides constitutive, developmental, tissue or environmental regulation of gene transcription. In this example, the 34S promoter (GenBank Accession numbers M59930 and X16673) can be used to provide constitutive expression of the trait gene.

The explants are cocultivated for 3 d in the dark on SH induction medium containing 288 mg/ L Prolin, 53 mg/ L thioproline, 4.35 g/ L K2SO4, and 100 µm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit Agrobacterium growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings are transplanted into pots and grown in a greenhouse.

The Tₒ transgenic plants are propagated by node cuttings and rooted in Turface growth medium. The plants are defoliated and grown to a height of about 10 cm (approximately 2 weeks after defoliation).

## Claims

1. A method for preparing a plant with faster growth and/or increased yield and/or increased biomass in comparison with a reference plant, which method comprises increasing the activity of SEQ ID NO: 2 in said plant or in one or more parts thereof in comparison with a reference organism,
wherein the activity of the SEQ ID NO: 2 polypeptide is increased by introducing a polynucleotide into the plant, or into one or more parts thereof, which polynucleotide codes for an SEQ ID NO: 2 polypeptide encoded by a nucleic acid molecule selected from the group consisting of:
(a) nucleic acid molecule encoding a SEQ ID NO: 2 polypeptide;
(b) nucleic acid molecule comprising a polynucleotide of the coding sequence according to SEQ ID NO: 1;
(c) nucleic acid molecule whose sequence is derivable from a polypeptide sequence encoded by a nucleic acid molecule according to (a) or (b), due to the degeneracy of the genetic code;
(d) nucleic acid molecule encoding a polypeptide whose sequence is at least 80% identical to the amino acid sequence of the polypeptide of SEQ ID NO:2, ;
or which comprises a complementary sequence thereof.

2. The method as claimed in claim 1 , wherein a polynucleotide encoding an endogenous SEQ ID NO: 2 polypeptide or activity is functionally linked to regulatory sequences causing increased expression of the SEQ ID NO: 2 polypeptide.

3. An expression cassette or a vector which comprises a nucleic acid molecule as defined in claim 1 (a) - (d)
or the complementary strand thereof,
wherein said expression cassette or vector comprises regulatory sequences which are capable of controlling gene expression in plant cells, and which comprise a plant promoter selected from the group consisting of seed-specific promoters, plant ubiquitin promoters, plant phaseolin promoters, inducible plant promoters, cytosolic FBPase promoter, potato ST-LSI promoter, Glycine max phosphoribosyl-pyrophosphate amidotransferase promoter, node-specific promoter, oil seed rape napin promoter, Arabidopsis oleosin promoter, Brassica Bce4 promoter, Legume LeB4 promoter, barley lpt-2- or lpt-1 promoter, barley hordein promoter, CaMV/35S, PRP1, lib4, usp, mas, STLS1, ScBV, B33, SAD1, SAD2, and nos promoter.

4. A plant cell which has been transformed or transfected stably or transiently with the plant vector or the expression cassette as claimed in claim 3..

5. A method for preparing a transgenic plant, plant cell, plant tissue, , which method comprises introducing into the genome thereof the expression cassette or the plant vector as claimed in claim 3.

6. A plant cell, which comprises the expression cassette or the plant vector as claimed in claim 3.

7. A plant tissue or plant, having an increased amount of SEQ ID NO: 2 activity or protein comprising the plant cell as claimed in claim 6.

8. Seed, tuber or propagation material of a plant as claimed in claim 7, comprising the expression cassette or the plant vector as claimed in claim 3 or the plant cell as claimed in claim 6.

9. A plant cell, a plant cell organelle, a plant tissue, a plant or a part thereof (over)expressing a nucleic acid having the sequence of SEQ ID NO: 1, wherein the dry weight is increased by 1%, 2,5%, 5%, 10%, 15%, 20%, 25%, 30%, 50% or more in comparison to wildtype plant, and whereby the dry weight of the plant cell, the plant cell organelle, the plant tissue, the plant or the part thereof means the weight of the organic material of the plant cell, the plant cell organelle, the plant tissue, the plant or the part thereof less the amount of water included in the plant cell, the plant cell organelle, the plant tissue, the plant or the part thereof.

10. A plant cell, a plant cell organelle, a plant tissue, a plant or a part thereof, (over)expressing a nucleic acid having the sequence of SEQ ID NO: 1, wherein the dry weight is increased by 1%, 2,5%, 5%, 10%, 15%, 20%, 25%, 30%, 50% or more in comparison to the dry weight of a variety selected from the group consisting of
(a) G. hirsutum IPK Accession Number GOS 6 (D 120), GOS 7 (ST 446), GOS 10 (D 1635), GOS 17 (D 4302), or GOS 21 (D 5553), or G. areysianum Deflers,
or G. incanum (Schwartz) Hillc., or G. raimondii Ulbr., or G. stocksii Masters, or G. thurberi Tod., or G. tomentosum Nutt. or G. triphyllum Hochr., or Gossypium arboreum IPK Accession Number GOS 13 (D 1634), GOS 16 (D 4240), GOS 18 (D 4505), GOS 19 (D 4506), GOS 20 (D 4750), or GOS 12 (D 1329), or Gossypium barbadense, or Gossypium herbaceum; and
(b) Brassica napus variety Mika, Brassica napus variety Digger, Brassica napus variety Artus, Brassica napus variety Terra, Brassica napus variety Smart, Brassica napus variety Olivine, Brassica napus variety Libretto, Brassica napus variety Wotan, Brassica napus variety Panther, Brassica napus variety Express, Brassica napus variety Oase, Brassica napus variety Elan, Brassica napus variety Ability, Brassica napus variety Mohican; and
(c) Linum usitatissimum variety Librina, Linum usitatissimum variety Flanders, Linum usitatissimum variety Scorpion, Linum usitatissimum variety Livia, Linum usitatissimum variety Lola, Linum usitatissimum variety Taurus, Linum usitatissimum variety Golda, Linum usitatissimum variety Lirima, and
(d) Zea mays variety Articat, Zea mays variety NK Dilitop, Zea mays variety Total, Zea mays variety Oldham, Zea mays variety Adenzo, Zea mays variety NK Lugan, Zea mays variety Liberal, Zea mays variety Peso; and
(e) Glycine max variety Oligata, Glycine max variety Lotus, Glycine max variety Primus,Glycine max variety Alma Ata, Glycine max variety OAC Vision, Glycine max variety Jutro; and
(f) Helianthus annus variety Helena, Helianthus annus variety Flavia, Helianthus annus variety Rigasol, Helianthus annus variety Flores, Helianthus annus variety Jazzy, Helianthus annus variety Pegaso, Helianthus annus variety Heliaroc, Helianthus annus variety Salut RM; and
(g) Camelina sativa variety Dolly, Camelina sativa variety Sonny, Camelina sativa variety Ligena, Camelina sativa variety Calinka; and
(h) Sinapis alba variety Martigena, Sinapis alba variety Silenda, Sinapis alba variety Sirola, Sinapis alba variety Sito, Sinapis alba variety Semper, Sinapis alba variety Seco; and
(i) Carthamus tinctorius variety Sabina, Carthamus tinctorius variety HUS-305, Carthamus tinctorius variety landrace, Carthamus tinctorius variety Thori-78, Carthamus tinctorius variety CR-34, Carthamus tinctorius variety CR-81; and
(j) Brassica juncea variety Vittasso, Brassica juncea variety Muscon M-973, Brassica juncea variety RAPD, Brassica juncea variety Co.J.86, Brassica juncea variety IAC 1-2, Brassica juncea variety Pacific Gold; and
(k) Cocos nucifera L. varietes Maypan, Ceylon Tall, Indian Tall, Jamaica Tall, Malayan Tall, Java Tall, Laguna, KingCRIC 60, CRIC 65, CRISL 98, Moorock tall, Plus palm tall, San Ramon, Typica, Nana or Aurantiaca; and
(l) Triticum aestivum L. variety Altos, Bundessortenamt file number 2646, Triticum aestivum L. variety Bussard, Bundessortenamt file number 1641, or Triticum aestivum L. variety Centrum, Bundessortenamt file number 2710; and
(m) Beta vulgaris variety Dieck 13, CPVO file number 19991828, Beta vulgaris variety FD 007, CPCO file number 20000506, or Beta vulgaris variety HI 0169, CPVO file number 20010315; and
(n) Hordeum vulgare variety Dorothea, CPVO file number 20031457, Hordeum vulgare variety Colibri, CPVO file number 20040122, Hordeum vulgare variety Brazil, CPVO file number 20010274, or Hordeum vulgare variety Christina, CPVO file number 20030277; and
(o) Secale cereale variety Esprit, CPVO file number 19950246, Secale cereale variety Resonanz, CPVO file number 20040651, or Secale cereale variety Ursus, CPVO file number 19970714; and
(p) Oryza sativa variety Gemini, CPVO file number 20010284, Oryza sativa variety Tanaro, CPVO file number 20020177, or Oryza sativa variety Zeus, CPVO file number 19980388; and
(q) Solanum tuberosum L. varieties Linda, Nicola, Solara, Agria, Sieglinde, or Russet Burbank; and
(r) Arachis hypogaea subsp. fastigiata cultivar Valencia; and
(s) Arachis hypogaea subsp. hypogaea cultivar Virginia variety 'Holland Jumbo', 'Virginia A23-7', or 'Florida 416'; and
(t) Arachis hypogaea subsp. hirsuta cultivar Peruvian runner variety 'Southeastern Runner 56-15', 'Dixie Runner', or'Early Runner'; and
(u) Arachis hypogaea subsp. vulgaris cultivar Spanish variety 'Dixie Spanish', 'Improved Spanish 2B', or 'GFA Spanish';
and whereby dry weight means the weight of the organic material of the plant cell, the plant cell organelle, the plant tissue, the plant or the part thereof less the amount of water included in the plant cell, the plant cell organelle,
the plant tissue, the plant or the part thereof.

## Patentansprüche

1. Verfahren zur Herstellung einer Pflanze mit schnellerem Wachstum und/oder erhöhtem Ertrag und/oder größerer Biomasse im Vergleich zu einer Referenzpflanze, wobei das Verfahren das Steigern der Aktivität von SEQ ID NO: 2 in der Pflanze oder in einem oder mehreren Teilen davon im Vergleich zu einem Referenzorganismus umfasst,
wobei die Aktivität des Polypeptids der SEQ ID NO: 2 durch Einbringen eines Polynukleotids in die Pflanze oder in einen oder mehrere Teile davon erhöht wird, wobei das Polynukleotid ein Polypeptid der SEQ ID NO: 2 codiert, das von einem Nukleinsäuremolekül codiert wird, ausgewählt aus der Gruppe, bestehend aus einem:
(a) Nukleinsäuremolekül, das ein Polypeptid der SEQ ID NO: 2 codiert;
(b) Nukleinsäuremolekül, das ein Polynukleotid der codierenden Sequenz gemäß SEQ ID NO: 1 umfasst;
(c) Nukleinsäuremolekül, dessen Sequenz sich von einer Polypeptidsequenz, die von einem Nukleinsäuremolekül gemäß (a) oder (b) codiert wird, aufgrund der Degeneration des genetischen Codes herleiten lässt;
(d) Nukleinsäuremolekül, das ein Polypeptid codiert, dessen Sequenz zu der Aminosäuresequenz des Polypeptids der SEQ ID NO: 2 mindestens 80% identisch ist;
oder das eine komplementäre Sequenz davon umfasst.

2. Verfahren nach Anspruch 1, wobei ein Polynukleotid, das ein endogenes Polypeptid oder die Aktivität von SEQ ID NO: 2 codiert, funktionell mit regulatorischen Sequenzen verknüpft ist, die eine erhöhte Expression des Polypeptids der SEQ ID NO: 2 verursachen.

3. Expressionscassette oder Vektor, umfassend ein Nukleinsäuremolekül nach Anspruch 1(a) - (d)
oder den komplementären Strang davon,
wobei die Expressionscassette oder der Vektor regulatorische Sequenzen umfasst, die die Genexpression in Pflanzenzellen steuern können, und die einen Pflanzenpromotor umfassen, ausgewählt aus der Gruppe, bestehend aus samenspezifischen Promotoren, Pflanzen-Ubiquitin-Promotoren, Pflanzen-Phaseolin-Promotoren, induzierbaren Pflanzenpromotoren, cytosolischem FBPase-Promotor, Kartoffel-ST-LSI-Promotor, Glycine max Phosphoribosyl-Pyrophosphatamidotransferase-Promotor, nodiumspezifischem Promotor, Ölsamen-Raps-Napin-Promotor, Arabidopsis-Oleosin-Promotor, Brassica-Bce4-Promotor, Leguminosen-LeB4-Promotor, Gerste-Ipt-2- oder -Ipt-1-Promotor, Gerste-Hordein-Promotor, CaMV/35S, PRP1, lib4, usp, mas, STLS1, ScBV, B33, SAD1, SAD2, und nos-Promotor.

4. Pflanzenzelle, die stabil oder transient mit dem Pflanzenvektor oder der Expressionscassette nach Anspruch 3 transformiert oder transfiziert wurde.

5. Verfahren zur Herstellung einer transgenen Pflanze, Pflanzenzelle, eines Pflanzengewebes, wobei das Verfahren das Einbringen der Expressionscassette oder des Pflanzenvektors nach Anspruch 3 in deren Genom umfasst.

6. Pflanzenzelle, die die Expressionscassette oder den Pflanzenvektor nach Anspruch 3 umfasst.

7. Pflanzengewebe oder Pflanze mit einer erhöhten Menge Aktivität oder Protein von SEQ ID NO: 2, umfassend die Pflanzenzelle nach Anspruch 6.

8. Samen, Knolle oder Vermehrungsmaterial einer Pflanze nach Anspruch 7, umfassend die Expressionscassette oder den Pflanzenvektor nach Anspruch 3 oder die Pflanzenzelle nach Anspruch 6.

9. Pflanzenzelle, Pflanzenzellorganelle, Pflanzengewebe, Pflanze oder Teil davon, die/das/der eine Nukleinsäure mit der Sequenz von SEQ ID NO: 1 (über)exprimiert, wobei das Trockengewicht um 1%, 2,5%, 5%, 10%, 15%, 20%, 25%, 30%, 50% oder mehr im Vergleich zu der Wildtyp-Pflanze erhöht wird und wobei das Trockengewicht der Pflanzenzelle, Pflanzenzellorganelle, des Pflanzengewebes, der Pflanze oder des Teils davon für das Gewicht des organischen Materials der Pflanzenzelle, Pflanzenzellorganelle, des Pflanzengewebes, der Pflanze oder des Teils davon steht, abzüglich der Menge Wasser, die in der Pflanzenzelle, Pflanzenzellorganelle, dem Pflanzengewebe, der Pflanze oder dem Teil davon enthalten ist.

10. Pflanzenzelle, Pflanzenzellorganelle, Pflanzengewebe, Pflanze oder Teil davon, die/das/der eine Nukleinsäure mit der Sequenz von SEQ ID NO: 1 (über)exprimiert, wobei das Trockengewicht um 1%, 2,5%, 5%, 10%, 15%, 20%, 25%, 30%, 50% oder mehr im Vergleich zu dem Trockengewicht einer Varietät erhöht wird, ausgewählt aus der Gruppe, bestehend aus
a) G. hirsutum IPK Zugangsnummer GOS 6 (D 120), GOS 7 (ST 446), GOS 10 (D 1635), GOS 17 (D 4302), oder GOS 21 (D 5553), oder G. areysianum Deflers, oder G. incanum (Schwartz) Hillc., oder G. raimondii Ulbr., oder G. stocksii Masters, oder G. thurberi Tod., oder G. tomentosum Nutt. oder G. triphyllum Hochr., oder Gossypium arboreum IPK Zugangsnummer GOS 13 (D 1634), GOS 16 (D 4240), GOS 18 (D 4505), GOS 19 (D 4506), GOS 20 (D 4750), oder GOS 12 (D 1329), oder Gossypium barbadense, oder Gossypium herbaceum; und
(b) Brassica napus Varietät Mika, Brassica napus Varietät Digger, Brassica napus Varietät Artus, Brassica napus Varietät Terra, Brassica napus Varietät Smart, Brassica napus Varietät Olivine, Brassica napus Varietät Libretto, Brassica napus Varietät Wotan, Brassica napus Varietät Panther, Brassica napus Varietät Express, Brassica napus Varietät Oase, Brassica napus Varietät Elan, Brassica napus Varietät Ability, Brassica napus Varietät Mohican; und
(c) Linum usitatissimum Varietät Librina, Linum usitatissimum Varietät Flanders, Linum usitatissimum Varietät Scorpion, Linum usitatissimum Varietät Livia, Linum usitatissimum Varietät Lola, Linum usitatissimum Varietät Taurus, Linum usitatissimum Varietät Golda, Linum usitatissimum Varietät Lirima, und
(d) Zea mays Varietät Articat, Zea mays Varietät NK Dilitop, Zea mays Varietät Total, Zea mays Varietät Oldham, Zea mays Varietät Adenzo, Zea mays Varietät NK Lugan, Zea mays Varietät Liberal, Zea mays Varietät Peso; und
(e) Glycine max Varietät Oligata, Glycine max Varietät Lotus, Glycine max Varietät Primus, Glycine max Varietät Alma Ata, Glycine max Varietät OAC Vision, Glycine max Varietät Jutro; und
(f) Helianthus annus Varietät Helena, Helianthus annus Varietät Flavia, Helianthus annus Varietät Rigasol, Helianthus annus Varietät Flores, Helianthus annus Varietät Jazzy, Helianthus annus Varietät Pegaso, Helianthus annus Varietät Heliaroc, Helianthus annus Varietät Salut RM; und
(g) Camelina sativa Varietät Dolly, Camelina sativa Varietät Sonny, Camelina sativa Varietät Ligena, Camelina sativa Varietät Calinka; und
(h) Sinapis alba Varietät Martigena, Sinapis alba Varietät Silenda, Sinapis alba Varietät Sirola, Sinapis alba Varietät Sito, Sinapis alba Varietät Semper, Sinapis alba Varietät Seco; und
(i) Carthamus tinctorius Varietät Sabina, Carthamus tinctorius Varietät HUS-305, Carthamus tinctorius Varietät landrace, Carthamus tinctorius Varietät Thori-78, Carthamus tinctorius Varietät CR-34, Carthamus tinctorius Varietät CR-81; und
(j) Brassica juncea Varietät Vittasso, Brassica juncea Varietät Muscon M-973, Brassica juncea Varietät RAPD, Brassica juncea Varietät Co.J.86, Brassica juncea Varietät IAC 1-2, Brassica juncea Varietät Pacific Gold; und
(k) Cocos nucifera L. Varietäten Maypan, Ceylon Tall, Indian Tall, Jamaica Tall, Malayan Tall, Java Tall, Laguna, KingCRIC 60, CRIC 65, CRISL 98, Moorock tall, Plus palm tall, San Ramon, Typica, Nana oder Aurantiaca; und
(l) Triticum aestivum L. Varietät Altos, Bundessortenamt Kenn-Nummer 2646, Triticum aestivum L. Varietät Bussard, Bundessortenamt Kenn-Nummer 1641, oder Triticum aestivum L. Varietät Centrum, Bundessortenamt Kenn-Nummer 2710; und
(m) Beta vulgaris Varietät Dieck 13, CPVO Kenn-Nummer 19991828, Beta vulgaris Varietät FD 007, CPVO Kenn-Nummer 20000506, oder Beta vulgaris Varietät HI 0169, CPVO Kenn-Nummer 20010315; und
(n) Hordeum vulgare Varietät Dorothea, CPVO Kenn-Nummer 20031457, Hordeum vulgare Varietät Colibri, CPVO Kenn-Nummer 20040122, Hordeum vulgare Varietät Brazil, CPVO Kenn-Nummer 20010274, oder Hordeum vulgare Varietät Christina, CPVO Kenn-Nummer 20030277; und
(o) Secale cereale Varietät Esprit, CPVO Kenn-Nummer 19950246, Secale cereale Varietät Resonanz, CPVO Kenn-Nummer 20040651, oder Secale cereale Varietät Ursus, CPVO Kenn-Nummer 19970714; und
(p) Oryza sativa Varietät Gemini, CPVO Kenn-Nummer 20010284, Oryza sativa Varietät Tanaro, CPVO Kenn-Nummer 20020177, oder Oryza sativa Varietät Zeus, CPVO Kenn-Nummer 19980388; und
(q) Solanum tuberosum L. Varietäten Linda, Nicola, Solara, Agria, Sieglinde, oder Russet Burbank; und
(r) Arachis hypogaea Subsp. fastigiata Kultursorte Valencia; und
(s) Arachis hypogaea Subsp. hypogaea Kultursorte Virginia Varietät 'Holland Jumbo', Virginia A23-7', oder 'Florida 416'; und
(t) Arachis hypogaea Subsp. hirsuta Kultursorte Peruvian runner Varietät 'Southeastern Runner 56-15', 'Dixie Runner', oder 'Early Runner'; und
(u) Arachis hypogaea Subsp. vulgaris Kultursorte Spanish Varietät 'Dixie Spanish', 'Improved Spanish 2B', oder 'GFA Spanish';
und wobei Trockengewicht für das Gewicht des organischen Materials der Pflanzenzelle, Pflanzenzellorganelle, des Pflanzengewebes, der Pflanze oder des Teils davon steht, abzüglich der Menge Wasser, die in der Pflanzenzelle, Pflanzenzellorganelle, dem Pflanzengewebe, der Pflanze oder dem Teil davon enthalten ist

## Revendications

1. Procédé pour préparer une plante ayant une croissance plus rapide et/ou un rendement augmenté et/ou une biomasse augmentée en comparaison à une plante de référence, ledit procédé comprenant l'augmentation de l'activité de SEQ ID NO: 2 dans ladite plante ou dans une ou plusieurs parties de celle-ci en comparaison à un organisme de référence, l'activité du polypeptide de SEQ ID NO: 2 étant augmentée par introduction d'un polynucléotide dans la plante, ou dans une ou plusieurs parties de celle-ci, ledit polynucléotide code pour un polypeptide de SEQ ID NO: 2 codé par une molécule d'acide nucléique choisie dans le groupe constitué de :
(a) une molécule d'acide nucléique codant pour un polypeptide de SEQ ID NO: 2 ;
(b) une molécule d'acide nucléique comprenant un polynucléotide de la séquence codante selon SEQ ID NO: 1 ;
(c) une molécule d'acide nucléique dont la séquence peut dériver d'une séquence polypeptidique codée par une molécule d'acide nucléique selon (a) ou (b), en raison de la dégénérescence du code génétique ;
(d) une molécule d'acide nucléique codant pour un polypeptide dont la séquence est au moins 80 % identique à la séquence d'acides aminés du polypeptide de SEQ ID NO: 2 ;
ou qui comprend une séquence complémentaire de celui-ci.

2. Procédé selon la revendication 1, dans lequel un polynucléotide codant pour un polypeptide ou une activité de SEQ ID NO: 2 endogène est fonctionnellement lié à des séquences régulatrices causant une expression augmentée du polypeptide de SEQ ID NO: 2.

3. Cassette d'expression ou vecteur qui comprend une molécule d'acide nucléique telle que définie dans la revendication 1 (a) - (d)
ou le brin complémentaire de celle-ci,
ladite cassette d'expression ou ledit vecteur comprenant des séquences régulatrices qui sont capables de contrôler l'expression génique dans des cellules de plante, et qui comprennent un promoteur de plante choisi dans le groupe constitué de promoteurs spécifiques de graine, promoteurs d'ubiquitine de plante, promoteurs de phaséoline de plante, promoteurs de plante inductibles, promoteur de FBPase cytosolique, promoteur ST-LSI de pomme de terre, promoteur de phosphoribosyl-pyrophosphate amidotransférase de Glycine max, promoteur spécifique de noeud, promoteur de napine de colza, promoteur d'oléosine d'Arabidopsis, promoteur Bce4 de Brassica, promoteur LeB4 de légumineuse, promoteur lpt-2 ou lpt-1 d'orge, promoteur d'hordéine d'orge, promoteur CaMV/35S, PRP1, lib4, usp, mas, STLS1, ScBV, B33, SAD1, SAD2, et nos.

4. Cellule de plante qui a été transformée ou transfectée de façon stable ou transitoire avec le vecteur de plante ou la cassette d'expression selon la revendication 3.

5. Procédé pour préparer une plante, cellule de plante, tissu de plante transgéniques, ledit procédé comprenant l'introduction dans le génome de ceux-ci de la cassette d'expression ou du vecteur de plante selon la revendication 3.

6. Cellule de plante, qui comprend la cassette d'expression ou le vecteur de plante selon la revendication 3.

7. Tissu de plante ou plante, ayant une quantité augmentée d'activité ou protéine de SEQ ID NO: 2 comprenant la cellule de plante selon la revendication 6.

8. Graine, tubercule ou matériau de propagation d'une plante selon la revendication 7, comprenant la cassette d'expression ou le vecteur de plante selon la revendication 3 ou la cellule de plante selon la revendication 6.

9. Cellule de plante, organite de cellule de plante, tissu de plante, plante ou partie de celle-ci (sur)exprimant un acide nucléique ayant la séquence de SEQ ID NO: 1, le poids sec étant augmenté de 1 %, 2,5 %, 5 %, 10 %, 15 %, 20 %, 25 %, 30 %, 50 % ou plus par rapport à la plante de type sauvage, et le poids sec de la cellule de plante, l'organite de cellule de plante, le tissu de plante, la plante ou la partie de celle-ci désignant le poids du matériau organique de la cellule de plante, l'organite de cellule de plante, le tissu de plante, la plante ou la partie de celle-ci moins la quantité d'eau comprise dans la cellule de plante, l'organite de cellule de plante, le tissu de plante, la plante ou la partie de celle-ci.

10. Cellule de plante, organite de cellule de plante, tissu de plante, plante ou partie de celle-ci (sur)exprimant un acide nucléique ayant la séquence de SEQ ID NO: 1, le poids sec étant augmenté de 1 %, 2,5 %, 5 %, 10 %, 15 %, 20 %, 25 %, 30 %, 50 % ou plus par rapport au poids sec d'une variété choisie dans le groupe constitué de
(a) G. hirsutum numéro d'accession IPK GOS 6 (D 120), GOS 7 (ST 446), GOS 10 (D 1635), GOS 17 (D 4302), ou GOS 21 (D 5553), ou G. areysianum Deflers,
ou G. incanum (Schwartz) Hillc., ou G. raimondii Ulbr., ou G. stocksii Masters, ou G. thurberi Tod., ou G. tomentosum Nutt, ou G. triphyllum Hochr., ou Gossypium arboreum numéro d'accession IPK GOS 13 (D 1634), GOS 16 (D 4240), GOS 18 (D 4505), GOS 19 (D 4506), GOS 20 (D 4750), ou GOS 12 (D 1329), ou Gossypium barbadense, ou Gossypium herbaceum ; et
(b) Brassica napus variété Mika, Brassica napus variété Digger, Brassica napus variété Artus, Brassica napus variété Terra, Brassica napus variété Smart, Brassica napus variété Olivine, Brassica napus variété Libretto, Brassica napus variété Wotan, Brassica napus variété Panther, Brassica napus variété Express, Brassica napus variété Oase, Brassica napus variété Elan, Brassica napus variété Ability, Brassica napus variété Mohican ; et
(c) Linum usitatissimum variété Librina, Linum usitatissimum variété Flanders, Linum usitatissimum variété Scorpion, Linum usitatissimum variété Livia, Linum usitatissimum variété Lola, Linum usitatissimum variété Taurus, Linum usitatissimum variété Golda, Linum usitatissimum variété Lirima, et
(d) Zea mays variété Articat, Zea mays variété NK Dilitop, Zea mays variété Total, Zea mays variété Oldham, Zea mays variété Adenzo, Zea mays variété NK Lugan, Zea mays variété Liberal, Zea mays variété Peso ; et
(e) Glycine max variété Oligata, Glycine max variété Lotus, Glycine max variété Primus,Glycine max variété Alma Ata, Glycine max variété OAC Vision, Glycine max variété Jutro ; et
(f) Helianthus annus variété Helena, Helianthus annus variété Flavia, Helianthus annus variété Rigasol, Helianthus annus variété Flores, Helianthus annus variété Jazzy, Helianthus annus variété Pegaso, Helianthus annus variété Heliaroc, Helianthus annus variété Salut RM ; et
(g) Camelina sativa variété Dolly, Camelina sativa variété Sonny, Camelina sativa variété Ligena, Camelina sativa variété Calinka ; et
(h) Sinapis alba variété Martigena, Sinapis alba variété Silenda, Sinapis alba variété Sirola, Sinapis alba variété Sito, Sinapis alba variété Semper, Sinapis alba variété Seco ; et
(i) Carthamus tinctorius variété Sabina, Carthamus tinctorius variété HUS-305, Carthamus tinctorius variété landrace, Carthamus tinctorius variété Thori-78, Carthamus tinctorius variété CR-34, Carthamus tinctorius variété CR-81 ; et
(j) Brassica juncea variété Vittasso, Brassica juncea variété Muscon M-973, Brassica juncea variété RAPD, Brassica juncea variété Co.J.86, Brassica juncea variété IAC 1-2, Brassica juncea variété Pacific Gold ; et
(k) Cocos nucifera L. variétés Maypan, Ceylon Tall, Indian Tall, Jamaica Tall, Malayan Tall, Java Tall, Laguna, KingCRIC 60, CRIC 65, CRISL 98, Moorock tall, Plus palm tall, San Ramon, Typica, Nana ou Aurantiaca ; et
(l) Triticum aestivum L. variété Altos, numéro de dossier Bundessortenamt 2646, Triticum aestivum L. variété Bussard, numéro de dossier Bundessortenamt 1641, ou Triticum aestivum L. variété Centrum, numéro de dossier Bundessortenamt 2710 ; et
(m) Beta vulgaris variété Dieck 13, numéro de dossier CPVO 19991828, Beta vulgaris variété FD 007, numéro de dossier CPVO 20000506, ou Beta vulgaris variété HI 0169, numéro de dossier CPVO 20010315 ; et
(n) Hordeum vulgare variété Dorothea, numéro de dossier CPVO 20031457, Hordeum vulgare variété Colibri, numéro de dossier CPVO 20040122, Hordeum vulgare variété Brazil, numéro de dossier CPVO 20010274, ou Hordeum vulgare variété Christina, numéro de dossier CPVO 20030277 ; et
(o) Secale cereale variété Esprit, numéro de dossier CPVO 19950246, Secale cereale variété Resonanz, numéro de dossier CPVO 20040651, ou Secale cereale variété Ursus, numéro de dossier CPVO 19970714 ; et
(p) Oryza sativa variété Gemini, numéro de dossier CPVO 20010284, Oryza sativa variété Tanaro, numéro de dossier CPVO 20020177, ou Oryza sativa variété Zeus, numéro de dossier CPVO 19980388 ; et
(q) Solanum tuberosum L. variétés Linda, Nicola, Solara, Agria, Sieglinde, ou Russet Burbank ; et
(r) Arachis hypogaea subsp. fastigiata cultivar Valencia ; et
(s) Arachis hypogaea subsp. hypogaea cultivar Virginia variété « Holland Jumbo », « Virginia A23-7 », ou « Florida 416 » ; et
(t) Arachis hypogaea subsp. hirsuta cultivar Peruvian runner variété « Southeastern Runner 56-15 », « Dixie Runner », ou « Early Runner » ; et
(u) Arachis hypogaea subsp. vulgaris cultivar Spanish variété « Dixie Spanish », « Improved Spanish 2B », ou « GFA Spanish » ;
et le poids sec désignaant le poids du matériau organique de la cellule de plante, l'organite de cellule de plante, le tissu de plante, la plante ou la partie de celle-ci moins la quantité d'eau comprise dans la cellule de plante, l'organite de cellule de plante, le tissu de plante, la plante ou la partie de celle-ci.
